# EUROPEAN PATENT APPLICATION

(11) **EP 2 514 747 A1**
(43) Date of publication of application: **24.10.2012**
(21) Application number: 10835704.7
(22) Date of filing: 08.12.2010
(51) Int. Cl.: C07D 279/06, A61K 31/54, A61K 31/5415, A61K 31/542, A61P 25/00, A61P 25/16, A61P 25/28, A61P 27/02, A61P 43/00, C07D 417/04, C07D 417/12, C07D 417/14, C07D 513/04

(54) **SUBSTITUTED AMINOTHIAZINE DERIVATIVE**

(30) Priority: 09.12.2009 JP 2009279933
(71) Applicant: Shionogi & Co., Ltd., Osaka-shi, Osaka 5410045 (JP)
(72) Inventor: ANAN, Kousuke, Toyonaka-shi Osaka 561-0825 (JP); TADA, Yukio, Osaka 530-0033 (JP); HORI, Akihiro, Toyonaka-shi Osaka 561-0825 (JP); MASUI, Moriyasu, Toyonaka-shi Osaka 561-0825 (JP)
(74) Representative: Vossius & Partner
(86) International application number: PCT/JP2010/007153
(87) International publication number: WO 2011/070781

(57) **Abstract**

The present invention relates to an agent for treating or preventing disease induced by production, secretion and/or deposition of amyloid β protein, for example, a compound of formula (I), wherein, R¹, R^{2a}, R^{2b} R³, R^{4a}, R^{4b}, ring A, and dashed line are described in this specification, its pharmaceutically acceptable salt or a solvate thereof.

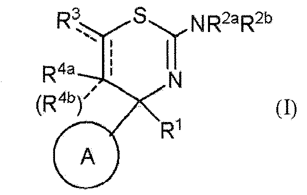

## Description

### [Technical field]

The present invention relates to a compound which has amyloid production inhibitory activity, and is useful as an agent for treating or preventing disease induced by production, secretion and/or deposition of amyloid β protein.

### [Background Art]

In the brain of Alzheimer's patient, the peptide composed of about 40 amino acids residue as is called amyloid β protein, that accumulates to form insoluble specks (senile specks) outside nerve cells is widely observed. It is concerned that this senile specks kill nerve cells to cause Alzheimer's disease, so the therapeutic agents for Alzheimer's disease, such as decomposition agents of amyloid β protein and amyloid vaccine, are under investigation.

Secretase is an enzyme which cleaves a protein called amyloid β precursor protein (APP) in cell and produces amyloid β protein. The enzyme which controls the production of N terminus of amyloid β protein is called as β -secretase (beta-site APP-cleaving enzyme 1, BACE1). It is thought that inhibition of this enzyme leads to reduction of producing amyloid β protein and that the therapeutic or preventive agent for Alzheimer's disease will be created due to the inhibition.

Patent Literature 8 and Non-patent Literature 2 describe the compounds which are similar to those of the present invention, and are useful for dye and antibacterial agent, respectively.

Patent Literatures 1 to 7, 9, 10 and Non-patent Literature 1 are known as β secretase inhibitor, however, all compounds in these literatures have different structures from the present invention.

### [Prior Art Literatures]

### [Patent Literatures]

[Patent Literature 1] International Patent Application Publication WO2007/049532
[Patent Literature 2] International Patent Application Publication WO2008/133274
[Patent Literature 3] International Patent Application Publication WO2008/133273
[Patent Literature 4] International Patent Application Publication WO2009/091016
[Patent Literature 5] International Patent Application Publication W02009/134617
[Patent Literature 6] International Patent Application Publication W02009/103626
[Patent Literature 7] International Patent Application Publication W02005/097767
[Patent Literature 8] Former Republic of East Germany Patent Application Publication 140144
[Patent Literature 9] International Patent Application Publication W02009/151098
[Patent Literature 10] International Patent Application Publication W020010/038686

### [Non-Patent Literature]

[Non-Patent Literature 1] Journal of Medicinal Chemistry, 50, 24, 5912-5925(2007)
[Non-Patent Literature 2] Journal of Chemical Research, 12, 726-728(2009)

### [Disclosure of Invention]

### [Problems to be solved by the Invention]

The present invention provides compounds which have reducing effects to produce amyloid β protein, especially β secretase inhibitory activity, and are useful as an agent for treating or preventing disease induced by production, secretion and/or deposition of amyloid β protein.

### [Means to Solve the Problems]

The present invention provides for examples as follows:

(1) A compound of formula (I):

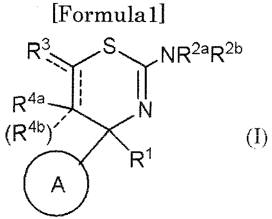

wherein R¹ is substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group, R^{2a} and R^{2b} are each independently hydrogen, substituted or unsubstituted alkyl, substituted or unsubstituted acyl, substituted or unsubstituted alkoxycarbonyl or substituted or unsubstituted carbamoyl,

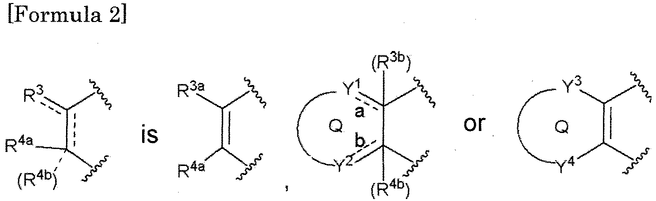

wherein R^{3a} is halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl, R^{4a} is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl, ring Q is a substituted or unsubstituted carbocycle or a substituted or unsubstituted heterocycle,
R^{3b} and R^{4b} are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, carboxy, substituted or unsubstituted alkoxycarbonyl, substituted or unsubstituted alkenyloxycarbonyl, substituted or unsubstituted alkynyloxycarbonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
dashed line a and dashed line b each independently mean the presence or absence of a bond,
when dashed line a means the presence of a bond, then R^{3b} is absent,
when dashed line b means the presence of a bond, then R^{4b} is absent,
Y¹ and Y² are each independently -C(R⁵)(R⁶)-, -C(R⁵)=, -N(R⁷)-, -N=, -S-, -SO-, -SO₂ -or -O-,
Y³ and Y⁴ are each independently -C(R⁵)(R⁶)-, -N(R⁷)-, -S-, -SO-, -SO₂ -or -O-,
R⁵ and R⁶ are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl,
R⁷ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, substituted or unsubstituted carbamoyl, substituted or unsubstituted thiocarbamoyl, substituted or unsubstituted sulfamoyl, substituted or unsubstituted amino, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl, and
ring A is a substituted or unsubstituted carbocycle or a substituted or unsubstituted heterocycle,
excluding the following compounds:

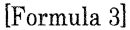

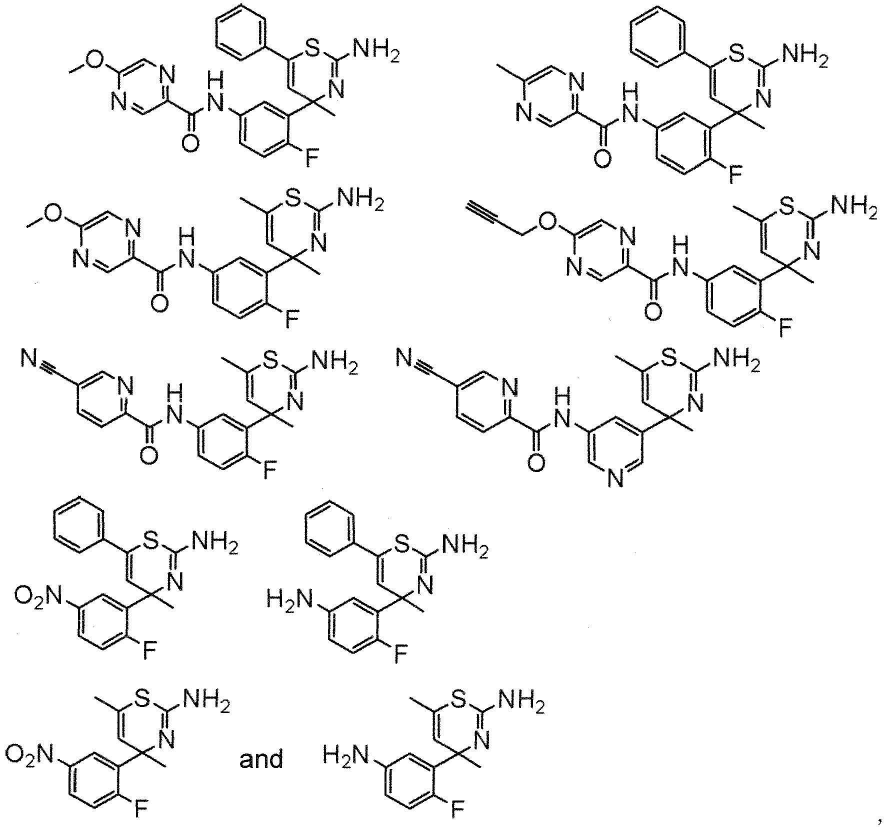

its pharmaceutically acceptable salt or a solvate thereof.

(1') A compound of formula (I):

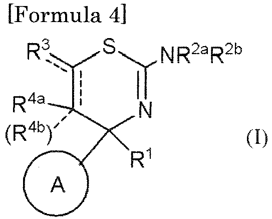

wherein
R¹ is substituted or unsubstituted alkyl, substituted or unsubstituted alkenyl, substituted or unsubstituted alkynyl, cyano, a substituted or unsubstituted carbocyclic group or a substituted or unsubstituted heterocyclic group,
R^{2a} and R^{2b} are each independently hydrogen, substituted or unsubstituted alkyl, substituted or unsubstituted acyl,

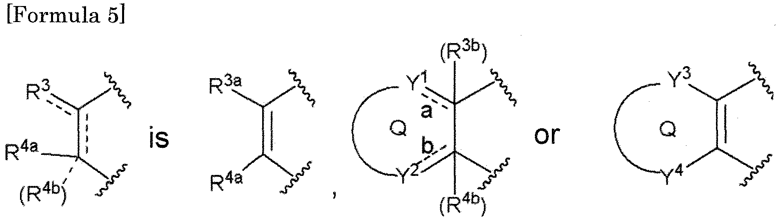

wherein
R^{3a} is halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl,
ring Q is a substituted or unsubstituted carbocycle or a substituted or unsubstituted heterocycle,
R^{3b} is hydrogen, halogen, substituted or unsubstituted alkyl, substituted or unsubstituted alkenyl, substituted or unsubstituted alkynyl, substituted or unsubstituted amino, substituted or unsubstituted carbamoyl, substituted or unsubstituted thiocarbamoyl, carboxy, substituted or unsubstituted alkoxycarbonyl, substituted or unsubstituted alkenyloxycarbonyl, a substituted or unsubstituted carbocyclic group, substituted or unsubstituted carbocyclyloxycarbonyl, substituted or unsubstituted heterocyclic group or substituted or unsubstituted heterocyclyloxycarbonyl,
R^{4a} and R^{4b} are each independently hydrogen, halogen, hydroxy, substituted or unsubstituted alkyl, substituted or unsubstituted alkenyl, or substituted or unsubstituted alkynyl,
when dashed line a means the presence of a bond, then R^{3b} is absent,
when dashed line b means the presence of a bond, then R^{4b} is absent,
Y¹ and Y² are each independently -C(R⁵)(R⁶)-, -C(R⁵)=, -N(R⁷)-, -N=, -S-, -SO-, -SO₂ -or -O-,
Y³ and Y⁴ are each independently -C(R⁵)(R⁶)-, -N(R⁷)-, -S-, -SO-, -SO₂ -or -O-,
R⁵ and R⁶ are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkylthio, substituted or unsubstituted alkenylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkylsulfonyl, substituted or unsubstituted alkenylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl,
R⁷ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted acyl, cyano, nitro, carboxy, substituted or unsubstituted alkoxycarbonyl, substituted or unsubstituted alkenyloxycarbonyl, substituted or unsubstituted alkylsulfinyl, substituted or unsubstituted alkenylsulfinyl, substituted or unsubstituted alkylsulfonyl, substituted or unsubstituted alkenylsulfonyl, substituted or unsubstituted carbamoyl, substituted or unsubstituted thiocarbamoyl, substituted or unsubstituted amino, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl, and
ring A is a substituted or unsubstituted carbocycle or a substituted or unsubstituted heterocycle,
excluding the following compounds:

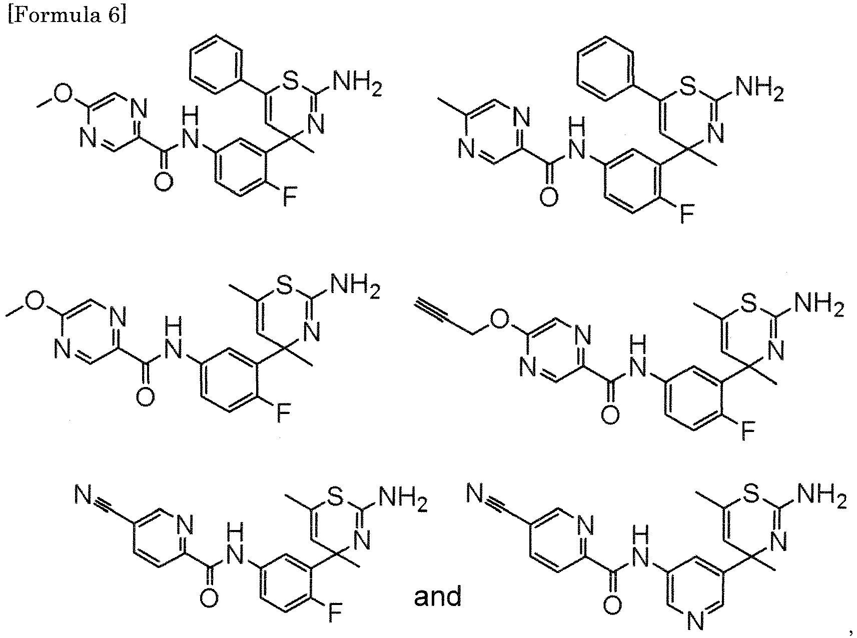

its pharmaceutically acceptable salt or a solvate thereof.

(2) The compound according to the above (1) or (1') wherein ring A is

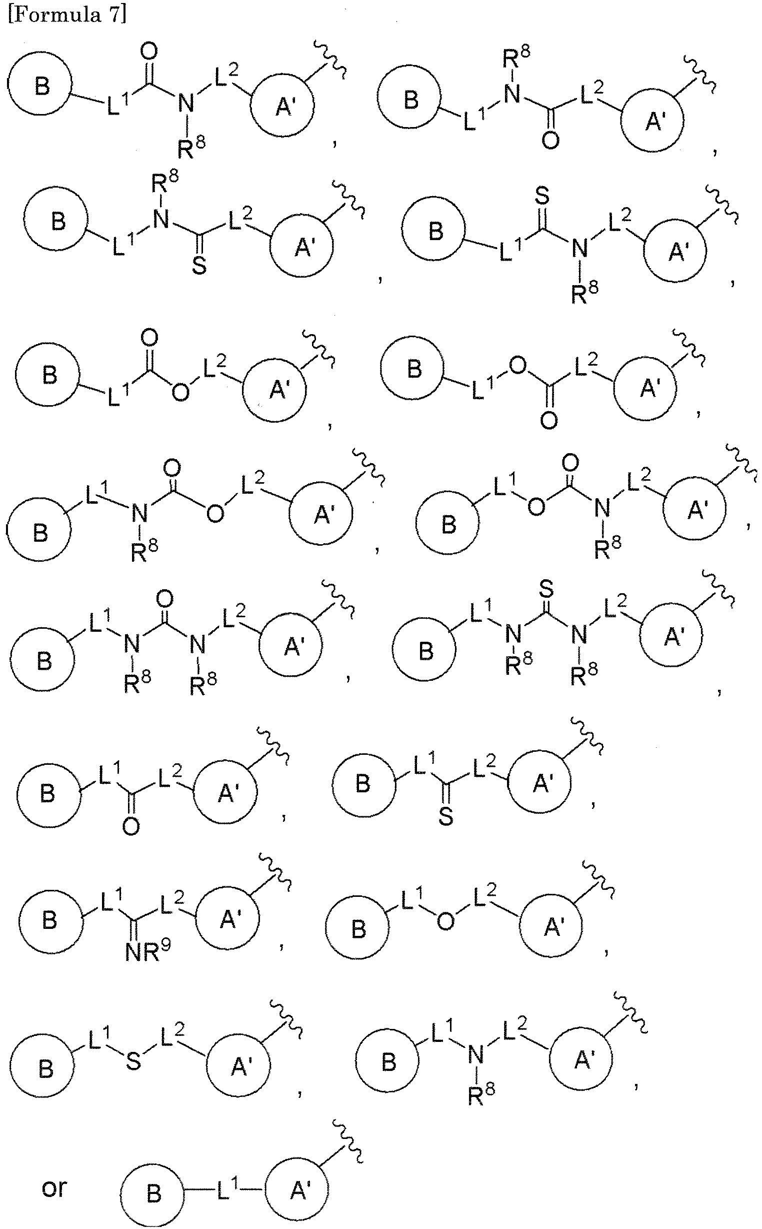

wherein ring A' and ring B are each independently a substituted or unsubstituted carbocycle or a substituted or unsubstituted heterocycle,
L¹ and L² are each independently a bond, substituted or unsubstituted alkylene, substituted or unsubstituted alkenylene or substituted or unsubstituted alkynylene,
R⁸ is hydrogen, substituted or unsubstituted alkyl, substituted or unsubstituted alkenyl, substituted or unsubstituted alkynyl or substituted or unsubstituted acyl,
R⁹ is hydrogen, hydroxy, substituted or unsubstituted alkyl, substituted or unsubstituted alkenyl, substituted or unsubstituted alkynyl or substituted or unsubstituted acyl, its pharmaceutically acceptable salt or a solvate thereof.

(3) The compound according to any one of the above (1), (1') and (2) wherein ring A is

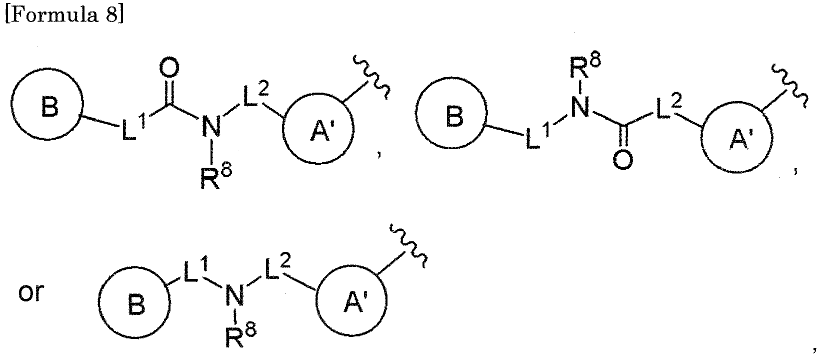

its pharmaceutically acceptable salt or a solvate thereof.

(4) The compound according to any one of the above (1), (1'), (2) and (3) wherein R^{3a} is substituted or unsubstituted alkyl, cyano, substituted or unsubstituted alkoxycarbonyl, substituted or unsubstituted carbamoyl or a substituted or unsubstituted heterocyclic group, its pharmaceutically acceptable salt or a solvate thereof.

(5) The compound according to any one of the above (1), (1'), and (2) to (4) wherein R^{4a} is hydrogen, its pharmaceutically acceptable salt or a solvate thereof.

(6)

The compound according to any one of the above (1), (1'), and (2) to (5) wherein

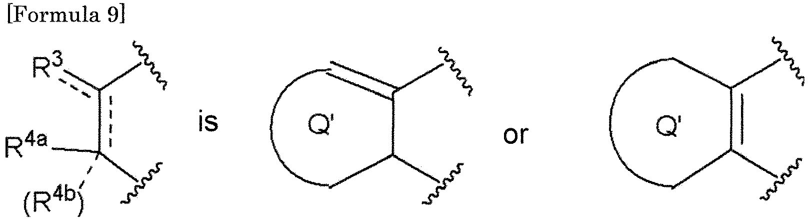

wherein ring Q' is a substituted or unsubstituted carbocycle, its pharmaceutically acceptable salt or a solvate thereof.

(7) The compound according to any one of the above (2) to (6) wherein ring A' is substituted or unsubstituted benzene, ring B is substituted or unsubstituted pyridine, substituted or unsubstituted pyrimidine, or substituted or unsubstituted pyrazine, its pharmaceutically acceptable salt or a solvate thereof.

(8) The compound according to any one of the above (1), (1'), and (2) to (7) wherein R¹ is C1 to C3 unsubstituted alkyl, its pharmaceutically acceptable salt or a solvate thereof.

(9) The compound according to any one of the above (1), (1'), and (2) to (8) wherein both of R^{2a} and R^{2b} are hydrogen, its pharmaceutically acceptable salt or a solvate thereof.

(10) A pharmaceutical composition comprising the compound according to any one of the above (1), (1'), and (2) to (9), its pharmaceutically acceptable salt or a solvate thereof as an active ingredient.

(11) A pharmaceutical composition having BACE1 inhibitory activity comprising the compound according to any one of the above (1), (1'), and (2) to (9), its pharmaceutically acceptable salt or a solvate thereof as an active ingredient.

(12) The pharmaceutical composition according to the above (10) or (11), which is a medicament for treating or preventing the diseases induced by production, secretion or deposition of amyloid-β proteins.

(13) The pharmaceutical composition according to any one of the above (10) to (12), which is a medicament for treating or preventing Alzheimer's disease.

(14) A method for inhibiting BACE1 activity comprising administering the compound according to any one of the above (1), (1'), and (2) to (9), its pharmaceutically acceptable salt or a solvate thereof.

(15) The compound according to any one of the above (1), (1'), and (2) to (9), its pharmaceutically acceptable salt or a solvate thereof for use in a method for inhibiting BACE1 activity.

(16) A method for treating or preventing diseases induced by production, secretion or deposition of amyloid-β proteins comprising administering the compound according to any one of the above (1), (1'), and (2) to (9), its pharmaceutically acceptable salt or a solvate thereof.

(17) The compound according to any one of the above (1), (1'), and (2) to (9), its pharmaceutically acceptable salt or a solvate thereof for use in a method for treating or preventing diseases induced by production, secretion or deposition of amyloid-β proteins.

(18) A method for treating or preventing Alzheimer's disease comprising administering the compound according to any one of the above (1), (1'), and (2) to (9), its pharmaceutically acceptable salt or a solvate thereof.

(19) The compound according to any one of the above (1), (1'), and (2) to (9), its pharmaceutically acceptable salt or a solvate thereof for use in a method for treating or preventing Alzheimer's disease.

(20) Use of the compound according to any one of the above (1), (1'), and (2) to (9), its pharmaceutically acceptable salt or a solvate thereof in the manufacture of a medicament for inhibiting BACE1 activity.

(21) Use of the compound according to any one of the above (1), (1'), and (2) to (9), its pharmaceutically acceptable salt or a solvate thereof in the manufacture of a medicament for treating or preventing disease induced by production, secretion or deposition of amyloid-β protein.

(22) Use of the compound according to any one of the above (1), (1'), and (2) to (9), its pharmaceutically acceptable salt or a solvate thereof in the manufacture of a medicament for treating or preventing Alzheimer's disease.

(23) A method, system, device, kit and the like for manufacturing the compound according to any one of the above (1), (1'), and (2) to (9), its pharmaceutically acceptable salt or a solvate.

(24) A method, system, device, kit and the like for preparing a pharmaceutical composition comprising the compound according to any one of the above (1), (1'), and (2) to (9), its pharmaceutically acceptable salt or a solvate.

(25) A method, system, device, kit and the like for using the compound according to any one of the above (1), (1'), and (2) to (9), its pharmaceutically acceptable salt or a solvate.

### [Effect of the Invention]

The compounds of the present invention are useful as an agent for treating or preventing disease induced by production, secretion or deposition of amyloid β protein such as Alzheimer's disease.

### [Best Mode for Carrying Out the Invention]

As used herein, the "halogen" includes fluorine, chlorine, bromine, and iodine.

The "alkyl" includes straight or branched alkyl of a carbon number of 1 to 15, e.g. 1 to 10, 1 to 6, and 1 to 3, and examples include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, and n-decyl.

An alkyl part of the " alkoxy", the "halogeno alkyl", the "hydroxy alkyl", the "halogeno alkoxy", the "hydroxy alkoxy", the " alkoxycarbonyl", the "halogeno alkoxycarbonyl", the " alkoxycarbonyl alkyl", the " alkylamino", the " alkoxy alkyl", the "hydroxyimino alkyl", the " alkoxyimino alkyl,", the "amino alkyl", the " alkoxy alkoxy", the " alkoxy, alkenyl", the " alkoxy alkenyloxy", the " alkoxycarbonyl alkenyl", the " alkoxy alkynyl", the " alkoxycarbonyl alkynyl", the " alkylcarbamoyl", the "hydroxy alkylcarbamoyl", the " alkoxyimino", the " alkylthio", the " alkylsulfonyl", the " alkylsulfonyloxy", the " alkylsulfonylamino", the " alkylsulfonyl alkylamino", the " alkylsulfonylimino", the " alkylsulfinylamino", the " alkylsulfinyl alkylamino", the " alkylsulfinylimino", the " alkylsulfamoyl", the " alkylsulfinyl", the "carbocyclyl alkyl", the "carbocyclyl alkoxy", the "carbocyclyl alkoxycarbonyl", the "carbocyclyl alkylamino", the "carbocyclyl alkylcarbamoyl", the "cycloalkyl alkyl", the "cycloalkyl alkoxy", the "cycloalkyl alkylamino", the "cycloalkyl alkoxycarbonyl", the "cycloalkyl alkylcarbamoyl", the "aryl alkyl", the "aryl alkoxy", the "aryl alkylamino", the "aryl alkoxycarbonyl", the "aryl alkylcarbamoyl", the "heterocyclyl alkyl", the "heterocyclyl alkoxy", the "heterocyclyl alkylamino", the "heterocyclyl alkoxycarbonyl", the "heterocyclyl alkylcarbamoyl", the "heteroaryl alkyl", and the "heteroaryl alkoxy" is the same as the above " alkyl".

The "substituted or unsubstituted alkyl," may be substituted with one or more substituents selected from substituent group α.

As used herein, substituent group α is a group consisting of halogen, hydroxy, alkoxy, halogeno alkoxy, hydroxyl alkoxy, alkoxy alkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyl alkylcarbamoyl, sulfamoyl, alkylsulfamoyl, alkylsulfinyl, alkylsulfonylamino, alkylsulfonyl alkylamino, alkylsulfonylimino, alkylsulfinylamino, alkylsulfinyl alkylamino, alkylsulfinylimino, cyano, nitro, a carbocyclic group and a heterocyclic group wherein the carbocycle and the heterocycle may be each substituted with one or more substituents selected from halogen, alkyl, hydroxyl, and alkoxy.

Examples of the substituent of the "substituted or unsubstituted alkoxy", the "substituted or unsubstituted alkoxycarbonyl", the "substituted or unsubstituted alkylthio", the "substituted or unsubstituted alkylsulfinyl" and the "substituted or unsubstituted alkylsulfonyl" include one or more substituents selected from the above substituent group α.

Examples of an embodiment of the "halogeno alkyl" include trifluoromethyl, fluoromethyl, and trichloromethyl.

Examples of an embodiment of the "halogeno alkoxy" include trifluoromethoxy, fluoromethoxy, and trichloromethoxy.

The "alkylidene" includes a divalent group of the above "alkyl", and examples include methylidene, ethylidene, propylidene, isopropylidene, butylidene, pentylidene, and hexylidene.

The "alkenyl" includes straight or branched alkenyl of a carbon number of 2 to 15, e.g. 2 to 10, 2 to 6, and 2 to 4, having one or more double bonds at any available position, and examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl and pentadecenyl.

The "alkynyl" includes straight or branched alkynyl of a carbon number of 2 to 10, e.g. 2 to 8, 3 to 6, having one or more triple bonds at any available position, and examples include ethynyl, propynyl, butynyl, pentynyl, hexynyl, heptynyl, octynyl, nonynyl, and decynyl. These may further a double bond at any available position.

Examples of the substituent of the "substituted or unsubstituted alkenyl", the "substituted or unsubstituted alkynyl", the "substituted or unsubstituted alkenyloxy ", the "substituted or unsubstituted alkynyloxy ", the "substituted or unsubstituted alkenylthio", the "substituted or unsubstituted alkynylthio", the "substituted or unsubstituted alkenyloxycarbonyl", the "substituted or unsubstituted alkynyloxycarbonyl", the "substituted or unsubstituted alkenylsulfinyl", the "substituted or unsubstituted alkynylsulfinyl", the "substituted or unsubstituted alkenylsulfonyl" and the "substituted or unsubstituted alkynylsulfonyl" include one or more substituents selected from the above substituent group α.

A alkenyl part of the "alkoxyalkenyl", the "alkoxycarbonylalkenyl", the "alkenyloxy", the ""alkenyloxycarbonyl", the "alkoxyalkenyloxy", the "alkenylthio", the "alkenylsulfinyl", the "alkenylsulfonyl", and the "alkenylamino" is the same as the above "alkenyl".

An alkynyl part of the "alkoxyalkynyl", the "alkoxycarbonylalkynyl", the "alkynyloxy", the "alkoxyalkynyloxy", the "alkynyloxycarbonyl", the "alkynylsulfinyl", the "alkynylsulfonyl", the "alkynylthio", and the "alkynylamino" is the same as the above "alkynyl".

Examples of the substituent of the "substituted or unsubstituted amino" , the "substituted or unsubstituted carbamoyl" , the "substituted or unsubstituted thiocarbamoyl" and the "substituted or unsubstituted sulfamoyl" include 1 to 2 substituents selected from alkyl, acyl, hydroxy, alkoxy, alkoxycarbonyl, a carbocyclic group and a heterocyclic group.

The "acyl" includes aliphatic acyl, carbocyclylcarbonyl and heterocyclylcarbonyl of a carbon number of 1 to 10. Examples include formyl, acetyl, propionyl, butyryl, isobutyryl, valeryl, pivaloyl, hexanoyl, acryloyl, propioloyl, methacryloyl, crotonoyl, benzoyl, cyclohexanecarbonyl, pyridinecarbonyl, furancarbonyl, thiophenecarbonyl, benzothiazolecarbonyl, pyrazinecarbonyl, piperidinecarbonyl, and thiomorpholino.

An acyl part of the "acyloxy" and the "acyl amino " is as described above.

Examples of the substituent of the "substituted or unsubstituted acyl" and the "substituted or unsubstituted acyloxy" include one or more substituents selected from the substituent group α. In addition, a ring part of the carbocyclylcarbonyl and the heterocyclylcarbonyl may be substituted with one or more substituents selected from alkyl, a substituent group α, and alkyl substituted with one or more substituents selected from the substituent group α.

The "carbocyclic group" includes cycloalkyl, cycloalkenyl, aryl and a non-aromatic fused carbocyclic group.

The "cycloalkyl" is a carbocyclic group of a carbon number of 3 to 10, e.g. 3 to 8, and 4 to 8, and examples include cyclopropyl, cyclobutyl, cyclopentyl, cyclohexyl, cycloheptyl, cyclooctyl, cyclononyl, and cyclodecyl.

A cycloalkyl part of the "cycloalkylalkyl", the "cycloalkoxy", the "cycloalkylalkoxy", the "cycloalkylthio", the "cycloalkylamino", the "cycloalkylalkylamino", the "cycloalkylsulfamoyl", the "cycloalkylsulfonyl", the "cycloalkylcarbamoyl", the "cycloalkyl alkyl carbamoyl", the "cycloalkyl alkoxycarbonyl", and the "cycloalkoxycarbonyl" is the same as the above "cycloalkyl".

The "cycloalkenyl" includes the cycloalkyl having one or more double bonds at any available position in the ring, and examples include cyclopropenyl, cyclobutenyl, cyclopentenyl, cyclohexenyl, cycloheptynyl, cyclooctynyl and cyclohexadienyl.

The "aryl" includes phenyl, naphthyl, anthryl and phenanthryl, and specific example is phenyl.

The "non-aromatic fused carbocyclic group" includes a non-aromatic group in which two or more cyclic groups selected from the above "cycloalkyl", the above "cycloalkenyl" and the above "aryl" are fused, and examples include indanyl, indenyl, tetrahydronaphthyl and fluorenyl.

A carbocyclyl part of the "carbocyclyl", the "carbocyclyloxy", the "carbocyclyl alkyl", the "carbocyclyl alkoxy", the "carbocyclyl alkoxycarbonyl", the "carbocyclylthio", the "carbocyclyl amino", the "carbocyclyl alkylamino", the "carbocyclyl carbonyl", the "carbocyclyl sulfamoyl", the "carbocyclyl sulfinyl", the "carbocyclyl sulfonyl", the "carbocyclylcarbamoyl", the "carbocyclyl alkylcarbamoyl", and the "carbocyclyloxycarbonyl" is the same as the "carbocyclic group".

An aryl part of the "arylalkyl", the "aryloxy", the "aryloxycarbonyl", the "aryl alkoxycarbonyl", the "arylthio", the "arylamino", the "aryl alkoxy", the "aryl alkylamino", the "arylsulfonyl", the "arylsulfamoyl", the "arylcarbamoyl", and the "aryl alkylcarbamoyl" is the same as the "aryl".

The "heterocyclic group" includes a heterocyclic group having one or more hetero atoms optionally selected from O, S and N in a ring, and examples include 5- to 6-membered heteroaryl such as pyrrolyl, imidazolyl, pyrazolyl, pyridyl, pyridazinyl, pyrimidinyl, pyrazinyl, triazolyl, triazinyl, tetrazolyl, furyl, thienyl, isoxazolyl, oxazolyl, oxadiazolyl, isothiazolyl, thiazolyl, and thiadiazolyl;
non-aromatic heterocyclic groups such as dioxanyl, thiiranyl, oxyranyl, oxetanyl, oxathioranyl, azetidinyl, thianyl, thiazolidinyl, pyrrolidinyl, pyrrolinyl, imidazolidinyl, imidazolinyl, pyrazolidinyl, pyrazolinyl, piperidyl, piperazinyl, morpholinyl, morpholino, thiomorpholinyl, thiomorpholino, dihydropyridyl, tetrahydropyridyl, tetrahydrofuryl, tetrahydropyranyl, dihydrothiazolyl, tetrahydrothiazolyl, tetrahydroisothiazolyl, dihydrooxazinyl, hexahydroazepinyl, tetrahydrodiazepinyl, and tetrahydropyridazinyl; fused bicyclic heterocyclic groups such as indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzotriazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, thienopyridyl, thienopyrrolyl, thienopyrazolyl, thienopyrazinyl, furopyrrolyl, thienothienyl, imidazopyridyl, imidazopyrazolyl, pyrazolopyridyl, pyrazolopyrazinyl, thiazolopyridyl, pyrazolopyrimidinyl, pyrazolotrianidyl, pyridazolopyridyl, triazolopyridyl, imidazothiazolyl, pyrazinopyridazinyl, quinazolinyl, quinolyl, isoquinolyl, naphthyridinyl, dihydrothiazolopyrimidinyl, tetrahydroquinolyl, tetrahydroisoquinolyl, dihydrobenzofuryl, dihydrobenzoxazinyl, dihydrobenzimidazolyl, tetrahydrobenzothienyl, tetrahydrobenzofuryl, benzodioxolyl, benzodioxonyl, chromanyl, chromenyl, octahydrochromenyl, dihydrobenzodioxinyl, dihydrobenzoxazinyl, dihydrobenzodioxepinyl, and dihydrothienodioxinyl;
fused tricyclic heterocyclic groups such as carbazolyl, acridinyl, xanthenyl, phenothiazinyl, phenoxathiinyl, phenoxazinyl, dibenzofuryl, imidazoquinolyl, and tetrahydrocarbazolyl. Examples include a 5- to 6-membered heteroaryl or non-aromatic heterocyclic group.

A heterocyclyl part of the "heterocyclyl", the "heterocyclyl alkyl", the "heterocyclyloxy", the "heterocyclylthio", the "heterocyclylcarbonyl", the "heterocyclyloxycarbonyl, the "heterocyclylalkoxy", the "heterocyclylamino", the "heterocyclylsulfamoyl", the "heterocyclylsulfinyl", the "heterocyclylsulfonyl", the "heterocyclylcarbamoyl", the "heterocyclyloxycarbonyl", the "heterocyclyl alkylamino", the "heterocyclyl alkoxycarbonyl" and the "heterocyclyl alkylcarbamoyl" is the same as the "heterocyclic group".

A bond of the "heterocyclic group" may be situated on any ring.

The "heteroaryl" includes an aromatic cyclic group among the "heterocyclic group". A heteroaryl part of the "heteroaryl alkyl" and the "heteroaryl alkoxy" is the same.

Examples of the ring A are as follows:

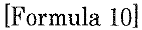

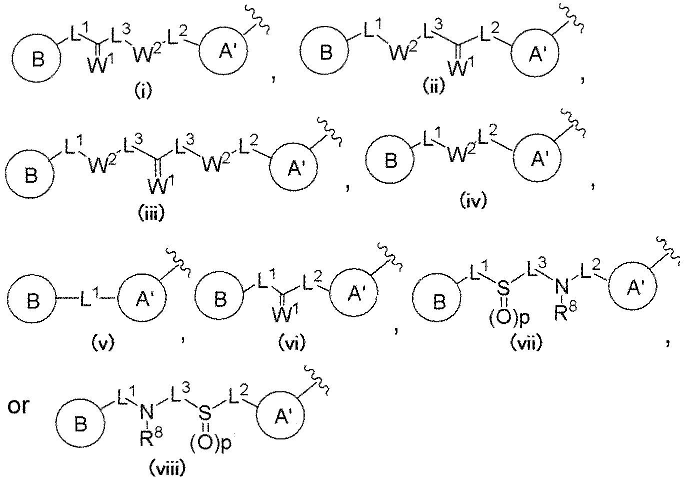

wherein ring A' and ring B are each independently a substituted or unsubstituted carbocycle or a substituted or unsubstituted heterocycle,
L¹ , L² and L³ are each independently a bond, substituted or unsubstituted alkylene, substituted or unsubstituted alkenylene or substituted or unsubstituted alkynylene, =W¹ is =O, =S or NR⁹⁻
W² is O, S or NR⁸;
R⁸ is hydrogen, substituted or unsubstituted alkyl, substituted or unsubstituted alkenyl, substituted or unsubstituted alkynyl or substituted or unsubstituted acyl,
R⁹ is hydrogen, hydroxy, substituted or unsubstituted alkyl, substituted or unsubstituted alkenyl, substituted or unsubstituted alkynyl or substituted or unsubstituted acyl,
when ring A is (i), a carbon atom in L¹ and a carbon atom in L² or a carbon atom in L¹ and a nitrogen atom in W², each may be bonded via a substituted or unsubstituted alkenylene to form a ring,
when ring A is (ii), a carbon atom in L¹ and a carbon atom in L² or a nitrogen atom in W² and a carbon atom in L² , each may be bonded via a substituted or unsubstituted alkenylene to form a ring,
when ring A is (iii), two nitrogen atoms in W²s may be bonded via a substituted or unsubstituted alkenylene to form a ring,
when ring A is (vi), a carbon atom in L¹ and a carbon atom in L² may be bonded via a substituted or unsubstituted alkenylene to form a ring,
p is 1 or 2,
when a plurality of L³s, a plurality of W²s and a plurality of R⁹s are present, each may be independently different.

### More specific examples are as follows;

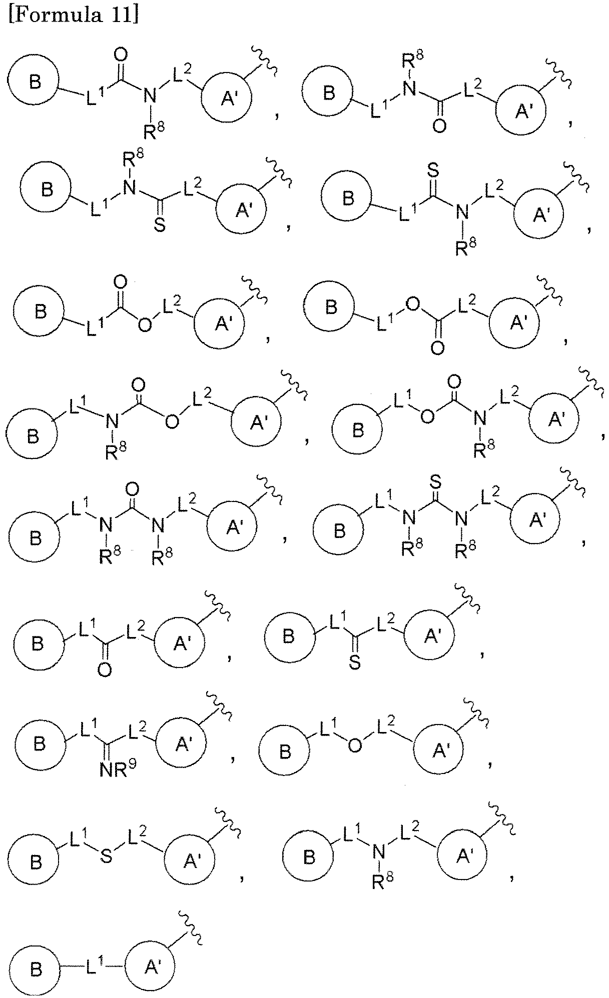

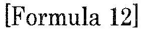

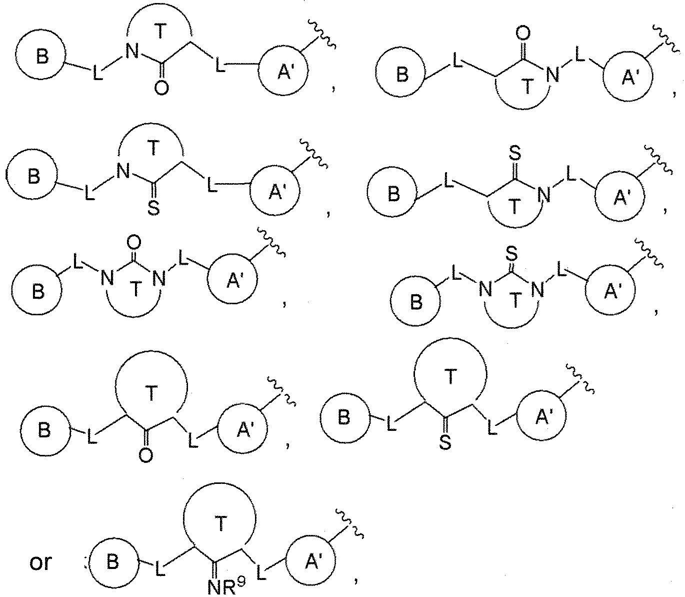

wherein all Ls are each independently a bond, optionally substituted lower alkylene, optionally substituted alkenylene, ring T is a carbocycle optionally substituted with the substituent(s) selected from the substituent group α or a heterocycle optionally substituted with the substituent(s) selected from the substituent group α, and the other symbols are as defined above.

More specific examples are as follows;

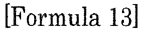

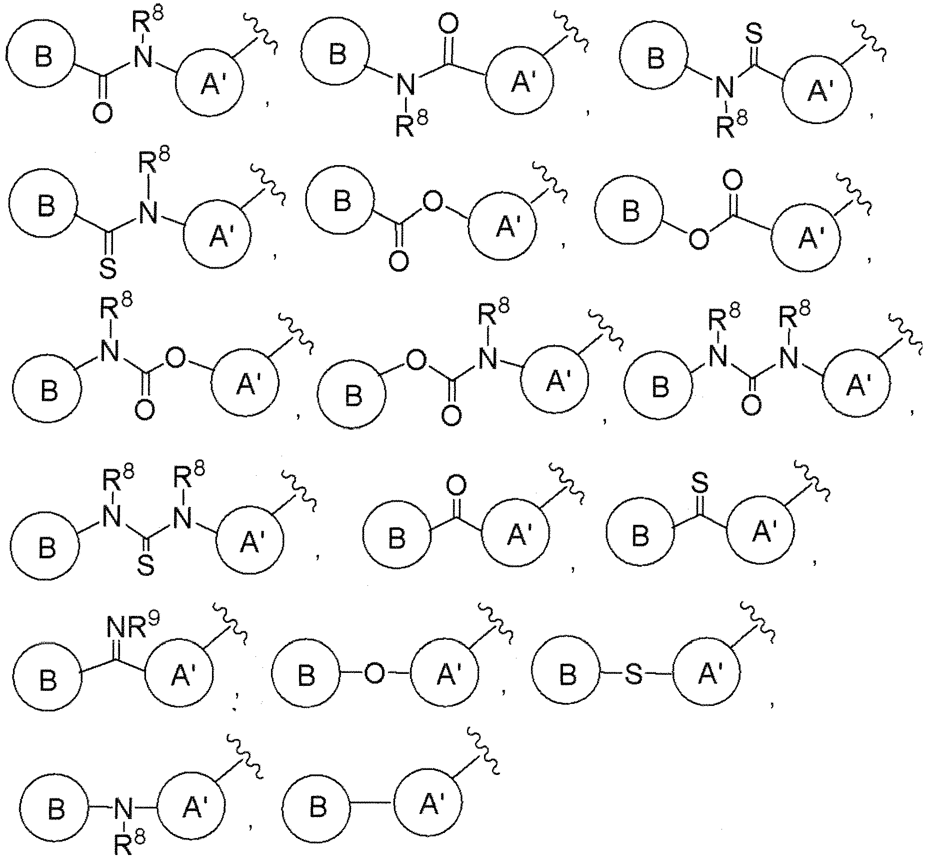

wherein each symbol is as defined above.

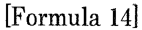

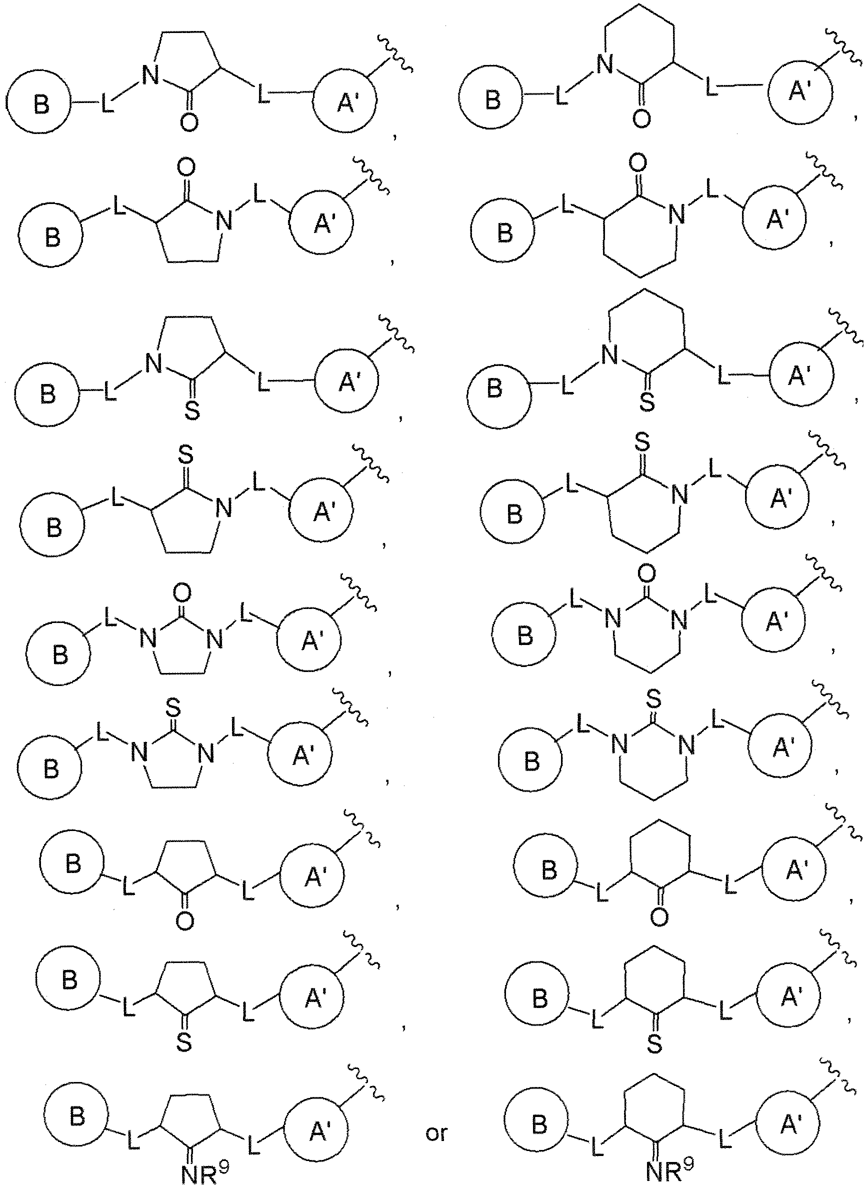

wherein each symbol is as defined above.

Examples of the substituent of the "substituted or unsubstituted carbocyclyl", the "substituted or unsubstituted heterocyclyl", the "substituted or unsubstituted benzene", the "substituted or unsubstituted pyridine", the "substituted or unsubstituted pyrimidine" and the "substituted or unsubstituted pyrazine" in ring A and ring B include:
a substituent group selected from a substituent group α, and e.g., halogen, hydroxy, alkoxy, acyl, acyloxy, carboxy, alkoxycarbonyl, carbamoyl, amino, cyano, alkylamino and/or alkylthio etc.;
alkyl substituted with one or more substituents selected from a substituent group α, hydroxyimino and alkoxyimino; herein, for example, the substituent is halogen, hydroxyl, alkoxy and/or alkoxycarbonyl etc., or unsubstituted alkyl;
aminoalkyl substituted with one or more substituents selected from a substituent group α; herein, for example, the substituent is acyl, alkyl and/or alkoxy etc.;
alkenyl substituted with one or more substituents selected from a substituent group α, herein, for example, the substituent is alkoxycarbonyl, halogen, and/or halogeno alkoxycarbonyl etc., or unsubstituted alkenyl;
alkynyl substituted with one or more substituents selected from a substituent group α, herein, for example, the substituent is alkoxycarbonyl etc., or unsubstituted alkynyl; alkoxy substituted with one or more substituents selected from a substituent group α, herein, for example, the substituent is halogen, carbamoyl, alkylcarbamoyl and/or hydroxy alkylcarbamoyl etc.;
alkoxyalkoxy substituted with one or more substituents selected from a substituent group α;
alkenyloxy substituted with one or more substituents selected from a substituent group α, herein, for example, the substituent is halogen, hydroxy, amino and/or lower alkylamino etc., or unsubstituted alkenyloxy;
alkoxyalkenyloxy substituted with one or more substituents selected from a substituent group α;
alkynyloxy substituted with one or more substituents selected from a substituent group α, herein, for example, the substituent is halogen and/or hydroxy etc., or unsubstituted alkynyloxy;
alkoxy alkynyloxy substituted with one or more substituents selected from a substituent group α;
alkylthio substituted with one or more substituents selected from a substituent group α or unsubstituted alkylthio;
alkenylthio substituted with one or more substituents selected from a substituent group α, or unsubstituted alkenylthio;
alkynylthio substituted with one or more substituents selected from a substituent group α, or unsubstituted alkynylthio;
alkylamino substituted with one or more substituents selected from a substituent group α;
alkenylamino substituted with one or more substituents selected from a substituent group α;
alkynylamino substituted with one or more substituents selected from a substituent group α;
aminooxy substituted with one or more substituents selected from a substituent group α and alkylidene, or unsubstituted aminooxy;
acyl substituted with one or more substituents selected from a substituent group α; alkylsulfonyl substituted with one or more substituents selected from a substituent group α, or unsubstituted alkylsulfonyl;
alkylsulfinyl substituted with one or more substituents selected from a substituent group α, or unsubstituted alkylsulfinyl;
alkylsulfamoyl substituted with one or more substituents selected from a substituent group α;
a carbocyclic group, e.g. cycloalkyl, aryl and the like, substituted with one or more substituents selected from a substituent group α, azide, alkyl and halogeno alkyl; heterocyclic group substituted with one or more substituents selected from a substituent group α, azide, alkyl and halogeno alkyl;
carbocyclyl alkyl, e.g. cycloalkyl alkyl, aryl alkyl and the like, substituted with one or more substituents selected from a substituent group α, azide, alkyl and halogeno alkyl, or unsubstituted carbocyclyl alkyl;
heterocyclyl alkyl substituted with one or more substituents selected from a substituent group α, azide, alkyl and halogeno alkyl, or unsubstituted heterocyclyl alkyl; carbocyclyloxy, e.g. cycloalkyloxy, aryloxy and the like, substituted with one or more substituents selected from a substituent group α, azide, alkyl and halogeno alkyl, or unsubstituted carbocyclyloxy, e.g. cycloalkyloxy, aryloxy and the like;
heterocyclyloxy substituted with one or more substituents selected from a substituent group α, azide, alkyl and halogeno alkyl, or unsubstituted heterocyclyloxy;
carbocyclyl alkoxy, e.g. cycloalkyl alkoxy, aryl alkoxy and the like, substituted with one or more substituents selected from a substituent group α, azide, alkyl and halogeno alkyl, or unsubstituted carbocyclyl alkoxy e.g. cycloalkyl alkoxy, aryl alkoxy and the like;
heterocyclyl alkoxy substituted with one or more substituents selected from a substituent group α, azide, alkyl and halogeno alkyl, or unsubstituted heterocyclyl alkoxy;
carbocyclyl alkoxycarbonyl, e.g. cycloalkyl alkoxycarbonyl, aryl alkoxycarbonyl and the like, substituted with one or more substituents selected from a substituent group α, azide, alkyl and halogeno alkyl, or unsubstituted carbocyclyl alkoxycarbonyl e.g. cycloalkyl alkoxycarbonyl, aryl alkoxycarbonyl and the like;
heterocyclyl alkoxycarbonyl substituted with one or more substituents selected from a substituent group α, azide, alkyl and halogeno alkyl, or unsubstituted heterocyclyl alkoxycarbonyl;
carbocyclylthio, e.g. cycloalkylthio, arylthio and the like, substituted with one or more substituents selected from a substituent group α, azide, alkyl and halogeno alkyl, or unsubstituted carbocyclylthio e.g. cycloalkylthio, arylthio and the like; heterocyclylthio substituted with one or more substituents selected from a substituent group α, azide, alkyl and halogeno alkyl, or unsubstituted heterocyclylthio; carbocyclylamino, e.g. cycloalkylamino, arylamino and the like, substituted with one or more substituents selected from a substituent group α, azide, alkyl and halogeno alkyl, or unsubstituted e.g. cycloalkylamino, arylamino and the like;
heterocyclylamino substituted with one or more substituents selected from a substituent group α, azide, alkyl and halogeno alkyl, or unsubstituted heterocyclylamino;
carbocyclyl alkylamino, e.g. cycloalkyl alkylamino, aryl alkylamino and the like, substituted with one or more substituents selected from a substituent group α, azide, alkyl and halogenoalky,l or unsubstituted carbocyclyl alkylamino e.g. cycloalkyl alkylamino, aryl alkylamino and the like;
heterocyclyl alkylamino substituted with one or more substituents selected from a substituent group α, azide, alkyl and halogeno alkyl, or unsubstituted heterocyclyl alkylamino;
carbocyclylsulfamoyl, e.g. cycloalkylsulfamoyl, arylsulfamoyl and the like, substituted with one or more substituents selected from a substituent group α, azide, alkyl and halogeno alkyl, or unsubstituted carbocyclyl sulfamoyl;
heterocyclylsulfamoyl substituted with one or more substituents selected from a substituent group α, azide, alkyl and halogeno alkyl, or unsubstituted heterocyclylsulfamoyl;
carbocyclylsulfonyl, e.g. cycloalkylsulfonyl, arylsulfonyl and the like, substituted with one or more substituents selected from a substituent group α, azide, alkyl and halogeno alkyl, or unsubstituted carbocyclylsulfonyl e.g. cycloalkylsulfonyl, arylsulfonyl and the like;
heterocyclylsulfonyl substituted with one or more substituents selected from a substituent group α, azide, alkyl and halogeno alkyl, or unsubstituted heterocyclylsulfonyl;
carbocyclylcarbamoyl, e.g. cycloalkylcarbamoyl, arylcarbamoyl and the like, substituted with one or more substituents selected from a substituent group α, azide, alkyl and halogeno alkyl, or unsubstituted carbocyclylcarbamoyl, e.g. cycloalkylcarbamoyl, arylcarbamoyl and the like;
heterocyclylcarbamoyl substituted with one or more substituents selected from a substituent group α, azide, alkyl and halogeno alkyl, or unsubstituted heterocyclylcarbamoyl;
carbocyclyl alkylcarbamoyl, e.g. cycloalkyl alkylcarbamoyl, aryl alkylcarbamoyl, substituted with one or more substituents selected from a substituent group α, azide, alkyl and halogeno alkyl, or unsubstituted carbocyclyl alkylcarbamoyl, e.g. cycloalkyl alkylcarbamoyl, aryl alkylcarbamoyl;
heterocyclyl alkylcarbamoyl substituted with one or more substituents selected from a substituent group α, azide, alkyl and halogeno alkyl, or unsubstituted heterocyclyl alkylcarbamoyl;
carbocyclyloxycarbonyl, e.g. cycloalkyloxycarbonyl, aryloxycarbonyl and the like, substituted with one or more substituents selected from a substituent group α, azide, alkyl and halogeno alkyl, or unsubstituted carbocyclyloxycarbonyl, e.g. cycloalkyloxycarbonyl, aryloxycarbonyl and the like;
heterocyclyloxycarbonyl substituted with one or more substituents selected from a substituent group α, azide, alkyl and halogeno alkyl, or unsubstituted heterocyclyloxycarbonyl;
alkylenedioxy substituted with halogen, or unsubstituted alkylenedioxy; oxo;
and azide.
The aforementioned ring of ring A and ring B each may be substituted with one or more substituents selected from them.

Examples of the substituent of the "substituted or unsubstituted carbocyclyl", the "substituted or unsubstituted benzene", the "substituted or unsubstituted heterocyclyl", the "substituted or unsubstituted pyridine" and the "substituted or unsubstituted pyrazine" in ring A' and ring B include:
halogen, cyano, hydroxy, nitro, carboxy, alkyl substituted with one or more substituents selected from a substituent group α, unsubstituted alkyl, alkoxy substituted with one or
more substituents selected from a substituent group α, unsubstituted alkoxy, amino substituted with one or more substituents selected from a substituent group α,
unsubstituted amino, carbamoyl substituted with one or more substituents selected from a substituent group α, unsubstituted carbamoyl, alkoxycarbonyl substituted with one or more substituents selected from a substituent group α, and unsubstituted alkoxycarbonyl.

Examples of the substituent except "-Z-ring B" of the "substituted or unsubstituted carbocycle" or the "substituted or unsubstituted heterocycle" in ring A include halogen, hydroxy, alkyl, alkenyl, alkynyl, alkoxy, alkenyloxy, alkynyloxy, acyl, carboxy, alkoxycarbonyl, amino or cyano.

Examples of the substituent of the "substituted or unsubstituted carbocycle", the "substituted or unsubstituted benzene" and the "substituted or unsubstituted heterocycle" in ring A' include halogen.

Examples of the substituent of the "substituted or unsubstituted carbocyclyl", the "substituted or unsubstituted heterocycle", "substituted or unsubstituted pyridine", "substituted or unsubstituted pyrimidine" and the "substituted or unsubstituted pyrazine" in ring B include halogen, alkyl, alkenyl, alkynyl, alkoxy, alkenyloxy, alkynyloxy or cyano.

In those other than ring A, ring A' and ring B, examples of the substituent of the "substituted or unsubstituted carbocyclic group", the "substituted or unsubstituted heterocyclic group", the "substituted or unsubstituted arylalkyl", the "substituted or unsubstituted arylalkoxy", the "substituted or unsubstituted heteroarylalkyl", the "substituted or unsubstituted heteroarylalkoxy", the "substituted or unsubstituted carbocyclylalkyl", the "substituted or unsubstituted carbocyclylalkoxy", the "substituted or unsubstituted carbocyclyloxy", the "substituted or unsubstituted carbocyclylthio", the "substituted or unsubstituted carbocyclyloxycarbonyl", the "substituted or unsubstituted carbocyclylsulfinyl", the "substituted or unsubstituted carbocyclylsulfonyl", the "substituted or unsubstituted heterocyclylalkyl", the "substituted or unsubstituted heterocyclylalkoxy", the "substituted or unsubstituted heterocyclyloxy", the "substituted or unsubstituted heterocyclylthio", the "substituted or unsubstituted heterocyclyloxycarbonyl", the "substituted or unsubstituted heterocyclylsulfinyl", the "substituted or unsubstituted heterocyclylsulfonyl", "substituted or unsubstituted carbocyclyl", and the "substituted or unsubstituted heterocyclyl " include one or more substituents selected from (i) alkyl substituted with one or more substituents selected from the substituent group α, (ii) unsubstituted alkyl and (iii) substituent group α.

The "alkylene" includes a straight or branched divalent carbon chain of a carbon number of 1 to 10, e.g. 1 to 6 and 1 to 3. Examples include methylene, dimethylene, trimethylene, tetramethylene, and methyltrimethylene.

An alkylene part of the " alkylenedioxy" is the same as the "alkylene".

The " alkenylene" includes a straight or branched divalent carbon chain of a carbon number of 2 to 10, e.g. 2 to 6, and 2 to 4, having a double bond at any available position. Examples include vinylene, propenylene, butenylene, butadienylene, methylpropenylene, pentenylene and hexenylene.

The "lower alkynylene" includes a straight or branched divalent carbon chain of a carbon number of 2 to 10, e.g. 2 to 6 and 2 to 4, having a triple bond at any available position and, further, optionally having a double bond. Examples include ethynylene, propynylene, butynylene, pentynylene and hexynylene.

Examples of the substituent of the "substituted or unsubstituted alkylene", the "substituted or unsubstituted alkenylene" and the "substituted or unsubstituted alkynylene" include a group(s) selected from the substituent group α, specific examples are halogen, and hydroxy.

In formula (I), an embodiment wherein

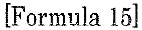

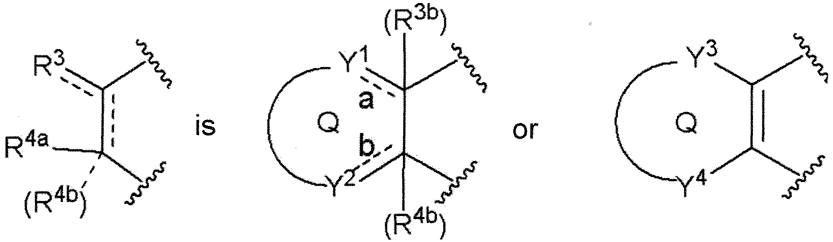

Each symbol is as defined above. Examples include the following:

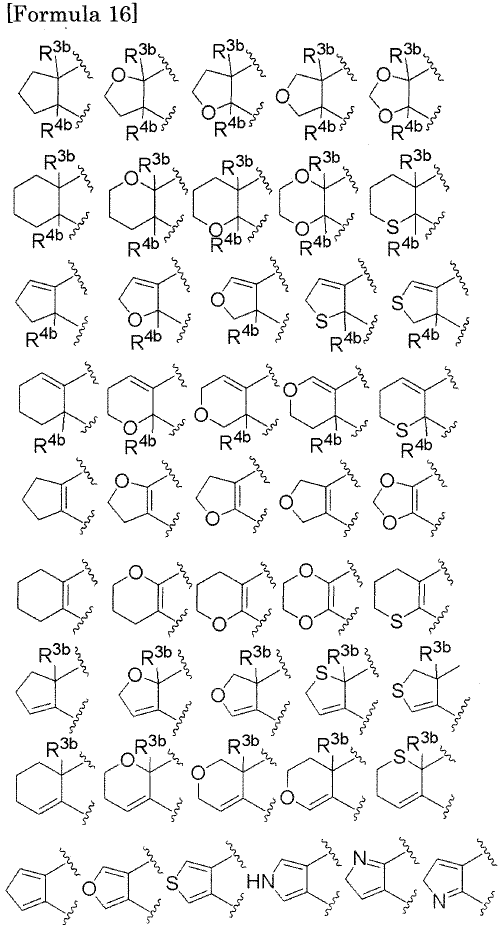

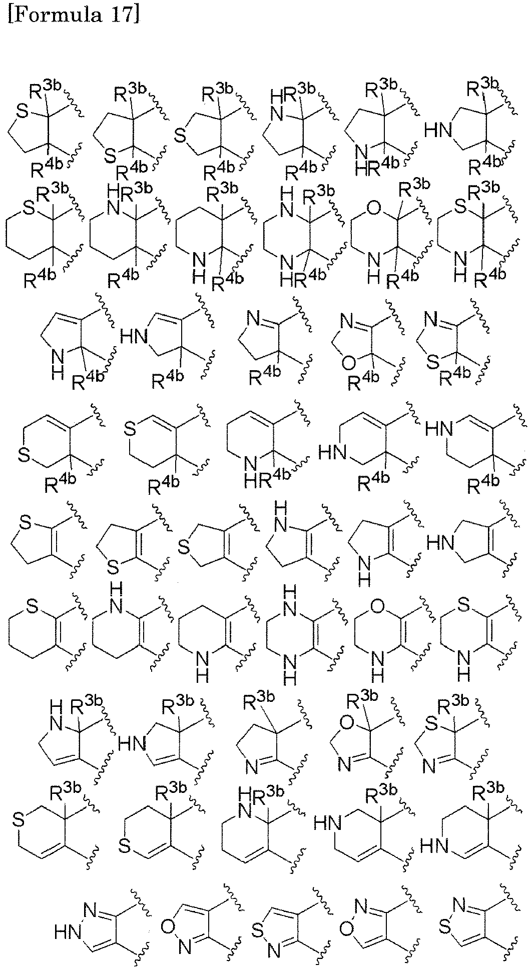

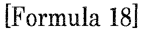

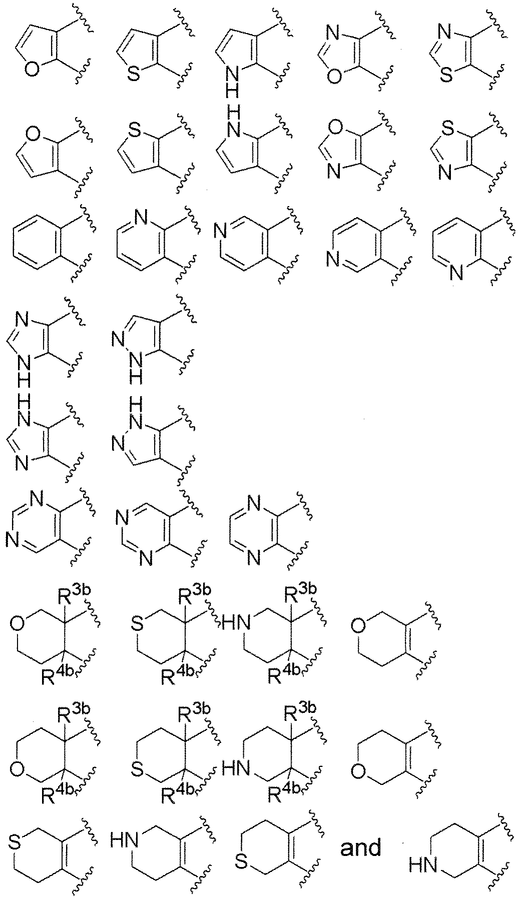

Any available position in these examples may be substituted with one or more substituents selected from substituent group α, unsubstituted alkyl and alkyl substituted with one or more substituents selected from the substituent group α.

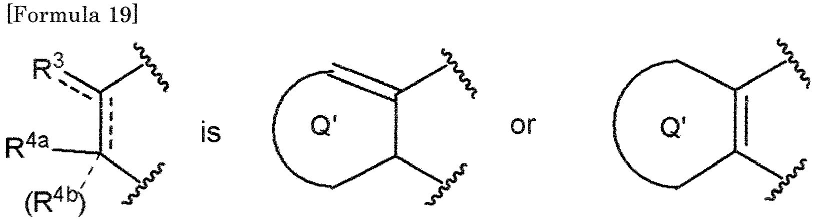

Each symbols as defined above. Examples include the followings:

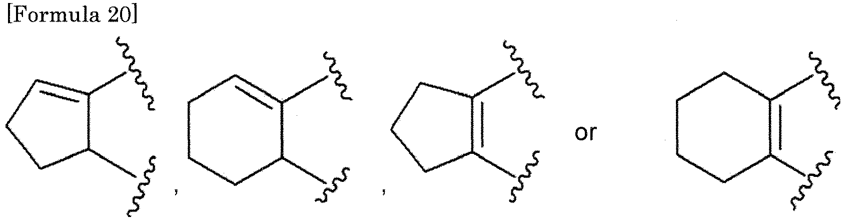

Any available position in these examples may be substituted with one or more substituents selected from substituent group α, unsubstituted alkyl and alkyl substituted with one or more substituents selected from the substituent group α.

In the present specification, the "solvate" includes, for example, a solvate with an organic solvent and a hydrate, and which can be prepared by known methods. Preferable solvates include a solvate with acetone, 2-butanol, 2-propanol, ethanol, ethyl acetate, tetrahydrofuran, diethyl ether etc. Examples include a hydrate or a solvate, which are nontoxic and water soluble, e.g. ethanol. When a solvate or a hydrate is formed, an arbitrary number of water molecules may be coordinated.

The compound of formula (I), (I') and (Ia) to (In) includes a pharmaceutically acceptable salt. Examples include salts with alkali metals such as lithium, sodium or potassium; alkaline earth metals such as calcium; magnesium; transition metals such as zinc and iron; ammonium; organic bases; and amino acids; or salts with inorganic acids such as hydrochloric acid, sulfuric acid, nitric acid, hydrobromic acid, phosphoric acid or hydroiodic acid; and organic acids such as acetic acid, trifluoroacetic acid, citric acid, lactic acid, tartaric acid, oxalic acid, maleic acid, fumaric acid, mandelic acid, glutaric acid, malic acid, benzoic acid, phthalic acid, benzenesulfonic acid, p-toluenesulfonic acid, methane sulfonic acid or ethane sulfonic acid. Examples include particularly, hydrochloric acid, phosphoric acid, tartaric acid or methanesulfonic acid. These salts can be formed by ordinary methods.

In addition, the compound of formula (I) is not limited to a specific isomer, but includes all possible isomers, such as keto-enol isomers, imine-enamine isomers, diastereoisomers, optical isomers and rotation isomers; and racemate. For example, the compound of formula (I) in which R^{2a} is hydrogen includes the following tautomers.

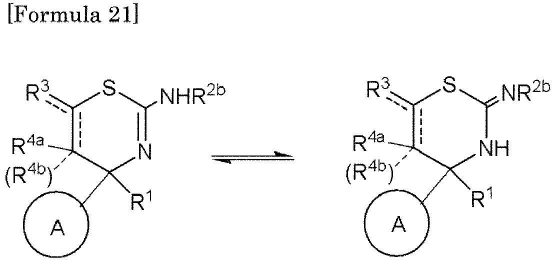

In addition, the compound of formula (I) which has an asymmetrical atom includes the following optical isomers.

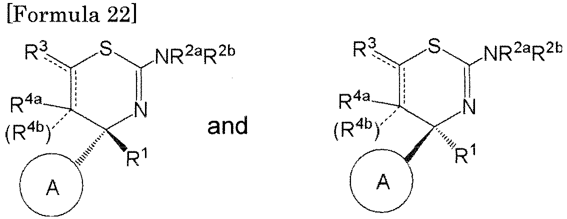

The compound of the present invention is preferably as follows.

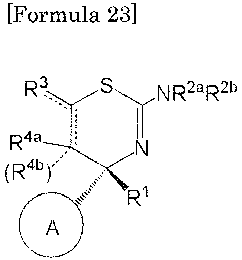

The compound of formula (I') and (Ia) to (In) include same tautomers.

In addition, one or more hydrogen, carbon or other atoms of a compound of formula (I), (I') and (Ia) to (In) can be replaced by an isotope of the hydrogen, carbon or other atoms. Compounds of formula (I) , (I') and (Ia) to (In) include all radiolabeled forms of compounds of formula (I), (I') and (Ia) to (In) "radiolabeled," "radiolabeled form", and the like of a compound of formula (I), (I') and (Ia) to (In), each of which is encompassed by the invention, is useful as a research and/or diagnostic tool in metabolism pharmacokinetic studies and in binding assays.

Examples of isotopes that can be incorporated into a compound of formula (I), (I') and (Ia) to (In) of the invention include isotopes of hydrogen, carbon, nitrogen, oxygen, phosphorous, sulfur, fluorine and chlorine, such as ²H, ³H, ¹¹C , ¹³C, ¹⁴C, ¹⁵N, ¹⁸O, ¹⁷O, ³¹P, ³²P, ³⁵S, ¹⁸F, ¹²³I and ³⁶Cl, respectively. Radiolabeled compounds of the invention can be prepared by methods known in the art. For example, tritiated compounds of formula (I) can be prepared by introducing tritium into the particular compound of formula (I), for example, by catalytic dehalogenation with tritium. This method may include reacting a suitably halogen-substituted precursor of a compound of formula (I) with tritium gas in the presence of a suitable catalyst, for example, Pd/C, in the presence or absence of a base. Other suitable methods for preparing tritiated compounds can be found in Filer, Isotopes in the Physical and Biomedical Sciences, Vol. 1, Labeled Compounds (Part A) Chapter 6 (1987). ¹⁴C-labeled compounds can be prepared by employing starting materials having a ¹⁴C carbon.

The present compound of formula (I), (I') and (Ia) to (In) can be produced, for example, according to the method such as those described in Journal of Heterocyclic Chemistry, 14, 717-723 (1977), or by the following method.

In the following all steps, when a substituent which interferes with the reaction, e.g. hydroxy, mercapto, amino, formyl, carbonyl, carboxy, is possessed, the substituent is protected by the method such as those described in Protective Groups in organic Synthesis, and Theodora W Greene (John Wiley & Sons) in advance, and the protective group may be removed at a desirable step.

In addition, in the all steps, an order of steps to be implemented may be appropriately changed, and each intermediate may be isolated, and used in a next step.

### General method for preparation

### Production of compound of formula (I)

The compound of formula (I) can be produced, for example, according to a method of synthesizing shown below.

1) Synthesis of compound (IIa)

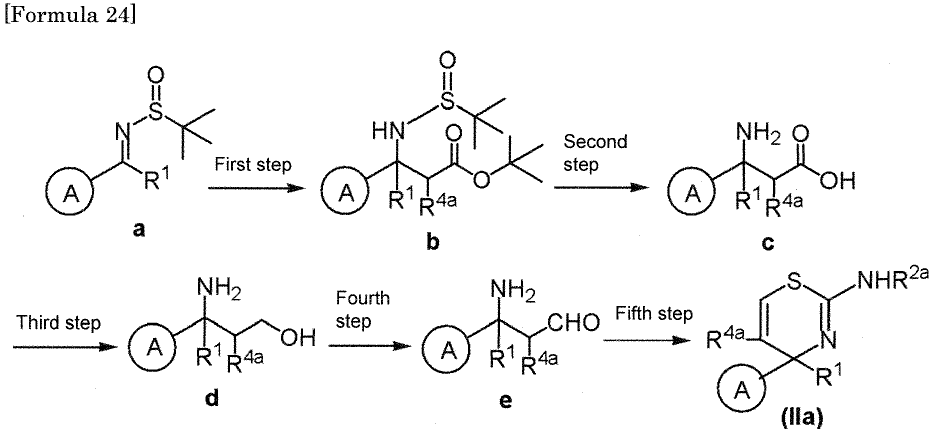

wherein each symbol is as defined above.

### First step

Compound b can be prepared by adding a titanium reagent such as chlorotitanium triisopropoxide to enolate, which is obtained by reacting an objective ester such as t-butyl propionate in the presence of a base such as lithium diisopropylamide in a solvent such as toluene, dichloromethane, and tetrahydrofuran, or a mixed solvent thereto, adding compound a which can be prepared by the known methods, and reacting them at -80°C to 30°C, preferably -80°C to 0°C, for 0.1 to 24 hours, preferably 0.1 to 12 hours.

### Second step

Compound c can be prepared by reacting the compound b at 0°C to 80°C, preferably 0°C to 30°C, for 0.5 to 48 hours, preferably 1 to 24 hours in the presence of an acid such as hydrochloric acid, hydrobromic acid, sulfuric acid and trifluoroacetic acid in a solvent such as dioxane, methanol, and dichloromethane, or a mixed solvent of them.

### Third step

A compound d can be prepared by adding a reducing agent such as borane, sodium borohydride and lithium aluminum hydride to the compound c and reacting at -80°C to 80°C, preferably -20°C to 30°C, for 0.5 to 48 hours, preferably 1 to 12 hours in a solvent such as dioxane, tetrahydrofuran, and toluene, or a mixed solvent of them.

### Fourth step

A compound e can be prepared by adding an oxidizing agent such as 2-iodoxybenzoic acid to the compound d and reacting at 0°C to 80°C, preferably 10°C to 40°C, for 0.5 to 48 hours, preferably 1 to 12 hours in a solvent such as dimethyl sulfoxide, and dichloromethane.

In third step and fourth step, amine and/or aldehyde groups of the compound d and the compound e can be protected by the method such as those described in Protective Groups in Organic Synthesis, Theodora W Green (John Wiley & Sons), and deprotected at an appropriate time, if necessary.

### Fifth step

A compound (IIa) can be prepared by adding isothiocyanate having a protective group, e.g. benzoyl isothiocyanate, which is commercially available or is prepared by the known methods, to the compound e, and reacting at -30°C to 50°C, preferably -10°C to 25°C, for 0.1 to 12 hours, preferably 0.1 to 3 hours in a solvent such as dioxane, tetrahydrofuran, toluene, and acetone, or a mixed solvent of them and, subsequently, adding concentrated sulfuric acid or concentrated nitric acid, followed by a reaction at 0°C to 100°C, preferably 0°C to 60°C, for 0.5 to 24 hours, preferably 1 to 12 hours.

2) Synthesis of compound (Ia)

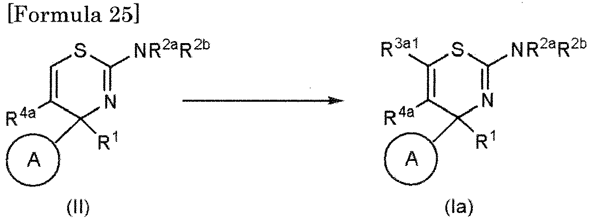

wherein R^{3a1} is halogen, hydroxy, substituted or unsubstituted alkyl, substituted or unsubstituted alkenyl, substituted or unsubstituted alkynyl, substituted or unsubstituted acyl, substituted or unsubstituted alkoxy, carboxy or substituted or unsubstituted alkoxycarbonyl, and other symbols are as defined above.

A compound (Ia) can be prepared by adding all sorts of electrophile, e.g. alkyl halide, aldehyde, to the compound (II) prepared by reacting in a solvent such as toluene, dichloromethane and tetrahydrofuran or a mixed solvent of them in the presence of a base such as tert-butyllithium, sec-butyllithium, n-butyllithium and in the presence or absence of additives such as tetramethylethylene diamine, and reacting them at -80°C to 30°C, preferably -80°C to -40°C, for 0.1 to 24 hours, preferably 0.1 to 6 hours.

3) Synthesis of compound (Ib)

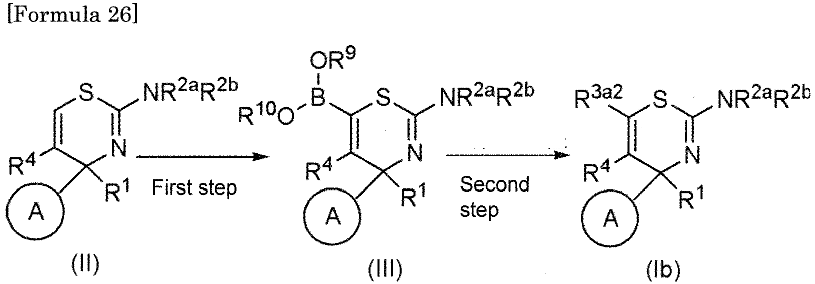

wherein R^{3a2} is substituted or unsubstituted carbocyclyloxy, substituted or unsubstituted heterocyclyloxy, substituted or unsubstituted alkylthio, substituted or unsubstituted amino, substituted or unsubstituted carbocyclyl or substituted or unsubstituted heterocyclyl, R⁹ and R¹⁰ are each hydrogen or substituted or unsubstituted alkyl, or, R⁹ and R¹⁰ taken together with the oxygen atoms binding thereto form a substituted or unsubstituted ring, and the other symbols are as defined above.

### First step

A compound (III) can be prepared by adding all sorts ofborane reagent to the compound (II) prepared by after reacting in a solvent such as toluene, dichloromethane and tetrahydrofuran or a mixed solvent of them in the presence of a base such as tert-butyllithium, sec-butyllithium, n-butyllithium and in the presence or absence of additives such as tetramethylethylene diamine, and reacting them at -80°C to 30°C, preferably -80°C to -40°C, for 0.1 to 24 hours, preferably 0.1 to 6 hours.

### Second step

A compound of general formula (Ib) can be prepared by adding trisbenzylideneacetone dipalladium, palladium acetate, or a Pd(0) catalyst which is prepared in situ and a ligand such as tri-tert-butylphosphine, dicyclohexylbiphenylphosphine, 9,9-dimethyl-4,5-bis (diphenylphosphino) xanthene (Xantphos), 2 - dicyclohexylphosphino-2', 4', 6'-triisopropyl biphenyl (X-Phos), 2 - dicyclohexylphosphino-2', 6'-diisopropoxy 1,1' - biphenyl (Ruphos) to compound (III) and carbocyclyl halide or heterocyclic which are commercially available or can be prepared by the known methods and equivalent to this, and reacting the mixture at 0°C to 150°C, preferably 10°C to 100°C, for 0.5 to 72 hours, preferably 1 to 24 hours in the presence of a base such as, triethylamine, sodium carbonate, potassium carbonate, and cesium carbonate in a solvent such as toluene, tetrahydrofuran, dimethylformamide, 1,2 - dimethoxyethane, 1,4 - dioxane and methanol, or a mixed solvent of them.

4) Synthesis of compound (Ic)

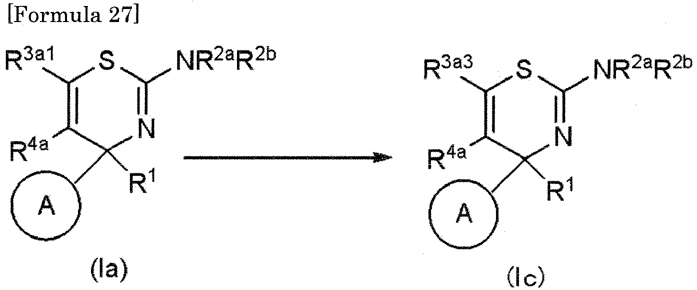

wherein R^{3a3} is cyano or substituted or unsubstituted carbamoyl, and the other symbols are as defined above.

A compound (Ic) can be prepared by derivatizing each compound (Ia) by the method such as those described in Shijikkenkagakukouza (Maruzen, 1978, synthesis and reaction of organic compounds).

In formula (I),

The compound wherein,

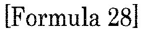

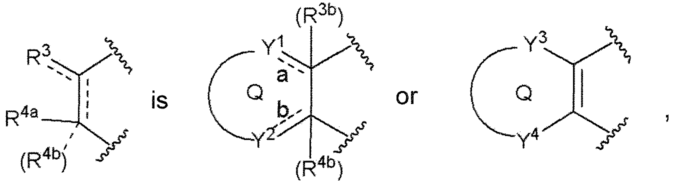

i.e., the compound of formula (Id) and (Ie) can be prepared, for example, according to a method of synthesis shown below.

5) Synthesis of compound (Id)

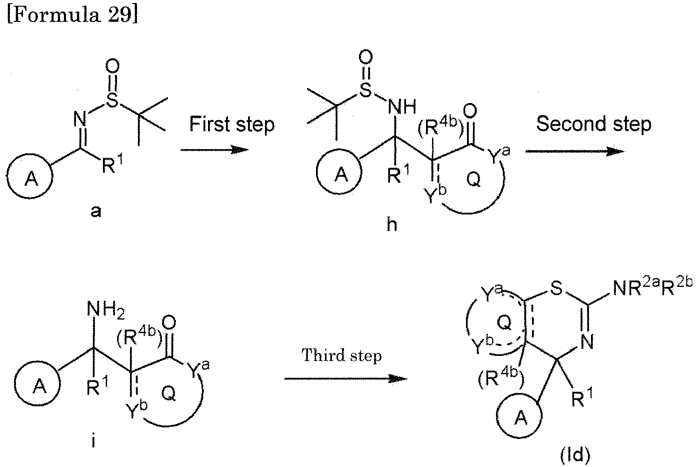

wherein Y^{a} is Y¹ or Y³, Y^{b} is Y² or Y⁴ and each dashed line means the presence or absence of a bond, the other symbols are as defined above.

### First step

A compound h can be prepared by adding a compound which can be prepared by the known methods, to an enolate obtained by reacting a carbonyl compound corresponding to an objective compound such as cyclopentanone in the presence of a base such as lithium diisopropylamide, and reacting them at -80°C to 30°C, preferably -80°C to 0°C, for 0.1 to 24 hours, preferably 0.1 to 12 hours in a solvent such as toluene, dichloromethane, and tetrahydrofuran, or a mixed solvent of them.

### Second step

A compound i can be prepared by reacting the compound h at 0°C to 80°C, preferably 0°C to 30°C, for 0.5 to 48 hours, preferably 1 to 24 hours in the presence of an acid such as hydrochloric acid, hydrobromic acid, sulfuric acid and trifluoroacetic acid in a solvent such as dioxane, methanol, and dichloromethane, or a mixed solvent of them.

### Third step

A compound j can be prepared by adding isothiocyanate having a protective group, e.g. benzoyl isothiocyanate, which is commercially available or prepared by the known methods to the compound i, reacting at -30°C to 50°C, preferably -10°C to 25°C, for 0.1 to 12 hours, preferably 0.1 to 3 hours in a solvent such as dioxane, tetrahydrofuran, toluene and acetone, or a mixed solvent of them, and subsequently, adding concentrated sulfuric acid or concentrated nitric acid, followed by a reaction at 0°C to 100°C, preferably 0°C to 60°C, for 0. 5 to 24 hours, preferably 1 to 12 hours.

A compound (Id) having wherein each dashed line means the presence of a bond can be prepared by the above method. A compound (Id) having wherein each dashed line means the absence of a bond can be prepared by hydrogen addition under a condition generally used.

6) Synthesis of compound (Ie)

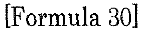

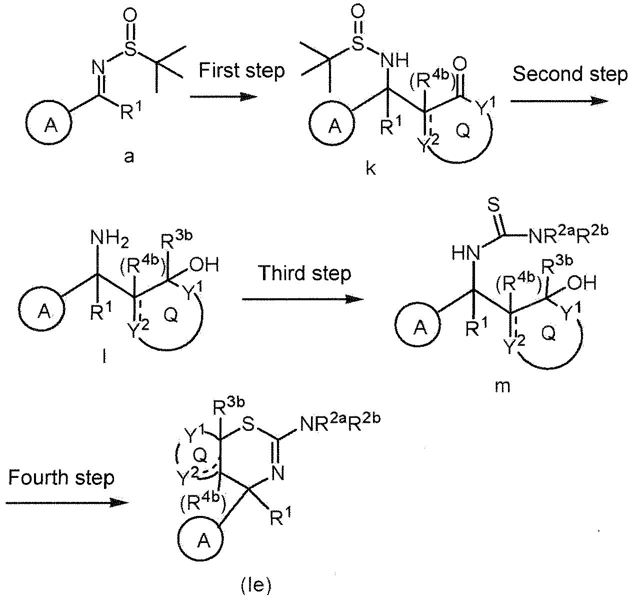

wherein each symbol is as defined above.

### First step

A compound k can be prepared by adding a compound a obtained by a same method as described above, to enolate prepared by reacting a carbonyl compound corresponding to an objective compound such as cyclopentanone in the presence of a base such as lithium diisopropylamide, and reacting them at -80°C to 30°C, preferably -80°C to 0°C, for 0.1 to 24 hours, preferably 0.1 to 12 hours in a solvent such as toluene, dichloromethane, and tetrahydrofuran, or a mixed solvent of them.

### Second step

A compound 1 can be prepared by adding a Grignard reagent such as methylmagnesium bromide which is commercially available or is prepared by the known methods to the compound k, and reacting at -80°C to 80°C, preferably -20°C to 30°C, for 0.5 to 48 hours, preferably 1 to 12 hours in a solvent such as dioxane, tetrahydrofuran, ether and toluene, or a mixed solvent of them, and reacting at 0°C to 80°C, preferably 0°C to 30°C, for 0.5 to 48 hours, preferably 1 to 24 hours in the presence of an acid such as hydrochloric acid, hydrobromic acid, sulfuric acid and trifluoroacetic acid.

### Third step

A compound m can be prepared by adding isothiocyanate having a protective group, e.g. benzoyl isothiocyanate, which is commercially available or is prepared by the known methods, to the compound 1, reacting at -30°C to 50°C, preferably -10°C to 25°C for 0.1 to 12 hours, preferably 0.1 to 3 hours in a solvent such as dioxane, tetrahydrofuran, toluene, and acetone, or a mixed solvent of them, subsequently, adding concentrated sulfuric acid, concentrated nitric acid or the like, and reacting them at 0°C to 100°C , preferably 0°C to 60°C for 0.5 to 24 hours, preferably 1 to 12 hours.

### Fourth step

A compound n can be prepared by adding oxalyl chloride, thionyl chloride or the like, and a catalytic amount of N,N-dimethylformamide, or adding a chlorinating reagent such as 1-chloro-2-trimethylpropenylamine to the compound m, and reacting at 0°C to 100°C, preferably 10°C to 50°C, for 0.5 to 72 hours, preferably 0.5 to 6 hours in a solvent such as dichloromethane, tetrahydrofuran, and toluene.

A compound (Ie) having dashed line which means the presence of a bond can be prepared by the method as described above. A compound (Ie) having dashed line which means the absence of a bond can be prepared by a method of hydrogen adding in a condition generally used.

7) Synthesis of compound (Ig)

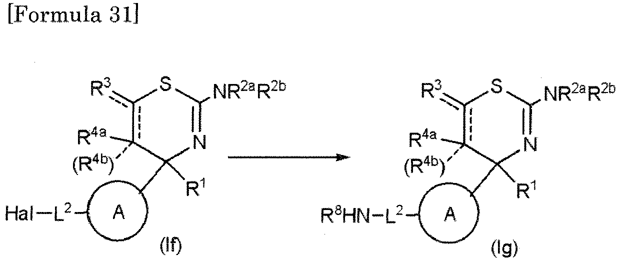

wherein Hal is halogen, and the other symbols are as defined above.

A compound (Ig) can be prepared by adding tris(dibenzylideneacetone) dipalladium, palladium acetate, palladium (0) prepared in situ or the like, and a phosphine ligand such as tert-butylphosphine, and dicyclohexylbiphenylphosphine to the compound (If) prepared by the above method in a solvent such as tetrahydrofuran, toluene, and xylene, adding a reagent having a substituent corresponding to an objective compound such as lithium hexamethyldisilazide, and benzophenoneimine at -10°C to 30°C, and reacting them at 30°C to reflux temperature of a solvent, preferably 50°C to 100°C, for 0.5 to 48 hours, preferably 3 to 20 hours.

8) Synthesis of compound (Ii)

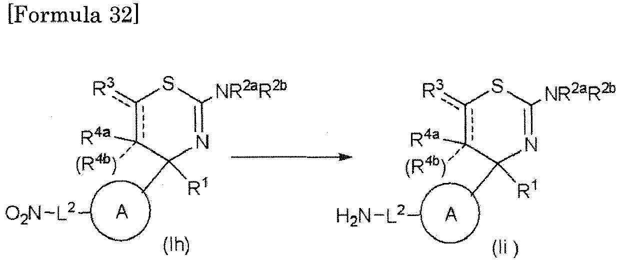

wherein each symbol is as defined above.

A compound (Ii) can be prepared by adding iron to a compound (Ih) in a mixed solvent of acetic acid and water, followed by a reaction at 20°C to 120°C, preferably 50°C to 80°C, for 0.5 to 48 hours, preferably 6 to 20 hours.

Besides, the compound (Ii) can be also prepared by adding a catalytic reducing catalyst such as 10% palladium/carbon to the compound (Ih) in a solvent such as tetrahydrofuran, ethyl acetate, and methanol, and reacting them at 30°C to 120°C, preferably 50°C to 80°C, for 0.5 to 48 hours, preferably 6 to 20 hours under the hydrogen atmosphere at a normal pressure to 5 atm, preferably a normal pressure to 2 atm, or by the method such as those described in Comprehensive Organic Transformations, Richard C Larock (Mcgraw-Hill).

9) Synthesis of compound (Ig)

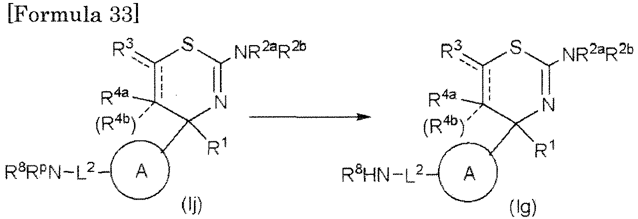

wherein R^{p} is an amino protective group, and other symbols are as defined above.

Compound of formula (Ig) can be prepared by deprotecting amino groups of the compound (Ij) by the method such as those described in Protective Groups in Organic Synthesis, Theodora W Green (John Wiley & Sons).

The amino protective group may be a substituent which can be deprotected by the method such as those described in Protective Groups in Organic Synthesis, Theodora W Green (John Wiley & Sons), and examples include alkoxycarbonyl, alkenyloxycarbonyl, trialkylsilyl, acyl, methanesulfonyl, trifluoroethanesulfonyl, toluenesulfonyl and the like.

10) Synthesis of compound (Ik)

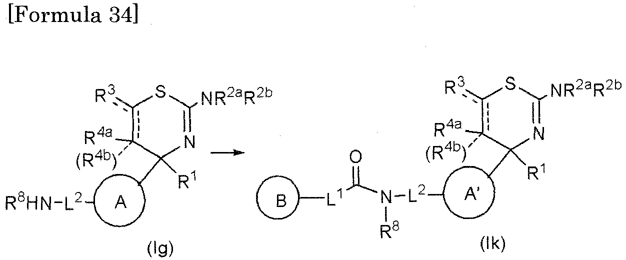

wherein each symbol is as defined above.

Compound (Ik) can be prepared by adding a reagent having a substituent corresponding to an objective compound such as acid chlorides, anhydride, chlorocarbonic acid esters, isocyanate, e.g. benzoyl chloride, 2-furoyl chloride, acetic anhydride, benzyl chlorocarbonate, di-tert-butyl dicarbonate, phenyl isocyanate, to compound (Ig) having a substituted or unsubstituted an amino group in ring A, in the presence or absence of a solvent such as tetrahydrofuran, dichloromethane, and in the presence or absence of a base such as pyridine, triethylamine, and reacting them at -80°C to 100°C, preferably -20°C to 40°C, for 0.1 to 24 hours, preferably 1 to 12 hours, or compound (Ik) can be prepared by adding a carboxylic acid having a substituent corresponding to an objective compound such as benzoic acid, 2-pyridinecarboxylic acid, to the compound (Ig) in the presence of a dehydration condensing agent such as dicyclohexylcarbodiimid, carbonyldiimidazole,
dicyclohexylcarbodiimide-N-hydroxybenzotriazole, and reacting them at -80°C to 100°C, preferably -20°C to 40°C, for 0.1 to 24 hours, preferably 1 to 12 hours in a solvent such as dimethylformamide, tetrahydrofuran, dichloromethane.

### 11) Synthesis of compound (II)

Compound (Il) can be prepared by method A or method B.

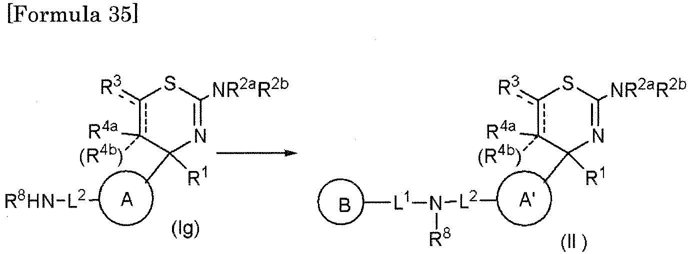

wherein each symbol is as defined above.

### Method A: condensation under the acid condition

Compound of the general formula (II) can be prepared by adding an acid such as hydrogen chloride, sulfuric acid, trifluoroacetic acid, methanesulfonic acid, trifluoromethanesulfonic acid, perchloric acid, to aryl halide or heteroaryl halide which is commercially available or is prepared by the known methods such as those described in Tetrahedron, vol.65, 757-764, 2009, or the method equivalent to those and compound (Ig), and reacting them at 0°C to reflux temperature of a solvent, preferably 20°C to 140°C, for 0.1 to 120 hours, preferably 0.5 to 72 hours in a solvent such as methanol, ethanol, isopropyl alcohol, butanol, isobutanol, sec-butanol, acetic acid, water, or a mixed solvent of them.

### Method B: condensation under the base condition

Compound (II) can be prepared by reacting an aryl halide or a hetero aryl halide which is commercially available or is prepared by the known methods such as those described in Tetrahedron, vol.65, 757-764, 2009, or the method equivalent to those and compound (Ig) in the presence of an base triethylamine, sodium carbonate, potassium carbonate, cesium carbonate, sodium methoxide, potassium tert-butoxide, n-butyl lithium, lithium hexamethyldisilazide, sodium hexamethyldisilazide, potassium hexamethyldisilazide at 0°C to reflux temperature of a solvent, preferably 20°C to 140°C, for 0.5 to 100 hours, preferably 0.5 to 72 hours in a solvent such as toluene, tetrahydrofuran, dimethylformamide, 1,2-dimethoxyethane, 1,4-dioxane, methanol.

The above reaction may be carried out by adding trisbenzylideneacetone dipalladium, palladium acetate, or a palladium(0) catalyst which is prepared in situ and a phosphine ligand such as triphenylphosphine, tri-tert-butylphosphine, dicyclohexylbiphenylphosphine, 9,9-dimethyl-4,5-bis (diphenylphosphino) xanthene (Xantphos), 2-dicyclohexylphosphino-2', 4', 6'-triisopropyl biphenyl (X-Phos), 2 - dicyclohexylphosphino-2', 6'-diisopropoxy 1,1'-biphenyl (Ruphos). In this case, compound of the general formula (II) can be prepared by reacting at 0°C to 150°C, preferably 10°C to 100°C, for 0.5 to 72 hours, preferably 1 to 24 hours under microwave irradiation or non-irradiation.

12) Synthesis of compound (In)

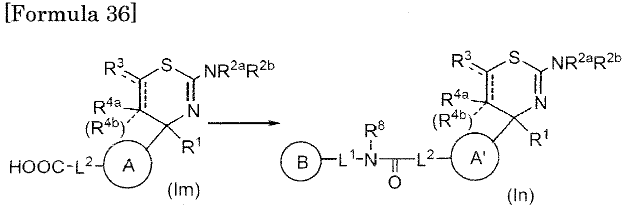

wherein each symbol is as defined above.

A compound having a protected carboxyl group is prepared by the above method 1), and (Ia), (Ib) and (Ic) are prepared by introducing substituent R^{3a} by the method 2) to 4). Or compound (Id) or (Ie) having a protected carboxy is prepared by the above-mentioned method 5) or 6).

Compound (Im) having a protected carboxyl group is prepared by deprotecting a thus-obtained compound according to the method such as those described in Protective Groups in Organic Synthesis, Theodora W Greene (John Wiley & Sons). Compound (In) can be prepared by reacting a primary amine or a secondary amine having a substituent corresponding to an objective compound such as aniline, 2-amino pyridine, and dimethyl amine, with compound (Im) in the presence of a dehydration condensing agent such as dicyclohexylcarbodiimid, carbonyldiimidazole, dicyclohexylcarbodiimide-N-hydroxybenzotriazole at -80°C to 100°C, preferably -20°C to 40°C, for 0.1 to 24 hours, preferably 1 to 12 hours in a solvent such as dimethylformamide, tetrahydrofuran, dichloromethane.

The compounds b, h, and k can be prepared, for example, according to the method such as those described in (1) T. Fujisawa et al., Tetrahedron Lett., 37,3881-3884 (1996), (2) D. H. Hua et al., Sulfur Reports, vol.21, pp.211-239 (1999), (3) Y. Koriyama et al., Tetrahedron, 58, 9621-9628 (2002) or (4) T. Vilavan et al., Current Organic Chemistry, 9, 1315-1392 (2005), Patent Literature 1, Patent Literature 2 and Patent Literature 3.

An optically active substance of compound (I) can be prepared by using an optical activate material, obtaining an optical activate intermediate by asymmetric synthesis in appropriate step, or optically resoluting an intermediate of each racemate and a final product in an appropriate step. Procedures of the optical resolution include a method of separating an optical isomer using an optically active column; kinetic optical resolution utilizing an enzymatic reaction and the like; crystallization resolution of a diastereomer by salt formation using a chiral acid or a chiral base; preferential crystallization method and the like.

Examples of a preferable embodiment include as follows. Each symbol is as defined above.

(A) Preferable example of formula (I):

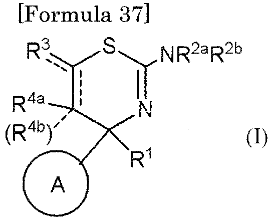

is formula (I')

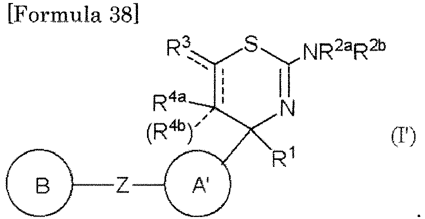

In formula (I) or (I'), examples of R¹ include substituted or unsubstituted alkyl, substituted or unsubstituted alkenyl, substituted or unsubstituted alkynyl, cyano, a substituted or unsubstituted carbocyclic group or a substituted or unsubstituted heterocyclic group.

Examples of R¹ include unsubstituted alkyl of a carbon number of 1 to 3.

Examples of R^{2a} and R^{2b} include each independently hydrogen, substituted or unsubstituted alkyl, or substituted or unsubstituted acyl.

Examples of R^{2a} and R^{2b} include hydrogen respectively.

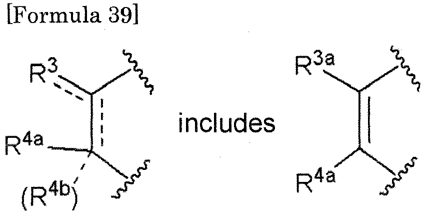

wherein examples of R^{3a} include halogen, hydroxy, substituted or unsubstituted alkyl, substituted or unsubstituted alkenyl, substituted or unsubstituted alkynyl, substituted or unsubstituted acyl, substituted or unsubstituted alkoxy, substituted or unsubstituted carbocyclyloxy, substituted or unsubstituted heterocyclyloxy, substituted or unsubstituted alkynylthio, carboxy, cyano, substituted or unsubstituted alkoxycarbonyl, substituted or unsubstituted amino, substituted or unsubstituted carbamoyl, a substituted or unsubstituted carbocyclic group or a substituted or unsubstituted heterocyclic group. Examples of R^{4a} include hydrogen or substituted or unsubstituted alkyl.

Examples of R^{3a} include substituted or unsubstituted alkyl, substituted or unsubstituted acyl, carboxy, cyano, substituted or unsubstituted alkoxycarbonyl, substituted or unsubstituted amino, substituted or unsubstituted carbamoyl, or a substituted or unsubstituted heterocyclic group. Examples of R^{4a} include hydrogen.

Examples of R^{3a} include substituted or unsubstituted alkyl, cyano, substituted or unsubstituted alkoxycarbonyl, substituted or unsubstituted carbamoyl, or a substituted or unsubstituted heterocyclic group. An example of R^{4a} includes hydrogen.

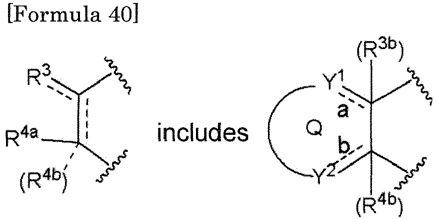

wherein examples include

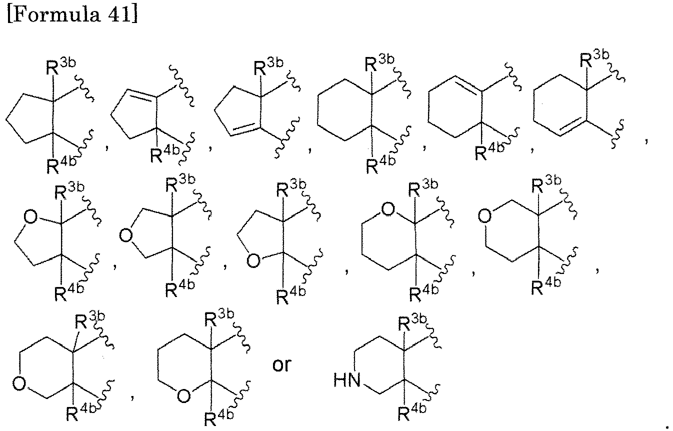

Any available position in these groups may be substituted with one or more substituents selected from substituent group α, unsubstituted alkyl and alkyl substituted with one or more substituents selected from the substituent group α.

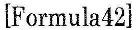

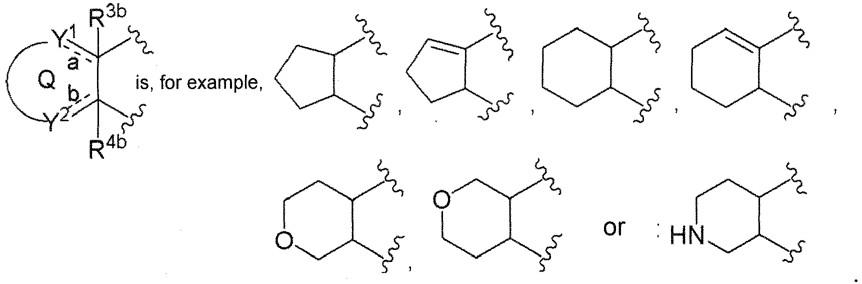

Any available position in these groups may be substituted with one or more substituents selected from substituent group α, unsubstituted alkyl, and alkyl substituted with one or more substituents selected from the substituent group α.

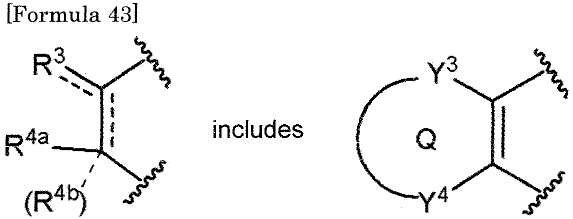

wherein examples include

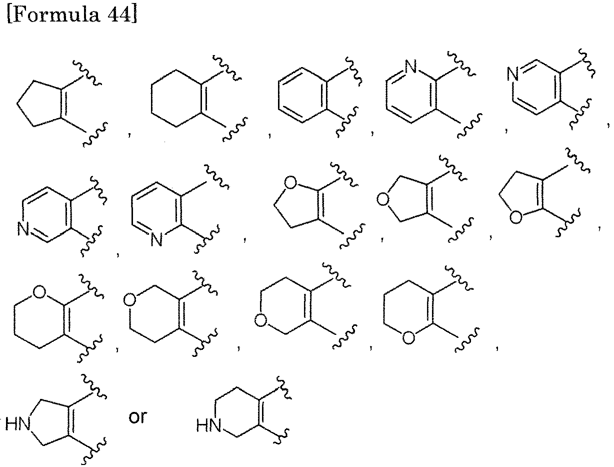

Any available position in these groups may be substituted with one or more substituents selected from substituent group α, unsubstituted alkyl, and alkyl substituted with one or more substituents selected from the substituent group α.

Examples of ring A, ring A' and ring B are each independently a substituted or unsubstituted carbocycle or a substituted or unsubstituted heterocycle.

Examples of ring A or ring A' are substituted or unsubstituted benzene or substituted or unsubstituted pyridine, preferably benzene substituted with halogen.

Examples of ring B are substituted or unsubstituted pyridine, substituted or unsubstituted pyrimidine, or substituted or unsubstituted pyrazine. Preferable substituents are one or more substituents selected from the halogen, cyano, alkyl and alkoxy.

Examples of Z are -L¹⁻C(=O)N(R⁸)-L²-, -L¹⁻N(R⁸)C(=O)-L²⁻, or -L¹⁻(R⁸)-L²⁻ wherein "L¹" bonds to ring B and "L²" bonds to ring A'.

L¹ and L² are each independently a bond, substituted or unsubstituted alkylene, substituted or unsubstituted alkenylene or substituted or unsubstituted alkynylene.

Examples of Z are -L¹⁻C(=O)N(R⁸)-L²⁻, -L¹⁻N(R⁸)C(=O)-L²⁻, or -L¹⁻(R⁸)-L²⁻ wherein L¹ and L² are a bond, and "L¹" bonds to ring B and "L²" bonds to ring A'.

Examples of Z are -L¹⁻-C(=O)N(R⁸)-L²⁻, or -N(R⁸)-, wherein L¹ and L² are a bond, and "L¹" bonds to ring B and "L²" bonds to ring A'.

Examples of R⁸ are hydrogen, substituted or unsubstituted alkyl, substituted or unsubstituted alkenyl, substituted or unsubstituted alkynyl, or substituted or unsubstituted acyl.

An example of R⁸ is hydrogen.

Excluding the following compounds:

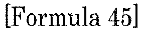

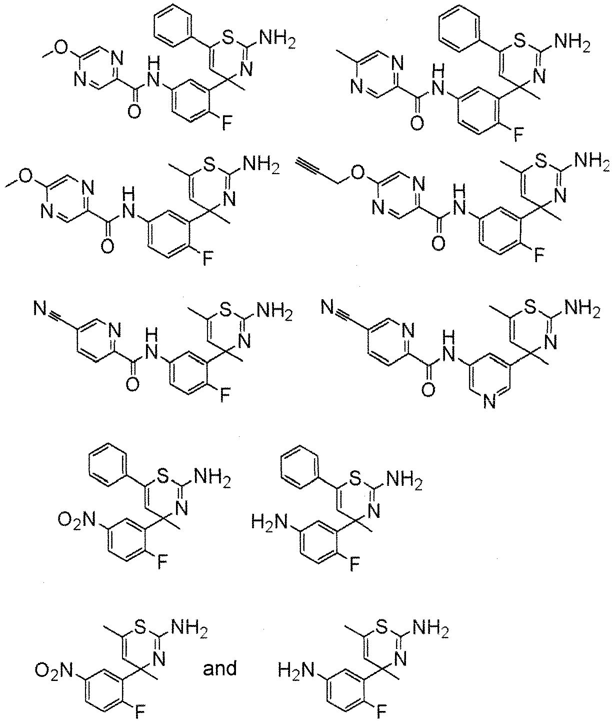

(B) In formula (I):

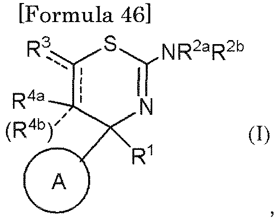

### examples of each symbols are as follows:

Examples of R¹are substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group.

Examples of R^{2a} and R^{2b} are each independently hydrogen, substituted or unsubstituted alkyl, substituted or unsubstituted acyl, substituted or unsubstituted alkoxycarbonyl or substituted or unsubstituted carbamoyl.

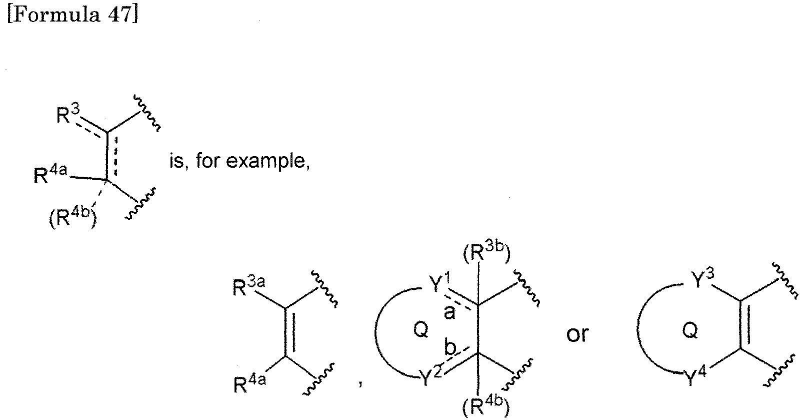

wherein examples of R^{3a} are halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl.

Examples of R^{4a} are hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl.

Examples of ring Q include a substituted or unsubstituted carbocycle or a substituted or unsubstituted heterocycle.

Examples of R^{3b} and R^{4b} are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, carboxy, substituted or unsubstituted alkoxycarbonyl, substituted or unsubstituted alkenyloxycarbonyl, substituted or unsubstituted alkynyloxycarbonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl.

Dashed line a and dashed line b each independently mean the presence or absence of a bond, and for example, when dashed line a means the presence of a bond, then R^{3b} is absent, and when dashed line b means the presence of a bond, then R^{4b} is absent.

Examples of Y¹ and Y² are each independently -C(R⁵)(R⁶)-, -C(R⁵)=, -N(R⁷)-, -N=, -S-, -SO-, -SO₂- or -O-.

Examples of Y³ and Y⁴ are each independently -C(R⁵)(R⁶)-, -N(R⁷)-, -S-, -SO-, -SO₂ -or -O-.

Examples of R⁵ and R⁶ are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl,

Examples of R⁷ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, substituted or unsubstituted carbamoyl, substituted or unsubstituted thiocarbamoyl, substituted or unsubstituted sulfamoyl, substituted or unsubstituted amino, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl.

Example of ring A is a substituted or unsubstituted carbocycle or a substituted or unsubstituted heterocycle.
Excluding the following compounds:

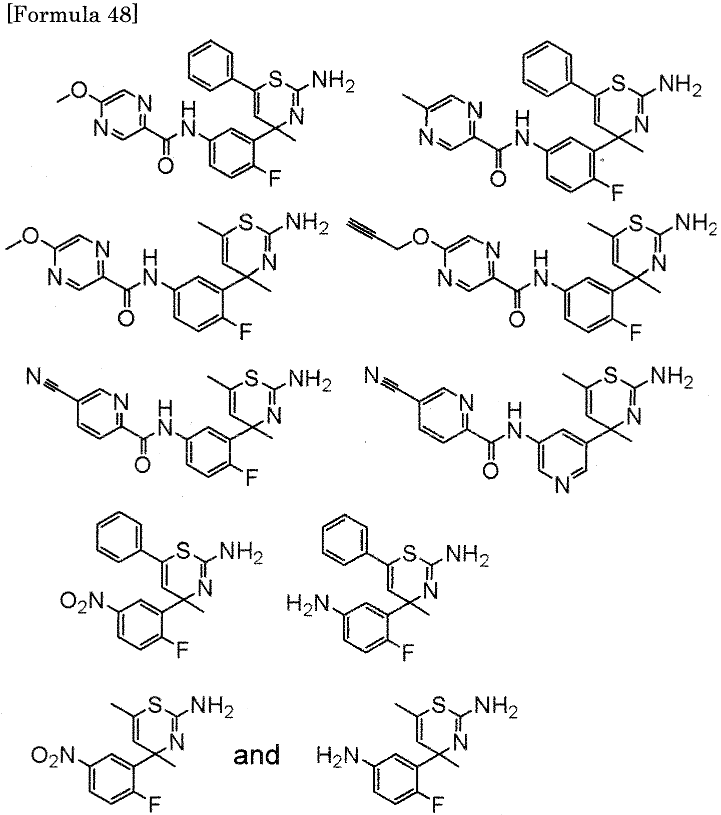

Embodiments of the above formula (I) are as follows.

(i) A compound wherein, R¹ is substituted or unsubstituted alkyl, R^{2a} and R^{2b} are hydrogen respectively,

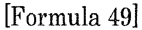

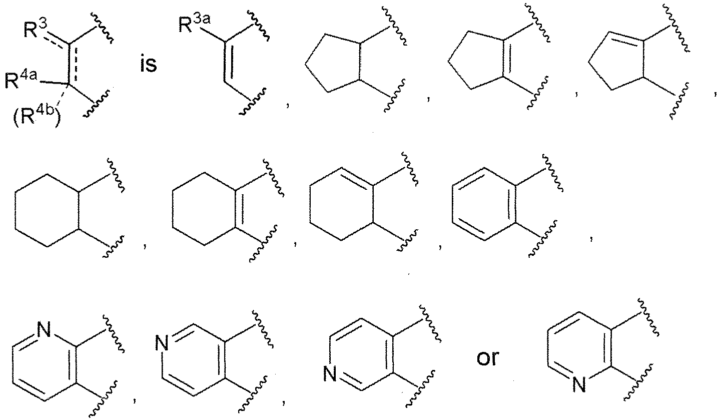

wherein R^{3a} is substituted or unsubstituted alkyl, substituted or unsubstituted alkenyl, cyano, carboxy, substituted or unsubstituted alkoxycarbonyl, substituted or unsubstituted alkenyloxycarbonyl, substituted or unsubstituted carbamoyl, substituted or unsubstituted thiocarbamoyl, a substituted or unsubstituted carbocyclic group, or a substituted or unsubstituted heterocyclic group,
ring A is a substituted or unsubstituted carbocycle or a substituted or unsubstituted heterocycle,
its pharmaceutically acceptable salt or a solvate thereof.

(ii) A compound wherein R¹ is unsubstituted alkyl of a carbon number of 1 to 3, R^{2a} and R^{2b} are hydrogen respectively,

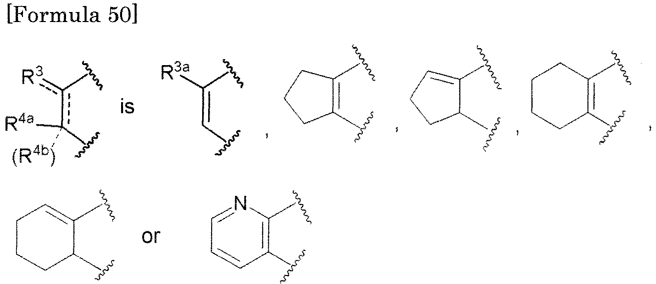

wherein R^{3a} is substituted or unsubstituted alkyl wherein the substituent is one or more substituents selected from halogen and hydroxy; cyano; alkoxycarbonyl; substituted or unsubstituted carbamoyl wherein the substituent is one or more substituents selected from alkyl and alkoxyalkyl; or a heterocyclic group,
ring A is ,

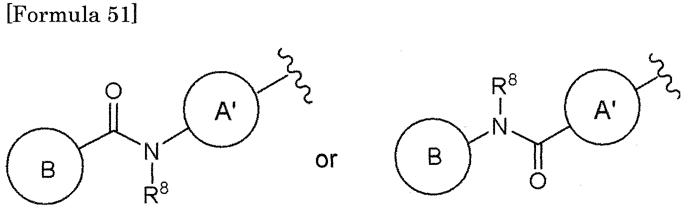

wherein ring A' is a substituted or unsubstituted carbocycle, ring B is a substituted or unsubstituted heterocycle, R⁸ is hydrogen, substituted or unsubstituted alkyl or substituted or unsubstituted acyl,
its pharmaceutically acceptable salt or a solvate thereof.

(iii) A compound of (ii) wherein, ring A' is a benzene ring which may be substituted with halogen, ring B is a substituted or unsubstituted pyridine or a substituted or unsubstituted pyrazine wherein the substituent is one or more substituents selected from halogen, cyano, alkyl and alkoxy, R⁸ is hydrogen,
its pharmaceutically acceptable salt or a solvate thereof.

(iv) A compound wherein

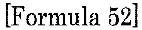

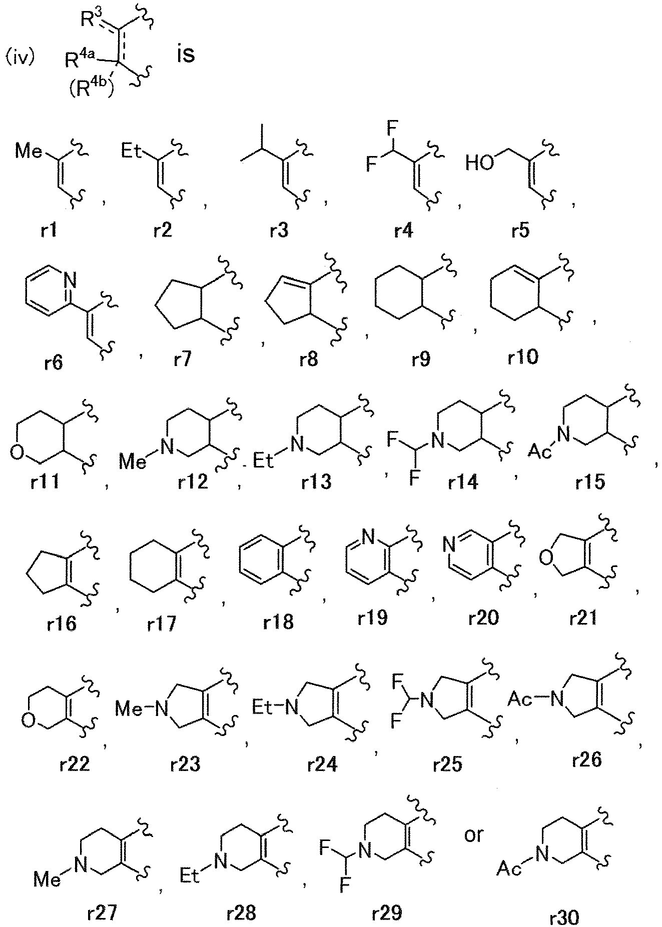

wherein Me is methyl, Et is ethyl and Ac is acetyl, (v) A compound wherein ring A is

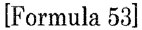

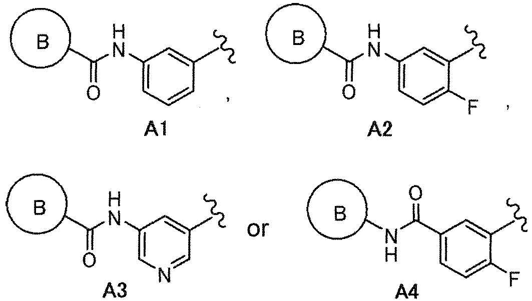

(vi) A compound wherein ring B is

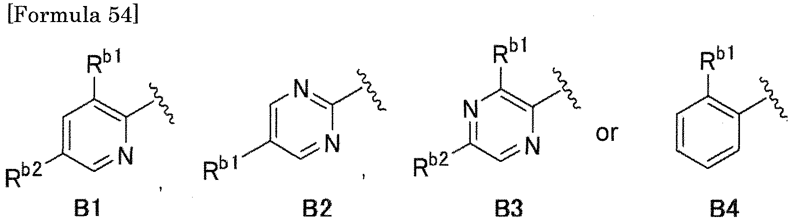

wherein R^{1b} and R^{2b} are each independently hydrogen, chloro, fluoro, methoxy, butynyloxy, cyano, amino or carbamoyl.

(vii) A compound wherein the combination of ring B, R^{b1} and R^{b2} (B, R^{b1}, R^{b2}) is as follows.
(B1, hydrogen, hydrogen) (hereinafter referred to as "ring B is b1"),
(B1 ,hydrogen, chloro) (hereinafter referred to as "ring B is b2"),
(B1, hydrogen, fluoro) (hereinafter referred to as "ring B is b3"),
(B1, hydrogen, methoxy) (hereinafter referred to as "ring B is b4"),
(B1, hydrogen, butynyloxy) (hereinafter referred to as "ring B is b5"),
(B1, hydrogen, cyano) (hereinafter referred to as "ring B is b6"),
(B1, hydrogen, amino) (hereinafter referred to as "ring B is b7"),
(B1, hydrogen, carbamoyl) (hereinafter referred to as "ring B is b8"),
(B1, chloro, hydrogen) (hereinafter referred to as "ring B is b9"),
(B1, chloro, chloro) (hereinafter referred to as "ring B is b10"),
(B1, chloro, fluoro) (hereinafter referred to as "ring B is b11"),
(B1, chloro, methoxy) (hereinafter referred to as "ring B is b 12"),
(B1, chloro, butynyloxy) (hereinafter referred to as "ring B is b13"),
(B1, chloro, cyano) (hereinafter referred to as ""ring B is b14"),
(B1, chloro, amino) (hereinafter referred to as "ring B is b15"),
(B1, chloro, carbamoyl) (hereinafter referred to as "ring B is b16"),
(B1, fluoro, hydrogen) (hereinafter referred to as "ring B is b17"),
(B1, fluoro, chloro) (hereinafter referred to as "ring B is b18"),
(B1, fluoro, fluoro) (hereinafter referred to as "ring B is b19"),
(B1, fluoro, methoxy) (hereinafter referred to as "ring B is b20"),
(B1, fluoro, butynyloxy) (hereinafter referred to as "ring B is b21"),
(B1, fluoro, cyano) (hereinafter referred to as "ring B is b22"),
(B1, fluoro, amino) (hereinafter referred to as "ring B is b23"),
(B1, fluoro, carbamoyl) (hereinafter referred to as "ring B is b24"),
(B1, methoxy, hydrogen) (hereinafter referred to as "ring B is b25"),
(B1, methoxy, chloro) (hereinafter referred to as "ring B is b26"),
(B1, methoxy, fluoro) (hereinafter referred to as "ring B is b27"),
(B1, methoxy, methoxy) (hereinafter referred to as "ring B is b28"),
(B1, methoxy, butynyloxy) (hereinafter referred to as "ring B is b29"),
(B1, methoxy, cyano) (hereinafter referred to as "ring B is b30"),
(B1, methoxy, amino) (hereinafter referred to as "ring B is b31"),
(B1, methoxy, carbamoyl) (hereinafter referred to as "ring B is b32"),
(B1, butynyloxy, hydrogen) (hereinafter referred to as "ring B is b33"),
(B1, butynyloxy, chloro) (hereinafter referred to as "ring B is b34"),
(B1, butynyloxy, fluoro) (hereinafter referred to as "ring B is b35"),
(B1, butynyloxy, methoxy) (hereinafter referred to as "ring B is b36"),
(B1, butynyloxy, cyano) (hereinafter referred to as "ring B is b37"),
(B1, butynyloxy, amino) (hereinafter referred to as "ring B is b38"),
(B1, butynyloxy, carbamoyl) (hereinafter referred to as "ring B is b39"),
(B1, cyano, hydrogen) (hereinafter referred to as "ring B is b40"),
(B1, cyano, chloro) (hereinafter referred to as "ring B is b41"),
(B1, cyano, fluoro) (hereinafter referred to as "ring B is b42"),
(B1, cyano, methoxy) (hereinafter referred to as "ring B is b43"),
(B1, cyano, butynyloxy) (hereinafter referred to as "ring B is b44"),
(B1, cyano, cyano) (hereinafter referred to as "ring B is b45").
(B1, cyano, amino) (hereinafter referred to as "ring B is b46"),
(B1, cyano, carbamoyl) (hereinafter referred to as "ring B is b47"),
(B1, amino, hydrogen) (hereinafter referred to as "ring B is b48"),
(B1, amino, chloro) (hereinafter referred to as "ring B is b49"),
(B1, amino, fluoro) (hereinafter referred to as "ring B is b50"),
(B1, amino, methoxy) (hereinafter referred to as "ring B is b51"),
(B1, amino, butynyloxy) (hereinafter referred to as "ring B is b52"),
(B1, amino, cyano) hereinafter referred to as "ring B is b53"),
(B1, carbamoyl, hydrogen) (hereinafter referred to as "ring B is b54"),
(B1, carbamoyl, chloro) (hereinafter referred to as "ring B is b55"),
(B1, carbamoyl, fluoro) (hereinafter referred to as "ring B is b56"),
(B1, carbamoyl, methoxy) (hereinafter referred to as "ring B is b57"),
(B1, carbamoyl, butynyloxy) (hereinafter referred to as "ring B is b58"),
(B1, carbamoyl, cyano) (hereinafter referred to as "ring B is b59"),

(B3, hydrogen, hydrogen) (hereinafter referred to as "ring B is b60"),
(B3, hydrogen, chloro) (hereinafter referred to as "ring B is b61"),
(B3, hydrogen, fluoro)(hereinafter referred to as "ring B is b62"),
(B3, hydrogen, methoxy) (hereinafter referred to as "ring B is b63"),
(B3, hydrogen, butynyloxy) (hereinafter referred to as "ring B is b64"),
(B3, hydrogen, cyano) (hereinafter referred to as "ring B is b65"),
(B3, hydrogen, amino) (hereinafter referred to as "ring B is b66"),
(B3, hydrogen, carbamoyl) (hereinafter referred to as "ring B is b67"),
(B3, chloro, hydrogen) (hereinafter referred to as "ring B is b68"),
(B3, chloro, chloro) (hereinafter referred to as "ring B is b69"),
(B3, chloro, fluoro) (hereinafter referred to as "ring B is b70"),
(B3, chloro, methoxy) (hereinafter referred to as "ring B is b71"),
(B3, chloro, butynyloxy) (hereinafter referred to as "ring B is b72"),
(B3, chloro, cyano) (hereinafter referred to as "ring B is b73"),
(B3, chloro, amino) (hereinafter referred to as "ring B is b74"),
(B3, chloro, carbamoyl) (hereinafter referred to as "ring B is b75"),
(B3, fluoro, hydrogen) (hereinafter referred to as "ring B is b76"),
(B3, fluoro, chloro) (hereinafter referred to as "ring B is b77"),
(B3, fluoro, fluoro) (hereinafter referred to as "ring B is b78"),
(B3, fluoro, methoxy) (hereinafter referred to as "ring B is b79"),
(B3, fluoro, butynyloxy) (hereinafter referred to as "ring B is b80"),
(B3, fluoro, cyano) (hereinafter referred to as "ring B is b81"),
(B3, fluoro, amino) (hereinafter referred to as "ring B is b82"),
(B3, fluoro, carbamoyl)(hereinafter referred to as "ring B is b83"),
(B3, methoxy, hydrogen) (hereinafter referred to as "ring B is b84"),
(B3, methoxy, chloro) (hereinafter referred to as "ring B is b85"),
(B3, methoxy, fluoro) (hereinafter referred to as "ring B is b86"),
(B3, methoxy, methoxy) (hereinafter referred to as "ring B is b87"),
(B3, methoxy, butynyloxy) (hereinafter referred to as "ring B is b88"),
(B3, methoxy, cyano) (hereinafter referred to as "ring B is b89"),
(B3, methoxy, amino) (hereinafter referred to as "ring B is b90"),
(B3, methoxy, carbamoyl) (hereinafter referred to as "ring B is b91"),
(B3, butynyloxy, hydrogen) (hereinafter referred to as "ring B is b92"),
(B3, butynyloxy, chloro) (hereinafter referred to as "ring B is b93"),
(B3, butynyloxy, fluoro) (hereinafter referred to as "ring B is b94"),
(B3, butynyloxy, methoxy) (hereinafter referred to as "ring B is b95"),
(B3, butynyloxy, cyano) (hereinafter referred to as "ring B is b96"),
(B3, butynyloxy, amino) (hereinafter referred to as "ring B is b97"),
(B3, butynyloxy, carbamoyl) (hereinafter referred to as "ring B is b98"),
(B3, cyano, hydrogen) (hereinafter referred to as "ring B is b99"),
(B3, cyano, chloro) (hereinafter referred to as "ring B is b 100"),
(B3, cyano, fluoro) (hereinafter referred to as "ring B is b 101"),
(B3, cyano, methoxy) (hereinafter referred to as "ring B is b102"),
(B3, cyano, butynyloxy) (hereinafter referred to as "ring B is b103"),
(B3, cyano, cyano) (hereinafter referred to as "ring B is b104"),
(B3, cyano, amino) (hereinafter referred to as "ring B is b 105"),
(B3, cyano, carbamoyl) (hereinafter referred to as "ring B is b106"),
(B3, amino, hydrogen) (hereinafter referred to as "ring B is b107"),
(B3, amino, chloro) (hereinafter referred to as "ring B is b108"),
(B3, amino, fluoro) (hereinafter referred to as "ring B is b109"),
(B3, amino, methoxy) (hereinafter referred to as "ring B is b110"),
(B3, amino, butynyloxy) (hereinafter referred to as "ring B is b111"),
(B3, amino, cyano) (hereinafter referred to as "ring B is b112"),
(B3, carbamoyl, hydrogen) (hereinafter referred to as "ring B is b113"),
(B3, carbamoyl, chloro) (hereinafter referred to as "ring B is b114"),
(B3, carbamoyl, fluoro) (hereinafter referred to as "ring B is b115"),
(B3, carbamoyl, methoxy) (hereinafter referred to as "ring B is b116"),
(B3, carbamoyl, butynyloxy) (hereinafter referred to as "ring B is b117"),
(B3, carbamoyl, cyano) (hereinafter referred to as "ring B is b118"),

A compound the combination of which ring B and R^{b1} (B, R^{b1}) is as follows
(B2, hydrogen) (hereinafter referred to as "ring B is b119"),
(B2, chloro) (hereinafter referred to as "ring B is b120"),
(B2, fluoro) (hereinafter referred to as "ring B is b121"),
(B2, methoxy) (hereinafter referred to as "ring B is b 122"),
(B2, butynyloxy) (hereinafter referred to as "ring B is b123"),
(B2, cyano) (hereinafter referred to as "ring B is b124"),
(B2, amino) (hereinafter referred to as "ring B is b125"),
(B2, carbamoyl) (hereinafter referred to as "ring B is b126"),
(B4, hydrogen) (hereinafter referred to as "ring B is b127"),
(B4, chloro) (hereinafter referred to as "ring B is b128"),
(B4, fluoro) (hereinafter referred to as "ring B is b129"),
(B4, methoxy) (hereinafter referred to as "ring B is b130"),
(B4, butynyloxy) (hereinafter referred to as "ring B is b131"),
(B4, cyano) (hereinafter referred to as "ring B is b132"),
(B4, amino) (hereinafter referred to as "ring B is b133"),
(B4, carbamoyl) (hereinafter referred to as "ring B is b134").

A compound of formula (I) wherein R^{2a} and R^{2b} are hydrogen, respectively, R¹ is methyl, the combination of

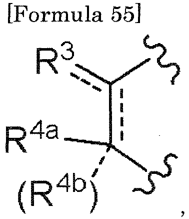

ring A and ring B (r, A, b) is as follows:
(r1,A1,b1),(r1,A1,b2),(r1,A1,b3),(r1,A1,b4),(r1,A1,b5),(r1,A1,b6),(r1,A1,b7),(r1,A1,b8),(r1 ,A1,b9),(r1,A1,b10),(r1,A1,b11),(r1,A1,b12),(r1,A1,b13),(r1,A1,b14),(r1,A1,b15),(r1,A1,b1 6),(r1,A1,b17),(r1,A1,b18),(r1,A1,b19),(r1,A1,b20),(r1,A1,b21),(r1,A1,b22),(r1,A1,b23),(r 1,A1,b24),(r1,A1,b25),(r1,A1,b26),(r1,A1,b27),(r1,A1,b28),(r1,A1,b29),(r1,A1,b30),(r1,A1 ,b31),(r1,A1,b32),(r1,A1,b33),(r1,A1,b34),(r1,A1,b35),(r1,A1,b36),(r1,A1,b37),(r1,A1,b38) ,(r1,A1,b39),(r1,A1,b40),(r1,A1,b41),(r1,A1,b42),(r1,A1,b43),(r1,A1,b44),(r1,A1,b45),(r1, A1,b46),(r1,A1,b47),(r1,A1,b48),(r1,A1,b49),(r1,A1,b50),(r1,A1,b51),(r1,A1,b52),(r1,A1,b 53),(r1,A1,b54),(r1,A1,b55),(r1,A1,b56),(r1,A1,b57),(r1,A1,b58),(r1,A1,b59),(r1,A1,b60),( r1,A1,b61),(r1,A1,b62),(r1,A1,b63),(r1,A1,b64),(r1,A1,b65),(r1,A1,b66),(r1,A1,b67),(r1,A 1,b68),(r1,A1,b69),(r1,A1,b70),(r1,A1,b71),(r1,A1,b72),(r1,A1,b73),(r1,A1,b74),(r1,A1,b7 5),(r1,A1,b76),(r1,A1,b77),(r1,A1,b78),(r1,A1,b79),(r1,A1,b80),(r1,A1,b81),(r1,A1,b82),(r 1,A1,b83),(r1,A1,b84),(r1,A1,b85),(r1,A1,b86),(r1,A1,b87),(r1,A1,b88),(r1,A1,b89),(r1,A1 ,b90),(r1,A1,b91),(r1,A1,b92),(r1,A1,b93),(r1,A1,b94),(r1,A1,b95),(r1,A1,b96),(r1,A1,b97) ,(r1,A1,b98),(r1,A1,b99),(r1,A1,b100),(r1,A1,b101),(r1,A1,b102),(r1,A1,b103),(r1,A1,b10 4),(r1,A1,b105),(r1,A1,b106),(r1,A1,b107),(r1,A1,b108),(r1,A1,b109),(r1,A1,b110),(r1,A1, b111),(r1,A1,b112),(r1,A1,b113),(r1,A1,b114),(r1,A1,b115),(r1,A1,b116),(r1,A1,b117),(r1, A1,b118),(r1,A1,b119),(r1,A1,b120),(r1,A1,b121),(r1,A1,b122),(r1,A1,b123),(r1,A1,b124); (r1,A1,b125),(r1,A1,b126),(r1,A1,b127),(r1,A1,b128),(r1,A1,b129),(r1,A1,b130),(r1,A1,b1 31),(r1,A1,b132),(r1,A1,b133),(r1,A1,b134),(r1,A2,b1),(r1,A2,b2),(r1,A2,b3),(r1,A2,b4),(r 1,A2,b5),(r1,A2,b6),(r1,A2,b7),(r1,A2,b8),(r1,A2,b9),(r1,A2,b10),(r1,A2,b11),(r1,A2,b12),( r1,A2,b13),(r1,A2,b14),(r1,A2,b15),(r1,A2,b16),(r1,A2,b17),(r1,A2,b18),(r1,A2,b19),(r1,A 2,b20),(r1,A2,b21),(r1,A2,b22),(r1,A2,b23),(r1,A2,b24),(r1,A2,b25),(r1,A2,b26),(r1,A2,b2 7),(r1,A2,b28),(r1,A2,b29),(r1,A2,b30),(r1,A2,b31),(r1,A2,b32),(r1,A2,b33),(r1,A2,b34),(r 1,A2,b35),(r1,A2,b36),(r1,A2,b37),(r1,A2,b38),(r1,A2,b39),(r1,A2,b40),(r1,A2,b41),(r1,A2 ,b42),(r1,A2,b43),(r1,A2,b44),(r1,A2,b45),(r1,A2,b46),(r1,A2,b47),(r1,A2,b48),(r1,A2,b49) ,(r1,A2,b50),(r1,A2,b51),(r1,A2,b52),(r1,A2,b53),(r1,A2,b54),(r1,A2,b55),(r1,A2,b56),(r1, A2,b57),(r1,A2,b58),(r1,A2,b59),(r1,A2,b60),(r1,A2,b61),(r1,A2,b62),(r1,A2,b63),(r1,A2,b 64),(r1,A2,b65),(r1,A2,b66),(r1,A2,b67),(r1,A2,b68),(r1,A2,b69),(r1,A2,b70),(r1,A2,b71),( r1,A2,b72),(r1,A2,b73),(r1,A2,b74),(r1,A2,b75),(r1,A2,b76),(r1,A2,b77),(r1,A2,b78),(r1,A 2,b79),(r1,A2,b80),(r1,A2,b81),(r1,A2,b82),(r1,A2,b83),(r1,A2,b84),(r1,A2,b85),(r1,A2,b8 6),(r1,A2,b87),(r1,A2,b88),(r1,A2,b89),(r1,A2,b90),(r1,A2,b91),(r1,A2,b92),(r1,A2,b93),(r 1,A2,b94),(r1,A2,b95),(r1,A2,b96),(r1,A2,b97),(r1,A2,b98),(r1,A2,b99),(r1,A2,b100),(r1,A 2,b101),(r1,A2,b102),(r1,A2,b103),(r1,A2,b104),(r1,A2,b105),(r1,A2,b106),(r1,A2,b107),(r 1,A2,b108),(r1,A2,b109),(r1,A2,b110),(r1,A2,b111),(r1,A2,b112),(r1,A2,b113),(r1,A2,b114 ),(r1,A2,b115),(r1,A2,b116),(r1,A2,b117),(r1,A2,b118),(r1,A2,b119),(r1,A2,b120),(r1,A2,b 121),(r1,A2,b122),(r1,A2,b123),(r1,A2,b124),(r1,A2,b125),(r1,A2,b126),(r1,A2,b127),(r1, A2,b128),(r1,A2,b129),(r1,A2,b130),(r1,A2,b131),(r1,A2,b132),(r1,A2,b133),(r1,A2,b134), (r1,A3,b1),(r1,A3,b2),(r1,A3,b3),(r1,A3,b4),(r1,A3,b5),(r1,A3,b6),(r1,A3,b7),(r1,A3,b8),(r1 ,A3,b9),(r1,A3,b10),(r1,A3,b11),(r1,A3,b12),(r1,A3,b13),(r1,A3,b14),(r1,A3,b15),(r1,A3,b1 6),(r1,A3,b17),(r1,A3,b18),(r1,A3,b19),(r1,A3,b20),(r1,A3,b21),(r1,A3,b22),(r1,A3,b23),(r 1,A3,b24),(r1,A3,b25),(r1,A3,b26),(r1,A3,b27),(r1,A3,b28),(r1,A3,b29),(r1,A3,b30),(r1,A3 ,b31),(r1,A3,b32),(r1,A3,b33),(r1,A3,b34),(r1,A3,b35),(r1,A3,b36),(r1,A3,b37),(r1,A3,b38) ,(r1,A3,b39),(r1,A3,b40),(r1,A3,b41),(r1,A3,b42),(r1,A3,b43),(r1,A3,b44),(r1,A3,b45),(r1, A3,b46),(r1,A3,b47),(r1,A3,b48),(r1,A3,b49),(r1,A3,b50),(r1,A3,b51),(r1,A3,b52),(r1,A3,b 53),(r1,A3,b54),(r1,A3,b55),(r1,A3,b56),(r1,A3,b57),(r1,A3,b58),(r1,A3,b59),(r1,A3,b60),( r1,A3,b61),(r1,A3,b62),(r1,A3,b63),(r1,A3,b64),(r1,A3,b65),(r1,A3,b66),(r1,A3,b67),(r1,A 3,b68),(r1,A3,b69),(r1,A3,b70),(r1,A3,b71),(r1,A3,b72),(r1,A3,b73),(r1,A3,b74),(r1,A3,b7 5),(r1,A3,b76),(r1,A3,b77),(r1,A3,b78),(r1,A3,b79),(r1,A3,b80),(r1,A3,b81),(r1,A3,b82),(r 1,A3,b83),(r1,A3,b84),(r1,A3,b85),(r1,A3,b86),(r1,A3,b87),(r1,A3,b88),(r1,A3,b89),(r1,A3 ,b90),(r1,A3,b91),(r1,A3,b92),(r1,A3,b93),(r1,A3,b94),(r1,A3,b95),(r1,A3,b96),(r1,A3,b97) ,(r1,A3,b98),(r1,A3,b99),(r1,A3,b100),(r1,A3,b101),(r1,A3,b102),(r1,A3,b103),(r1,A3,b10 4),(r1,A3,b105),(r1,A3,b106),(r1,A3,b107),(r1,A3,b108),(r1,A3,b109),(r1,A3,b110),(r1,A3, b111),(r1,A3,b112),(r1,A3,b113),(r1,A3,b114),(r1,A3,b115),(r1,A3,b116),(r1,A3,b117),(r1, A3,b118),(r1,A3,b119),(r1,A3,b120),(r1,A3,b121),(r1,A3,b122),(r1,A3,b123),(r1,A3,b124), (r1,A3,b125),(r1,A3,b126),(r1,A3,b127),(r1,A3,b128),(r1,A3,b129),(r1,A3,b130),(r1,A3,b1 31),(r1,A3,b132),(r1,A3,b133),(r1,A3,b134),
(r1,A4,b1),(r1,A4,b2),(r1,A4,b3),(r1,A4,b4),(r1,A4,b5),(r1,A4,b6),(r1,A4,b7),(r1,A4,b8),(r1 ,A4,b9),(r1,A4,b10),(r1,A4,b11),(r1,A4,b12),(r1,A4,b13),(r1,A4,b14),(r1,A4,b15),(r1,A4,b1 6),(r1,A4,b17),(r1,A4,b18),(r1,A4,b19),(r1,A4,b20),(r1,A4,b21),(r1,A4,b22),(r1,A4,b23),(r 1,A4,b24),(r1,A4,b25),(r1,A4,b26),(r1,A4,b27),(r1,A4,b28),(r1,A4,b29),(r1,A4,b30),(r1,A4 ,b31),(r1,A4,b32),(r1,A4,b33),(r1,A4,b34),(r1,A4,b35),(r1,A4,b36),(r1,A4,b37),(r1,A4,b38) ,(r1,A4,b39),(r1,A4,b40),(r1,A4,b41),(r1,A4,b42),(r1,A4,b43),(r1,A4,b44),(r1,A4,b45),(r1, A4,b46),(r1,A4,b47),(r1,A4,b48),(r1,A4,b49),(r1,A4,b50),(r1,A4,b51),(r1,A4,b52),(r1,A4,b 53),(r1,A4,b54),(r1,A4,b55),(r1,A4,b56),(r1,A4,b57),(r1,A4,b58),(r1,A4,b59),(r1,A4,b60),( r1,A4,b61),(r1,A4,b62),(r1,A4,b63),(r1,A4,b64),(r1,A4,b65),(r1,A4,b66),(r1,A4,b67),(r1,A 4,b68),(r1,A4,b69),(r1,A4,b70),(r1,A4,b71),(r1,A4,b72),(r1,A4,b73),(r1,A4,b74),(r1,A4,b7 5),(r1,A4,b76),(r1,A4,b77),(r1,A4,b78),(r1,A4,b79),(r1,A4,b80),(r1,A4,b81),(r1,A4,b82),(r 1,A4,b83),(r1,A4,b84),(r1,A4,b85),(r1,A4,b86),(r1,A4,b87),(r1,A4,b88),(r1,A4,b89),(r1,A4 ,b90),(r1,A4,b91),(r1,A4,b92),(r1,A4,b93),(r1,A4,b94),(r1,A4,b95),(r1,A4,b96),(r1,A4,b97) ,(r1,A4,b98),(r1,A4,b99),(r1,A4,b100),(r1,A4,b101),(r1,A4,b102),(r1,A4,b103),(r1,A4,b10 4),(r1,A4,b105),(r1,A4,b106),(r1,A4,b107),(r1,A4,b108),(r1,A4,b109),(r1,A4,b110),(r1,A4, b111),(r1,A4,b112),(r1,A4,b113),(r1,A4,b114),(r1,A4,b115),(r1,A4,b116),(r1,A4,b117),(r1, A4,b118),(r1,A4,b119),(r1,A4,b120),(r1,A4,b121),(r1,A4,b122),(r1,A4,b123),(r1,A4,b124), (r1,A4,b125),(r1,A4,b126),(r1,A4,b127),(r1,A4,b128),(r1,A4,b129),(r1,A4,b130),(r1,A4,b1 31),(r1,A4,b132),(r1,A4,b133),(r1,A4,b134),(r2,A1,b1),(r2,A1,b2),(r2,A1,b3),(r2,A1,b4),(r 2,A1,b5),(r2,A1,b6),(r2,A1,b7),(r2,A1,b8),(r2,A1,b9),(r2,A1,b10),(r2,A1,b11),(r2,A1,b12),( r2,A1,b13),(r2,A1,b14),(r2,A1,b15),(r2,A1,b16),(r2,A1,b17),(r2,A1,b18),(r2,A1,b19),(r2,A 1,b20),(r2,A1,b21),(r2,A1,b22),(r2,A1,b23),(r2,A1,b24),(r2,A1,b25),(r2,A1,b26),(r2,A1,b2 7),(r2,A1,b28),(r2,A1,b29),(r2,A1,b30),(r2,A1,b31),(r2,A1,b32),(r2,A1,b33),(r2,A1,b34),(r 2,A1,b35),(r2,A1,b36),(r2,A1,b37),(r2,A1,b38),(r2,A1,b39),(r2,A1,b40),(r2,A1,b41),(r2,A1 ,b42),(r2,A1,b43),(r2,A1,b44),(r2,A1,b45),(r2,A1,b46),(r2,A1,b47),(r2,A1,b48),(r2,A1,b49) ,(r2,A1,b50),(r2,A1,b51),(r2,A1,b52),(r2,A1,b53),(r2,A1,b54),(r2,A1,b55),(r2,A1,b56),(r2, A1,b57),(r2,A1,b58),(r2,A1,b59),(r2,A1,b60),(r2,A1,b61),(r2,A1,b62),(r2,A1,b63),(r2,A1,b 64),(r2,A1,b65),(r2,A1,b66),(r2,A1,b67),(r2,A1,b68),(r2,A1,b69),(r2,A1,b70),(r2,A1,b71), ( r2,A1,b72),(r2,A1,b73),(r2,A1,b74),(r2,A1,b75),(r2,A1,b76),(r2,A1,b77),(r2,A1,b78),(r2,A 1,b79),(r2,A1,b80),(r2,A1,b81),(r2,A1,b82),(r2,A1,b83),(r2,A1,b84),(r2,A1,b85),(r2,A1,b8 6),(r2,A1,b87),(r2,A1,b88),(r2,A1,b89),(r2,A1,b90),(r2,A1,b91),(r2,A1,b92),(r2,A1,b93),(r 2,A1,b94),(r2,A1,b95),(r2,A1,b96),(r2,A1,b97),(r2,A1,b98),(r2,A1,b99),(r2,A1,b100),(r2,A 1,b101),(r2,A1,b102),(r2,A1,b103),(r2,A1,b104),(r2,A1,b105),(r2,A1,b106),(r2,A1,b107),(r 2,A1,b108),(r2,A1,b109),(r2,A1,b110),(r2,A1,b111),(r2,A1,b112),(r2,A1,b113),(r2,A1,b114 ),(r2,A1,b115),(r2,A1,b116),(r2,A1,b117),(r2,A1,b118),(r2,A1,b119),(r2,A1,b120),(r2,A1,b 121),(r2,A1,b122),(r2,A1,b123),(r2,A1,b124),(r2,A1,b125),(r2,A1,b126),(r2,A1,b127),(r2, A1,b128),(r2,A1,b129),(r2,A1,b130),(r2,A1,b131),(r2,A1,b132),(r2,A1,b133),(r2,A1,b134), (r2,A2,b1),(r2,A2,b2),(r2,A2,b3),(r2,A2,b4),(r2,A2,b5),(r2,A2,b6),(r2,A2,b7),(r2,A2,b8),(r2 ,A2,b9),(r2,A2,b10),(r2,A2,b11),(r2,A2,b12),(r2,A2,b13),(r2,A2,b14),(r2,A2,b15),(r2,A2,b1 6),(r2,A2,b17),(r2,A2,b18),(r2,A2,b19),(r2,A2,b20),(r2,A2,b21),(r2,A2,b22),(r2,A2,b23),(r 2,A2,b24),(r2,A2,b25),(r2,A2,b26),(r2,A2,b27),(r2,A2,b28),(r2,A2,b29),(r2,A2,b30),(r2,A2 ,b31),(r2,A2,b32),(r2,A2,b33),(r2,A2,b34),(r2,A2,b35),(r2,A2,b36),(r2,A2,b37),(r2,A2,b38) ,(r2,A2,b39),(r2,A2,b40),(r2,A2,b41),(r2,A2,b42),(r2,A2,b43),(r2,A2,b44),(r2,A2,b45),(r2, A2,b46),(r2,A2,b47),(r2,A2,b48),(r2,A2,b49),(r2,A2,b50),(r2,A2,b51),(r2,A2,b52),(r2,A2,b 53),(r2,A2,b54),(r2,A2,b55),(r2,A2,b56),(r2,A2,b57),(r2,A2,b58),(r2,A2,b59),(r2,A2,b60),( r2,A2,b61),(r2,A2,b62),(r2,A2,b63),(r2,A2,b64),(r2,A2,b65),(r2,A2,b66),(r2,A2,b67),(r2,A 2,b68),(r2,A2,b69),(r2,A2,b70),(r2,A2,b71),(r2,A2,b72),(r2,A2,b73),(r2,A2,b74),(r2,A2,b7 5),(r2,A2,b76),(r2,A2,b77),(r2,A2,b78),(r2,A2,b79),(r2,A2,b80),(r2,A2,b81),(r2,A2,b82),(r 2,A2,b83),(r2,A2,b84),(r2,A2,b85),(r2,A2,b86),(r2,A2,b87),(r2,A2,b88),(r2,A2,b89),(r2,A2 ,b90),(r2,A2,b91),(r2,A2,b92),(r2,A2,b93),(r2,A2,b94),(r2,A2,b95),(r2,A2,b96),(r2,A2,b97) ,(r2,A2,b98),(r2,A2,b99),(r2,A2,b100),(r2,A2,b101),(r2,A2,b102),(r2,A2,b103),(r2,A2,b10 4),(r2,A2,b105),(r2,A2,b106),(r2,A2,b107),(r2,A2,b108),(r2,A2,b109),(r2,A2,b110),(r2,A2, b111),(r2,A2,b112),(r2,A2,b113),(r2,A2,b114),(r2,A2,b115),(r2,A2,b116),(r2,A2,b117),(r2, A2,b 118),(r2,A2,b 119),(r2,A2,b 120),(r2,A2,b 121),(r2,A2,b 122),(r2,A2,b 123),(r2,A2,b 124), (r2,A2,b125),(r2,A2,b126),(r2,A2,b127),(r2,A2,b128),(r2,A2,b129),(r2,A2,b130),(r2,A2,b1 31),(r2,A2,b132),(r2,A2,b133),(r2,A2,b134),
(r2,A3,b1),(r2,A3,b2),(r2,A3,b3),(r2,A3,b4),(r2,A3,b5),(r2,A3,b6),(r2,A3,b7),(r2,A3,b8),(r2 ,A3,b9),(r2,A3,b10),(r2,A3,b11),(r2,A3,b12),(r2,A3,b13),(r2,A3,b14),(r2,A3,b15),(r2,A3,b1 6),(r2,A3,b17),(r2,A3,b18),(r2,A3,b19),(r2,A3,b20),(r2,A3,b21),(r2,A3,b22),(r2,A3,b23),(r 2,A3,b24),(r2,A3,b25),(r2,A3,b26),(r2,A3,b27),(r2,A3,b28),(r2,A3,b29),(r2,A3,b30),(r2,A3 ,b31),(r2,A3,b32),(r2,A3,b33),(r2,A3,b34),(r2,A3,b35),(r2,A3,b36),(r2,A3,b37),(r2,A3,b38) ,(r2,A3,b39),(r2,A3,b40),(r2,A3,b41),(r2,A3,b42),(r2,A3,b43),(r2,A3,b44),(r2,A3,b45),(r2, A3,b46),(r2,A3,b47),(r2,A3,b48),(r2,A3,b49),(r2,A3,b50),(r2,A3,b51),(r2,A3,b52),(r2,A3,b 53),(r2,A3,b54),(r2,A3,b55),(r2,A3,b56),(r2,A3,b57),(r2,A3,b58),(r2,A3,b59),(r2,A3,b60),( r2,A3,b61),(r2,A3,b62),(r2,A3,b63),(r2,A3,b64),(r2,A3,b65),(r2,A3,b66),(r2,A3,b67),(r2,A 3,b68),(r2,A3,b69),(r2,A3,b70),(r2,A3,b71),(r2,A3,b72),(r2,A3,b73),(r2,A3,b74),(r2,A3,b7 5),(r2,A3,b76),(r2,A3,b77),(r2,A3,b78),(r2,A3,b79),(r2,A3,b80),(r2,A3,b81),(r2,A3,b82),(r 2,A3,b83),(r2,A3,b84),(r2,A3,b85),(r2,A3,b86),(r2,A3,b87),(r2,A3,b88),(r2,A3,b89),(r2,A3 ,b90),(r2,A3,b91),(r2,A3,b92),(r2,A3,b93),(r2,A3,b94),(r2,A3,b95),(r2,A3,b96),(r2,A3,b97) ,(r2,A3,b98),(r2,A3,b99),(r2,A3,b100),(r2,A3,b101),(r2,A3,b102),(r2,A3,b103),(r2,A3,b10 4),(r2,A3,b105),(r2,A3,b106),(r2,A3,b107),(r2,A3,b108),(r2,A3,b109),(r2,A3,b110),(r2,A3, b111),(r2,A3,b112),(r2,A3,b113),(r2,A3,b114),(r2,A3,b115),(r2,A3,b116),(r2,A3,b117),(r2, A3,b118),(r2,A3,b119),(r2,A3,b120),(r2,A3,b121),(r2,A3,b122),(r2,A3,b123),(r2,A3,b124), (r2,A3,b125),(r2,A3,b126),(r2,A3,b127),(r2,A3,b128),(r2,A3,b129),(r2,A3,b130),(r2,A3,b1 31), (r2,A3,b 132), (r2,A3,b 133), (r2,A3,b 134), (r2,A4,b 1),(r2,A4,b2), (r2,A4,b3), (r2,A4,b4), (r 2,A4,b5),(r2,A4,b6),(r2,A4,b7),(r2,A4,b8),(r2,A4,b9),(r2,A4,b10),(r2,A4,b11),(r2,A4,b12),( r2,A4,b13),(r2,A4,b14),(r2,A4,b15),(r2,A4,b16),(r2,A4,b17),(r2,A4,b18),(r2,A4,b19),(r2,A 4,b20),(r2,A4,b21),(r2,A4,b22),(r2,A4,b23),(r2,A4,b24),(r2,A4,b25),(r2,A4,b26),(r2,A4,b2 7),(r2,A4,b28),(r2,A4,b29),(r2,A4,b30),(r2,A4,b31),(r2,A4,b32),(r2,A4,b33),(r2,A4,b34),(r 2,A4,b35),(r2,A4,b36),(r2,A4,b37),(r2,A4,b38),(r2,A4,b39),(r2,A4,b40),(r2,A4,b41),(r2,A4 ,b42),(r2,A4,b43),(r2,A4,b44),(r2,A4,b45),(r2,A4,b46),(r2,A4,b47),(r2,A4,b48),(r2,A4,b49) ,(r2,A4,b50),(r2,A4,b51),(r2,A4,b52),(r2,A4,b53),(r2,A4,b54),(r2,A4,b55),(r2,A4,b56),(r2, A4,b57),(r2,A4,b58),(r2,A4,b59),(r2,A4,b60),(r2,A4,b61),(r2,A4,b62),(r2,A4,b63),(r2,A4,b 64),(r2,A4,b65),(r2,A4,b66),(r2,A4,b67),(r2,A4,b68),(r2,A4,b69),(r2,A4,b70),(r2,A4,b71),( r2,A4,b72),(r2,A4,b73),(r2,A4,b74),(r2,A4,b75),(r2,A4,b76),(r2,A4,b77),(r2,A4,b78),(r2,A 4,b79),(r2,A4,b80),(r2,A4,b81),(r2,A4,b82),(r2,A4,b83),(r2,A4,b84),(r2,A4,b85),(r2,A4,b8 6),(r2,A4,b87),(r2,A4,b88),(r2,A4,b89),(r2,A4,b90),(r2,A4,b91),(r2,A4,b92),(r2,A4,b93),(r 2,A4,b94),(r2,A4,b95),(r2,A4,b96),(r2,A4,b97),(r2,A4,b98),(r2,A4,b99),(r2,A4,b100),(r2,A 4,b101),(r2,A4,b102),(r2,A4,b103),(r2,A4,b104),(r2,A4,b105),(r2,A4,b106),(r2,A4,b107),(r 2,A4,b108),(r2,A4,b109),(r2,A4,b110),(r2,A4,b111),(r2,A4,b112),(r2,A4,b113),(r2,A4,b114 ), (r2,A4,b 115), (r2,A4,b 116), (r2,A4,b 117),(r2,A4,b 118), (r2,A4,b 119), (r2,A4,b 120), (r2,A4,b 121),(r2,A4,b 122),(r2,A4,b 123), (r2,A4,b 124), (r2,A4,b 125), (r2,A4,b 126), (r2,A4,b 127), (r2, A4,b128),(r2,A4,b129),(r2,A4,b130),(r2,A4,b131),(r2,A4,b132),(r2,A4,b133),(r2,A4,b134), (r3,A1,b1),(r3,A1,b2),(r3,A1,b3),(r3,A1,b4),(r3,A1,b5),(r3,A1,b6),(r3,A1,b7),(r3,A1,b8),(r3 ,A1,b9),(r3,A1,b10),(r3,A1,b11),(r3,A1,b12),(r3,A1,b13),(r3,A1,b14),(r3,A1,b15),(r3,A1,b1 6),(r3,A1,b17),(r3,A1,b18),(r3,A1,b19),(r3,A1,b20),(r3,A1,b21),(r3,A1,b22),(r3,A1,b23),(r 3,A1,b24),(r3,A1,b25),(r3,A1,b26),(r3,A1,b27),(r3,A1,b28),(r3,A1,b29),(r3,A1,b30),(r3,A1 ,b31),(r3,A1,b32),(r3,A1,b33),(r3,A1,b34),(r3,A1,b35),(r3,A1,b36),(r3,A1,b37),(r3,A1,b38) ,(r3,A1,b39),(r3,A1,b40),(r3,A1,b41),(r3,A1,b42),(r3,A1,b43),(r3,A1,b44),(r3,A1,b45),(r3, A1,b46),(r3,A1,b47),(r3,A1,b48),(r3,A1,b49),(r3,A1,b50),(r3,A1,b51),(r3,A1,b52),(r3,A1,b 53),(r3,A1,b54),(r3,A1,b55),(r3,A1,b56),(r3,A1,b57),(r3,A1,b58),(r3,A1,b59),(r3,A1,b60),( r3,A1,b61),(r3,A1,b62),(r3,A1,b63),(r3,A1,b64),(r3,A1,b65),(r3,A1,b66),(r3,A1,b67),(r3,A 1,b68),(r3,A1,b69),(r3,A1,b70),(r3,A1,b71),(r3,A1,b72),(r3,A1,b73),(r3,A1,b74),(r3,A1,b7 5),(r3,A1,b76),(r3,A1,b77),(r3,A1,b78),(r3,A1,b79),(r3,A1,b80),(r3,A1,b81),(r3,A1,b82),(r 3,A1,b83),(r3,A1,b84),(r3,A1,b85),(r3,A1,b86),(r3,A1,b87),(r3,A1,b88),(r3,A1,b89),(r3,A1 ,b90),(r3,A1,b91),(r3,A1,b92),(r3,A1,b93),(r3,A1,b94),(r3,A1,b95),(r3,A1,b96),(r3,A1,b97) ,(r3,A1,b98),(r3,A1,b99),(r3,A1,b100),(r3,A1,b101),(r3,A1,b102),(r3,A1,b103),(r3,A1,b10 4),(r3,A1,b105),(r3,A1,b106),(r3,A1,b107),(r3,A1,b108),(r3,A1,b109),(r3,A1,b110),(r3,A1, b111),(r3,A1,b112),(r3,A1,b113),(r3,A1,b114),(r3,A1,b115),(r3,A1,b116),(r3,A1,b117),(r3, A1,b118),(r3,A1,b119),(r3,A1,b120),(r3,A1,b121),(r3,A1,b122),(r3,A1,b123),(r3,A1,b124), (r3,A1,b125),(r3,A1,b126),(r3,A1,b127),(r3,A1,b128),(r3,A1,b129),(r3,A1,b130),(r3,A1,b1 31),(r3,A1,b132),(r3,A1,b133),(r3,A1,b134),
(r3,A2,b1),(r3,A2,b2),(r3,A2,b3),(r3,A2,b4),(r3,A2,b5),(r3,A2,b6),(r3,A2,b7),(r3,A2,b8),(r3 ,A2,b9),(r3,A2,b10),(r3,A2,b11),(r3,A2,b12),(r3,A2,b13),(r3,A2,b14),(r3,A2,b15),(r3,A2,b1 6),(r3,A2,b17),(r3,A2,b18),(r3,A2,b19),(r3,A2,b20),(r3,A2,b21),(r3,A2,b22),(r3,A2,b23),(r 3,A2,b24),(r3,A2,b25),(r3,A2,b26),(r3,A2,b27),(r3,A2,b28),(r3,A2,b29),(r3,A2,b30),(r3,A2 ,b31),(r3,A2,b32),(r3,A2,b33),(r3,A2,b34),(r3,A2,b35),(r3,A2,b36),(r3,A2,b37),(r3,A2,b38) ,(r3,A2,b39),(r3,A2,b40),(r3,A2,b41),(r3,A2,b42),(r3,A2,b43),(r3,A2,b44),(r3,A2,b45),(r3, A2,b46),(r3,A2,b47),(r3,A2,b48),(r3,A2,b49),(r3,A2,b50),(r3,A2,b51),(r3,A2,b52),(r3,A2,b 53),(r3,A2,b54),(r3,A2,b55),(r3,A2,b56),(r3,A2,b57),(r3,A2,b58),(r3,A2,b59),(r3,A2,b60),( r3,A2,b61),(r3,A2,b62),(r3,A2,b63),(r3,A2,b64),(r3,A2,b65),(r3,A2,b66),(r3,A2,b67),(r3,A 2,b68),(r3,A2,b69),(r3,A2,b70),(r3,A2,b71),(r3,A2,b72),(r3,A2,b73),(r3,A2,b74),(r3,A2,b7 5),(r3,A2,b76),(r3,A2,b77),(r3,A2,b78),(r3,A2,b79),(r3,A2,b80),(r3,A2,b81),(r3,A2,b82),(r 3,A2,b83),(r3,A2,b84),(r3,A2,b85),(r3,A2,b86),(r3,A2,b87),(r3,A2,b88),(r3,A2,b89),(r3,A2 ,b90),(r3,A2,b91),(r3,A2,b92),(r3,A2,b93),(r3,A2,b94),(r3,A2,b95),(r3,A2,b96),(r3,A2,b97) ,(r3,A2,b98),(r3,A2,b99),(r3,A2,b100),(r3,A2,b101),(r3,A2,b102),(r3,A2,b103),(r3,A2,b10 4),(r3,A2,b105),(r3,A2,b106),(r3,A2,b107),(r3,A2,b108),(r3,A2,b109),(r3,A2,b110),(r3,A2, b111),(r3,A2,b112),(r3,A2,b113),(r3,A2,b114),(r3,A2,b115),(r3,A2,b116),(r3,A2,b117),(r3, A2,b118),(r3,A2,b119),(r3,A2,b120),(r3,A2,b121),(r3,A2,b122),(r3,A2,b123),(r3,A2,b124), (r3,A2,b125),(r3,A2,b126),(r3,A2,b127),(r3,A2,b128),(r3,A2,b129),(r3,A2,b130),(r3,A2,b1 31),(r3,A2,b132),(r3,A2,b133),(r3,A2,b134),(r3,A3,b1),(r3,A3,b2),(r3,A3,b3),(r3,A3,b4),(r 3,A3,b5),(r3,A3,b6),(r3,A3,b7),(r3,A3,b8),(r3,A3,b9),(r3,A3,b10),(r3,A3,b11),(r3,A3,b12),( r3,A3,b13),(r3,A3,b14),(r3,A3,b15),(r3,A3,b16),(r3,A3,b17),(r3,A3,b18),(r3,A3,b19),(r3,A 3,b20),(r3,A3,b21),(r3,A3,b22),(r3,A3,b23),(r3,A3,b24),(r3,A3,b25),(r3,A3,b26),(r3,A3,b2 7),(r3,A3,b28),(r3,A3,b29),(r3,A3,b30),(r3,A3,b31),(r3,A3,b32),(r3,A3,b33),(r3,A3,b34),(r 3,A3,b35),(r3,A3,b36),(r3,A3,b37),(r3,A3,b38),(r3,A3,b39),(r3,A3,b40),(r3,A3,b41),(r3,A3 ,b42),(r3,A3,b43),(r3,A3,b44),(r3,A3,b45),(r3,A3,b46),(r3,A3,b47),(r3,A3,b48),(r3,A3,b49) ,(r3,A3,b50),(r3,A3,b51),(r3,A3,b52),(r3,A3,b53),(r3,A3,b54),(r3,A3,b55),(r3,A3,b56),(r3, A3,b57),(r3,A3,b58),(r3,A3,b59),(r3,A3,b60),(r3,A3,b61),(r3,A3,b62),(r3,A3,b63),(r3,A3,b 64),(r3,A3,b65),(r3,A3,b66),(r3,A3,b67),(r3,A3,b68),(r3,A3,b69),(r3,A3,b70),(r3,A3,b71),( r3,A3,b72),(r3,A3,b73),(r3,A3,b74),(r3,A3,b75),(r3,A3,b76),(r3,A3,b77),(r3,A3,b78),(r3,A 3,b79),(r3,A3,b80),(r3,A3,b81),(r3,A3,b82),(r3,A3,b83),(r3,A3,b84),(r3,A3,b85),(r3,A3,b8 6),(r3,A3,b87),(r3,A3,b88),(r3,A3,b89),(r3,A3,b90),(r3,A3,b91),(r3,A3,b92),(r3,A3,b93),(r 3,A3,b94),(r3,A3,b95),(r3,A3,b96),(r3,A3,b97),(r3,A3,b98),(r3,A3,b99),(r3,A3,b100),(r3,A 3,b101),(r3,A3,b102),(r3,A3,b103),(r3,A3,b104),(r3,A3,b105),(r3,A3,b106),(r3,A3,b107),(r 3,A3,b108),(r3,A3,b109),(r3,A3,b110),(r3,A3,b111),(r3,A3,b112),(r3,A3,b113),(r3,A3,b114 ),(r3,A3,b115),(r3,A3,b116),(r3,A3,b117),(r3,A3,b118),(r3,A3,b119),(r3,A3,b120),(r3,A3,b 121),(r3,A3,b122),(r3,A3,b123),(r3,A3,b124),(r3,A3,b125),(r3,A3,b126),(r3,A3,b127),(r3, A3,b128),(r3,A3,b129),(r3,A3,b130),(r3,A3,b131),(r3,A3,b132),(r3,A3,b133),(r3,A3,b134), (r3,A4,b1),(r3,A4,b2),(r3,A4,b3),(r3,A4,b4),(r3,A4,b5),(r3,A4,b6),(r3,A4,b7),(r3,A4,b8),(r3 ,A4,b9),(r3,A4,b10),(r3,A4,b11),(r3,A4,b12),(r3,A4,b13),(r3,A4,b14),(r3,A4,b15),(r3,A4,b1 6),(r3,A4,b17),(r3,A4,b18),(r3,A4,b19),(r3,A4,b20),(r3,A4,b21),(r3,A4,b22),(r3,A4,b23),(r 3,A4,b24),(r3,A4,b25),(r3,A4,b26),(r3,A4,b27),(r3,A4,b28),(r3,A4,b29),(r3,A4,b30),(r3,A4 ,b31),(r3,A4,b32),(r3,A4,b33),(r3,A4,b34),(r3,A4,b35),(r3,A4,b36),(r3,A4,b37),(r3,A4,b38) ,(r3,A4,b39),(r3,A4,b40),(r3,A4,b41),(r3,A4,b42),(r3,A4,b43),(r3,A4,b44),(r3,A4,b45),(r3, A4,b46),(r3,A4,b47),(r3,A4,b48),(r3,A4,b49),(r3,A4,b50),(r3,A4,b51),(r3,A4,b52),(r3,A4,b 53),(r3,A4,b54),(r3,A4,b55),(r3,A4,b56),(r3,A4,b57),(r3,A4,b58),(r3,A4,b59),(r3,A4,b60),( r3,A4,b61),(r3,A4,b62),(r3,A4,b63),(r3,A4,b64),(r3,A4,b65),(r3,A4,b66),(r3,A4,b67),(r3,A 4,b68),(r3,A4,b69),(r3,A4,b70),(r3,A4,b71),(r3,A4,b72),(r3,A4,b73),(r3,A4,b74),(r3,A4,b7 5),(r3,A4,b76),(r3,A4,b77),(r3,A4,b78),(r3,A4,b79),(r3,A4,b80),(r3,A4,b81),(r3,A4,b82),(r 3,A4,b83),(r3,A4,b84),(r3,A4,b85),(r3,A4,b86),(r3,A4,b87),(r3,A4,b88),(r3,A4,b89),(r3,A4 ,b90),(r3,A4,b91),(r3,A4,b92),(r3,A4,b93),(r3,A4,b94),(r3,A4,b95),(r3,A4,b96),(r3,A4,b97) ,(r3,A4,b98),(r3,A4,b99),(r3,A4,b100),(r3,A4,b101),(r3,A4,b102),(r3,A4,b103),(r3,A4,b10 4),(r3,A4,b105),(r3,A4,b106),(r3,A4,b107),(r3,A4,b108),(r3,A4,b109),(r3,A4,b110),(r3,A4, b111),(r3,A4,b112),(r3,A4,b113),(r3,A4,b114),(r3,A4,b115),(r3,A4,b116),(r3,A4,b117),(r3, A4,b118),(r3,A4,b119),(r3,A4,b120),(r3,A4,b121),(r3,A4,b122),(r3,A4,b123),(r3,A4,b124), (r3,A4,b125),(r3,A4,b126),(r3,A4,b127),(r3,A4,b128),(r3,A4,b129),(r3,A4,b130),(r3,A4,b1 31),(r3,A4,b132),(r3,A4,b133),(r3,A4,b134),
(r4,A1,b1),(r4,A1,b2),(r4,Al,b3),(r4,A1,b4),(r4,A1,b5),(r4,A1,b6),(r4,A1,b7),(r4,A1,b8),(r4 ,A1,b9),(r4,A1,b10),(r4,A1,b11),(r4,A1,b12),(r4,A1,b13),(r4,A1,b14),(r4,A1,b15),(r4,A1,b1 6),(r4,A1,b17),(r4,A1,b18),(r4,A1,b19),(r4,A1,b20),(r4,A1,b21),(r4,A1,b22),(r4,A1,b23),(r 4,A1,b24),(r4,A1,b25),(r4,A1,b26),(r4,A1,b27),(r4,A1,b28),(r4,A1,b29),(r4,A1,b30),(r4,A1 ,b31),(r4,A1,b32),(r4,A1,b33),(r4,A1,b34),(r4,A1,b35),(r4,A1,b36),(r4,A1,b37),(r4,A1,b38) ,(r4,A1,b39),(r4,A1,b40),(r4,A1,b41),(r4,A1,b42),(r4,A1,b43),(r4,A1,b44),(r4,A1,b45),(r4, A1,b46),(r4,A1,b47),(r4,A1,b48),(r4,A1,b49),(r4,A1,b50),(r4,A1,b51),(r4,A1,b52),(r4,A1,b 53),(r4,A1,b54),(r4,A1,b55),(r4,A1,b56),(r4,A1,b57),(r4,A1,b58),(r4,A1,b59),(r4,A1,b60),( r4,A1,b61),(r4,A1,b62),(r4,A1,b63),(r4,A1,b64),(r4,A1,b65),(r4,A1,b66),(r4,A1,b67),(r4,A 1,b68),(r4,A1,b69),(r4,A1,b70),(r4,A1,b71),(r4,A1,b72),(r4,A1,b73),(r4,A1,b74),(r4,A1,b7 5),(r4,A1,b76),(r4,A1,b77),(r4,A1,b78),(r4,A1,b79),(r4,A1,b80),(r4,A1,b81),(r4,A1,b82),(r 4,A1,b83),(r4,A1,b84),(r4,A1,b85),(r4,A1,b86),(r4,A1,b87),(r4,A1,b88),(r4,A1,b89),(r4,A1 ,b90),(r4,A1,b91),(r4,A1,b92),(r4,A1,b93),(r4,A1,b94),(r4,A1,b95),(r4,A1,b96),(r4,A1,b97) ,(r4,A1,b98),(r4,A1,b99),(r4,A1,b100),(r4,A1,b101),(r4,A1,b102),(r4,A1,b103),(r4,A1,b10 4),(r4,A1,b105),(r4,A1,b106),(r4,A1,b107),(r4,A1,b108),(r4,A1,b109),(r4,A1,b110),(r4,A1, b111),(r4,A1,b112),(r4,A1,b113),(r4,A1,b114),(r4,A1,b115),(r4,A1,b116),(r4,A1,b117),(r4, A1,b118),(r4,A1,b119),(r4,A1,b120),(r4,A1,b121),(r4,A1,b122),(r4,A1,b123),(r4,A1,b124), (r4,A1,b125),(r4,A1,b126),(r4,A1,b127),(r4,A1,b128),(r4,A1,b129),(r4,A1,b130),(r4,A1,b1 31),(r4,A1,b132),(r4,A1,b133),(r4,A1,b134),(r4,A2,b1),(r4,A2,b2),(r4,A2,b3),(r4,A2,b4),(r 4,A2,b5),(r4,A2,b6),(r4,A2,b7),(r4,A2,b8),(r4,A2,b9),(r4,A2,b10),(r4,A2,b11),(r4,A2,b12),( r4,A2,b 13),(r4,A2,b 14),(r4,A2,b 15),(r4,A2,b 16),(r4,A2,b 17),(r4,A2,b 18),(r4,A2,b 19),(r4,A 2,b20),(r4,A2,b21),(r4,A2,b22),(r4,A2,b23),(r4,A2,b24),(r4,A2,b25),(r4,A2,b26),(r4,A2,b2 7),(r4,A2,b28),(r4,A2,b29),(r4,A2,b30),(r4,A2,b31),(r4,A2,b32),(r4,A2,b33),(r4,A2,b34),(r 4,A2,b35),(r4,A2,b36),(r4,A2,b37),(r4,A2,b38),(r4,A2,b39),(r4,A2,b40),(r4,A2,b41),(r4,A2 ,b42),(r4,A2,b43),(r4,A2,b44),(r4,A2,b45),(r4,A2,b46),(r4,A2,b47),(r4,A2,b48),(r4,A2,b49) ,(r4,A2,b50),(r4,A2,b51),(r4,A2,b52),(r4,A2,b53),(r4,A2,b54),(r4,A2,b55),(r4,A2,b56),(r4, A2,b57),(r4,A2,b58),(r4,A2,b59),(r4,A2,b60),(r4,A2,b61),(r4,A2,b62),(r4,A2,b63),(r4,A2,b 64),(r4,A2,b65),(r4,A2,b66),(r4,A2,b67),(r4,A2,b68),(r4,A2,b69),(r4,A2,b70),(r4,A2,b71),( r4,A2,b72),(r4,A2,b73),(r4,A2,b74),(r4,A2,b75),(r4,A2,b76),(r4,A2,b77),(r4,A2,b78),(r4,A 2,b79),(r4,A2,b80),(r4,A2,b81),(r4,A2,b82),(r4,A2,b83),(r4,A2,b84),(r4,A2,b85),(r4,A2,b8 6),(r4,A2,b87),(r4,A2,b88),(r4,A2,b89),(r4,A2,b90),(r4,A2,b91),(r4,A2,b92),(r4,A2,b93),(r 4,A2,b94),(r4,A2,b95),(r4,A2,b96),(r4,A2,b97),(r4,A2,b98),(r4,A2,b99),(r4,A2,b100),(r4,A 2,b101),(r4,A2,b102),(r4,A2,b103),(r4,A2,b104),(r4,A2,b105),(r4,A2,b106),(r4,A2,b107),(r 4,A2,b108),(r4,A2,b109),(r4,A2,b110),(r4,A2,b111),(r4,A2,b112),(r4,A2,b113),(r4,A2,b114 ),(r4,A2,b115),(r4,A2,b116),(r4,A2,b117),(r4,A2,b118),(r4,A2,b119),(r4,A2,b120),(r4,A2,b 121),(r4,A2,b 122), (r4,A2,b 123), (r4,A2,b 124), (r4,A2,b 125), (r4,A2,b 126),(r4,A2,b 127),(r4, A2,b128),(r4,A2,b129),(r4,A2,b130),(r4,A2,b131),(r4,A2,b132),(r4,A2,b133),(r4,A2,b134), (r4,A3,b1),(r4,A3,b2),(r4,A3,b3),(r4,A3,b4),(r4,A3,b5),(r4,A3,b6),(r4,A3,b7),(r4,A3,b8),(r4 ,A3,b9),(r4,A3,b10),(r4,A3,b11),(r4,A3,b12),(r4,A3,b13),(r4,A3,b14),(r4,A3,b15),(r4,A3,b1 6),(r4,A3,b17),(r4,A3,b18),(r4,A3,b19),(r4,A3,b20),(r4,A3,b21),(r4,A3,b22),(r4,A3,b23),(r 4,A3,b24),(r4,A3,b25),(r4,A3,b26),(r4,A3,b27),(r4,A3,b28),(r4,A3,b29),(r4,A3,b30),(r4,A3 ,b31),(r4,A3,b32),(r4,A3,b33),(r4,A3,b34),(r4,A3,b35),(r4,A3,b36),(r4,A3,b37),(r4,A3,b38) ,(r4,A3,b39),(r4,A3,b40),(r4,A3,b41),(r4,A3,b42),(r4,A3,b43),(r4,A3,b44),(r4,A3,b45),(r4, A3,b46),(r4,A3,b47),(r4,A3,b48),(r4,A3,b49),(r4,A3,b50),(r4,A3,b51),(r4,A3,b52),(r4,A3,b 53),(r4,A3,b54),(r4,A3,b55),(r4,A3,b56),(r4,A3,b57),(r4,A3,b58),(r4,A3,b59),(r4,A3,b60),( r4,A3,b61),(r4,A3,b62),(r4,A3,b63),(r4,A3,b64),(r4,A3,b65),(r4,A3,b66),(r4,A3,b67),(r4,A 3,b68),(r4,A3,b69),(r4,A3,b70),(r4,A3,b71),(r4,A3,b72),(r4,A3,b73),(r4,A3,b74),(r4,A3,b7 5),(r4,A3,b76),(r4,A3,b77),(r4,A3,b78),(r4,A3,b79),(r4,A3,b80),(r4,A3,b81),(r4,A3,b82),(r 4,A3,b83),(r4,A3,b84),(r4,A3,b85),(r4,A3,b86),(r4,A3,b87),(r4,A3,b88),(r4,A3,b89),(r4,A3 ,b90), (r4,A3,b91), (r4,A3,b92),(r4,A3,b93), (r4,A3,b94), (r4,A3,b95), (r4,A3,b96), (r4,A3,b97) ,(r4,A3,b98),(r4,A3,b99),(r4,A3,b100),(r4,A3,b101),(r4,A3,b102),(r4,A3,b103),(r4,A3,b10 4),(r4,A3,b105),(r4,A3,b106),(r4,A3,b107),(r4,A3,b108),(r4,A3,b109),(r4,A3,b110),(r4,A3, b111),(r4,A3,b112),(r4,A3,b113),(r4,A3,b114),(r4,A3,b115),(r4,A3,b116),(r4,A3,b117),(r4, A3,b118),(r4,A3,b119),(r4,A3,b120),(r4,A3,b121),(r4,A3,b122),(r4,A3,b123),(r4,A3,b124), (r4,A3,b125),(r4,A3,b126),(r4,A3,b127),(r4,A3,b128),(r4,A3,b129),(r4,A3,b130),(r4,A3,b1 31),(r4,A3,b132),(r4,A3,b133),(r4,A3,b134),
(r4,A4,b1),(r4,A4,b2),(r4,A4,b3),(r4,A4,b4),(r4,A4,b5),(r4,A4,b6),(r4,A4,b7),(r4,A4,b8),(r4 ,A4,b9),(r4,A4,b10),(r4,A4,b11),(r4,A4,b12),(r4,A4,b13),(r4,A4,b14),(r4,A4,b15),(r4,A4,b1 6),(r4,A4,b17),(r4,A4,b18),(r4,A4,b19),(r4,A4,b20),(r4,A4,b21),(r4,A4,b22),(r4,A4,b23),(r 4,A4,b24),(r4,A4,b25),(r4,A4,b26),(r4,A4,b27),(r4,A4,b28),(r4,A4,b29),(r4,A4,b30),(r4,A4 ,b31),(r4,A4,b32),(r4,A4,b33),(r4,A4,b34),(r4,A4,b35),(r4,A4,b36),(r4,A4,b37),(r4,A4,b38) ,(r4,A4,b39),(r4,A4,b40),(r4,A4,b41),(r4,A4,b42),(r4,A4,b43),(r4,A4,b44),(r4,A4,b45),(r4, A4,b46),(r4,A4,b47),(r4,A4,b48),(r4,A4,b49),(r4,A4,b50),(r4,A4,b51),(r4,A4,b52),(r4,A4,b 53),(r4,A4,b54),(r4,A4,b55),(r4,A4,b56),(r4,A4,b57),(r4,A4,b58),(r4,A4,b59),(r4,A4,b60),( r4,A4,b61),(r4,A4,b62),(r4,A4,b63),(r4,A4,b64),(r4,A4,b65),(r4,A4,b66),(r4,A4,b67),(r4,A 4,b68),(r4,A4,b69),(r4,A4,b70),(r4,A4,b71),(r4,A4,b72),(r4,A4,b73),(r4,A4,b74),(r4,A4,b7 5),(r4,A4,b76),(r4,A4,b77),(r4,A4,b78),(r4,A4,b79),(r4,A4,b80),(r4,A4,b81),(r4,A4,b82),(r 4,A4,b83),(r4,A4,b84),(r4,A4,b85),(r4,A4,b86),(r4,A4,b87),(r4,A4,b88),(r4,A4,b89),(r4,A4 ,b90),(r4,A4,b91),(r4,A4,b92),(r4,A4,b93),(r4,A4,b94),(r4,A4,b95),(r4,A4,b96),(r4,A4,b97) ,(r4,A4,b98),(r4,A4,b99),(r4,A4,b100),(r4,A4,b101),(r4,A4,b102),(r4,A4,b103),(r4,A4,b10 4),(r4,A4,b105),(r4,A4,b106),(r4,A4,b107),(r4,A4,b108),(r4,A4,b109),(r4,A4,b110),(r4,A4, b111),(r4,A4,b112),(r4,A4,b113),(r4,A4,b114),(r4,A4,b115),(r4,A4,b116),(r4,A4,b117),(r4, A4,b118),(r4,A4,b119),(r4,A4,b120),(r4,A4,b121),(r4,A4,b122),(r4,A4,b123),(r4,A4,b124), (r4,A4,b125),(r4,A4,b126),(r4,A4,b127),(r4,A4,b128),(r4,A4,b129),(r4,A4,b130),(r4,A4,b1 31),(r4,A4,b132),(r4,A4,b133),(r4,A4,b134),(r5,A1,b1),(r5,A1,b2),(r5,A1,b3),(r5,A1,b4),(r 5,A1,b5),(r5,A1,b6),(r5,A1,b7),(r5,A1,b8),(r5,A1,b9),(r5,A1,b10),(r5,A1,b11),(r5,A1,b12),( r5,A1,b13),(r5,A1,b14),(r5,A1,b15),(r5,A1,b16),(r5,A1,b17),(r5,A1,b18),(r5,A1,b19),(r5,A 1,b20),(r5,A1,b21),(r5,A1,b22),(r5,A1,b23),(r5,A1,b24),(r5,A1,b25),(r5,A1,b26),(r5,A1,b2 7),(r5,A1,b28),(r5,A1,b29),(r5,A1,b30),(r5,A1,b31),(r5,A1,b32),(r5,A1,b33),(r5,A1,b34),(r 5,A1,b35),(r5,A1,b36),(r5,A1,b37),(r5,A1,b38),(r5,A1,b39),(r5,A1,b40),(r5,A1,b41),(r5,A1 ,b42),(r5,A1,b43),(r5,A1,b44),(r5,A1,b45),(r5,A1,b46),(r5,A1,b47),(r5,A1,b48),(r5,A1,b49) ,(r5,A1,b50),(r5,A1,b51),(r5,A1,b52),(r5,A1,b53),(r5,A1,b54),(r5,A1,b55),(r5,A1,b56),(r5, A1,b57),(r5,A1,b58),(r5,A1,b59),(r5,A1,b60),(r5,A1,b61),(r5,A1,b62),(r5,A1,b63),(r5,A1,b 64),(r5,A1,b65),(r5,A1,b66),(r5,A1,b67),(r5,A1,b68),(r5,A1,b69),(r5,A1,b70),(r5,A1,b71),( r5,A1,b72),(r5,A1,b73),(r5,A1,b74),(r5,A1,b75),(r5,A1,b76),(r5,A1,b77),(r5,A1,b78),(r5,A 1,b79),(r5,A1,b80),(r5,A1,b81),(r5,A1,b82),(r5,A1,b83),(r5,A1,b84),(r5,A1,b85),(r5,A1,b8 6),(r5,A1,b87),(r5,A1,b88),(r5,A1,b89),(r5,A1,b90),(r5,A1,b91),(r5,A1,b92),(r5,A1,b93),(r 5,A1,b94),(r5,A1,b95),(r5,A1,b96),(r5,A1,b97),(r5,A1,b98),(r5,A1,b99),(r5,A1,b100),(r5,A 1,b101),(r5,A1,b102),(r5,A1,b103),(r5,A1,b104),(r5,A1,b105),(r5,A1,b106),(r5,A1,b107),(r 5,A1,b108),(r5,A1,b109),(r5,A1,b110),(r5,A1,b111),(r5,A1,b112),(r5,A1,b113),(r5,A1,b114 ),(r5,A1,b115),(r5,A1,b116),(r5,A1,b117),(r5,A1,b118),(r5,A1,b119),(r5,A1,b120),(r5,A1,b 121),(r5,A1,b122),(r5,A1,b123),(r5,A1,b124),(r5,A1,b125),(r5,A1,b126),(r5,A1,b127),(r5, A1,b128),(r5,A1,b129),(r5,A1,b130),(r5,A1,b131),(r5,A1,b132),(r5,A1,b133),(r5,A1,b134), (r5,A2,b1),(r5,A2,b2),(r5,A2,b3),(r5,A2,b4),(r5,A2,b5),(r5,A2,b6),(r5,A2,b7),(r5,A2,b8),(r5 ,A2,b9),(r5,A2,b10),(r5,A2,b11),(r5,A2,b12),(r5,A2,b13),(r5,A2,b14),(r5,A2,b15),(r5,A2,b1 6),(r5,A2,b17),(r5,A2,b18),(r5,A2,b19),(r5,A2,b20),(r5,A2,b21),(r5,A2,b22),(r5,A2,b23),(r 5,A2,b24),(r5,A2,b25),(r5,A2,b26),(r5,A2,b27),(r5,A2,b28),(r5,A2,b29),(r5,A2,b30),(r5,A2 ,b31),(r5,A2,b32),(r5,A2,b33),(r5,A2,b34),(r5,A2,b35),(r5,A2,b36),(r5,A2,b37),(r5,A2,b38) ,(r5,A2,b39),(r5,A2,b40),(r5,A2,b41),(r5,A2,b42),(r5,A2,b43),(r5,A2,b44),(r5,A2,b45),(r5, A2,b46),(r5,A2,b47),(r5,A2,b48),(r5,A2,b49),(r5,A2,b50),(r5,A2,b51),(r5,A2,b52),(r5,A2,b 53),(r5,A2,b54),(r5,A2,b55),(r5,A2,b56),(r5,A2,b57),(r5,A2,b58),(r5,A2,b59),(r5.A2,b60),( r5,A2,b61),(r5,A2,b62),(r5,A2,b63),(r5,A2,b64),(r5,A2,b65),(r5,A2,b66),(r5,A2,b67),(r5,A 2,b68),(r5,A2,b69),(r5,A2,b70),(r5,A2,b71),(r5,A2,b72),(r5,A2,b73),(r5,A2,b74),(r5,A2,b7 5),(r5,A2,b76),(r5,A2,b77),(r5,A2,b78),(r5,A2,b79),(r5,A2,b80),(r5,A2,b81),(r5,A2,b82),(r 5,A2,b83),(r5,A2,b84),(r5,A2,b85),(r5,A2,b86),(r5,A2,b87),(r5,A2,b88),(r5,A2,b89),(r5,A2 ,b90),(r5,A2,b91),(r5,A2,b92),(r5,A2,b93),(r5,A2,b94),(r5,A2,b95),(r5,A2,b96),(r5,A2,b97) ,(r5,A2,b98),(r5,A2,b99),(r5,A2,b100),(r5,A2,b101),(r5,A2,b102),(r5,A2,b103),(r5,A2,b10 4),(r5,A2,b105),(r5,A2,b106),(r5,A2,b107),(r5,A2,b108),(r5,A2,b109),(r5,A2,b110),(r5,A2, b111),(r5,A2,b112),(r5,A2,b113),(r5,A2,b114),(r5,A2,b115),(r5,A2,b116),(r5,A2,b117),(r5, A2,b118),(r5,A2,b119),(r5,A2,b120),(r5,A2,b121),(r5,A2,b122),(r5,A2,b123),(r5,A2,b124), (r5,A2,b125),(r5,A2,b126),(r5,A2,b127),(r5,A2,b128),(r5,A2,b129),(r5,A2,b130),(r5,A2,b 1 31),(r5,A2,b132),(r5,A2,b133),(r5,A2,b134),
(r5,A3,b1),(r5,A3,b2),(r5,A3,b3),(r5,A3,b4),(r5,A3,b5),(r5,A3,b6),(r5,A3,b7),(r5,A3,b8),(r5 ,A3,b9),(r5,A3,b10),(r5,A3,b11),(r5,A3,b12),(r5,A3,b13),(r5,A3,b14),(r5,A3,b15),(r5,A3,b1 6),(r5,A3,b17),(r5,A3,b18),(r5,A3,b19),(r5,A3,b20),(r5,A3,b21),(r5,A3,b22),(r5,A3,b23),(r 5,A3,b24),(r5,A3,b25),(r5,A3,b26),(r5,A3,b27),(r5,A3,b28),(r5,A3,b29),(r5,A3,b30),(r5,A3 ,b31),(r5,A3,b32),(r5,A3,b33),(r5,A3,b34),(r5,A3,b35),(r5,A3,b36),(r5,A3,b37),(r5,A3,b38) ,(r5,A3,b39),(r5,A3,b40),(r5,A3,b41),(r5,A3,b42),(r5,A3,b43),(r5,A3,b44),(r5,A3,b45),(r5, A3,b46),(r5,A3,b47),(r5,A3,b48),(r5,A3,b49),(r5,A3,b50),(r5,A3,b51),(r5,A3,b52),(r5,A3,b 53),(r5,A3,b54),(r5,A3,b55),(r5,A3,b56),(r5,A3,b57),(r5,A3,b58),(r5,A3,b59),(r5,A3,b60),( r5,A3,b61),(r5,A3,b62),(r5,A3,b63),(r5,A3,b64),(r5,A3,b65),(r5,A3,b66),(r5,A3,b67),(r5,A 3,b68),(r5,A3,b69),(r5,A3,b70),(r5,A3,b71),(r5,A3,b72),(r5,A3,b73),(r5,A3,b74),(r5,A3,b7 5),(r5,A3,b76),(r5,A3,b77),(r5,A3,b78),(r5,A3,b79),(r5,A3,b80),(r5,A3,b81),(r5,A3,b82),(r 5,A3,b83),(r5,A3,b84),(r5,A3,b85),(r5,A3,b86),(r5,A3,b87),(r5,A3,b88),(r5,A3,b89),(r5,A3 ,b90),(r5,A3,b91),(r5,A3,b92),(r5,A3,b93),(r5,A3,b94),(r5,A3,b95),(r5,A3,b96),(r5,A3,b97) ,(r5,A3,b98),(r5,A3,b99),(r5,A3,b100),(r5,A3,b101),(r5,A3,b102),(r5,A3,b103),(r5,A3,b10 4),(r5,A3,b105),(r5,A3,b106),(r5,A3,b107),(r5,A3,b108),(r5,A3,b109),(r5,A3,b110),(r5,A3, b111),(r5,A3,b112),(r5,A3,b113),(r5,A3,b114),(r5,A3,b115),(r5,A3,b116),(r5,A3,b117),(r5, A3,b118),(r5,A3,b119),(r5,A3,b120),(r5,A3,b121),(r5,A3,b122),(r5,A3,b123),(r5,A3,b124), (r5,A3,b125),(r5,A3,b126),(r5,A3,b127),(r5,A3,b128),(r5,A3,b129),(r5,A3,b130),(r5,A3,b 1 31),(r5,A3,b132),(r5,A3,b133),(r5,A3,b134),(r5,A4,b1),(r5,A4,b2),(r5,A4,b3),(r5,A4,b4),(r 5,A4,b5),(r5,A4,b6),(r5,A4,b7),(r5,A4,b8),(r5,A4,b9),(r5,A4,b10),(r5,A4,b11),(r5,A4,b12),( r5,A4,b13),(r5,A4,b14),(r5,A4,b15),(r5,A4,b16),(r5,A4,b17),(r5,A4,b18),(r5,A4,b19),(r5,A 4,b20),(r5,A4,b21),(r5,A4,b22),(r5,A4,b23),(r5,A4,b24),(r5,A4,b25),(r5,A4,b26),(r5,A4,b2 7),(r5,A4,b28),(r5,A4,b29),(r5,A4,b30),(r5,A4,b31),(r5,A4,b32),(r5,A4,b33),(r5,A4,b34),(r 5,A4,b35),(r5,A4,b36),(r5,A4,b37),(r5,A4,b38),(r5,A4,b39),(r5,A4,b40),(r5,A4,b41),(r5,A4 ,b42),(r5,A4,b43),(r5,A4,b44),(r5,A4,b45),(r5,A4,b46),(r5,A4,b47),(r5,A4,b48),(r5,A4,b49) ,(r5,A4,b50),(r5,A4,b51),(r5,A4,b52),(r5,A4,b53),(r5,A4,b54),(r5,A4,b55),(r5,A4,b50),(r5, A4,b57),(r5,A4,b58),(r5,A4,b59),(r5,A4,b60),(r5,A4,b61),(r5,A4,b62),(r5,A4,b63),(r5,A4,b 64),(r5,A4,b65),(r5,A4,b66),(r5,A4,b67),(r5,A4,b68),(r5,A4,b69),(r5,A4,b70),(r5,A4,b71),( r5,A4,b72),(r5,A4,b73),(r5,A4,b74),(r5,A4,b75),(r5,A4,b76),(r5,A4,b77),(r5,A4,b78),(r5,A 4,b79),(r5,A4,b80),(r5,A4,b81),(r5,A4,b82),(r5,A4,b83),(r5,A4,b84),(r5,A4,b85),(r5,A4,b8 6),(r5,A4,b87),(r5,A4,b88),(r5,A4,b89),(r5,A4,b90),(r5,A4,b91),(r5,A4,b92),(r5,A4,b93),(r 5,A4,b94),(r5,A4,b95),(r5,A4,b96),(r5,A4,b97),(r5,A4,b98),(r5,A4,b99),(r5,A4,b100),(r5,A 4,b101),(r5,A4,b102),(r5,A4,b103),(r5,A4,b104),(r5,A4,b105),(r5,A4,b106),(r5,A4,b107),(r 5,A4,b108),(r5,A4,b109),(r5,A4,b110),(r5,A4,b111),(r5,A4,b112),(r5,A4,b113),(r5,A4,b114 ),(r5,A4,b115),(r5,A4,b116),(r5,A4,b117),(r5,A4,b118),(r5,A4,b119),(r5,A4,b120),(r5,A4,b 121),(r5,A4,b122),(r5,A4,b123),(r5,A4,b124),(r5,A4,b125),(r5,A4,b126),(r5,A4,b127),(r5, A4,b128),(r5,A4,b129),(r5,A4,b130),(r5,A4,b131),(r5,A4,b132),(r5,A4,b133),(r5,A4,b134), (r6,A1,b1),(r6,A1,b2),(r6,A1,b3),(r6,A1,b4),(r6,A1,b5),(r6,A1,b6),(r6,A1,b7),(r6,A1,b8),(r6 ,A1,b9),(r6,A1,b10),(r6,A1,b11),(r6,A1,b12),(r6,A1,b13),(r6,A1,b14),(r6,A1,b15),(r6,A1,b1 6),(r6,A1,b17),(r6,A1,b18),(r6,A1,b19),(r6,A1,b20),(r6,A1,b21),(r6,A1,b22),(r6,A1,b23),(r 6,A1,b24),(r6,A1,b25),(r6,A1,b26),(r6,A1,b27),(r6,A1,b28),(r6,A1,b29),(r6,A1,b30),(r6,A1 ,b31),(r6,A1,b32),(r6,A1,b33),(r6,A1,b34),(r6,A1,b35),(r6,A1,b36),(r6,A1,b37),(r6,A1,b38) ,(r6,A1,b39),(r6,A1,b40),(r6,A1,b41),(r6,A1,b42),(r6,A1,b43),(r6,A1,b44),(r6,A1,b45),(r6, A1,b46),(r6,A1,b47),(r6,A1,b48),(r6,A1,b49),(r6,A1,b50),(r6,A1,b51),(r6,A1,b52),(r6,A1,b 53),(r6,A1,b54),(r6,A1,b55),(r6,A1,b56),(r6,A1,b57),(r6,A1,b58),(r6,A1,b59),(r6,A1,b60),( r6,A1,b61),(r6,A1,b62),(r6,A1,b63),(r6,A1,b64),(r6,A1,b65),(r6,A1,b66),(r6,A1,b67),(r6,A 1,b68),(r6,A1,b69),(r6,A1,b70),(r6,A1,b71),(r6,A1,b72),(r6,A1,b73),(r6,A1,b74),(r6,A1,b7 5),(r6,A1,b76),(r6,A1,b77),(r6,A1,b78),(r6,A1,b79),(r6,A1,b80),(r6,A1,b81),(r6,A1,b82),(r 6,A1,b83),(r6,A1,b84),(r6,A1,b85),(r6,A1,b86),(r6,A1,b87),(r6,A1,b88),(r6,A1,b89),(r6,A1 ,b90),(r6,A1,b91),(r6,A1,b92),(r6,A1,b93),(r6,A1,b94),(r6,A1,b95),(r6,A1,b96),(r6,A1,b97) ,(r6,A1,b98),(r6,A1,b99),(r6,A1,b100),(r6,A1,b101),(r6,A1,b102),(r6,A1,b103),(r6,A1,b10 4),(r6,A1,b105),(r6,A1,b106),(r6,A1,b107),(r6,A1,b108),(r6,A1,b109),(r6,A1,b110),(r6,A1, b111),(r6,A1,b112),(r6,A1,b113),(r6,A1,b114),(r6,A1,b115),(r6,A1,b116),(r6,A1,b117),(r6, A1,b118),(r6,A1,b119),(r6,A1,b120),(r6,A1,b121),(r6,A1,b122),(r6,A1,b123),(r6,A1,b124), (r6,A1,b125),(r6,A1,b126),(r6,A1,b127),(r6,A1,b128),(r6,A1,b129),(r6,A1,b130),(r6,A1,b1 31),(r6,A1,b132),(r6,A1,b133),(r6,A1,b134),
(r6,A2,b1),(r6,A2,b2),(r6,A2,b3),(r6,A2,b4),(r6,A2,b5),(r6,A2,b6),(r6,A2,b7),(r6,A2,b8),(r6 ,A2,b9),(r6,A2,b10),(r6,A2,b11),(r6,A2,b12),(r6,A2,b13),(r6,A2,b14),(r6,A2,b15),(r6,A2,b1 6),(r6,A2,b17),(r6,A2,b18),(r6,A2,b19),(r6,A2,b20),(r6,A2,b21),(r6,A2,b22),(r6,A2,b23),(r 6,A2,b24),(r6,A2,b25),(r6,A2,b26),(r6,A2,b27),(r6,A2,b28),(r6,A2,b29),(r6,A2,b30),(r6,A2 ,b31),(r6,A2,b32),(r6,A2,b33),(r6,A2,b34),(r6,A2,b35),(r6,A2,b36),(r6,A2,b37),(r6,A2,b38) ,(r6,A2,b39),(r6,A2,b40),(r6,A2,b41),(r6,A2,b42),(r6,A2,b43),(r6,A2,b44),(r6,A2,b45),(r6, A2,b46),(r6,A2,b47),(r6,A2,b48),(r6,A2,b49),(r6,A2,b50),(r6,A2,b51),(r6,A2,b52),(r6,A2,b 53),(r6,A2,b54),(r6,A2,b55),(r6,A2,b56),(r6,A2,b57),(r6,A2,b58),(r6,A2,b59),(r6,A2,b60),( r6,A2,b61),(r6,A2,b62),(r6,A2,b63),(r6,A2,b64),(r6,A2,b65),(r6,A2,b66),(r6,A2,b67),(r6,A 2,b68),(r6,A2,b69),(r6,A2,b70),(r6,A2,b71),(r6,A2,b72),(r6,A2,b73),(r6,A2,b74),(r6,A2,b7 5),(r6,A2,b76),(r6,A2,b77),(r6,A2,b78),(r6,A2,b79),(r6,A2,b80),(r6,A2,b81),(r6,A2,b82),(r 6,A2,b83),(r6,A2,b84),(r6,A2,b85),(r6,A2,b86),(r6,A2,b87),(r6,A2,b88),(r6,A2,b89),(r6,A2 ,b90),(r6,A2,b91),(r6,A2,b92),(r6,A2,b93),(r6,A2,b94),(r6,A2,b95),(r6,A2,b96),(r6,A2,b97) ,(r6,A2,b98),(r6,A2,b99),(r6,A2,b100),(r6,A2,b101),(r6,A2,b102),(r6,A2,b103),(r6,A2,b10 4),(r6,A2,b105),(r6,A2,b106),(r6,A2,b107),(r6,A2,b108),(r6,A2,b109),(r6,A2,b110),(r6,A2, b111),(r6,A2,b112),(r6,A2,b113),(r6,A2,b114),(r6,A2,b115),(r6,A2,b116),(r6,A2,b117),(r6, A2,b118),(r6,A2,b119),(r6,A2,b120),(r6,A2,b121),(r6,A2,b122),(r6,A2,b123),(r6,A2,b124), (r6,A2,b125),(r6,A2,b126),(r6,A2,b127),(r6,A2,b128),(r6,A2,b129),(r6,A2,b130),(r6,A2,b1 31),(r6,A2,b132),(r6,A2,b133),(r6,A2,b134),(r6,A3,b1),(r6,A3,b2),(r6,A3,b3),(r6,A3,b4),(r 6,A3,b5),(r6,A3,b6),(r6,A3,b7),(r6,A3,b8),(r6,A3,b9),(r6,A3,b10),(r6,A3,b11),(r6,A3,b12),( r6,A3,b13),(r6,A3,b14),(r6,A3,b15),(r6,A3,b16),(r6,A3,b17),(r6,A3,b18),(r6,A3,b19),(r6,A 3,b20),(r6,A3,b21),(r6,A3,b22),(r6,A3,b23),(r6,A3,b24),(r6,A3,b25),(r6,A3,b26),(r6,A3,b2 7),(r6,A3,b28),(r6,A3,b29),(r6,A3,b30),(r6,A3,b31),(r6,A3,b32),(r6,A3,b33),(r6,A3,b34),(r 6,A3,b35),(r6,A3,b36),(r6,A3,b37),(r6,A3,b38),(r6,A3,b39),(r6,A3,b40),(r6,A3,b41),(r6,A3 ,b42),(r6,A3,b43),(r6,A3,b44),(r6,A3,b45),(r6,A3,b46),(r6,A3,b47),(r6,A3,b48),(r6,A3,b49) ,(r6,A3,b50),(r6,A3,b51),(r6,A3,b52),(r6,A3,b53),(r6,A3,b54),(r6,A3,b55),(r6,A3,b56),(r6, A3,b57),(r6,A3,b58),(r6,A3,b59),(r6,A3,b60),(r6,A3,b61),(r6,A3,b62),(r6,A3,b63),(r6,A3,b 64),(r6,A3,b65),(r6,A3,b66),(r6,A3,b67),(r6,A3,b68),(r6,A3,b69),(r6,A3,b70),(r6,A3,b71),( r6,A3,b72),(r6,A3,b73),(r6,A3,b74),(r6,A3,b75),(r6,A3,b76),(r6,A3,b77),(r6,A3,b78),(r6,A 3,b79),(r6,A3,b80),(r6,A3,b81),(r6,A3,b82),(r6,A3,b83),(r6,A3,b84),(r6,A3,b85),(r6,A3,b8 6),(r6,A3,b87),(r6,A3,b88),(r6,A3,b89),(r6,A3,b90),(r6,A3,b91),(r6,A3,b92),(r6,A3,b93),(r 6,A3,b94),(r6,A3,b95),(r6,A3,b96),(r6,A3,b97),(r6,A3,b98),(r6,A3,b99),(r6,A3,b100),(r6,A 3,b101),(r6,A3,b102),(r6,A3,b103),(r6,A3,b104),(r6,A3,b105),(r6,A3,b106),(r6,A3,b107),(r 6,A3,b108),(r6,A3,b109),(r6,A3,b110),(r6,A3,b111),(r6,A3,b112),(r6,A3,b113),(r6,A3,b114 ),(r6,A3,b115),(r6,A3,b116),(r6,A3,b117),(r6,A3,b118),(r6,A3,b119),(r6,A3,b120),(r6,A3,b 121),(r6,A3,b 122),(r6,A3,b 123),(r6,A3,b124),(r6,A3,b 125),(r6,A3,b 126),(r6,A3,b 127),(r6, A3,b128),(r6,A3,b129),(r6,A3,b130),(r6,A3,b131),(r6,A3,b132),(r6,A3,b133),(r6,A3,b134), (r6,A4,b1),(r6,A4,b2),(r6,A4,b3),(r6,A4,b4),(r6,A4,b5),(r6,A4,b6),(r6,A4,b7),(r6,A4,b8),(r6 ,A4,b9),(r6,A4,b10),(r6,A4,b11),(r6,A4,b12),(r6,A4,b13),(r6,A4,b14),(r6,A4,b15),(r6,A4,b1 6),(r6,A4,b17),(r6,A4,b18),(r6,A4,bl9),(r6,A4,b20),(r6,A4,b21),(r6,A4,b22),(r6,A4,b23),(r 6,A4,b24),(r6,A4,b25),(r6,A4,b26),(r6,A4,b27),(r6,A4,b28),(r6,A4,b29),(r6,A4,b30),(r6,A4 ,b31),(r6,A4,b32),(r6,A4,b33),(r6,A4,b34),(r6,A4,b35),(r6,A4,b36),(r6,A4,b37),(r6,A4,b38) ,(r6,A4,b39),(r6,A4,b40),(r6,A4,b41),(r6,A4,b42),(r6,A4,b43),(r6,A4,b44),(r6,A4,b45),(r6, A4,b46),(r6,A4,b47),(r6,A4,b48),(r6,A4,b49),(r6,A4,b50),(r6,A4,b51),(r6,A4,b52),(r6,A4,b 53),(r6,A4,b54),(r6,A4,b55),(r6,A4,b56),(r6,A4,b57),(r6,A4,b58),(r6,A4,b59),(r6,A4,b60),( r6,A4,b61),(r6,A4,b62),(r6,A4,b63),(r6,A4,b64),(r6,A4,b65),(r6,A4,b66),(r6,A4,b67),(r6,A 4,b68),(r6,A4,b69),(r6,A4,b70),(r6,A4,b71),(r6,A4,b72),(r6,A4,b73),(r6,A4,b74),(r6,A4,b7 5),(r6,A4,b76),(r6,A4,b77),(r6,A4,b78),(r6,A4,b79),(r6,A4,b80),(r6,A4,b81),(r6,A4,b82),(r 6,A4,b83),(r6,A4,b84),(r6,A4,b85),(r6,A4,b86),(r6,A4,b87),(r6,A4,b88),(r6,A4,b89),(r6,A4 ,b90),(r6,A4,b91),(r6,A4,b92),(r6,A4,b93),(r6,A4,b94),(r6,A4,b95),(r6,A4,b96),(r6,A4,b97) ,(r6,A4,b98),(r6,A4,b99),(r6,A4,b100),(r6,A4,b101),(r6,A4,b102),(r6,A4,b103),(r6,A4,b10 4),(r6,A4,b105),(r6,A4,b106),(r6,A4,b107),(r6,A4,b108),(r6,A4,b109),(r6,A4,b110),(r6,A4, b111),(r6,A4,b112),(r6,A4,b113),(r6,A4,b114),(r6,A4,b115),(r6,A4,b116),(r6,A4,b117),(r6, A4,b118),(r6,A4,b119),(r6,A4,b120),(r6,A4,b121),(r6,A4,b122),(r6,A4,b123),(r6,A4,b124), (r6,A4,b125),(r6,A4,b126),(r6,A4,b127),(r6,A4,b128),(r6,A4,b129),(r6,A4,b130),(r6,A4,b1 31),(r6,A4,b132),(r6,A4,b133),(r6,A4,b134),
(r7,A1,b1),(r7,A1,b2),(r7,A1,b3),(r7,A1,b4),(r7,A1,b5),(r7,A1,b6),(r7,A1,b7),(r7,A1,b8),(r7 ,A1,b9),(r7,A1,b10),(r7,A1,b11),(r7,A1,b12),(r7,A1,b13),(r7,A1,b14),(r7,A1,b15),(r7,A1,b1 6),(r7,A1,b17),(r7,A1,b18),(r7,A1,b19),(r7,A1,b20),(r7,A1,b21),(r7,A1,b22),(r7,A1,b23),(r 7,A1,b24),(r7,A1,b25),(r7,A1,b26),(r7,A1,b27),(r7,A1,b28),(r7,A1,b29),(r7,A1,b30),(r7,A1 ,b31),(r7,A1,b32),(r7,A1,b33),(r7,A1,b34),(r7,A1,b35),(r7,A1,b36),(r7,A1,b37),(r7,A1,b38) ,(r7,A1,b39),(r7,A1,b40),(r7,A1,b41),(r7,A1,b42),(r7,A1,b43),(r7,A1,b44),(r7,A1,b45),(r7, A1,b46),(r7,A1,b47),(r7,A1,b48),(r7,A1,b49),(r7,A1,b50),(r7,A1,b51),(r7,A1,b52),(r7,A1,b 53),(r7,A1,b54),(r7,A1,b55),(r7,A1,b56),(r7,A1,b57),(r7,A1,b58),(r7,A1,b59),(r7,A1,b60),( r7,A1,b61),(r7,A1,b62),(r7,A1,b63),(r7,A1,b64),(r7,A1,b65),(r7,A1,b66),(r7,A1,b67),(r7,A 1,b68),(r7,A1,b69),(r7,A1,b70),(r7,A1,b71),(r7,A1,b72),(r7,A1,b73),(r7,A1,b74),(r7,A1,b7 5),(r7,A1,b76),(r7,A1,b77),(r7,A1,b78),(r7,A1,b79),(r7,A1,b80),(r7,A1,b81),(r7,A1,b82),(r 7,A1,b83),(r7,A1,b84),(r7,A1,b85),(r7,A1,b86),(r7,A1,b87),(r7,A1,b88),(r7,A1,b89),(r7,A1 ,b90),(r7,A1,b91),(r7,A1,b92),(r7,A1,b93),(r7,A1,b94),(r7,A1,b95),(r7,A1,b96),(r7,A1,b97) ,(r7,A1,b98),(r7,A1,b99),(r7,A1,b100),(r7,A1,b101),(r7,A1,b102),(r7,A1,b103),(r7,A1,b10 4),(r7,A1,b105),(r7,A1,b106),(r7,A1,b107),(r7,A1,b108),(r7,A1,b109),(r7,A1,b110),(r7,A1, b111),(r7,A1,b112),(r7,A1,b113),(r7,A1,b114),(r7,A1,b115),(r7,A1,b116),(r7,A1,b117),(r7, A1,b118),(r7,A1,b119),(r7,A1,b120),(r7,A1,b121),(r7,A1,b122),(r7,A1,b123),(r7,A1,b124), (r7,A1,b125),(r7,A1,b 126),(r7,A1,b 127),(r7,A1,b 128),(r7,A1,b129),(r7,A1,b130),(r7,A1,b1 31),(r7,A1,b132),(r7,A1,b133),(r7,A1,b134),(r7,A2,b1),(r7,A2,b2),(r7,A2,b3),(r7,A2,b4),(r 7,A2,b5),(r7,A2,b6),(r7,A2,b7),(r7,A2,b8),(r7,A2,b9),(r7,A2,b10),(r7,A2,b11),(r7,A2,b12),( r7,A2,b13),(r7,A2,b14),(r7,A2,b15),(r7,A2,b16),(r7,A2,b17),(r7,A2,b18),(r7,A2,b19),(r7,A 2,b20),(r7,A2,b21),(r7,A2,b22),(r7,A2,b23),(r7,A2,b24),(r7,A2,b25),(r7,A2,b26),(r7,A2,b2 7),(r7,A2,b28),(r7,A2,b29),(r7,A2,b30),(r7,A2,b31),(r7,A2,b32),(r7,A2,b33),(r7,A2,b34),(r 7,A2,b35),(r7,A2,b36),(r7,A2,b37),(r7,A2,b38),(r7,A2,b39),(r7,A2,b40),(r7,A2,b411),(r7,A2 ,b42),(r7,A2,b43),(r7,A2,b44),(r7,A2,b45),(r7,A2,b46),(r7,A2,b47),(r7,A2,b48),(r7,A2,b49) ,(r7,A2,b50),(r7,A2,b51),(r7,A2,b52),(r7,A2,b53),(r7,A2,b54),(r7,A2,b55),(r7,A2,b56),(r7, A2,b57),(r7,A2,b58),(r7,A2,b59),(r7,A2,b60),(r7,A2,b61),(r7,A2,b62),(r7,A2,b63),(r7,A2,b 64),(r7,A2,b65),(r7,A2,b66),(r7,A2,b67),(r7,A2,b68),(r7,A2,b69),(r7,A2,b70),(r7,A2,b71),( r7,A2,b72),(r7,A2,b73),(r7,A2,b74),(r7,A2,b75),(r7,A2,b76),(r7,A2,b77),(r7,A2,b78),(r7,A 2,b79),(r7,A2,b80),(r7,A2,b81),(r7,A2,b82),(r7,A2,b83),(r7,A2,b84),(r7,A2,b85),(r7,A2,b8 6),(r7,A2,b87),(r7,A2,b88),(r7,A2,b89),(r7,A2,b90),(r7,A2,b91),(r7,A2,b92),(r7,A2,b93),(r 7,A2,b94),(r7,A2,b95),(r7,A2,b96),(r7,A2,b97),(r7,A2,b98),(r7,A2,b99),(r7,A2,b100),(r7,A 2,b101),(r7,A2,b102),(r7,A2,b103),(r7,A2,b104),(r7,A2,b105),(r7,A2,b106),(r7,A2,b107),(r 7,A2,b108),(r7,A2,b109),(r7,A2,b110),(r7,A2,b111),(r7,A2,b112),(r7,A2,b113),(r7,A2,b114 ),(r7,A2,b115),(r7,A2,b116),(r7,A2,b117),(r7,A2,b118),(r7,A2,b119),(r7,A2,b120),(r7,A2,b 121),(r7,A2,b122),(r7,A2,b123),(r7,A2,b124),(r7,A2,b125),(r7,A2,b126),(r7,A2,b127),(r7, A2,b128),(r7,A2,b129),(r7,A2,b130),(r7,A2,b131),(r7,A2,b132),(r7,A2,b133),(r7,A2,b134), (r7,A3,b1),(r7,A3,b2),(r7,A3,b3),(r7,A3,b4),(r7,A3,b5),(r7,A3,b6),(r7,A3,b7),(r7,A3,b8),(r7 ,A3,b9),(r7,A3,b10),(r7,A3,b11),(r7,A3,b12),(r7,A3,b13),(r7,A3,b14),(r7,A3,b15),(r7,A3,b1 6),(r7,A3,b17),(r7,A3,b18),(r7,A3,b19),(r7,A3,b20),(r7,A3,b21),(r7,A3,b22),(r7,A3,b23),(r 7,A3,b24),(r7,A3,b25),(r7,A3,b26),(r7,A3,b27),(r7,A3,b28),(r7,A3,b29),(r7,A3,b30),(r7,A3 ,b31),(r7,A3,b32),(r7,A3,b33),(r7,A3,b34),(r7,A3,b35),(r7,A3,b36),(r7,A3,b37),(r7,A3,b38) ,(r7,A3,b39),(r7,A3,b40),(r7,A3,b41),(r7,A3,b42),(r7,A3,b43),(r7,A3,b44),(r7,A3,b45),(r7, A3,b46),(r7,A3,b47),(r7,A3,b48),(r7,A3,b49),(r7,A3,b50),(r7,A3,b51),(r7,A3,b52),(r7,A3,b 53),(r7,A3,b54),(r7,A3,b55),(r7,A3,b56),(r7,A3,b57),(r7,A3,b58),(r7,A3,b59),(r7,A3,b60),( r7,A3,b61),(r7,A3,b62),(r7,A3,b63),(r7,A3,b64),(r7,A3,b65),(r7,A3,b66),(r7,A3,b67),(r7,A 3,b68),(r7,A3,b69),(r7,A3,b70),(r7,A3,b71),(r7,A3,b72),(r7,A3,b73),(r7,A3,b74),(r7,A3,b7 5),(r7,A3,b76),(r7,A3,b77),(r7,A3,b78),(r7,A3,b79),(r7,A3,b80),(r7,A3,b81),(r7,A3,b82),(r 7,A3,b83),(r7,A3,b84),(r7,A3,b85),(r7,A3,b86),(r7,A3,b87),(r7,A3,b88),(r7,A3,b89),(r7,A3 ,b90),(r7,A3,b91),(r7,A3,b92),(r7,A3,b93),(r7,A3,b94),(r7,A3,b95),(r7,A3,b96),(r7,A3,b97) ,(r7,A3,b98),(r7,A3,b99),(r7,A3,b100),(r7,A3,b101),(r7,A3,b102),(r7,A3,b103),(r7,A3,b10 4),(r7,A3,b105),(r7,A3,b106),(r7,A3,b107),(r7,A3,b108),(r7,A3,b109),(r7,A3,b110),(r7,A3, b111),(r7,A3,b112),(r7,A3,b113),(r7,A3,b114),(r7,A3,b115),(r7,A3,b116),(r7,A3,b117),(r7, A3,b118),(r7,A3,b119),(r7,A3,b120),(r7,A3,b121),(r7,A3,b122),(r7,A3,b123),(r7,A3,b124), (r7,A3,b125),(r7,A3,b126),(r7,A3,b127),(r7,A3,b128),(r7,A3,b129),(r7,A3,b130),(r7,A3,b1 31),(r7,A3,b132),(r7,A3,b133),(r7,A3,b134),
(r7,A4,b1),(r7,A4,b2),(r7,A4,b3),(r7,A4,b4),(r7,A4,b5),(r7,A4,b6),(r7,A4,b7),(r7,A4,b8),(r7 ,A4,b9),(r7,A4,b10),(r7,A4,b11),(r7,A4,b12),(r7,A4,b13),(r7,A4,b14),(r7,A4,b15),(r7,A4,b1 6),(r7,A4,b17),(r7,A4,b18),(r7,A4,b19),(r7,A4,b20),(r7,A4,b21),(r7,A4,b22),(r7,A4,b23),(r 7,A4,b24),(r7,A4,b25),(r7,A4,b26),(r7,A4,b27),(r7,A4,b28),(r7,A4,b29),(r7,A4,b30),(r7,A4 ,b31),(r7,A4,b32),(r7,A4,b33),(r7,A4,b34),(r7,A4,b35),(r7,A4,b36),(r7,A4,b37),(r7,A4,b38) ,(r7,A4,b39),(r7,A4,b40),(r7,A4,b41),(r7,A4,b42),(r7,A4,b43),(r7,A4,b44),(r7,A4,b45),(r7, A4,b46),(r7,A4,b47),(r7,A4,b48),(r7,A4,b49),(r7,A4,b50),(r7,A4,b51),(r7,A4,b52),(r7,A4,b 53),(r7,A4,b54),(r7,A4,b55),(r7,A4,b56),(r7,A4,b57),(r7,A4,b58),(r7,A4,b59),(r7,A4,b60),( r7,A4,b61),(r7,A4,b62),(r7,A4,b63),(r7,A4,b64),(r7,A4,b65),(r7,A4,b66),(r7,A4,b67),(r7,A 4,b68),(r7,A4,b69),(r7,A4,b70),(r7,A4,b71),(r7,A4,b72),(r7,A4,b73),(r7,A4,b74),(r7,A4,b7 5),(r7,A4,b76),(r7,A4,b77),(r7,A4,b78),(r7,A4,b79),(r7,A4,b80),(r7,A4,b81),(r7,A4,b82),(r 7,A4,b83),(r7,A4,b84),(r7,A4,b85),(r7,A4,b86),(r7,A4,b87),(r7,A4,b88),(r7,A4,b89),(r7,A4 ,b90),(r7,A4,b91),(r7,A4,b92),(r7,A4,b93),(r7,A4,b94),(r7,A4,b95),(r7,A4,b96),(r7,A4,b97) ,(r7,A4,b98),(r7,A4,b99),(r7,A4,b100),(r7,A4,b101),(r7,A4,b102),(r7,A4,b103),(r7,A4,b10 4),(r7,A4,b105),(r7,A4,b106),(r7,A4,b107),(r7,A4,b108),(r7,A4,b109),(r7,A4,b110),(r7,A4, b111),(r7,A4,b112),(r7,A4,b113),(r7,A4,b114),(r7,A4,b115),(r7,A4,b116),(r7,A4,b117),(r7, A4,b118),(r7,A4,b119),(r7,A4,b120),(r7,A4,b121),(r7,A4,b122),(r7,A4,b123),(r7,A4,b124), (r7,A4,b125),(r7,A4,b126),(r7,A4,b127),(r7,A4,b128),(r7,A4,b129),(r7,A4,b130),(r7,A4,b1 31),(r7,A4,b132),(r7,A4,b133),(r7,A4,b134),(r8,A1,b1),(r8,A1,b2),(r8,A1,b3),(r8,A1,b4),(r 8,A1,b5),(r8,A1,b6),(r8,A1,b7),(r8,A1,b8),(r8,A1,b9),(r8,A1,b10),(r8,A1,b11),(r8,A1,b12),( r8,A1,b13),(r8,A1,b14),(r8,A1,b15),(r8,A1,b16),(r8,A1,b17),(r8,A1,b18),(r8,A1,b19),(r8,A 1,b20),(r8,A1,b21),(r8,A1,b22),(r8,A1,b23),(r8,A1,b24),(r8,A1,b25),(r8,A1,b26),(r8,A1,b2 7),(r8,A1,b28),(r8,A1,b29),(r8,A1,b30),(r8,A1,b31),(r8,A1,b32),(r8,A1,b33),(r8,A1,b34),(r 8,A1,b35),(r8,A1,b36),(r8,A1,b37),(r8,A1,b38),(r8,A1,b39),(r8,A1,b40),(r8,A1,b41),(r8,A1 ,b42),(r8,A1,b43),(r8,A1,b44),(r8,A1,b45),(r8,A1,b46),(r8,A1,b47),(r8,A1,b48),(r8,A1,b49) ,(r8,A1,b50),(r8,A1,b51),(r8,A1,b52),(r8,A1,b53),(r8,A1,b54),(r8,A1,b55),(r8,A1,b56),(r8, A1,b57),(r8,A1,b58),(r8,A1,b59),(r8,A1,b60),(r8,A1,b61),(r8,A1,b62),(r8,A1,b63),(r8,A1,b 64),(r8,A1,b65),(r8,A1,b66),(r8,A1,b67),(r8,A1,b68),(r8,A1,b69),(r8,A1,b70),(r8,A1,b71),( r8,A1,b72),(r8,A1,b73),(r8,A1,b74),(r8,A1,b75),(r8,A1,b76),(r8,A1,b77),(r8,A1,b78),(r8,A 1,b79),(r8,A1,b80),(r8,A1,b81),(r8,A1,b82),(r8,A1,b83),(r8,A1,b84),(r8,A1,b85),(r8,A1,b8 6),(r8,A1,b87),(r8,A1,b88),(r8,A1,b89),(r8,A1,b90),(r8,A1,b91),(r8,A1,b92),(r8,A1,b93),(r 8,A1,b94),(r8,A1,b95),(r8,A1,b96),(r8,A1,b97),(r8,A1,b98),(r8,A1,b99),(r8,A1,b100),(r8,A 1,b101),(r8,A1,b102),(r8,A1,b103),(r8,A1,b104),(r8,A1,b105),(r8,A1,b106),(r8,A1,b107),(r 8,A1,b108),(r8,A1,b109),(r8,A1,b110),(r8,A1,b111),(r8,A1,b112),(r8,A1,b113),(r8,A1,b114 ),(r8,A1,b115),(r8,A1,b116),(r8,A1,b117),(r8,A1,b118),(r8,A1,b119),(r8,A1,b120),(r8,A1,b 121),(r8,A1,b122),(r8,A1,b123),(r8,A1,b124),(r8,A1,b125),(r8,A1,b126),(r8,A1,b127),(r8, A1,b128),(r8,A1,b129),(r8,A1,b130),(r8,A1,b131),(r8,A1,b132),(r8,A1,b133),(r8,A1,b134), (r8,A2,b 1),(r8,A2,b2),(r8,A2,b3),(r8,A2,b4),(r8,A2,b5),(r8,A2,b6),(r8,A2,b7),(r8,A2,b8),(r8 ,A2,b9),(r8,A2,b10),(r8,A2,b11),(r8,A2,b12),(r8,A2,b13),(r8,A2,b14),(r8,A2,b15),(r8,A2,b1 6),(r8,A2,b17),(r8,A2,b18),(r8,A2,b19),(r8,A2,b20),(r8,A2,b21),(r8,A2,b22),(r8,A2,b23),(r 8,A2,b24),(r8,A2,b25),(r8,A2,b26),(r8,A2,b27),(r8,A2,b28),(r8,A2,b29),(r8,A2,b30),(r8,A2 ,b31),(r8,A2,b32),(r8,A2,b33),(r8,A2,b34),(r8,A2,b35),(r8,A2,b36),(r8,A2,b37),(r8,A2,b38) ,(r8,A2,b39),(r8,A2,b40),(r8,A2,b41),(r8,A2,b42),(r8,A2,b43),(r8,A2,b44),(r8,A2,b45),(r8, A2,b46),(r8,A2,b47),(r8,A2,b48),(r8,A2,b49),(r8,A2,b50),(r8,A2,b51),(r8,A2,b52),(r8,A2,b 53),(r8,A2,b54),(r8,A2,b55),(r8,A2,b56),(r8,A2,b57),(r8,A2,b58),(r8,A2,b59),(r8,A2,b60),( r8,A2,b61),(r8,A2,b62),(r8,A2,b63),(r8,A2,b64),(r8,A2,b65),(r8,A2,b66),(r8,A2,b67),(r8,A 2,b68),(r8,A2,b69),(r8,A2,b70),(r8,A2,b71),(r8,A2,b72),(r8,A2,b73),(r8,A2,b74),(r8,A2,b7 5),(r8,A2,b76),(r8,A2,b77),(r8,A2,b78),(r8,A2,b79),(r8,A2,b80),(r8,A2,b81),(r8,A2,b82),(r 8,A2,b83),(r8,A2,b84),(r8,A2,b85),(r8,A2,b86),(r8,A2,b87),(r8,A2,b88),(r8,A2,b89),(r8,A2 ,b90),(r8,A2,b91),(r8,A2,b92),(r8,A2,b93),(r8,A2,b94),(r8,A2,b95),(r8,A2,b96),(r8,A2,b97) ,(r8,A2,b98),(r8,A2,b99),(r8,A2,b100),(r8,A2,b101),(r8,A2,b102),(r8,A2,b103),(r8,A2,b10 4),(r8,A2,b105),(r8,A2,b106),(r8,A2,b107),(r8,A2,b108),(r8,A2,b109),(r8,A2,b110),(r8,A2, b111),(r8,A2,b112),(r8,A2,b113),(r8,A2,b114),(r8,A2,b115),(r8,A2,b116),(r8,A2,b117),(r8, A2,b118),(r8,A2,b119),(r8,A2,b120),(r8,A2,b121),(r8,A2,b122),(r8,A2,b123),(r8,A2,b124), (r8,A2,b125),(r8,A2,b126),(r8,A2,b127),(r8,A2,b128),(r8,A2,b129),(r8,A2,b130),(r8,A2,b1 31),(r8,A2,b132),(r8,A2,b133),(r8,A2,b134),
(r8,A3,b1),(r8,A3,b2),(r8,A3,b3),(r8,A3,b4),(r8,A3,b5),(r8,A3,b6),(r8,A3,b7),(r8,A3,b8),(r8 ,A3,b9),(r8,A3,b10),(r8,A3,b11),(r8,A3,b12),(r8,A3,b13),(r8,A3,b14),(r8,A3,b15),(r8,A3,b1 6),(r8,A3,b17),(r8,A3,b18),(r8,A3,b19),(r8,A3,b20),(r8,A3,b21),(r8,A3,b22),(r8,A3,b23),(r 8,A3,b24),(r8,A3,b25),(r8,A3,b26),(r8,A3,b27),(r8,A3,b28),(r8,A3,b29),(r8,A3,b30),(r8,A3 ,b31),(r8,A3,b32),(r8,A3,b33),(r8,A3,b34),(r8,A3,b35),(r8,A3,b36),(r8,A3,b37),(r8,A3,b38) ,(r8,A3,b39),(r8,A3,b40),(r8,A3,b4l),(r8,A3,b42),(r8,A3,b43),(r8,A3,b44),(r8,A3,b45),(r8, A3,b46),(r8,A3,b47),(r8,A3,b48),(r8,A3,b49),(r8,A3,b50),(r8,A3,b51),(r8,A3,b52),(r8,A3,b 53),(r8,A3,b54),(r8,A3,b55),(r8,A3,b56),(r8,A3,b57),(r8,A3,b58),(r8,A3,b59),(r8,A3,b60),( r8,A3,b61),(r8,A3,b62),(r8,A3,b63),(r8,A3,b64),(r8,A3,b65),(r8,A3,b66),(r8,A3,b67),(r8,A 3,b68),(r8,A3,b69),(r8,A3,b70),(r8,A3,b71),(r8,A3,b72),(r8,A3,b73),(r8,A3,b74),(r8,A3,b7 5),(r8,A3,b76),(r8,A3,b77),(r8,A3,b78),(r8,A3,b79),(r8,A3,b80),(r8,A3,b81),(r8,A3,b82),(r 8,A3,b83),(r8,A3,b84),(r8,A3,b85),(r8,A3,b86),(r8,A3,b87),(r8,A3,b88),(r8,A3,b89),(r8,A3 ,b90),(r8,A3,b91),(r8,A3,b92),(r8,A3,b93),(r8,A3,b94),(r8,A3,b95),(r8,A3,b96),(r8,A3,b97) ,(r8,A3,b98),(r8,A3,b99),(r8,A3,b100),(r8,A3,b101),(r8,A3,b102),(r8,A3,b103),(r8,A3,b10 4), (r8,A3,b 105), (r8,A3,b 106), (r8,A3,b 107), (r8,A3,b 108), (r8,A3,b 109), (r8,A3,b 110),(r8,A3, b111),(r8,A3,b112),(r8,A3,b113),(r8,A3,b114),(r8,A3,b115),(r8,A3,b116),(r8,A3,b117),(r8, A3,b118),(r8,A3,b119),(r8,A3,b120),(r8,A3,b121),(r8,A3,b122),(r8,A3,b123),(r8,A3,b124). (r8,A3,b125),(r8,A3,b126),(r8,A3,b127),(r8,A3,b128),(r8,A3,b129),(r8,A3,b130),(r8,A3,b 1 31),(r8,A3,b132),(r8,A3,b133),(r8,A3,b134),(r8,A4,b1),(r8,A4,b2),(r8,A4,b3),(r8,A4,b4),(r 8,A4,b5),(r8,A4,b6),(r8,A4,b7),(r8,A4,b8),(r8,A4,b9),(r8,A4,b10),(r8,A4,b11),(r8,A4,b12),( r8,A4,b13),(r8,A4,b14),(r8,A4,b15),(r8,A4,b16),(r8,A4,b17),(r8,A4,b18),(r8,A4,b19),(r8,A 4,b20),(r8,A4,b21),(r8,A4,b22),(r8,A4,b23),(r8,A4,b24),(r8,A4,b25),(r8,A4,b26),(r8,A4,b2 7),(r8,A4,b28),(r8,A4,b29),(r8,A4,b30),(r8,A4,b31),(r8,A4,b32),(r8,A4,b33),(r8,A4,b34),(r 8,A4,b35),(r8,A4,b36),(r8,A4,b37),(r8,A4,b38),(r8,A4,b39),(r8,A4,b40),(r8,A4,b41),(r8,A4 ,b42),(r8,A4,b43),(r8,A4,b44),(r8,A4,b45),(r8,A4,b46),(r8,A4,b47),(r8,A4,b48),(r8,A4,b49) ,(r8,A4,b50),(r8,A4,b51),(r8,A4,b52),(r8,A4,b53),(r8,A4,b54),(r8,A4,b55),(r8,A4,b56),(r8, A4,b57),(r8,A4,b58),(r8,A4,b59),(r8,A4,b60),(r8,A4,b61),(r8,A4,b62),(r8,A4,b63),(r8,A4,b 64),(r8,A4,b65),(r8,A4,b66),(r8,A4,b67),(r8,A4,b68),(r8,A4,b69),(r8,A4,b70),(r8,A4,b71),( r8,A4,b72),(r8,A4,b73),(r8,A4,b74),(r8,A4,b75),(r8,A4,b76),(r8,A4,b77),(r8,A4,b78),(r8,A 4,b79),(r8,A4,b80),(r8,A4,b81),(r8,A4,b82),(r8,A4,b83),(r8,A4,b84),(r8,A4,b85),(r8,A4,b8 6),(r8,A4,b87),(r8,A4,b88),(r8,A4,b89),(r8,A4,b90),(r8,A4,b9l),(r8,A4,b92),(r8,A4,b93),(r 8,A4,b94),(r8,A4,b95),(r8,A4,b96),(r8,A4,b97),(r8,A4,b98),(r8,A4,b99),(r8,A4,b100),(r8,A 4,b101),(r8,A4,b102),(r8,A4,b103),(r8,A4,b104),(r8,A4,b105),(r8,A4,b106),(r8,A4,b107),(r 8,A4,b108),(r8,A4,b109),(r8,A4,b110),(r8,A4,b111),(r8,A4,b112),(r8,A4,b113),(r8,A4,b114 ),(r8,A4,b115),(r8,A4,b116),(r8,A4,b117),(r8,A4,b118),(r8,A4,b119),(r8,A4,b120),(r8,A4,b 121),(r8,A4,b122),(r8,A4,b123),(r8,A4,b124),(r8,A4,b125),(r8,A4,b126),(r8,A4,b127),(r8, A4,b 128),(r8,A4,b 129),(r8,A4,b 130),(r8,A4,b 131),(r8,A4,b 132),(r8,A4,b 133),(r8,A4,b 134), (r9,A1,b1),(r9,A1,b2),(r9,A1,b3),(r9,A1,b4),(r9,A1,b5),(r9,A1,b6),(r9,A1,b7),(r9,A1,b8),(r9 ,A1,b9),(r9,A1,b10),(r9,A1,b11),(r9,A1,b12),(r9,A1,b13),(r9,A1,b14),(r9,A1,b15),(r9,A1,b1 6),(r9,A1,b17),(r9,A1,b18),(r9,A1,b19),(r9,A1,b20),(r9,A1,b21),(r9,A1,b22),(r9,A1,b23),(r 9,A1,b24),(r9,A1,b25),(r9,A1,b26),(r9,A1,b27),(r9,A1,b28),(r9,A1,b29),(r9,A1,b30),(r9,A1 ,b31),(r9,A1,b32),(r9,A1,b33),(r9,A1,b34),(r9,A1,b35),(r9,A1,b36),(r9,A1,b37),(r9,A1,b38) ,(r9,A1,b39),(r9,A1,b40),(r9,A1,b41),(r9,A1,b42),(r9,A1,b43),(r9,A1,b44),(r9,A1,b45),(r9, A1,b46),(r9,A1,b47),(r9,A1,b48),(r9,A1,b49),(r9,A1,b50),(r9,A1,b51),(r9,A1,b52),(r9,Al,b 53),(r9,A1,b54),(r9,A1,b55),(r9,A1,b56),(r9,A1,b57),(r9,A1,b58),(r9,A1,b59),(r9,A1,b60),( r9,A1,b61),(r9,A1,b62),(r9,A1,b63),(r9,A1,b64),(r9,A1,b65),(r9,A1,b66),(r9,A1,b67),(r9,A 1,b68),(r9,A1,b69),(r9,A1,b70),(r9,A1,b71),(r9,A1,b72),(r9,A1,b73),(r9,A1,b74),(r9,Al,b7 5),(r9,A1,b76),(r9,A1,b77),(r9,A1,b78),(r9,A1,b79),(r9,A1,b80),(r9,A1,b81),(r9,A1,b82),(r 9,A1,b83),(r9,A1,b84),(r9,A1,b85),(r9,A1,b86),(r9,A1,b87),(r9,A1,b88),(r9,A1,b89),(r9,A1 ,b90),(r9,A1,b91),(r9,A1,b92),(r9,A1,b93),(r9,A1,b94),(r9,A1,b95),(r9,A1,b96),(r9,A1,b97) ,(r9,A1,b98),(r9,A1,b99),(r9,A1,b100),(r9,A1,b101),(r9,A1,b102),(r9,A1,b103),(r9,A1,b10 4),(r9,A1,b105),(r9,A1,b106),(r9,A1,b107),(r9,A1,b108),(r9,A1,b109),(r9,A1,b110),(r9,A1, b111),(r9,A1,b112),(r9,A1,b113),(r9,A1,b114),(r9,A1,b115),(r9,A1,b116),(r9,A1,b117),(r9, A1,b118),(r9,A1,b119),(r9,A1,b120),(r9,A1,b121),(r9,A1,b122),(r9,A1,b123),(r9,A1,b124), (r9,A1,b125),(r9,A1,b126),(r9,A1,b127),(r9,A1,b128),(r9,A1,b129),(r9,A1,b130),(r9,A1,b1 31),(r9,A1,b132),(r9,A1,b133),(r9,A1,b134),
(r9,A2,b1),(r9,A2,b2),(r9,A2,b3),(r9,A2,b4),(r9,A2,b5),(r9,A2,b6),(r9,A2,b7),(r9,A2,b8),(r9 ,A2,b9),(r9,A2,b10),(r9,A2,b11),(r9,A2,b12),(r9,A2,b13),(r9,A2,b14),(r9,A2,b15),(r9,A2,b1 6),(r9,A2,b17),(r9,A2,b18),(r9,A2,b19),(r9,A2,b20),(r9,A2,b21),(r9,A2,b22),(r9,A2,b23),(r 9,A2,b24),(r9,A2,b25),(r9,A2,b26),(r9,A2,b27),(r9,A2,b28),(r9,A2,b29),(r9,A2,b30),(r9,A2 ,b31),(r9,A2,b32),(r9,A2,b33),(r9,A2,b34),(r9,A2,b35),(r9,A2,b36),(r9,A2,b37),(r9,A2,b38) ,(r9,A2,b39),(r9,A2,b40),(r9,A2,b41),(r9,A2,b42),(r9,A2,b43),(r9,A2,b44),(r9,A2,b45),(r9, A2,b46),(r9,A2,b47),(r9,A2,b48),(r9,A2,b49),(r9,A2,b50),(r9,A2,b51),(r9,A2,b52),(r9,A2,b 53),(r9,A2,b54),(r9,A2,b55),(r9,A2,b56),(r9,A2,b57),(r9,A2,b58),(r9,A2,b59),(r9,A2,b60),( r9,A2,b61),(r9,A2,b62),(r9,A2,b63),(r9,A2,b64),(r9,A2,b65),(r9,A2,b66),(r9,A2,b67),(r9,A 2,b68),(r9,A2,b69),(r9,A2,b70),(r9,A2,b71),(r9,A2,b72),(r9,A2,b73),(r9,A2,b74),(r9,A2,b7 5),(r9,A2,b76),(r9,A2,b77),(r9,A2,b78),(r9,A2,b79),(r9,A2,b80),(r9,A2,b81),(r9,A2,b82),(r 9,A2,b83),(r9,A2,b84),(r9,A2,b85),(r9,A2,b86),(r9,A2,b87),(r9,A2,b88),(r9,A2,b89),(r9,A2 ,b90),(r9,A2,b91),(r9,A2,b92),(r9,A2,b93),(r9,A2,b94),(r9,A2,b95),(r9,A2,b96),(r9,A2,b97) ,(r9,A2,b98),(r9,A2,b99),(r9,A2,b100),(r9,A2,b101),(r9,A2,b102),(r9,A2,b103),(r9,A2,b10 4),(r9,A2,b105),(r9,A2,b106),(r9,A2,b107),(r9,A2,b108),(r9,A2,b109),(r9,A2,b110),(r9,A2, b111),(r9,A2,b112),(r9,A2,b113),(r9,A2,b114),(r9,A2,b115),(r9,A2,b116),(r9,A2,b117),(r9, A2,b118),(r9,A2,b119),(r9,A2,b120),(r9,A2,b121),(r9,A2,b122),(r9,A2,b123),(r9,A2,b124), (r9,A2,b125),(r9,A2,b126),(r9,A2,b127),(r9,A2,b128),(r9,A2,b129),(r9,A2,b130),(r9,A2,b1 31),(r9,A2,b132),(r9,A2,b133),(r9,A2,b134),(r9,A3,b1),(r9,A3,b2),(r9,A3,b3),(r9,A3,b4),(r 9,A3,b5),(r9,A3,b6),(r9,A3,b7),(r9,A3,b8),(r9,A3,b9),(r9,A3,b10),(r9,A3,b11),(r9,A3,b12),( r9,A3,b13),(r9,A3,b14),(r9,A3,b15),(r9,A3,b16),(r9,A3,b17),(r9,A3,b18),(r9,A3,b19),(r9,A 3,b20),(r9,A3,b21),(r9,A3,b22),(r9,A3,b23),(r9,A3,b24),(r9,A3,b25),(r9,A3,b26),(r9,A3,b2 7),(r9,A3,b28),(r9,A3,b29),(r9,A3,b30),(r9,A3,b31),(r9,A3,b32),(r9,A3,b33),(r9,A3,b34),(r 9,A3,b35),(r9,A3,b36),(r9,A3,b37),(r9,A3,b38),(r9,A3,b39),(r9,A3,b40),(r9,A3,b41),(r9,A3 ,b42),(r9,A3,b43),(r9,A3,b44),(r9,A3,b45),(r9,A3,b46),(r9,A3,b47),(r9,A3,b48),(r9,A3,b49) ,(r9,A3,b50),(r9,A3,b51),(r9,A3,b52),(r9,A3,b53),(r9,A3,b54),(r9,A3,b55),(r9,A3,b56),(r9, A3,b57),(r9,A3,b58),(r9,A3,b59),(r9,A3,b60),(r9,A3,b61),(r9,A3,b62),(r9,A3,b63),(r9,A3,b 64),(r9,A3,b65),(r9,A3,b66),(r9,A3,b67),(r9,A3,b68),(r9,A3,b69),(r9,A3,b70),(r9,A3,b71),( r9,A3,b72),(r9,A3,b73),(r9,A3,b74),(r9,A3,b75),(r9,A3,b76),(r9,A3,b77),(r9,A3,b78),(r9,A 3,b79),(r9,A3,b80),(r9,A3,b81),(r9,A3,b82),(r9,A3,b83),(r9,A3,b84),(r9,A3,b85),(r9,A3,b8 6),(r9,A3,b87),(r9,A3,b88),(r9,A3,b89),(r9,A3,b90),(r9,A3,b91),(r9,A3,b92),(r9,A3,b93),(r 9,A3,b94),(r9,A3,b95),(r9,A3,b96),(r9,A3,b97),(r9,A3,b98),(r9,A3,b99),(r9,A3,b100),(r9,A 3,b101),(r9,A3,b102),(r9,A3,b103),(r9,A3,b104),(r9,A3,b105),(r9,A3,b106),(r9,A3,b107),(r 9,A3,b108),(r9,A3,b109),(r9,A3,b110),(r9,A3,b111),(r9,A3,b112),(r9,A3,b113),(r9,A3,b114 ),(r9,A3,b115),(r9,A3,b116),(r9,A3,b117),(r9,A3,b118),(r9,A3,b119),(r9,A3,b120),(r9,A3,b 121),(r9,A3,b122),(r9,A3,b 123),(r9,A3,b 124),(r9,A3,b 125),(r9,A3,b 126),(r9,A3,b 127),(r9, A3,b128),(r9,A3,b129),(r9,A3,b130),(r9,A3,b131),(r9,A3,b132),(r9,A3,b133),(r9,A3,b134), (r9,A4,b1),(r9,A4,b2),(r9,A4,b3),(r9,A4,b4),(r9,A4,b5),(r9,A4,b6),(r9,A4,b7),(r9,A4,b8),(r9 ,A4,b9),(r9,A4,b10),(r9,A4,b11),(r9,A4,b12),(r9,A4,b13),(r9,A4,b14),(r9,A4,b15),(r9,A4,b1 6),(r9,A4,b17),(r9,A4,b18),(r9,A4,b19),(r9,A4,b20),(r9,A4,b21),(r9,A4,b22),(r9,A4,b23),(r 9,A4,b24),(r9,A4,b25),(r9,A4,b26),(r9,A4,b27),(r9,A4,b28),(r9,A4,b29),(r9,A4,b30),(r9,A4 ,b31),(r9,A4,b32),(r9,A4,b33),(r9,A4,b34),(r9,A4,b35),(r9,A4,b36),(r9,A4,b37),(r9,A4,b38) ,(r9,A4,b39),(r9,A4,b40),(r9,A4,b41),(r9,A4,b42),(r9,A4,b43),(r9,A4,b44),(r9,A4,b45),(r9, A4,b46),(r9,A4,b47),(r9,A4,b48),(r9,A4,b49),(r9,A4,b50),(r9,A4,b51),(r9,A4,b52),(r9,A4,b 53),(r9,A4,b54),(r9,A4,b55),(r9,A4,b56),(r9,A4,b57),(r9,A4,b58),(r9,A4,b59),(r9,A4,b60),( r9,A4,b61),(r9,A4,b62),(r9,A4,b63),(r9,A4,b64),(r9,A4,b65),(r9,A4,b66),(r9,A4,b67),(r9,A 4,b68),(r9,A4,b69),(r9,A4,b70),(r9,A4,b71),(r9,A4,b72),(r9,A4,b73),(r9,A4,b74),(r9,A4,b7 5),(r9,A4,b76),(r9,A4,b77),(r9,A4,b78),(r9,A4,b79),(r9,A4,b80),(r9,A4,b81),(r9,A4,b82),(r 9,A4,b83),(r9,A4,b84),(r9,A4,b85),(r9,A4,b86),(r9,A4,b87),(r9,A4,b88),(r9,A4,b89),(r9,A4 ,b90),(r9,A4,b91),(r9,A4,b92),(r9,A4,b93),(r9,A4,b94),(r9,A4,b95),(r9,A4,b96),(r9,A4,b97) ,(r9,A4,b98),(r9,A4,b99),(r9,A4,b100),(r9,A4,b101),(r9,A4,b102),(r9,A4,b103),(r9,A4,b10 4),(r9,A4,b105),(r9,A4,b106),(r9,A4,b107),(r9,A4,b108),(r9,A4,b109),(r9,A4,b110),(r9,A4, b111),(r9,A4,b112),(r9,A4,b113),(r9,A4,b114),(r9,A4,b115),(r9,A4,b116),(r9,A4,b117),(r9, A4,b118),(r9,A4,b119),(r9,A4,b120),(r9,A4,b121),(r9,A4,b122),(r9,A4,b123),(r9,A4,b124), (r9,A4,b125),(r9,A4,b126),(r9,A4,b127),(r9,A4,b128),(r9,A4,b129),(r9,A4,b130),(r9,A4,b1 31),(r9,A4,b132),(r9,A4,b133),(r9,A4,b134),
(r10,A1,b1),(r10,A1,b2),(r10,A1,b3),(r10,A1,b4),(r10,A1,b5),(r10,A1,b6),(r10,A1,b7),(r10, A1,b8),(r10,A1,b9),(r10,A1,b10),(r10,A1,b11),(r10,A1,b12),(r10,A1,b13),(r10,A1,b14),(r1 0,A1,b15),(r10,A1,b16),(r10,A1,b17),(r10,A1,b18),(r10,A1,b19),(r10,A1,b20),(r10,A1,b21) ,(r10,A1,b22),(r10,A1,b23),(r10,A1,b24),(r10,A1,b25),(r10,A1,b26),(r10,A1,b27),(r10,A1, b28),(r10,A1,b29),(r10,A1,b30),(r10,A1,b31),(r10,A1,b32),(r10,A1,b33),(r10,A1,b34),(r10, A1,b35),(r10,A1,b36),(r10,A1,b37),(r10,A1,b38),(r10,A1,b39),(r10,A1,b40),(r10,A1,b41),( r10,A1,b42),(r10,A1,b43),(r10,A1,b44),(r10,A1,b45),(r10,A1,b46),(r10,A1,b47),(r10,A1,b 48),(r10,A1,b49),(r10,A1,b50),(r10,A1,b51),(r10,A1,b52),(r10,A1,b53),(r10,A1,b54),(r10, A1,b55),(r10,A1,b56),(r10,A1,b57),(r10,A1,b58),(r10,A1,b59),(r10,A1,b60),(r10,A1,b61),( r10,A1,b62),(r10,A1,b63),(r10,A1,b64),(r10,A1,b65),(r10,A1,b66),(r10,A1,b67),(r10,A1,b 68),(r10,A1,b69),(r10,A1,b70),(r10,A1,b71),(r10,A1,b72),(r10,A1,b73),(r10,A1,b74),(r10, A1,b75),(r10,A1,b76),(r10,A1,b77),(r10,A1,b78),(r10,A1,b79),(r10,A1,b80),(r10,A1,b81),( r10,A1,b82),(r10,A1,b83),(r10,A1,b84),(r10,A1,b85),(r10,A1,b86),(r10,A1,b87),(r10,A1,b 88),(r10,A1,b89),(r10,A1,b90),(r10,A1,b91),(r10,A1,b92),(r10,A1,b93),(r10,A1,b94),(r10, A1,b95),(r10,A1,b96),(r10,A1,b97),(r10,A1,b98),(r10,A1,b99),(r10,A1,b100),(r10,A1,b101 ),(r10,A1,b102),(r10,A1,b103),(r10,A1,b104),(r10,A1,b105),(r10,A1,b106),(r10,A1,b107),( r10,A1,b108),(r10,A1,b109),(r10,A1,b110),(r10,A1,b111),(r10,A1,b112),(r10,A1,b113),(r1 0,A1,b114),(r10,A1,b115),(r10,A1,b116),(r10,A1,b117),(r10,A1,b118),(r10,A1,b119),(r10, A1,b120),(r10,A1,b121),(r10,A1,b122),(r10,A1,b123),(r10,A1,b124),(r10,A1,b125),(r10,A 1,b126),(r10,A1,b127),(r10,A1,b128),(r10,A1,b129),(r10,A1,b130),(r10,A1,b131),(r10,A1, b132),(r10,A1,b133),(r10,A1,b134),(r10,A2,b1),(r10,A2,b2),(r10,A2,b3),(r10,A2,b4),(r10, A2,b5),(r10,A2,b6),(r10,A2,b7),(r10,A2,b8),(r10,A2,b9),(r10,A2,b10),(r10,A2,b11),(r10,A2 ,b12),(r10,A2,b13),(r10,A2,b14),(r10,A2,b15),(r10,A2,b16),(r10,A2,b17),(r10,A2,b18),(r10 ,A2,b19),(r10,A2,b20),(r10,A2,b21),(r10,A2,b22),(r10,A2,b23),(r10,A2,b24),(r10,A2,b25),( r10,A2,b26),(r10,A2,b27),(r10,A2,b28),(r10,A2,b29),(r10,A2,b30),(r10,A2,b31),(r10,A2,b 32),(r10,A2,b33),(r10,A2,b34),(r10,A2,b35),(r10,A2,b36),(r10,A2,b37),(r10,A2,b38),(r10, A2,b39),(r10,A2,b40),(r10,A2,b41),(r10,A2,b42),(r10,A2,b43),(r10,A2,b44),(r10,A2,b45),( r10,A2,b46),(r10,A2,b47),(r10,A2,b48),(r10,A2,b49),(r10,A2,b50),(r10,A2,b51),(r10,A2,b 52),(r10,A2,b53),(r10,A2,b54),(r10,A2,b55),(r10,A2,b56),(r10,A2,b57),(r10,A2,b58),(r10, A2,b59),(r10,A2,b60),(r10,A2,b61),(r10,A2,b62),(r10,A2,b63),(r10,A2,b64),(r10,A2,b65),( r10,A2,b66),(r10,A2,b67),(r10,A2,b68),(r10,A2,b69),(r10,A2,b70),(r10,A2,b71),(r10,A2,b 72),(r10,A2,b73),(r10,A2,b74),(r10,A2,b75),(r10,A2,b76),(r10,A2,b77),(r10,A2,b78),(r10, A2,b79),(r10,A2,b80),(r10,A2,b81),(r10,A2,b82),(r10,A2,b83),(r10,A2,b84),(r10,A2,b85),( r10,A2,b86),(r10,A2,b87),(r10,A2,b88),(r10,A2,b89),(r10,A2,b90),(r10,A2,b91),(r10,A2,b 92),(r10,A2,b93),(r10,A2,b94),(r10,A2,b95),(r10,A2,b96),(r10,A2,b97),(r10,A2,b98),(r10, A2,b99),(r10,A2,b100),(r10,A2,b101),(r10,A2,b102),(r10,A2,b103),(r10,A2,b104),(r10,A2, b05),(r10,A2,b106),(r10,A2,b107),(r10,A2,b108),(r10,A2,b109),(r10,A2,b110),(r10,A2,b1 11),(r10,A2,b112),(r10,A2,b113),(r10,A2,b114),(r10,A2,b115),(r10,A2,b116),(r10,A2,b117) ,(r10,A2,b118),(r10,A2,b119),(r10,A2,b120),(r10,A2,b121),(r10,A2,b122),(r10,A2,b123),(r 10,A2,b124),(r10,A2,b125),(r10,A2,b126),(r10,A2,b127),(r10,A2,b128),(r10,A2,b129),(r10 ,A2,b130),(r10,A2,b131),(r10,A2,b132),(r10,A2,b133),(r10,A2,b134),(r10,A3,b1),(r10,A3, b2),(r10,A3,b3),(r10,A3,b4),(r10,A3,b5),(r10,A3,b6),(r10,A3,b7),(r10,A3,b8),(r10,A3,b9),( r10,A3,b10),(r10,A3,b11),(r10,A3,b12),(r10,A3,b13),(r10,A3,b14),(r10,A3,b15),(r10,A3,b1 6),(r10,A3,b17),(r10,A3,b18),(r10,A3,b19),(r10,A3,b20),(r10,A3,b21),(r10,A3,b22),(r10,A 3,b23),(r10,A3,b24),(r10,A3,b25),(r10,A3,b26),(r10,A3,b27),(r10,A3,b28),(r10,A3,b29),(r1 0,A3,b30),(r10,A3,b31),(r10,A3,b32),(r10,A3,b33),(r10,A3,b34),(r10,A3,b35),(r10,A3,b36) ,(r10,A3,b37),(r10,A3,b38),(r10,A3,b39),(r10,A3,b40),(r10,A3,b41),(r10,A3,b42),(r10,A3, b43),(r10,A3,b44),(r10,A3,b45),(r10,A3,b46),(r10,A3,b47),(r10,A3,b48),(r10,A3,b49),(r10, A3,b50),(r10,A3,b51),(r10,A3,b52),(r10,A3,b53),(r10,A3,b54),(r10,A3,b55),(r10,A3,b56),( r10,A3,b57),(r10,A3,b58),(r10,A3,b59),(r10,A3,b60),(r10,A3,b61),(r10,A3,b62),(r10,A3,b 63),(r10,A3,b64),(r10,A3,b65),(r10,A3,b66),(r10,A3,b67),(r10,A3,b68),(r10,A3,b69),(r10, A3,b70),(r10,A3,b71),(r10,A3,b72),(r10,A3,b73),(r10,A3,b74),(r10,A3,b75),(r10,A3,b76),( r10,A3,b77),(r10,A3,b78),(r10,A3,b79),(r10,A3,b80),(r10,A3,b81),(r10,A3,b82),(r10,A3,b 83),(r10,A3,b84),(r10,A3,b85),(r10,A3,b86),(r10,A3,b87),(r10,A3,b88),(r10,A3,b89),(r10, A3,b90),(r10,A3,b91),(r10,A3,b92),(r10,A3,b93),(r10,A3,b94),(r10,A3,b95),(r10,A3,b96),( r10,A3,b97),(r10,A3,b98),(r10,A3,b99),(r10,A3,b100),(r10,A3,b101),(r10,A3,b102),(r10,A 3,b103),(r10,A3,b104),(r10,A3,b105),(r10,A3,b106),(r10,A3,b107),(r10,A3,b108),(r10,A3, b109),(r10,A3,b110),(r10,A3,b111),(r10,A3,b112),(r10,A3,b113),(r10,A3,b114),(r10,A3,b1 15),(r10,A3,b116),(r10,A3,b117),(r10,A3,b118),(r10,A3,b119),(r10,A3,b120),(r10,A3,b121 ),(r10,A3,b122),(r10,A3,b123),(r10,A3,b124),(r10,A3,b125),(r10,A3,b126),(r10,A3,b127),( r10,A3,b128),(r10,A3,b129),(r10,A3,b130),(r10,A3,b131),(r10,A3,b132),(r10,A3,b133),(r1 0,A3,b134),
(r10,A4,b1),(r10,A4,b2),(r10,A4,b3),(r10,A4,b4),(r10,A4,b5),(r10,A4,b6),(r10,A4,b7),(r10, A4,b8),(r10,A4,b9),(r10,A4,b10),(r10,A4,b11),(r10,A4,b12),(r10,A4,b13),(r10,A4,b14),(r1 0,A4,b15),(r10,A4,b16),(r10,A4,b17),(r10,A4,b18),(r10,A4,b19),(r10,A4,b20),(r10,A4,b21) ,(r10,A4,b22),(r10,A4,b23),(r10,A4,b24),(r10,A4,b25),(r10,A4,b26),(r10,A4,b27),(r10,A4, b28),(r10,A4,b29),(r10,A4,b30),(r10,A4,b31),(r10,A4,b32),(r10,A4,b33),(r10,A4,b34),(r10, A4,b35),(r10,A4,b36),(r10,A4,b37),(r10,A4,b38),(r10,A4,b39),(r10,A4,b40),(r10,A4,b41),( r10,A4,b42),(r10,A4,b43),(r10,A4,b44),(r10,A4,b45),(r10,A4,b46),(r10,A4,b47),(r10,A4,b 48),(r10,A4,b49),(r10,A4,b50),(r10,A4,b51),(r10,A4,b52),(r10,A4,b53),(r10,A4,b54),(r10, A4,b55),(r10,A4,b56),(r10,A4,b57),(r10,A4,b58),(r10,A4,b59),(r10,A4,b60),(r10,A4,b61),( r10,A4,b62),(r10,A4,b63),(r10,A4,b64),(r10,A4,b65),(r10,A4,b66),(r10,A4,b67),(r10,A4,b 68),(r10,A4,b69),(r10,A4,b70),(r10,A4,b71),(r10,A4,b72),(r10,A4,b73),(r10,A4,b74),(r10, A4,b75),(r10,A4,b76),(r10,A4,b77),(r10,A4,b78),(r10,A4,b79),(r10,A4,b80),(r10,A4,b81),( r10,A4,b82),(r10,A4,b83),(r10,A4,b84),(r10,A4,b85),(r10,A4,b86),(r10,A4,b87),(r10,A4,b 88),(r10,A4,b89),(r10,A4,b90),(r10,A4,b91),(r10,A4,b92),(r10,A4,b93),(r10,A4,b94),(r10, A4,b95),(r10,A4,b96),(r10,A4,b97),(r10,A4,b98),(r10,A4,b99),(r10,A4,b100),(r10,A4,b101 ),(r10,A4,b102),(r10,A4,b103),(r10,A4,b104),(r10,A4,b105),(r10,A4,b106),(r10,A4,b107),( r10,A4,b108),(r10,A4,b109),(r10,A4,b110),(r10,A4,b111),(r10,A4,b112),(r10,A4,b113),(r1 0,A4,b114),(r10,A4,b115),(r10,A4,b116),(r10,A4,b117),(r10,A4,b118),(r10,A4,b119),(r10, A4,b120),(r10,A4,b121),(r10,A4,b122),(r10,A4,b123),(r10,A4,b124),(r10,A4,b125),(r10,A 4,b126),(r10,A4,b127),(r10,A4,b128),(r10,A4,b129),(r10,A4,b130),(r10,A4,b131),(r10,A4, b132),(r10,A4,b133),(r10,A4,b134),(r11,A1,b1),(r11,A1,b2),(r11,A1,b3),(r11,A1,b4),(r11,A 1,b5),(r11,A1,b6),(r11,A1,b7),(r11,A1,b8),(r11,A1,b9),(r11,A1,b10),(r11,A1,b11),(r11,A1,b 12),(r11,A1,b13),(r11,A1,b14),(r11,A1,b15),(r11,A1,b16),(r11,A1,b17),(r11,A1,b18),(r11,A 1,b19),(r11,A1,b20),(r11,A1,b21),(r11,A1,b22),(r11,A1,b23),(r11,A1,b24),(r11,A1,b25),(r1 1,A1,b26),(r11,A1,b27),(r11,A1,b28),(r11,A1,b29),(r11,A1,b30),(r11,A1,b31),(r11,A1,b32), (r11,A1,b33),(r11,A1,b34),(r11,A1,b35),(r11,A1,b36),(r11,A1,b37),(r11,A1,b38),(r11,A1,b3 9),(r11,A1,b40),(r11,A1,b41),(r11,A1,b42),(r11,A1,b43),(r11,A1,b44),(r11,A1,b45),(r11,A1 ,b46),(r11,A1,b47),(r11,A1,b48),(r11,A1,b49),(r11,A1,b50),(r11,A1,b51),(r11,A1,b52),(r11, A1,b53),(r11,A1,b54),(r11,A1,b55),(r11,A1,b56),(r11,A1,b57),(r11,A1,b58),(r11,A1,b59),(r 11,A1,b60),(r11,A1,b61),(r11,A1,b62),(r11,A1,b63),(r11,A1,b64),(r11,A1,b65),(r11,A1,b66 ),(r11,A1,b67),(r11,A1,b68),(r11,A1,b69),(r11,A1,b70),(r11,A1,b71),(r11,A1,b72),(r11,A1, b73),(r11,A1,b74),(r11,A1,b75),(r11,A1,b76),(r11,A1,b77),(r11,A1,b78),(r11,A1,b79),(r11, A1,b80),(r11,A1,b81),(r11,A1,b82),(r11,A1,b83),(r11,A1,b84),(r11,A1,b85),(r11,A1,b86),(r 11,A1,b87),(r11,A1,b88),(r11,A1,b89),(r11,A1,b90),(r11,A1,b91),(r11,A1,b92),(r11,A1,b93 ),(r11,A1,b94),(r11,A1,b95),(r11,A1,b96),(r11,A1,b97),(r11,A1,b98),(r11,A1,b99),(r11,A1, b100),(r11,A1,b101),(r11,A1,b102),(r11,A1,b103),(r11,A1,b104),(r11,A1,b105),(r11,A1,b1 06),(r11,A1,b107),(r11,A1,b108),(r11,A1,b109),(r11,A1,b110),(r11,A1,b111),(r11,A1,b112) ,(r11,A1,b113),(r11,A1,b114),(r11,A1,b115),(r11,A1,b116),(r11,A1,b117),(r11,A1,b118),(r1 1,A1,b119),(r11,A1,b120),(r11,A1,b121),(r11,A1,b122),(r11,A1,b123),(r11,A1,b124),(r11, A1,b125),(r11,A1,b126),(r11,A1,b127),(r11,A1,b128),(r11,A1,b129),(r11,A1,b130),(r11,A1 ,b131),(r11,A1,b132),(r11,A1,b133),(r11,A1,b134),(r11,A2,b1),(r11,A2,b2),(r11,A2,b3),(r1 1,A2,b4),(r11,A2,b5),(r11,A2,b6),(r11,A2,b7),(r11,A2,b8),(r11,A2,b9),(r11,A2,b10),(r11,A2 ,b11),(r11,A2,b12),(r11,A2,b13),(r11,A2,b14),(r11,A2,b15),(r11,A2,b16),(r11,A2,b17),(r11, A2,b18),(r11,A2,b19),(r11,A2,b20),(r11,A2,b21),(r11,A2,b22),(r11,A2,b23),(r11,A2,b24),(r 11,A2,b25),(r11,A2,b26),(r11,A2,b27),(r11,A2,b28),(r11,A2,b29),(r11,A2,b30),(r11,A2,b31 ),(r11,A2,b32),(r11,A2,b33),(r11,A2,b34),(r11,A2,b35),(r11,A2,b36),(r11,A2,b37),(r11,A2, b38),(r11,A2,b39),(r11,A2,b40),(r11,A2,b41),(r11,A2,b42),(r11,A2,b43),(r11,A2,b44),(r11, A2,b45),(r11,A2,b46),(r11,A2,b47),(r11,A2,b48),(r11,A2,b49),(r11,A2,b50),(r11,A2,b51),(r 11,A2,b52),(r11,A2,b53),(r11,A2,b54),(r11,A2,b55),(r11,A2,b56),(r11,A2,b57),(r11,A2,b58 ),(r11,A2,b59),(r11,A2,b60),(r11,A2,b61),(r11,A2,b62),(r11,A2,b63),(r11,A2,b64),(r11,A2, b65),(r11,A2,b66),(r11,A2,b67),(r11,A2,b68),(r11,A2,b69),(r11,A2,b70),(r11,A2,b71),(r11, A2,b72),(r11,A2,b73),(r11,A2,b74),(r11,A2,b75),(r11,A2,b76),(r11,A2,b77),(r11,A2,b78),(r 11,A2,b79),(r11,A2,b80),(r11,A2,b81),(r11,A2,b82),(r11,A2,b83),(r11,A2,b84),(r11,A2,b85 ),(r11,A2,b86),(r11,A2,b87),(r11,A2,b88),(r11,A2,b89),(r11,A2,b90),(r11,A2,b91),(r11,A2, b92),(r11,A2,b93),(r11,A2,b94),(r11,A2,b95),(r11,A2,b96),(r11,A2,b97),(r11,A2,b98),(r11, A2,b99),(r11,A2,b100),(r11,A2,b101),(r11,A2,b102),(r11,A2,b103),(r11,A2,b104),(r11,A2, b105),(r11,A2,b106),(r11,A2,b107),(r11,A2,b108),(r11,A2,b109),(r11,A2,b110),(r11,A2,b1 11),(r11,A2,b112),(r11,A2,b113),(r11,A2,b114),(r11,A2,b115),(r11,A2,b116),(r11,A2,b117), (r11,A2,b118),(r11,A2,b119),(r11,A2,b120),(r11,A2,b121),(r11,A2,b122),(r11,A2,b123),(r1 1,A2,b124),(r11,A2,b125),(r11,A2,b126),(r11,A2,b127),(r11,A2,b128),(r11,A2,b129),(r11, A2,b130),(r11,A2,b131),(r11,A2,b132),(r11,A2,b133),(r11,A2,b134),
(r11,A3,b1),(r11,A3,b2),(r11,A3,b3),(r11,A3,b4),(r11,A3,b5),(r11,A3,b6),(r11,A3,b7),(r11, A3,b8),(r11,A3,b9),(r11,A3,b10),(r11,A3,b11),(r11,A3,b12),(r11,A3,b13),(r11,A3,b14),(r11, A3,b15),(r11,A3,b16),(r11,A3,b17),(r11,A3,b18),(r11,A3,b19),(r11,A3,b20),(r11,A3,b21),(r 11,A3,b22),(r11,A3,b23),(r11,A3,b24),(r11,A3,b25),(r11,A3,b26),(r11,A3,b27),(r11,A3,b28 ),(r11,A3,b29),(r11,A3,b30),(r11,A3,b31),(r11,A3,b32),(r11,A3,b33),(r11,A3,b34),(r11,A3, b35),(r11,A3,b36),(r11,A3,b37),(r11,A3,b38),(r11,A3,b39),(r11,A3,b40),(r11,A3,b41),(r11, A3,b42),(r11,A3,b43),(r11,A3,b44),(r11,A3,b45),(r11,A3,b46),(r11,A3,b47),(r11,A3,b48),(r 11,A3,b49),(r11,A3,b50),(r11,A3,b51),(r11,A3,b52),(r11,A3,b53),(r11,A3,b54),(r11,A3,b55 ),(r11,A3,b56),(r11,A3,b57),(r11,A3,b58),(r11,A3,b59),(r11,A3,b60),(r11,A3,b61),(r11,A3, b62),(r11,A3,b63),(r11,A3,b64),(r11,A3,b65),(r11,A3,b66),(r11,A3,b67),(r11,A3,b68),(r11, A3,b69),(r11,A3,b70),(r11,A3,b71),(r11,A3,b72),(r11,A3,b73),(r11,A3,b74),(r11,A3,b75),(r 11,A3,b76),(r11,A3,b77),(r11,A3,b78),(r11,A3,b79),(r11,A3,b80),(r11,A3,b81),(r11,A3,b82 ),(r11,A3,b83),(r11,A3,b84),(r11,A3,b85),(r11,A3,b86),(r11,A3,b87),(r11,A3,b88),(r11,A3, b89),(r11,A3,b90),(r11,A3,b91),(r11,A3,b92),(r11,A3,b93),(r11,A3,b94),(r11,A3,b95),(r11, A3,b96),(r11,A3,b97),(r11,A3,b98),(r11,A3,b99),(r11,A3,b100),(r11,A3,b101),(r11,A3,b10 2),(r11,A3,b103),(r11,A3,b104),(r11,A3,b105),(r11,A3,b106),(r11,A3,b107),(r11,A3,b108), (r11,A3,b109),(r11,A3,b110),(r11,A3,b111),(r11,A3,b112),(r11,A3,b113),(r11,A3,b114),(r1 1,A3,b115),(r11,A3,b116),(r11,A3,b117),(r11,A3,b118),(r11,A3,b119),(r11,A3,b120),(r11,A 3,b121),(r11,A3,b122),(r11,A3,b123),(r11,A3,b124),(r11,A3,b125),(r11,A3,b126),(r11,A3,b 127),(r11,A3,b128),(r11,A3,b129),(r11,A3,b130),(r11,A3,b131),(r11,A3,b132),(r11,A3,b13 3),(r11,A3,b134),(r11,A4,b1),(r11,A4,b2),(r11,A4,b3),(r11,A4,b4),(r11,A4,b5),(r11,A4,b6),( r11,A4,b7),(r11,A4,b8),(r11,A4,b9),(r11,A4,b10),(r11,A4,b11),(r11,A4,b12),(r11,A4,b13),(r 11,A4,b14),(r11,A4,b15),(r11,A4,b16),(r11,A4,b17),(r11,A4,b18),(r11,A4,b19),(r11,A4,b20 ),(r11,A4,b21),(r11,A4,b22),(r11,A4,b23),(r11,A4,b24),(r11,A4,b25),(r11,A4,b26),(r11,A4, b27),(r11,A4,b28),(r11,A4,b29),(r11,A4,b30),(r11,A4,b31),(r11,A4,b32),(r11,A4,b33),(r11, A4,b34),(r11,A4,b35),(r11,A4,b36),(r11,A4,b37),(r11,A4,b38),(r11,A4,b39),(r11,A4,b40),(r 11,A4,b41),(r11,A4,b42),(r11,A4,b43),(r11,A4,b44),(r11,A4,b45),(r11,A4,b46),(r11,A4,b47 ),(r11,A4,b48),(r11,A4,b49),(r11,A4,b50),(r11,A4,b51),(r11,A4,b52),(r11,A4,b53),(r11,A4, b54),(r11,A4,b55),(r11,A4,b56),(r11,A4,b57),(r11,A4,b58),(r11,A4,b59),(r11,A4,b60),(r11, A4,b61),(r11,A4,b62),(r11,A4,b63),(r11,A4,b64),(r11,A4,b65),(r11,A4,b66),(r11,A4,b67),(r 11,A4,b68),(r11,A4,b69),(r11,A4,b70),(r11,A4,b71),(r11,A4,b72),(r11,A4,b73),(r11,A4,b74 ),(r11,A4,b75),(r11,A4,b76),(r11,A4,b77),(r11,A4,b78),(r11,A4,b79),(r11,A4,b80),(r11,A4, b81),(r11,A4,b82),(r11,A4,b83),(r11,A4,b84),(r11,A4,b85),(r11,A4,b86),(r11,A4,b87),(r11, A4,b88),(r11,A4,b89),(r11,A4,b90),(r11,A4,b91),(r11,A4,b92),(r11,A4,b93),(r11,A4,b94),(r 11,A4,b95),(r11,A4,b96),(r11,A4,b97),(r11,A4,b98),(r11,A4,b99),(r11,A4,b100),(r11,A4,b1 01),(r11,A4,b102),(r11,A4,b103),(r11,A4,b104),(r11,A4,b105),(r11,A4,b106),(r11,A4,b107) ,(r11,A4,b108),(r11,A4,b109),(r11,A4,b110),(r11,A4,b111),(r11,A4,b112),(r11,A4,b113),(r1 1,A4,b114),(r11,A4,b115),(r11,A4,b116),(r11,A4,b117),(r11,A4,b118),(r11,A4,b119),(r11,A 4,b120),(r11,A4,b121),(r11,A4,b122),(r11,A4,b123),(r11,A4,b124),(r11,A4,b125),(r11,A4,b 126),(r11,A4,b127),(r11,A4,b128),(r11,A4,b129),(r11,A4,b130),(r11,A4,b131),(r11,A4,b13 2),(r11,A4,b133),(r11,A4,b134),(r12,A1,b1),(r12,A1,b2),(r12,A1,b3),(r12,A1,b4),(r12,A1,b 5),(r12,A1,b6),(r12,A1,b7),(r12,A1,b8),(r12,A1,b9),(r12,A1,b10),(r12,A1,b11),(r12,A1,b12 ),(r12,A1,b13),(r12,A1,b14),(r12,A1,b15),(r12,A1,b16),(r12,A1,b17),(r12,A1,b18),(r12,Al, b19),(r12,A1,b20),(r12,A1,b21),(r12,A1,b22),(r12,A1,b23),(r12,A1,b24),(r12,A1,b25),(r12, A1,b26),(r12,A1,b27),(r12,A1,b28),(r12,A1,b29),(r12,A1,b30),(r12,A1,b31),(r12,A1,b32),( r12,A1,b33),(r12,A1,b34),(r12,A1,b35),(r12,A1,b36),(r12,A1,b37),(r12,A1,b38),(r12,A1,b 39),(r12,A1,b40),(r12,A1,b41),(r12,A1,b42),(r12,A1,b43),(r12,A1,b44),(r12,A1,b45),(r12, A1,b46),(r12,A1,b47),(r12,A1,b48),(r12,A1,b49),(r12,A1,b50),(r12,A1,b51),(r12,A1,b52),( r12,A1,b53),(r12,A1,b54),(r12,A1,b55),(r12,A1,b56),(r12,A1,b57),(r12,A1,b58),(r12,A1,b 59),(r12,A1,b60),(r12,A1,b61),(r12,A1,b62),(r12,A1,b63),(r12,A1,b64),(r12,A1,b65),(r12, A1,b66),(r12,A1,b67),(r12,A1,b68),(r12,A1,b69),(r12,A1,b70),(r12,A1,b71),(r12,A1,b72),( r12,A1,b73),(r12,A1,b74),(r12,A1,b75),(r12,A1,b76),(r12,A1,b77),(r12,A1,b78),(r12,A1,b 79),(r12,A1,b80),(r12,A1,b81),(r12,A1,b82),(r12,A1,b83),(r12,A1,b84),(r12,A1,b85),(r12, A1,b86),(r12,A1,b87),(r12,A1,b88),(r12,A1,b89),(r12,A1,b90),(r12,A1,b91),(r12,A1,b92),( r12,A1,b93),(r12,A1,b94),(r12,A1,b95),(r12,A1,b96),(r12,A1,b97),(r12,A1,b98),(r12,A1,b 99),(r12,A1,b100),(r12,A1,b101),(r12,A1,b102),(r12,A1,b103),(r12,A1,b104),(r12,A1,b105 ),(r12,A1,b106),(r12,A1,b107),(r12,A1,b108),(r12,A1,b109),(r12,A1,b110),(r12,A1,b111),( r12,A1,b112),(r12,A1,b113),(r12,A1,b114),(r12,A1,b115),(r12,A1,b116),(r12,A1,b117),(r1 2,A1,b118),(r12,A1,b119),(r12,A1,b120),(r12,A1,b121),(r12,A1,b122),(r12,A1,b123),(r12, A1,b124),(r12,A1,b125),(r12,A1,b126),(r12,A1,b127),(r12,A1,b128),(r12,A1,b129),(r12,A 1,b130),(r12,A1,b131),(r12,A1,b132),(r12,A1,b133),(r12,A1,b134),
(r12,A2,b1),(r12,A2,b2),(r12,A2,b3),(r12,A2,b4),(r12,A2,b5),(r12,A2,b6),(r12,A2,b7),(r12, A2,b8),(r12,A2,b9),(r12,A2,b10),(r12,A2,b11),(r12,A2,b12),(r12,A2,b13),(r12,A2,b14),(r1 2,A2,b15),(r12,A2,b16),(r12,A2,b17),(r12,A2,b18),(r12,A2,b19),(r12,A2,b20),(r12,A2,b21) ,(r12,A2,b22),(r12,A2,b23),(r12,A2,b24),(r12,A2,b25),(r12,A2,b26),(r12,A2,b27),(r12,A2, b28),(r12,A2,b29),(r12,A2,b30),(r12,A2,b31),(r12,A2,b32),(r12,A2,b33),(r12,A2,b34),(r12, A2,b35),(r12,A2,b36),(r12,A2,b37),(r12,A2,b38),(r12,A2,b39),(r12,A2,b40),(r12,A2,b41),( r12,A2,b42),(r12,A2,b43),(r12,A2,b44),(r12,A2,b45),(r12,A2,b46),(r12,A2,b47),(r12,A2,b 48),(r12,A2,b49),(r12,A2,b50),(r12,A2,b51),(r12,A2,b52),(r12,A2,b53),(r12,A2,b54),(r12, A2,b55),(r12,A2,b56),(r12,A2,b57),(r12,A2,b58),(r12,A2,b59),(r12,A2,b60),(r12,A2,b61),( r12,A2,b62),(r12,A2,b63),(r12,A2,b64),(r12,A2,b65),(r12,A2,b66),(r12,A2,b67),(r12,A2,b 68),(r12,-A2,b69),(r12,A2,b70),(r12,A2,b71),(r12,A2,b72),(r12,A2,b73),(r12,A2,b74),(r12, A2,b75),(r12,A2,b76),(r12,A2,b77),(r12,A2,b78),(r12,A2,b79),(r12,A2,b80),(r12,A2,b81),( r12,A2,b82),(r12,A2,b83),(r12,A2,b84),(r12,A2,b85),(r12,A2,b86),(r12,A2,b87),(r12,A2,b 88),(r12,A2,b89),(r12,A2,b90),(r12,A2,b91),(r12,A2,b92),(r12,A2,b93),(r12,A2,b94),(r12, A2,b95),(r12,A2,b96),(r12,A2,b97),(r12,A2,b98),(r12,A2,b99),(r12,A2,b100),(r12,A2,b101 ),(r12,A2,b102),(r12,A2,b103),(r12,A2,b104),(r12,A2,b105),(r12,A2,b106),(r12,A2,b107),( r12,A2,b108),(r12,A2,b109),(r12,A2,b110),(r12,A2,b111),(r12,A2,b112),(r12,A2,b113),(r1 2,A2,b114),(r12,A2,b115),(r12,A2,b116),(r12,A2,b117),(r12,A2,b118),(r12,A2,b119),(r12, A2,b120),(r12,A2,b121),(r12,A2,b122),(r12,A2,b123),(r12,A2,b124),(r12,A2,b125),(r12,A 2,b126),(r12,A2,b127),(r12,A2,b128),(r12,A2,b129),(r12,A2,b130),(r12,A2,b131),(r12,A2, b132),(r12,A2,b133),(r12,A2,b134),(r12,A3,b1),(r12,A3,b2),(r12,A3,b3),(r12,A3,b4),(r12, A3,b5),(r12,A3,b6),(r12,A3,b7),(r12,A3,b8),(r12,A3,b9),(r12,A3,b10),(r12,A3,b11),(r12,A3 ,b12),(r12,A3,b13),(r12,A3,b14),(r12,A3,b15),(r12,A3,b16),(r12,A3,b17),(r12,A3,b18),(r12 ,A3,b19),(r12,A3,b20),(r12,A3,b21),(r12,A3,b22),(r12,A3,b23),(r12,A3,b24),(r12,A3,b25),( r12,A3,b26),(r12,A3,b27),(r12,A3,b28),(r12,A3,b29),(r12,A3,b30),(r12,A3,b31),(r12,A3,b 32),(r12,A3,b33),(r12,A3,b34),(r12,A3,b35),(r12,A3,b36),(r12,A3,b37),(r12,A3,b38),(r12, A3,b39),(r12,A3,b40),(r12,A3,b41),(r12,A3,b42),(r12,A3,b43),(r12,A3,b44),(r12,A3,b45),( r12,A3,b46),(r12,A3,b47),(r12,A3,b48),(r12,A3,b49),(r12,A3,b50),(r12,A3,b51),(r12,A3,b 52),(r12,A3,b53),(r12,A3,b54),(r12,A3,b55),(r12,A3,b56),(r12,A3,b57),(r12,A3,b58),(r12, A3,b59),(r12,A3,b60),(r12,A3,b61),(r12,A3,b62),(r12,A3,b63),(r12,A3,b64),(r12,A3,b65),( r12,A3,b66),(r12,A3,b67),(r12,A3,b68),(r12,A3,b69),(r12,A3,b70),(r12,A3,b71),(r12,A3,b 72),(r12,A3,b73),(r12,A3,b74),(r12,A3,b75),(r12,A3,b76),(r12,A3,b77),(r12,A3,b78),(r12, A3,b79),(r12,A3,b80),(r12,A3,b81),(r12,A3,b82),(r12,A3,b83),(r12,A3,b84),(r12,A3,b85),( r12,A3,b86),(r12,A3,b87),(r12,A3,b88),(r12,A3,b89),(r12,A3,b90),(r12,A3,b91),(r12,A3,b 92),(r12,A3,b93),(r12,A3,b94),(r12,A3,b95),(r12,A3,b96),(r12,A3,b97),(r12,A3,b98),(r12, A3,b99),(r12,A3,b100),(r12,A3,b101),(r12,A3,b102),(r12,A3,b103),(r12,A3,b104),(r12,A3, b105),(r12,A3,b106),(r12,A3,b107),(r12,A3,b108),(r12,A3,b109),(r12,A3,b110),(r12,A3,b1 11),(r12,A3,b112),(r12,A3,b113),(r12,A3,b114),(r12,A3,b115),(r12,A3,b116),(r12,A3,b117) ,(r12,A3,b118),(r12,A3,b119),(r12,A3,b120),(r12,A3,b121),(r12,A3,b122),(r12,A3,b123),(r 12,A3,b124),(r12,A3,b125),(r12,A3,b126),(r12,A3,b127),(r12,A3,b128),(r12,A3,b129),(r12 ,A3,b130),(r12,A3,b131),(r12,A3,b132),(r12,A3,b133),(r12,A3,b134),(r12,A4,b1),(r12,A4, b2),(r12,A4,b3),(r12,A4,b4),(r12,A4,b5),(r12,A4,b6),(r12,A4,b7),(r12,A4,b8),(r12,A4,b9),( r12,A4,b10),(r12,A4,b11),(r12,A4,b12),(r12,A4,b13),(r12,A4,b14),(r12,A4,b15),(r12,A4,b1 6),(r12,A4,b17),(r12,A4,b18),(r12,A4,b19),(r12,A4,b20),(r12,A4,b21),(r12,A4,b22),(r12,A 4,b23),(r12,A4,b24),(r12,A4,b25),(r12,A4,b26),(r12,A4,b27),(r12,A4,b28),(r12,A4,b29),(r1 2,A4,b30),(r12,A4,b31),(r12,A4,b32),(r12,A4,b33),(r12,A4,b34),(r12,A4,b35),(r12,A4,b36) ,(r12,A4,b37),(r12,A4,b38),(r12,A4,b39),(r12,A4,b40),(r12,A4,b41),(r12,A4,b42),(r12,A4, b43),(r12,A4,b44),(r12,A4,b45),(r12,A4,b46),(r12,A4,b47),(r12,A4,b48),(r12,A4,b49),(r12, A4,b50),(r12,A4,b51),(r12,A4,b52),(r12,A4,b53),(r12,A4,b54),(r12,A4,b55),(r12,A4,b56),( r12,A4,b57),(r12,A4,b58),(r12,A4,b59),(r12,A4,b60),(r12,A4,b61),(r12,A4,b62),(r12,A4,b 63),(r12,A4,b64),(r12,A4,b65),(r12,A4,b66),(r12,A4,b67),(r12,A4,b68),(r12,A4,b69),(r12, A4,b70),(r12,A4,b71),(r12,A4,b72),(r12,A4,b73),(r12,A4,b74),(r12,A4,b75),(r12,A4,b76),( r12,A4,b77),(r12,A4,b78),(r12,A4,b79),(r12,A4,b80),(r12,A4,b81),(r12,A4,b82),(r12,A4,b 83),(r12,A4,b84),(r12,A4,b85),(r12,A4,b86),(r12,A4,b87),(r12,A4,b88),(r12,A4,b89),(r12, A4,b90),(r12,A4,b91),(r12,A4,b92),(r12,A4,b93),(r12,A4,b94),(r12,A4,b95),(r12,A4,b96),( r12,A4,b97),(r12,A4,b98),(r12,A4,b99),(r12,A4,b100),(r12,A4,b101),(r12,A4,b102),(r12,A 4,b103),(r12,A4,b104),(r12,A4,b105),(r12,A4,b106),(r12,A4,b107),(r12,A4,b108),(r12,A4, b109),(r12,A4,b110),(r12,A4,b111),(r12,A4,b112),(r12,A4,b113),(r12,A4,b114),(r12,A4,b1 15),(r12,A4,b116),(r12,A4,b117),(r12,A4,b118),(r12,A4,b119),(r12,A4,b120),(r12,A4,b121 ),(r12,A4,b122),(r12,A4,b123),(r12,A4,b124),(r12,A4,b125),(r12,A4,b126),(r12,A4,b127),( r12,A4,b128),(r12,A4,b129),(r12,A4,b130),(r12,A4,b131),(r12,A4,b132),(r12,A4,b133),(r1 2,A4,b134),
(r13,A1,b1),(r13,A1,b2),(r13,A1,b3),(r13,A1,b4),(r13,A1,b5),(r13,A1,b6),(r13,A1,b7),(r13, A1,b8),(r13,A1,b9),(r13,A1,b10),(r13,A1,b11),(r13,A1,b12),(r13,A1,b13),(r13,A1,b14),(r1 3,A1,b15),(r13,A1,b16),(r13,A1,b17),(r13,A1,b18),(r13,A1,b19),(r13,A1,b20),(r13,A1,b21) ,(r13,A1,b22),(r13,A1,b23),(r13,A1,b24),(r13,A1,b25),(r13,A1,b26),(r13,A1,b27),(r13,A1, b28),(r13,A1,b29),(r13,A1,b30),(r13,A1,b31),(r13,A1,b32),(r13,A1,b33),(r13,A1,b34),(r13, A1,b35),(r13,A1,b36),(r13,A1,b37),(r13,A1,b38),(r13,A1,b39),(r13,A1,b40),(r13,A1,b41),( r13,A1,b42),(r13,A1,b43),(r13,A1-b44),(r13,A1,b45),(r13,A1,b46),(r13,A1,b47),(r13,A1,b 48),(r13,A1,b49),(r13,A1,b50),(r13,A1,b51),(r13,A1,b52),(r1A,A1,b53),(r13,A1,b54),(r13, A1,b55),(r13,A1,b56),(r13,A1,b57),(r13,A1,b58),(r13,A1,b59),(r13,A1,b60),(r13,A1,b61),( r13,A1,b62),(r13,A1,b63),(r13,A1,b64),(r13,A1,b65),(r13,A1,b66),(r13,A1,b67),(r13,A1,b 68),(r13,A1,b69),(r13,A1,b70),(r13,A1,b71),(r13,A1,b72),(r13,A1,b73),(r13,A1,b74),(r13, A1,b75),(r13,A1,b76),(r13,A1,b77),(r13,A1,b78),(r13,A1,b79),(r13,A1,b80),(r13,A1,b81),( r13,A1,b82),(r13,A1,b83),(r13,A1,b84),(r13,A1,b85),(r13,A1,b86),(r13,A1,b87),(r13,Al,b 88),(r13,A1,b89),(r13,A1,b90),(r13,A1,b91),(r13,A1,b92),(r13,A1,b93),(r13,A1,b94),(r13, A1,b95),(r13,A1,b96),(r13,A1,b97),(r13,A1,b98),(r13,A1,b99),(r13,A1,b100),(r13,A1,b101 ),(r13,A1,b102),(r13,A1,b103),(r13,A1,b104),(r13,A1,b105),(r13,A1,b106),(r13,A1,b107),( r13,A1,b108),(r13,A1,b109),(r13,A1,b110),(r13,A1,b111),(r13,A1,b112),(r13,A1,b113),(r1 3,A1,b114),(r13,A1,b115),(r13,A1,b116),(r13,A1,b117),(r13,A1,b118),(r13,A1,b119),(r13, A1,b120),(r13,A1,b121),(r13,A1,b122),(r13,A1,b123),(r13,A1,b124),(r13,A1,b125),(r13,A 1,b126),(r13,A1,b127),(r13,A1,b128),(r13,A1,b129),(r13,A1,b130),(r13,A1,b131),(r13,A1, b132),(r13,A1,b133),(r13,A1,b134),(r13,A2,b1),(r13,A2,b2),(r13,A2,b3),(r13,A2,b4),(r13, A2,b5),(r13,A2,b6),(r13,A2,b7),(r13,A2,b8),(r13,A2,b9),(r13,A2,b10),(r13,A2,b11),(r13,A2 ,b12),(r13,A2,b13),(r13,A2,b14),(r13,A2,b15),(r13,A2,b16),(r13,A2,b17),(r13,A2,b18),(r13 ,A2,b19),(r13,A2,b20),(r13,A2,b21),(r13,A2,b22),(r13,A2,b23),(r13,A2,b24),(r13,A2,b25),( r13,A2,b26),(r13,A2,b27),(r13,A2,b28),(r13,A2,b29),(r13,A2,b30),(r13,A2,b31),(r13,A2,b 32),(r13,A2,b33),(r13,A2,b34),(r13,A2,b35),(r13,A2,b36),(r13,A2,b37),(r13,A2,b38),(r13, A2,b39),(r13,A2,b40),(r13,A2,b41),(r13,A2,b42),(r13,A2,b43),(r13,A2,b44),(r13,A2,b45),( r13,A2,b46),(r13,A2,b47),(r13,A2,b48),(r13,A2,b49),(r13,A2,b50),(r13,A2,b51),(r13,A2,b 52),(r13,A2,b53),(r13,A2,b54),(r13,A2,b55),(r13,A2,b56),(r13,A2,b57),(r13,A2,b58),(r13, A2,b59),(r13,A2,b60),(r13,A2,b61),(r13,A2,b62),(r13,A2,b63),(r13,A2,b64),(r13,A2,b65),( r13,A2,b66),(r13,A2,b67),(r13,A2,b68),(r13,A2,b69),(r13,A2,b70),(r13,A2,b71),(r13,A2,b 72),(r13,A2,b73),(r13,A2,b74),(r13,A2,b75),(r13,A2,b76),(r13,A2,b77),(r13,A2,b78),(r13, A2,b79),(r13,A2,b80),(r13,A2,b81),(r13,A2,b82),(r13,A2,b83),(r13,A2,b84),(r13,A2,b85),( r13,A2,b86),(r13,A2,b87),(r13,A2,b88),(r13,A2,b89),(r13,A2,b90),(r13,A2,b91),(r13,A2,b 92),(r13,A2,b93),(r13,A2,b94),(r13,A2,b95),(r13,A2,b96),(r13,A2,b97),(r13,A2,b98),(r13, A2,b99),(r13,A2,b100),(r13,A2,b101),(r13,A2,b102),(r13,A2,b103),(r13,A2,b104),(r13,A2, b105),(r13,A2,b106),(r13,A2,b107),(r13,A2,b108),(r13,A2,b109),(r13,A2,b110),(r13,A2,b1 11),(r13,A2,b112),(r13,A2,b113),(r13,A2,b114),(r13,A2,b115),(r13,A2,b116),(r13,A2,b117) ,(r13,A2,b118),(r13,A2,b119),(r13,A2,b120),(r13,A2,b121),(r13,A2,b122),(r13,A2,b123),(r 13,A2,b124),(r13,A2,b125),(r13,A2,b126),(r13,A2,b127),(r13,A2,b128),(r13,A2,b129),(r13 ,A2,b130),(r13,A2,b131),(r13,A2,b132),(r13,A2,b133),(r13,A2,b134),(r13,A3,b1),(r13,A3, b2),(r13,A3,b3),(r13,A3,b4),(r13,A3,b5),(r13,A3,b6),(r13,A3,b7),(r13,A3,b8),(r13,A3,b9),( r13,A3,b10),(r13,A3,b11),(r13,A3,b12),(r13,A3,b13),(r13,A3,b14),(r13,A3,b15),(r13,A3,b1 6),(r13,A3,b17),(r13,A3,b18),(r13,A3,b19),(r13,A3,b20),(r13,A3,b21),(r13,A3,b22),(r13,A 3,b23),(r13,A3,b24),(r13,A3,b25),(r13,A3,b26),(r13,A3,b27),(r13,A3,b28),(r13,A3,b29),(r1 3,A3,b30),(r13,A3,b31),(r13,A3,b32),(r13,A3,b33),(r13,A3,b34),(r13,A3,b35),(r13,A3,b36) ,(r13,A3,b37),(r13,A3,b38),(r13,A3,b39),(r13,A3,b40),(r13,A3,b41),(r13,A3,b42),(r13,A3, b43),(r13,A3,b44),(r13,A3,b45),(r13,A3,b46),(r13,A3,b47),(r13,A3,b48),(r13,A3,b49),(r13, A3,b50),(r13,A3,b51),(r13,A3,b52),(r13,A3,b53),(r13,A3,b54),(r13,A3,b55),(r13,A3,b56),( r13,A3,b57),(r13,A3,b58),(r13,A3,b59),(r13,A3,b60),(r13,A3,b61),(r13,A3,b62),(r13,A3,b 63),(r13,A3,b64),(r13,A3,b65),(r13,A3,b66),(r13,A3,b67),(r13,A3,b68),(r13,A3,b69),(r13, A3,b70),(r13,A3,b71),(r13,A3,b72),(r13,A3,b73),(r13,A3,b74),(r13,A3,b75),(r13,A3,b76),( r13,A3,b77),(r13,A3,b78),(r13,A3,b79),(r13,A3,b80),(r13,A3,b81),(r13,A3,b82),(r13,A3,b 83),(r13,A3,b84),(r13,A3,b85),(r13,A3,b86),(r13,A3,b87),(r13,A3,b88),(r13,A3,b89),(r13, A3,b90),(r13,A3,b91),(r13,A3,b92),(r13,A3,b93),(r13,A3,b94),(r13,A3,b95),(r13,A3,b96),( r13,A3,b97),(r13,A3,b98),(r13,A3,b99),(r13,A3,b100),(r13,A3,b101),(r13,A3,b102),(r13,A 3,b103),(r13,A3,b104),(r13,A3,b105),(r13,A3,b106),(r13,A3,b107),(r13,A3,b108),(r13,A3, b109),(r13,A3,b110),(r13,A3,b111),(r13,A3,b112),(r13,A3,b113),(r13,A3,b114),(r13,A3,b1 15),(r13,A3,b116),(r13,A3,b117),(r13,A3,b118),(r13,A3,b119),(r13,A3,b120),(r13,A3,b121 ),(r13,A3,b122),(r13,A3,b123),(r13,A3,b124),(r13,A3,b125),(r13,A3,b126),(r13,A3,b127),( r13,A3,b128),(r13,A3,b129),(r13,A3,b130),(r13,A3,b131),(r13,A3,b132),(r13,A3,b133),(r1 3,A3,b134),
(r13,A4,b1),(r13,A4,b2),(r13,A4,b3),(r13,A4,b4),(r13,A4,b5),(r13,A4,b6),(r13,A4,b7),(r13, A4,b8),(r13,A4,b9),(r13,A4,b10),(r13,A4,b11),(r13,A4,b12),(r13,A4,b13),(r13,A4,b14),(r1 3,A4,b15),(r13,A4,b16),(r13,A4,b17),(r13,A4,b18),(r13,A4,b19),(r13,A4,b20),(r13,A4,b21) ,(r13,A4,b22),(r13,A4,b23),(r13,A4,b24),(r13,A4,b25),(r13,A4,b26),(r13,A4,b27),(r13,A4, b28),(r13,A4,b29),(r13,A4,b30),(r13,A4,b31),(r13,A4,b32),(r13,A4,b33),(r13,A4,b34),(r13, A4,b35),(r13,A4,b36),(r13,A4,b37),(r13,A4,b38),(r13,A4,b39),(r13,A4,b40),(r13,A4,b41),( r13,A4,b42),(r13,A4,b43),(r13,A4,b44),(r13,A4,b45),(r13,A4,b46),(r13,A4,b47),(r13,A4,b 48),(r13,A4,b49),(r13,A4,b50),(r13,A4,b51),(r13,A4,b52),(r13,A4,b53),(r13,A4,b54),(r13, A4,b55),(r13,A4,b56),(r13,A4,b57),(r13,A4,b58),(r13,A4,b59),(r13,A4,b60),(r13,A4,b61),( r13,A4,b62),(r13,A4,b63),(r13,A4,b64),(r13,A4,b65),(r13,A4,b66),(r13,A4,b67),(r13,A4,b 68),(r13,A4,b69),(r13,A4,b70),(r13,A4,b71),(r13,A4,b72),(r13,A4,b73),(r13,A4,b74),(r13, A4,b75),(r13,A4,b76),(r13,A4,b77),(r13,A4,b78),(r13,A4,b79),(r13,A4,b80),(r13,A4,b81),( r13,A4,b82),(r13,A4,b83),(r13,A4,b84),(r13,A4,b85),(r13,A4,b86),(r13,A4,b87),(r13,A4,b 88),(r13,A4,b89),(r13,A4,b90),(r13,A4,b91),(r13,A4,b92),(r13,A4,b93),(r13,A4,b94),(r13, A4,b95),(r13,A4,b96),(r13,A4,b97),(r13,A4,b98),(r13,A4,b99),(r13,A4,b100),(r13,A4,b101 ),(r13,A4,b102),(r13,A4,b103),(r13,A4,b104),(r13,A4,b105),(r13,A4,b106),(r13,A4,b107),( r13,A4,b108),(r13,A4,b109),(r13,A4,b110),(r13,A4,b111),(r13,A4,b112),(r13,A4,b113),(r1 3,A4,b114),(r13,A4,b115),(r13,A4,b116),(r13,A4,b117),(r13,A4,b118),(r13,A4,b119),(r13, A4,b120),(r13,A4,b121),(r13,A4,b122),(r13,A4,b123),(r13,A4,b124),(r13,A4,b125),(r13,A 4,b126),(r13,A4,b127),(r13,A4,b128),(r13,A4,b129),(r13,A4,b130),(r13,A4,b131),(r13,A4, b132),(r13,A4,b133),(r13,A4,b134),(r14,A1,b1),(r14,A1,b2),(r14,A1,b3),(r14,A1,b4),(r14, A1,b5),(r14,A1,b6),(r14,A1,b7),(r14,A1,b8),(r14,A1,b9),(r14,A1,b10),(r14,A1,b11),(r14,A1 ,b12),(r14,A1,b13),(r14,A1,b14),(r14,A1,b15),(r14,A1,b16),(r14,A1,b17),(r14,A1,b18),(r14 ,A1,b19),(r14,A1,b20),(r14,A1,b21),(r14,A1,b22),(r14,A1,b23),(r14,A1,b24),(r14,A1,b25),( r14,A1,b26),(r14,A1,b27),(r14,A1,b28),(r14,A1,b29),(r14,A1,b30),(r14,A1,b31),(r14,A1,b 32),(r14,A1,b33),(r14,A1,b34),(r14,A1,b35),(r14,A1,b36),(r14,A1,b37),(r14,A1,b38),(r14, A1,b39),(r14,A1,b40),(r14,A1,b41),(r14,A1,b42),(r14,A1,b43),(r14,A1,b44),(r14,A1,b45),( r14,A1,b46),(r14,A1,b47),(r14,A1,b48),(r14,A1,b49),(r14,A1,b50),(r14,A1,b51),(r14,A1,b 52),(r14,A1,b53),(r14,A1,b54),(r14,A1,b55),(r14,A1,b56),(r14,A1,b57),(r14,A1,b58),(r14, Al,b59),(r14,A1,b60),(r14,A1,b61),(r14,A1,b62),(r14,A1,b63),(r14,A1,b64),(r14,A1,b65),( r14,A1,b66),(r14,A1,b67),(r14,A1,b68),(r14,A1,b69),(r14,A1,b70),(r14,A1,b71),(r14,A1,b 72),(r14,A1,b73),(r14,A1,b74),(r14,A1,b75),(r14,A1,b76),(r14,A1,b77),(r14,A1,b78),(r14, A1,b79),(r14,A1,b80),(r14,A1,b81),(r14,A1,b82),(r14,A1,b83),(r14,A1,b84),(r14,A1,b85),( r14,A1,b86),(r14,A1,b87),(r14,A1,b88),(r14,A1,b89),(r14,A1,b90),(r14,A1,b91),(r14,A1,b 92),(r14,A1,b93),(r14,A1,b94),(r14,A1,b95),(r14,A1,b96),(r14,A1,b97),(r14,A1,b98),(r14, A1,b99),(r14,A1,b100),(r14,A1,b101),(r14,A1,b102),(r14,A1,b103),(r14,A1,b104),(r14,A1, b105),(r14,A1,b106),(r14,A1,b107),(r14,A1,b108),(r14,A1,b109),(r14,A1,b110),(r14,A1,b1 11),(r14,A1,b112),(r14,A1,b113),(r14,A1,b114),(r14,A1,b115),(r14,A1,b116),(r14,A1,b117) ,(r14,A1,b118),(r14,A1,b119),(r14,A1,b120),(r14,A1,b121),(r14,A1,b122),(r14,A1,b123),(r 14,A1,b124),(r14,A1,b125),(r14,A1,b126),(r14,A1,b127),(r14,A1,b128),(r14,A1,b129),(r14 ,A1,b130),(r14,A1,b131),(r14,A1,b132),(r14,A1,b133),(r14,A1,b134),(r14,A2,b1),(r14,A2, b2),(r14,A2,b3),(r14,A2,b4),(r14,A2,b5),(r14,A2,b6),(r14,A2,b7),(r14,A2,b8),(r14,A2,b9),( r14,A2,b10),(r14,A2,b11),(r14,A2,b12),(r14,A2,b13),(r14,A2,b14),(r14,A2,b15),(r14,A2,b1 6),(r14,A2,b17),(r14,A2,b18),(r14,A2,b19),(r14,A2,b20),(r14,A2,b21),(r14,A2,b22),(r14,A 2,b23),(r14,A2,b24),(r14,A2,b25),(r14,A2,b26),(r14,A2,b27),(r14,A2,b28),(r14,A2,b29),(r1 4,A2,b30),(r14,A2,b31),(r14,A2,b32),(r14,A2,b33),(r14,A2,b34),(r14,A2,b35),(r14,A2,b36) ,(r14,A2,b37),(r14,A2,b38),(r14,A2,b39),(r14,A2,b40),(r14,A2,b41),(r14,A2,b42),(r14,A2, b43),(r14,A2,b44),(r14,A2,b45),(r14,A2,b46),(r14,A2,b47),(r14,A2,b48),(r14,A2,b49),(r14, A2,b50),(r14,A2,b51),(r14,A2,b52),(r14,A2,b53),(r14,A2,b54),(r14,A2,b55),(r14,A2,b56),( r14,A2,b57),(r14,A2,b58),(r14,A2,b59),(r14,A2,b60),(r14,A2,b61),(r14,A2,b62),(r14,A2,b 63),(r14,A2,b64),(r14,A2,b65),(r14,A2,b66),(r14,A2,b67),(r14,A2,b68),(r14,A2,b69),(r14, A2,b70),(r14,A2,b71),(r14,A2,b72),(r14,A2,b73),(r14,A2,b74),(r14,A2,b75),(r14,A2,b76),( r14,A2,b77),(r14,A2,b78),(r14,A2,b79),(r14,A2,b80),(r14,A2,b81),(r14,A2,b82),(r14,A2,b 83),(r14,A2,b84),(r14,A2,b85),(r14,A2,b86),(r14,A2,b87),(r14,A2,b88),(r14,A2,b89),(r14, A2,b90),(r14,A2,b91),(r14,A2,b92),(r14,A2,b93),(r14,A2,b94),(r14,A2,b95),(r14,A2,b96),( r14,A2,b97),(r14,A2,b98),(r14,A2,b99),(r14,A2,b100),(r14,A2,b101),(r14,A2,b102),(r14,A 2,b103),(r14,A2,b104),(r14,A2,b105),(r14,A2,b106),(r14,A2,b107),(r14,A2,b108),(r14,A2, b109),(r14,A2,b110),(r14,A2,b111),(r14,A2,b112),(r14,A2,b113),(r14,A2,b114),(r14,A2,b1 15),(r14,A2,b116),(r14,A2,b117),(r14,A2,b118),(r14,A2,b119),(r14,A2,b120),(r14,A2,b121 ),(r14,A2,b122),(r14,A2,b123),(r14,A2,b124),(r14,A2,b125),(r14,A2,b126),(r14,A2,b127),( r14,A2,b128),(r14,A2,b129),(r14,A2,b130),(r14,A2,b131),(r14,A2,b132),(r14,A2,b133),(r1 4,A2,b134),
(r14,A3,b1),(r14,A3,b2),(r14,A3,b3),(r14,A3,b4),(r14,A3,b5),(r14,A3,b6),(r14,A3,b7),(r14, A3,b8),(r14,A3,b9),(r14,A3,b10),(r14,A3,b11),(r14,A3,b12),(r14,A3,b13),(r14,A3,b14),(r1 4,A3,b15),(r14,A3,b16),(r14,A3,b17),(r14,A3,b18),(r14,A3,b19),(r14,A3,b20),(r14,A3,b21) ,(r14,A3,b22),(r14,A3,b23),(r14,A3,b24),(r14,A3,b25),(r14,A3,b26),(r14,A3,b27),(r14,A3, b28),(r14,A3,b29),(r14,A3,b30),(r14,A3,b31),(r14,A3,b32),(r14,A3,b33),(r14,A3,b34),(r14, A3,b35),(r14,A3,b36),(r14,A3,b37),(r14,A3,b38),(r14,A3,b39),(r14,A3,b40),(r14,A3,b41),( r14,A3,b42),(r14,A3,b43),(r14,A3,b44),(r14,A3,b45),(r14,A3,b46),(r14,A3,b47),(r14,A3,b 48),(r14,A3,b49),(r14,A3,b50),(r14,A3,b51),(r14,A3,b52),(r14,A3,b53),(r14,A3,b54),(r14, A3,b55),(r14,A3,b56),(r14,A3,b57),(r14,A3,b58),(r14,A3,b59),(r14,A3,b60),(r14,A3,b61),( r14,A3,b62),(r14,A3,b63),(r14,A3,b64),(r14,A3,b65),(r14,A3,b66),(r14,A3,b67),(r14,A3,b 68),(r14,A3,b69),(r14,A3,b70),(r14,A3,b71),(r14,A3,b72),(r14,A3,b73),(r14,A3,b74),(r14, A3,b75),(r14,A3,b76),(r14,A3,b77),(r14,A3,b78),(r14,A3,b79),(r14,A3,b80),(r14,A3,b81),( r14,A3,b82),(r14,A3,b83),(r14,A3,b84),(r14,A3,b85),(r14,A3,b86),(r14,A3,b87),(r14,A3,b 88),(r14,A3,b89),(r14,A3,b90),(r14,A3,b91),(r14,A3,b92),(r14,A3,b93),(r14,A3,b94),(r14, A3,b95),(r14,A3,b96),(r14,A3,b97),(r14,A3,b98),(r14,A3,b99),(r14,A3,b100),(r14,A3,b101 ),(r14,A3,b102),(r14,A3,b103),(r14,A3,b104),(r14,A3,b105),(r14,A3,b106),(r14,A3,b107),( r14,A3,b108),(r14,A3,b109),(r14,A3,b110),(r14,A3,b111),(r14,A3,b112),(r14,A3,b113),(r1 4,A3,b114),(r14,A3,b115),(r14,A3,b116),(r14,A3,b117),(r14,A3,b118),(r14,A3,b119),(r14, A3,b120),(r14,A3,b121),(r14,A3,b122),(r14,A3,b123),(r14,A3,b124),(r14,A3,b125),(r14,A 3,b126),(r14,A3,b127),(r14,A3,b128),(r14,A3,b129),(r14,A3,b130),(r14,A3,b131),(r14,A3, b132),(r14,A3,b133),(r14,A3,b134),(r14,A4,b1),(r14,A4,b2),(r14,A4,b3),(r14,A4,b4),(r14, A4,b5),(r14,A4,b6),(r14,A4,b7),(r14,A4,b8),(r14,A4,b9),(r14,A4,b10),(r14,A4,b11),(r14,A4 ,b12),(r14,A4,b13),(r14,A4,b14),(r14,A4,b15),(r14,A4,b16),(r14,A4,b17),(r14,A4,b18),(r14 ,A4,b19),(r14,A4,b20),(r14,A4,b21),(r14,A4,b22),(r14,A4,b23),(r14,A4,b24),(r14,A4,b25),( r14,A4,b26),(r14,A4,b27),(r14,A4,b28),(r14,A4,b29),(r14,A4,b30),(r14,A4,b31),(r14,A4,b 32),(r14,A4,b33),(r14,A4,b34),(r14,A4,b35),(r14,A4,b36),(r14,A4,b37),(r14,A4,b38),(r14, A4,b39),(r14,A4,b40),(r14,A4,b41),(r14,A4,b42),(r14,A4,b43),(r14,A4,b44),(r14,A4,b45),( r14,A4,b46),(r14,A4,b47),(r14,A4,b48),(r14,A4,b49),(r14,A4,b50),(r14,A4,b51),(r14,A4,b 52),(r14,A4,b53),(r14,A4,b54),(r14,A4,b55),(r14,A4,b56),(r14,A4,b57),(r14,A4,b58),(r14, A4,b59),(r14,A4,b60),(r14,A4,b61),(r14,A4,b62),(r14,A4,b63),(r14,A4,b64),(r14,A4,b65),( r14,A4,b66),(r14,A4,b67),(r14,A4,b68),(r14,A4,b69),(r14,A4,b70),(r14,A4,b71),(r14,A4,b 72),(r14,A4,b73),(r14,A4,b74),(r14,A4,b75),(r14,A4,b76),(r14,A4,b77),(r14,A4,b78),(r14, A4,b79),(r14,A4,b80),(r14,A4,b81),(r14,A4,b82),(r14,A4,b83),(r14,A4,b84),(r14,A4,b85),( r14,A4,b86),(r14,A4,b87),(r14,A4,b88),(r14,A4,b89),(r14,A4,b90),(r14,A4,b91),(r14,A4,b 92),(r14,A4,b93),(r14,A4,b94),(r14,A4,b95),(r14,A4,b96),(r14,A4,b97),(r14,A4,b98),(r14, A4,b99),(r14,A4,b100),(r14,A4,b101),(r14,A4,b102),(r14,A4,b103),(r14,A4,b104),(r14,A4, b105),(r14,A4,b106),(r14,A4,b107),(r14,A4,b108),(r14,A4,b109),(r14,A4,b110),(r14,A4,b1 11),(r14,A4,b112),(r14,A4,b113),(r14,A4,b114),(r14,A4,b115),(r14,A4,b116),(r14,A4,b117) ,(r14,A4,b118),(r14,A4,b119),(r14,A4,b120),(r14,A4,b121),(r14,A4,b122),(r14,A4,b123),(r 14,A4,b124),(r14,A4,b125),(r14,A4,b126),(r14,A4,b127),(r14,A4,b128),(r14,A4,b129),(r14 ,A4,b130),(r14,A4,b131),(r14,A4,b132),(r14,A4,b133),(r14,A4,b134),(r15,A1,b1),(r15,A1, b2),(r15,A1,b3),(r15,A1,b4),(r15,A1,b5),(r15,A1,b6),(r15,A1,b7),(r15,A1,b8),(r15,A1,b9),( r15,A1,b10),(r15,A1,b11),(r15,A1,b12),(r15,A1,b13),(r15,A1,b14),(r15,A1,b15),(r15,A1,b1 6),(r15,A1,b17),(r15,A1,b18),(r15,A1,b19),(r15,A1,b20),(r15,A1,b21),(r15,A1,b22),(r15,A 1,b23),(r15,A1,b24),(r15,A1,b25),(r15,A1,b26),(r15,A1,b27),(r15,A1,b28),(r15,A1,b29),(r1 5,A1,b30),(r15,A1,b31),(r15,A1,b32),(r15,A1,b33),(r15,A1,b34),(r15,A1,b35),(r15,A1,b36) ,(r15,A1,b37),(r15,A1,b38),(r15,A1,b39),(r15,A1,b40),(r15,A1,b41),(r15,A1,b42),(r15,A1, b43),(r15,A1,b44),(r15,A1,b45),(r45,A1,b46),(r15,A1,b47),(r15,A1,b48),(r15,A1,b49),(r15, A1,b50),(r15,A1,b51),(r15,A1,b52),(r15,A1,b53),(r15,A1,b54),(r15,A1,b55),(r15,A1,b56),( r15,A1,b57),(r15,A1,b58),(r15,A1,b59),(r15,A1,b60),(r15,A1,b61),(r15,A1,b62),(r15,A1,b 63),(r15,A1,b64),(r15,A1,b65),(r15,A1,b66),(r15,A1,b67),(r15,A1,b68),(r15,A1,b69),(r15, A1,b70),(r15,A1,b71),(r15,A1,b72),(r15,A1,b73),(r15,A1,b74),(r15,A1,b75),(r15,A1,b76),( r15,A1,b77),(r15,A1,b78),(r15,A1,b79),(r15,A1,b80),(r15,A1,b81),(r15,A1,b82),(r15,Al,b 83),(r15,A1,b84),(r15,A1,b85),(r15,A1,b86),(r15,A1,b87),(r15,A1,b88),(r15,A1,b89),(r15, A1,b90),(r15,A1,b91),(r15,A1,b92),(r15,A1,b93),(r15,A1,b94),(r15,A1,b95),(r15,A1,b96),( r15,A1,b97),(r15,A1,b98),(r15,A1,b99),(r15,A1,b100),(r15,A1,b101),(r15,A1,b102),(r15,A 1,b103),(r15,A1,b104),(r15,A1,b105),(r15,A1,b106),(r15,A1,b107),(r15,A1,b108),(r15,A1, b109),(r15,A1,b110),(r15,A1,b111),(r15,A1,b112),(r15,A1,b113),(r15,A1,b114),(r15,A1,b1 15),(r15,A1,b116),(r15,A1,b117),(r15,A1,b118),(r15,A1,b119),(r15,A1,b120),(r15,A1,b121 ),(r15,A1,b122),(r15,A1,b123),(r15,A1,b124),(r15,A1,b125),(r15,A1,b126),(r15,A1,b127),( r15,A1,b128),(r15,A1,b129),(r15,A1,b130),(r15,A1,b131),(r15,A1,b132),(r15,A1,b133),(r1 5,A1,b134),
(r15,A2,b1),(r15,A2,b2),(r15,A2,b3),(r15,A2,b4),(r15,A2,b5),(r15,A2,b6),(r15,A2,b7),(r15, A2,b8),(r15,A2,b9),(r15,A2,b10),(r15,A2,b11),(r15,A2,b12),(r15,A2,b13),(r15,A2,b14),(r1 5,A2,b15),(r15,A2,b16),(r15,A2,b17),(r15,A2,b18),(r15,A2,b19),(r15,A2,b20),(r15,A2,b21) ,(r15,A2,b22),(r15,A2,b23),(r15,A2,b24),(r15,A2,b25),(r15,A2,b26),(r15,A2,b27),(r15,A2, b28),(r15,A2,b29),(r15,A2,b30),(r15,A2,b31),(r15,A2,b32),(r15,A2,b33),(r15,A2,b34),(r15, A2,b35),(r15,A2,b36),(r15,A2,b37),(r15,A2,b38),(r15,A2,b39),(r15,A2,b40),(r15,A2,b41),( r15,A2,b42),(r15,A2,b43),(r15,A2,b44),(r15,A2,b45),(r15,A2,b46),(r15,A2,b47),(r15,A2,b 48),(r15,A2,b49),(r15,A2,b50),(r15,A2,b51),(r15,A2,b52),(r15,A2,b53),(r15,A2,b54),(r15, A2,b55),(r15,A2,b56),(r15,A2,b57),(r15,A2,b58),(r15,A2,b59),(r15,A2,b60),(r15,A2,b61),( r15,A2,b62),(r15,A2,b63),(r15,A2,b64),(r15,A2,b65),(r15,A2,b66),(r15,A2,b67),(r15,A2,b 68),(r15,A2,b69),(r15,A2,b70),(r15,A2,b71),(r15,A2,b72),(r15,A2,b73),(r15,A2,b74),(r15, A2,b75),(r15,A2,b76),(r15,A2,b77),(r15,A2,b78),(r15,A2,b79),(r15,A2,b80),(r15,A2,b81),( r15,A2,b82),(r15,A2,b83),(r15,A2,b84),(r15,A2,b85),(r15,A2,b86),(r15,A2,b87),(r15,A2,b 88),(r15,A2,b89),(r15,A2,b90),(r15,A2,b91),(r15,A2,b92),(r15,A2,b93),(r15,A2,b94),(r15, A2,b95),(r15,A2,b96),(r15,A2,b97),(r15,A2,b98),(r15,A2,b99),(r15,A2,b100),(r15,A2,b101 ),(r15,A2,b102),(r15,A2,b103),(r15,A2,b104),(r15,A2,b105),(r15,A2,b106),(r15,A2,b107),( r15,A2,b108),(r15,A2,b109),(r15,A2,b110),(r15,A2,b111),(r15,A2,b112),(r15,A2,b113),(r1 5,A2,b114),(r15,A2,b115),(r15,A2,b116),(r15,A2,b117),(r15,A2,b118),(r15,A2,b119),(r15, A2,b120),(r15,A2,b121),(r15,A2,b122),(r15,A2,b123),(r15,A2,b124),(r15,A2,b125),(r15,A 2,b126),(r15,A2,b127),(r15,A2,b128),(r15,A2,b129),(r15,A2,b130),(r15,A2,b131),(r15,A2, b132),(r15,A2,b133),(r15,A2,b134),(r15,A3,b1),(r15,A3,b2),(r15,A3,b3),(r15,A3,b4),(r15, A3,b5),(r15,A3,b6),(r15,A3,b7),(r15,A3,b8),(r15,A3,b9),(r15,A3,b10),(r15,A3,b11),(r15,A3 ,b12),(r15,A3,b13),(r15,A3,b14),(r15,A3,b15),(r15,A3,b16),(r15,A3,b17),(r15,A3,b18),(r15 ,A3,b19),(r15,A3,b20),(r15,A3,b21),(r15,A3,b22),(r15,A3,b23),(r15,A3,b24),(r15,A3,b25),( r15,A3,b26),(r15,A3,b27),(r15,A3,b28),(r15,A3,b29),(r15,A3,b30),(r15,A3,b31),(r15,A3,b 32),(r15,A3,b33),(r15,A3,b34),(r15,A3,b35),(r15,A3,b36),(r15,A3,b37),(r15,A3,b38),(r15, A3,b39),(r15,A3,b40),(r15,A3,b41),(r15,A3,b42),(r15,A3,b43),(r15,A3,b44),(r15,A3,b45),( r15,A3,b46),(r15,A3,b47),(r15,A3,b48),(r15,A3,b49),(r15,A3,b50),(r15,A3,b51),(r15,A3,b 52),(r15,A3,b53),(r15,A3,b54),(r15,A3,b55),(r15,A3,b56),(r15,A3,b57),(r15,A3,b58),(r15, A3,b59),(r15,A3,b60),(r15,A3,b61),(r15,A3,b62),(r15,A3,b63),(r15,A3,b64),(r15,A3,b65),( r15,A3,b66),(r15,A3,b67),(r15,A3,b68),(r15,A3,b69),(r15,A3,b70),(r15,A3,b71),(r15,A3,b 72),(r15,A3,b73),(r15,A3,b74),(r15,A3,b75),(r15,A3,b76),(r15,A3,b77),(r15,A3,b78),(r15, A3,b79),(r15,A3,b80),(r15,A3,b81),(r15,A3,b82),(r15,A3,b83),(r15,A3,b84),(r15,A3,b85),( r15,A3,b86),(r15,A3,b87),(r15,A3,b88),(r15,A3,b89),(r15,A3,b90),(r15,A3,b91),(r15,A3,b 92),(r15,A3,b93),(r15,A3,b94),(r15,A3,b95),(r15,A3,b96),(r15,A3,b97),(r15,A3,b98),(r15, A3,b99),(r15,A3,b100),(r15,A3,b101),(r15,A3,b102),(r15,A3,b103),(r15,A3,b104),(r15,A3, b105),(r15,A3,b106),(r15,A3,b107),(r15,A3,b108),(r15,A3,b109),(r15,A3,b110),(r15,A3,b1 11),(r15,A3,b112),(r15,A3,b113),(r15,A3,b114),(r15,A3,b115),(r115,A3,b116),(r15,A3,b117) ,(r15,A3,b118),(r15,A3,b119),(r15,A3,b120),(r15,A3,b121),(r15,A3,b122),(r15,A3,b123),(r 15,A3,b124),(r15,A3,b125),(r15,A3,b126),(r15,A3,b127),(r15,A3,b128),(r15,A3,b129),(r15 ,A3,b130),(r15,A3,b131),(r15,A3,b132),(r15,A3,b133),(r15,A3,b134),(r15,A4,b1),(r15,A4, b2),(r15,A4,b3),(r15,A4,b4),(r15,A4,b5),(r15,A4,b6),(r15,A4,b7),(r15,A4,b8),(r15,A4,b9),( r15,A4,b10),(r15,A4,b11),(r15,A4,b12),(r15,A4,b13),(r15,A4,b14),(r15,A4,b15),(r15,A4,b1 6),(r15,A4,b17),(r15,A4,b18),(r15,A4,b19),(r15,A4,b20),(r15,A4,b21),(r15,A4,b22),(r15,A 4,b23),(r15,A4,b24),(r15,A4,b25),(r15,A4,b26),(r15,A4,b27),(r15,A4,b28),(r15,A4,b29),(r1 5,A4,b30),(r15,A4,b31),(r15,A4,b32),(r15,A4,b33),(r15,A4,b34),(r15,A4,b35),(r15,A4,b36) ,(r15,A4,b37),(r15,A4,b38),(r15,A4,b39),(r15,A4,b40),(r15,A4,b41),(r15,A4,b42),(r15,A4, b43),(r15,A4,b44),(r15,A4,b45),(r15,A4,b46),(r15,A4,b47),(r15,A4,b48),(r15,A4,b49),(r15, A4,b50),(r15,A4,b51),(r15,A4,b52),(r15,A4,b53),(r15,A4,b54),(r15,A4,b55),(r15,A4,b56),( r15,A4,b57),(r15,A4,b58),(r15,A4,b59),(r15,A4,b60),(r15,A4,b61),(r15,A4,b62),(r15,A4,b 63),(r15,A4,b64),(r15,A4,b65),(r15,A4,b66),(r15,A4,b67),(r15,A4,b68),(r15,A4,b69),(r15, A4,b70),(r15,A4,b71),(r15,A4,b72),(r15,A4,b73),(r15,A4,b74),(r15,A4,b75),(r15,A4,b76),( r15,A4,b77),(r15,A4,b78),(r15,A4,b79),(r15,A4,b80),(r15,A4,b81),(r15,A4,b82),(r15,A4,b 83),(r15,A4,b84),(r15,A4,b85),(r15,A4,b86),(r15,A4,b87),(r15,A4,b88),(r15,A4,b89),(r15, A4,b90),(r15,A4,b91),(r15,A4,b92),(r15,A4,b93),(r15,A4,b94),(r15,A4,b95),(r15,A4,b96),( r15,A4,b97),(r15,A4,b98),(r15,A4,b99),(r15,A4,b100),(r15,A4,b101),(r15,A4,b102),(r15,A 4,b103),(r15,A4,b104),(r15,A4,b105),(r15,A4,b106),(r15,A4,b107),(r15,A4,b108),(r15,A4, b109),(r15,A4,b110),(r15,A4,b111),(b15,A4,b112),(r15,A4,b113),(r15,A4,b114),(r15,A4,b1 15),(r15,A4,b116),(r15,A4,b117),(r15,A4,b118),(r15,A4,b119),(r15,A4,b120),(r15,A4,b121 ),(r15,A4,b122),(r15,A4,b123),(r15,A4,b124),(r15,A4,b125),(r15,A4,b126),(r15,A4,b127),( r15,A4,b128),(r15,A4,b129),(r15,A4,b130),(r15,A4,b131),(r15,A4,b132),(r15,A4,b133),(r1 5,A4,b134),
(r16,A1,b1),(r16,A1,b2),(r16,A1,b3),(r16,A1,b4),(r16,A1,b5),(r16,A1,b6),(r16,A1,b7),(r16, Al,b8),(r16,A1,b9),(r16,A1,b10),(r16,A1,b11),(r16,A1,b12),(r16,A1,b13),(r16,A1,b14),(r1 6,A1,b15),(r16,A1,b16),(r16,A1,b17),(r16,A1,b18),(r16,A1,b19),(r16,A1,b20),(r16,A1,b21) ,(r16,A1,b22),(r16,A1,b23),(r16,A1,b24),(r16,A1,b25),(r16,A1,b26),(r16,A1,b27),(r16,A1, b28),(r16,A1,b29),(r16,A1,b30),(r16,A1,b31),(r16,A1,b32),(r16,A1,b33),(r16,A1,b34),(r16, A1,b35),(r16,A1,b36),(r16,A1,b37),(r16,A1,b38),(r16,A1,b39),(r16,A1,b40),(r16,A1,b41),( r16,A1,b42),(r16,A1,b43),(r16,A1,b44),(r16,A1,b45),(r16,A1,b46),(r16,A1,b47),(r16,A1,b 48),(r16,A1,b49),(r16,A1,b50),(r16,A1,b51),(r16,A1,b52),(r16,A1,b53),(r16,A1,b54),(r16, A1,b55),(r16,A1,b56),(r16,A1,b57),(r16,A1,b58),(r16,A1,b59),(r16,A1,b60),(r16,A1,b61),( r16,A1,b62),(r16,A1,b63),(r16,A1,b64),(r16,A1,b65),(r16,A1,b66),(r16,A1,b67),(r16,A1,b 68),(r16,A1,b69),(r16,A1,b70),(r16,A1,b71),(r16,A1,b72),(r16,A1,b73),(r16,A1,b74),(r16, A1,b75),(r16,A1,b76),(r16,A1,b77),(r16,A1,b78),(r16,A1,b79),(r16,A1,b80),(r16,A1,b81),( r16,A1,b82),(r16,A1,b83),(r16,A1,b84),(r16,A1,b85),(r16,A1,b86),(r16,A1,b87),(r16,A1,b 88),(r16,A1,b89),(r16,A1,b90),(r16,A1,b91),(r16,A1,b92),(r16,A1,b93),(r16,A1,b94),(r16, A1,b95),(r16,A1,b96),(r16,A1,b97),(r16,A1,b98),(r16,A1,b99),(r16,A1,b100),(r16,A1,b101 ),(r16,A1,b102),(r16,A1,b103),(r16,A1,b104),(r16,A1,b105),(r16,A1,b106),(r16,A1,b107),( r16,A1,b108),(r16,A1,b109),(r16,A1,b110),(r16,A1,b111),(r16,A1,b112),(r16,A1,b113),(r1 6,A1,b114),(r16,A1,b115),(r16,A1,b116),(r16,A1,b117),(r16,A1,b118),(r16,A1,b119),(r16, A1,b120),(r16,A1,b121),(r16,A1,b122),(r16,A1,b123),(r16,A1,b124),(r16,A1,b125),(r16,A 1,b126),(r16,A1,b127),(r16,A1,b128),(r16,A1,b129),(r16,A1,b130),(r16,A1,b131),(r16,A1, b132),(r16,A1,b133),(r16,A1,b134),(r16,A2,b1),(r16,A2,b2),(r16,A2,b3),(r16,A2,b4),(r16, A2,b5),(r16,A2,b6),(r16,A2,b7),(r16,A2,b8),(r16,A2,b9),(r16,A2,b10),(r16,A2,b11),(r16,A2 ,b12),(r16,A2,b13),(r16,A2,b14),(r16,A2,b15),(r16,A2,b16),(r16,A2,b17),(r16,A2,b18),(r16 ,A2,b19),(r16,A2,b20),(r16,A2,b21),(r16,A2,b22),(r16,A2,b23),(r16,A2,b24),(r16,A2,b25),( r16,A2,b26),(r16,A2,b27),(r16,A2,b28),(r16,A2,b29),(r16,A2,b30),(r16,A2,b31),(r16,A2,b 32),(r16,A2,b33),(r16,A2,b34),(r16,A2,b35),(r16,A2,b36),(r16,A2,b37),(r16,A2,b38),(r16, A2,b39),(r16,A2,b40),(r16,A2,b41),(r16,A2,b42),(r16,A2,b43),(r16,A2,b44),(r16,A2,b45),( r16,A2,b46),(r16,A2,b47),(r16,A2,b48),(r16,A2,b49),(r16,A2,b50),(r16,A2,b51),(r16,A2,b 52),(r16,A2,b53),(r16,A2,b54),(r16,A2,b55),(r16,A2,b56),(r16,A2,b57),(r16,A2,b58),(r16, A2,b59),(r16,A2,b60),(r16,A2,b61),(r16,A2,b62),(r16,A2,b63),(r16,A2,b64),(r16,A2,b65),( r16,A2,b66),(r16,A2,b67),(r16,A2,b68),(r16,A2,b69),(r16,A2,b70),(r16,A2,b71),(r16,A2,b 72),(r16,A2,b73),(r16,A2,b74),(r16,A2,b75),(r16,A2,b76),(r16,A2,b77),(r16,A2,b78),(r16, A2,b79),(r16,A2,b80),(r16,A2,b81),(r16,A2,b82),(r16,A2,b83),(r16,A2,b84),(r16,A2,b85),( r16,A2,b86),(r16,A2,b87),(r16,A2,b88),(r16,A2,b89),(r16,A2,b90),(r16,A2,b91),(r16,A2,b 92),(r16,A2,b93),(r16,A2,b94),(r16,A2,b95),(r16,A2,b96),(r16,A2,b97),(r16,A2,b98),(r16, A2,b99),(r16,A2,b100),(r16,A2,b101),(r16,A2,b102),(r16,A2,b103),(r16,A2,b104),(r16,A2, b105),(r16,A2,b106),(r16,A2,b107),(r16,A2,b108),(r16,A2,b109),(r16,A2,b110),(r16,A2,b1 11),(r16,A2,b112),(r16,A2,b113),(r16,A2,b114),(r16,A2,b115),(r16,A2,b116),(r16,A2,b117) ,(r16,A2,b118),(r16,A2,b119),(r16,A2,b120),(r16,A2,b121),(r16,A2,b122),(r16,A2,b123),(r 16,A2,b124),(r16,A2,b125),(r16,A2,b126),(r16,A2,b127),(r16,A2,b128),(r16,A2,b129),(r16 ,A2,b130),(r16,A2,b131),(r16,A2,b132),(r16,A2,b133),(r16,A2,b134),(r16,A3,b1),(r16,A3, b2),(r16,A3,b3),(r16,A3,b4),(r16,A3,b5),(r16,A3,b6),(r16,A3,b7),(r16,A3,b8),(r16,A3,b9),( r16,A3,b10),(r16,A3,b11),(r16,A3,b12),(r16,A3,b13),(r16,A3,b14),(r16,A3,b15),(r16,A3,b1 6),(r16,A3,b17),(r16,A3,b18),(r16,A3,b19),(r16,A3,b20),(r16,A3,b21),(r16,A3,b22),(r16,A 3,b23),(r16,A3,b24),(r16,A3,b25),(r16,A3,b26),(r16,A3,b27),(r16,A3,b28),(r16,A3,b29),(r1 6,A3,b30),(r16,A3,b31),(r16,A3,b32),(r16,A3,b33),(r16,A3,b34),(r16,A3,b35),(r16,A3,b36) ,(r16,A3,b37),(r16,A3,b38),(r16,A3,b39),(r16,A3,b40),(r16,A3,b41),(r16,A3,b42),(r16,A3, b43),(r16,A3,b44),(r16,A3,b45),(r16,A3,b46),(r16,A3,b47),(r16,A3,b48),(r16,A3,b49),(r16, A3,b50),(r16,A3,b51),(r16,A3,b52),(r16,A3,b53),(r16,A3,b54),(r16,A3,b55),(r16,A3,b56),( r16,A3,b57),(r16,A3,b58),(r16,A3,b59),(r16,A3,b60),(r16,A3,b61),(r16,A3,b62),(r16,A3,b 63),(r16,A3,b64),(r16,A3,b65),(r16,A3,b66),(r16,A3,b67),(r16,A3,b68),(r16,A3,b69),(r16, A3,b70),(r16,A3,b71),(r16,A3,b72),(r16,A3,b73),(r16,A3,b74),(r16,A3,b75),(r16,A3,b76),( r16,A3,b77),(r16,A3,b78),(r16,A3,b79),(r16,A3,b80),(r16,A3,b81),(r16,A3,b82),(r16,A3,b 83),(r16,A3,b84),(r16,A3,b85),(r16,A3,b86),(r16,A3,b87),(r16,A3,b88),(r16,A3,b89),(r16, A3,b90),(r16,A3,b91),(r16,A3,b92),(r16,A3,b93),(r16,A3,b94),(r16,A3,b95),(r16,A3,b96),( r16,A3,b97),(r16,A3,b98),(r16,A3,b99),(r16,A3,b100),(r16,A3,b101),(r16,A3,b102),(r16,A 3,b103),(r16,A3,b104),(r16,A3,b105),(r16,A3,b106),(r16,A3,b107),(r16,A3,b108),(r16,A3, b109),(r16,A3,b110),(r16,A3,b111),(r16,A3,b112),(r16,A3,b113),(r16,A3,b114),(r16,A3,b1 15),(r16,A3,b116),(r16,A3,b117),(r16,A3,b118),(r16,A3,b119),(r16,A3,b120),(r16,A3,b121 ),(r16,A3,b122),(r16,A3,b123),(r16,A3,b124),(r16,A3,b125),(r16,A3,b126),(r16,A3,b127),( r16,A3,b128),(r16,A3,b129),(r16,A3,b130),(r16,A3,b131),(r16,A3,b132),(r16,A3,b133),(r1 6,A3,b134),
(r16,A4,b1),(r16,A4,b2),(r16,A4,b3),(r16,A4,b4),(r16,A4,b5),(r16,A4,b6),(r16,A4,b7),(r16, A4,b8),(r16,A4,b9),(r16,A4,b10),(r16,A4,b11),(r16,A4,b12),(r16,A4,b13),(r16,A4,b14),(r1 6,A4,b15),(r16,A4,b16),(r16,A4,b17),(r16,A4,b18),(r16,A4,b19),(r16,A4,b20),(r16,A4,b21) ,(r16,A4,b22),(r16,A4,b23),(r16,A4,b24),(r16,A4,b25),(r16,A4,b26),(r16,A4,b27),(r16,A4, b28),(r16,A4,b29),(r16,A4,b30),(r16,A4,b31),(r16,A4,b32),(r16,A4,b33),(r16,A4,b34),(r16, A4,b35),(r16,A4,b36),(r16,A4,b37),(r16,A4,b38),(r16,A4,b39),(r16,A4,b40),(r16,A4,b41),( r16,A4,b42),(r16,A4,b43),(r16,A4,b44),(r16,A4,b45),(r16,A4,b46),(r16,A4,b47),(r16,A4,b 48),(r16,A4,b49),(r16,A4,b50),(r16,A4,b51),(r16,A4,b52),(r16,A4,b53),(r16,A4,b54),(r16, A4,b55),(r16,A4,b56),(r16,A4,b57),(r16,A4,b58),(r16,A4,b59),(r16,A4,b60),(r16,A4,b61),( r16,A4,b62),(r16,A4,b63),(r16,A4,b64),(r16,A4,b65),(r16,A4,b66),(r16,A4,b67),(r16,A4,b 68),(r16,A4,b69),(r16,A4,b70),(r16,A4,b71),(r16,A4,b72),(r16,A4,b73),(r16,A4,b74),(r16, A4,b75),(r16,A4,b76),(r16,A4,b77),(r16,A4,b78),(r16,A4,b79),(r16,A4,b80),(r16,A4,b81),( r16,A4,b82),(r16,A4,b83),(r16,A4,b84),(r16,A4,b85),(r16,A4,b86),(r16,A4,b87),(r16,A4,b 88),(r16,A4,b89),(r16,A4,b90),(r16,A4,b91),(r16,A4,b92),(r16,A4,b93),(r16,A4,b94),(r16, A4,b95),(r16,A4,b96),(r16,A4,b97),(r16,A4,b98),(r16,A4,b99),(r16,A4,b100),(r16,A4,b101 ),(r16,A4,b102),(r16,A4,b103),(r16,A4,b104),(r16,A4,b105),(r16,A4,b106),(r16,A4,b107),( r16,A4,b108),(r16,A4,b109),(r16,A4,b110),(r16,A4,b111),(r16,A4,b112),(r16,A4,b113),(r1 6,A4,b114),(r16,A4,b115),(r16,A4,b116),(r16,A4,b117),(r16,A4,b118),(r16,A4,b119),(r16, A4,b120),(r16,A4,b121),(r16,A4,b122),(r16,A4,b123),(r16,A4,b124),(r16,A4,b125),(r16,A 4,b126),(r16,A4,b127),(r16,A4,b128),(r16,A4,b129),(r16,A4,b130),(r16,A4,b131),(r16,A4, b132),(r16,A4,b133),(r16,A4,b134),(r17,A1,b1),(r17,A1,b2),(r17,A1,b3),(r17,A1,b4),(r17, A1,b5),(r17,A1,b6),(r17,A1,b7),(r17,A1,b8),(r17,A1,b9),(r17,A1,b10),(r17,A1,b11),(r17,A1 ,b12),(r17,A1,b13),(r17,A1,b14),(r17,A1,b15),(r17,A1,b16),(r17,A1,b17),(r17,A1,b18),(r17 ,A1,b19),(r17,A,b20),(r17,A1,b21),(r17,A1,b22),(r17,A1,b23),(r17,A1,b24),(r17,A1,b25),( r17,A1,b26),(r17,A1,b27),(r17,A1,b28),(r17,A1,b29),(r17,A1,b30),(r17,A1,b31),(r17,A1,b 32),(r17,A1,b33),(r17,A1,b34),(r17,A1,b35),(r17,A1,b36),(r17,A1,b37),(r17,A1,b38),(r17, A1,b39),(r17,A1,b40),(r17,A1,b41),(r17,A1,b42),(r17,A1,b43),(r17,A1,b44),(r17,A1,b45),( r17,A1,b46),(r17,A1,b47),(r17,A1,b48),(r17,A1,b49),(r17,A1,b50),(r17,A1,b51),(r17,A1,b 52),(r17,A1,b53),(r17,A1,b54),(r17,A1,b55),(r17,A1,b56),(r17,A1,b57),(r17,A1,b58),(r17, A1,b59),(r17,A1,b60),(r17,A1,b61),(r17,A1,b62),(r17,A1,b63),(r17,A1,b64),(r17,A1,b65),( r17,A1,b66),(r17,A1,b67),(r17,A1,b68),(r17,A1,b69),(r17,A1,b70),(r17,A1,b71),(r17,A1,b 72),(r17,A1,b73),(r17,A1,b74),(r17,A1,b75),(r17,A1,b76),(r17,A1,b77),(r17,A1,b78),(r17, A1,b79),(r17,A1,b80),(r17,A1,b81),(r17,A1,b82),(r17,A1,b83),(r17,A1,b84),(r17,A1,b85),( r17,A1,b86),(r17,A1,b87),(r17,A1,b88),(r17,A1,b89),(r17,A1,b90),(r17,A1,b91),(r17,A1,b 92),(r17,A1,b93),(r17,A1,b94),(r17,A1,b95),(r17,A1,b96),(r17,A1,b97),(r17,A1,b98),(r17, A1,b99),(r17,A1,b100),(r17,A1,b101),(r17,A1,b102),(r17,A1,b103),(r17,A1,b104),(r17,A1, b105),(r17,A1,b106),(r17,A1,b107),(r17,A1,b108),(r17,A1,b109),(r17,A1,b110),(r17,A1,b1 11),(r17,A1,b112),(r17,A1,b113),(r17,A1,b114),(r17,A1,b115),(r17,A1,b116),(r17,A1,b117) ,(r17,A1,b118),(r17,A1,b119),(r17,A1,b120),(r17,A1,b121),(r17,A1,b122),(r17,A1,b123),(r 17,A1,b124),(r17,A1,b125),(r17,A1,b126),(r17,A1,b127),(r17,A1,b128),(r17,A1,b129),(r17 ,A1,b130),(r17,A1,b131),(r17,A1,b132),(r17,A1,b133),(r17,A1,b134),(r17,A2,b1),(r17,A2, b2),(r17,A2,b3),(r17,A2,b4),(r17,A2,b5),(r17,A2,b6),(r17,A2,b7),(r17,A2,b8),(r17,A2,b9),( r17,A2,b10),(r17,A2,b11),(r17,A2,b12),(r17,A2,b13),(r17,A2,b14),(r17,A2,b15),(r17,A2,b1 6),(r17,A2,b17),(r17,A2,b18),(r17,A2,b19),(r17,A2,b20),(r17,A2,b21),(r17,A2,b22),(r17,A 2,b23),(r17,A2,b24),(r17,A2,b25),(r17,A2,b26),(r17,A2,b27),(r17,A2,b28),(r17,A2,b29),(r1 7,A2,b30),(r17,A2,b31),(r17,A2,b32),(r17,A2,b33),(r17,A2,b34),(r17,A2,b35),(r17,A2,b36) ,(r17,A2,b37),(r17,A2,b38),(r17,A2,b39),(r17,A2,b40),(r17,A2,b41),(r17,A2,b42),(r17,A2, b43),(r17,A2,b44),(r17,A2,b45),(r17,A2,b46),(r17,A2,b47),(r17,A2,b48),(r17,A2,b49),(r17, A2,b50),(r17,A2,b51),(r17,A2,b52),(r17,A2,b53),(r17,A2,b54),(r7,A2,b55),(r17,A2,b56),( r17,A2,b57),(r17,A2,b58),(r17,A2,b59),(r17,A2,b60),(r17,A2,b61),(r17,A2,b62),(r17,A2,b 63),(r17,A2,b64),(r17,A2,b65),(r17,A2,b66),(r17,A2,b67),(r17,A2,b68),(r17,A2,b69),(r17, A2,b70),(r17,A2,b71),(r17,A2,b72),(r17,A2,b73),(r17,A2,b74),(r17,A2,b75),(r17,A2,b76),( r17,A2,b77),(r17,A2,b78),(r17,A2,b79),(r17,A2,b80),(r17,A2,b81),(r17,A2,b82),(r17,A2,b 83),(r17,A2,b84),(r17,A2,b85),(r17,A2,b86),(r17,A2,b87),(r17,A2,b88),(r17,A2,b89),(r17, A2,b90),(r17,A2,b91),(r17,A2,b92),(r17,A2,b93),(r17,A2,b94),(r17,A2,b95),(r17,A2,b96),( r17,A2,b97),(r17,A2,b98),(r17,A2,b99),(r17,A2,b100),(r17,A2,b101),(r17,A2,b102),(r17,A 2,b103),(r17,A2,b104),(r17,A2,b105),(r17,A2,b106),(r17,A2,b107),(r17,A2,b108),(r17,A2, b109),(r17,A2,b110),(r17,A2,b111),(r17,A2,b112),(r17,A2,b113),(r17,A2,b114),(r17,A2,b11 15),(r17,A2,b116),(r17,A2,b117),(r17,A2,b118),(r17,A2,b119),(r17,A2,b120),(r17,A2,b121 ),(r17,A2,b122),(r17,A2,b123),(r17,A2,b124),(r17,A2,b125),(r17,A2,b126),(r17,A2,b127),( r17,A2,b128),(r17,A2,b129),(r17,A2,b130),(r17,A2,b131),(r17,A2,b132),(r17,A2,b133),(r1 7,A2,b134),
(r17,A3,b1),(r17,A3,b2),(r17,A3,b3),(r17,A3,b4),(r17,A3,b5),(r17,A3,b6),(r17,A3,b7),(r17, A3,b8),(r17,A3,b9),(r17,A3,b10),(r17,A3,b11),(r17,A3,b12),(r17,A3,b13),(r17,A3,b14),(r1 7,A3,b15),(r17,A3,b16),(r17,A3,b17),(r17,A3,b18),(r17,A3,b19),(r17,A3,b20),(r17,A3,b21) ,(r17,A3,b22),(r17,A3,b23),(r17,A3,b24),(r17,A3,b25),(r17,A3,b26),(r17,A3,b27),(r17,A3, b28),(r17,A3,b29),(r17,A3,b30),(r17,A3,b31),(r17,A3,b32),(r17,A3,b33),(r17,A3,b34),(r17, A3,b35),(r17,A3,b36),(r17,A3,b37),(r17,A3,b38),(r17,A3,b39),(r17,A3,b40),(r17,A3,b41),( r17,A3,b42),(r17,A3,b43),(r17,A3,b44),(r17,A3,b45),(r17,A3,b46),(r17,A3,b47),(r17,A3,b 48),(r17,A3,b49),(r17,A3,b50),(r17,A3,b51),(r17,A3,b52),(r17,A3,b53),(r17,A3,b54),(r17, A3,b55),(r17,A3,b56),(r17,A3,b57),(r17,A3,b58),(r17,A3,b59),(r17,A3,b60),(r17,A3,b61),( r17,A3,b62),(r17,A3,b63),(r17,A3,b64),(r17,A3,b65),(r17,A3,b66),(r17,A3,b67),(r17,A3,b 68),(r17,A3,b69),(r17,A3,b70),(r17,A3,b71),(r17,A3,b72),(r17,A3,b73),(r17,A3,b74),(r17, A3,b75),(r17,A3,b76),(r17,A3,b77),(r17,A3,b78),(r17,A3,b79),(r17,A3,b80),(r17,A3,b81),( r17,A3,b82),(r17,A3,b83),(r17,A3,b84),(r17,A3,b85),(r17,A3,b86),(r17,A3,b87),(r17,A3,b 88),(r17,A3,b89),(r17,A3,b90),(r17,A3,b91),(r17,A3,b92),(r17,A3,b93),(r17,A3,b94),(r17, A3,b95),(r17,A3,b96),(r17,A3,b97),(r17,A3,b98),(r17,A3,b99),(r17,A3,b100),(r17,A3,b101 ),(r17,A3,b102),(r17,A3,b103),(r17,A3,b104),(r17,A3,b105),(r17,A3,b106),(r17,A3,b107),( r17,A3,b108),(r17,A3,b109),(r17,A3,b110),(r17,A3,b111),(r17,A3,b112),(r17,A3,b113),(r1 7,A3,b114),(r17,A3,b116),(r17,A3,b116),(r17,A3,b117),(r17,A3,b118),(r17,A3,b119),(r17, A3,b120),(r17,A3,b121),(r17,A3,b122),(r17,A3,b123),(r17,A3,b124),(r17,A3,b125),(r17,A 3,b126),(r17,A3,b127),(r17,A3,b128),(r17,A3,b129),(r17,A3,b130),(r17,A3,b131),(r17,A3, b132),(r17,A3,b133),(r17,A3,b134),(r17,A4,b1),(r17,A4,b2),(r17,A4,b3),(r17,A4,b4),(r17, A4,b5),(r17,A4,b6),(r17,A4,b7),(r17,A4,b8),(r17,A4,b9),(r17,A4,b10),(r17,A4,b11),(r17,A4 ,b12),(r17,A4,b13),(r17,A4,b14),(r17,A4,b15),(r17,A4,b16),(r17,A4,b17),(r17,A4,b18),(r17 ,A4,b19),(r17,A4,b20),(r17,A4,b21),(r17,A4,b22),(r17,A4,b23),(r17,A4,b24),(r17,A4,b25),( r17,A4,b26),(r17,A4,b27),(r17,A4,b28),(r17,A4,b29),(r17,A4,b30),(r17,A4,b31),(r17,A4,b 32),(r17,A4,b33),(r17,A4,b34),(r17,A4,b35),(r17,A4,b36),(r17,A4,b37),(r17,A4,b38),(r17, A4,b39),(r17,A4,b40),(r17,A4,b41),(r17,A4,b42),(r17,A4,b43),(r17,A4,b44),(r17,A4,b45),( r17,A4,b46),(r17,A4,b47),(r17,A4,b48),(r17,A4,b49),(r17,A4,b50),(r17,A4,b51),(r17,A4,b 52),(r17,A4,b53),(r17,A4,b54),(r17,A4,b55),(r17,A4,b56),(r17,A4,b57),(r17,A4,b58),(r17, A4,b59),(r17,A4,b60),(r17,A4,b61),(r17,A4,b62),(r17,A4,b63),(r17,A4,b64),(r17,A4,b65),( r17,A4,b66),(r17,A4,b67),(r17,A4,b68),(r17,A4,b69),(r17,A4,b70),(r17,A4,b71),(r17,A4,b 72),(r17,A4,b73),(r17,A4,b74),(r17,A4,b75),(r17,A4,b76),(r17,A4,b77),(r17,A4,b78),(r17, A4,b79),(r17,A4,b80),(r17,A4,b81),(r17,A4,b82),(r17,A4,b83),(r17,A4,b84),(r17,A4,b85),( r17,A4,b86),(r17,A4,b87),(r17,A4,b88),(r17,A4,b89),(r17,A4,b90),(r17,A4,b91),(r17,A4,b 92),(r17,A4,b93),(r17,A4,b94),(r17,A4,b95),(r17,A4,b96),(r17,A4,b97),(r17,A4,b98),(r17, A4,b99),(r17,A4,b100),(r17,A4,b101),(r17,A4,b102),(r17,A4,b103),(r17,A4,b104),(r17,A4, b105),(r17,A4,b106),(r17,A4,b107),(r17,A4,b108),(r17,A4,b109),(r17,A4,b110),(r17,A4,b1 11),(r17,A4,b112),(r17,A4,b113),(r17,A4,b114),(r17,A4,b115),(r17,A4,b116),(r17,A4,b117) ,(r17,A4,b118), (r17,A4,b119), (r17,A4,b120), (r17,A4,b121), (r17,A4,b122), (r17,A4,b123), (r 17,A4,b124), (r17,A4,b125), (r17,A4,b126),(r17,A4,b127),(r17,A4,b128),(r17,A4,b129),(r17 ,A4,b130),(r17,A4,b131),(r17,A4,b132),(r17,A4,b133),(r17,A4,b134),(r18,A1,b1),(r18,A1, b2),(r18,A1,b3),(r18,A1,b4),(r18,A1,b5),(r18,A1,b6),(r18,A1,b7),(r18,A1,b8),(r18,A1,b9),( r18,A1,b10),(r18,A1,b11),(r18,A1,b12),(r18,A1,b13),(r18,A1,b14),(r18,A1,b18),(r18,A1,b1 6),(r18,A1,b17),(r18,A1,b18),(r18,A1,b19),(r18,A1,b20),(r18,A1,b21),(r18,A1,b22),(r18,A 1,b23),(r18,A1,b24),(r18,A1,b25),(r18,A1,b26),(r18,A1,b27),(r18,A1,b28),(r18,A1,b29),(r1 8,A1,b30),(r18,A1,b31),(r18,A1,b32),(r18,A1,b33),(r18,A1,b34),(r18,A1,b35),(r18,A1,b36) ,(r18,A1,b37),(r18,A1,b38),(r18,A1,b39),(r18,A1,b40),(r18,A1,b41),(r18,A1,b42),(r18,A1, b43),(r18,A1,b44),(r18,A1,b45),(r18,A1,b46),(r18,A1,b47),(r18,A1,b48),(r18,A1,b49),(r18, A1,b50),(r18,A1,b51),(r18,A1,b52),(r18,A1,b53),(r18,A1,b54),(r18,A1,b55),(r18,A1,b56),( r18,A1,b57),(r18,A1,b58),(r18,A1,b59),(r18,A1,b60),(r18,A1,b61),(r18,A1,b62),(r18,A1,b 63),(r18,A1,b64),(r18,A1,b65),(rl8,A1,b66),(r18,A1,b67),(r18,A1,b68),(r18,A1,b69),(r18, A1,b70),(r18,A1,b71),(r18,A1,b72),(r18,A1,b73),(r18,A1,b74),(r18,A1,b75),(r18,A1,b76),( r18,A1,b77),(r18,A1,b78),(r18,A1,b79),(r18,A1,b80),(r18,A1,b81),(r18,A1,b82),(r18,A1,b 83),(r18,A1,b84),(r18,A1,b85),(r18,A1,b86),(r18,A1,b87),(r18,A1,b88),(r18,A1,b89),(r18, A1,b90),(r1B,A1,b91),(r18,A1,b92),(r18,A1,b93),(r18,A1,b94),(r18,A1,b95),(r18,A1,b96),( r18,A1,b97),(r18,A1,b98),(r18,A1,b99),(r18,A1,b100), (r18,A1,b101),(r18,A1,b102),(r28,A 1,b103),(r18,A1,b104),(r18,A1,b105),(r18,A1,b106),(r18,A1,b107),(r18,A1,b108),(r18,A1, b109),(r18,A1,b110),(r18,A1,b111),(r18,A1,b112),(r18,A1,b113),(r18,A1,b114),(r18,A1,b1 15),(r18,A1,b116),(r18,A1,b117),(r18,A1,b118),(r18,A1,b119),(r18,A1,b120),(r18,A1,b121 ),(r1B,A1,b122),(r18,A1,b123),(r18,A1,b124),(r18,A1,b125),(r18,A1,b126),(r18,A1,b127),( r18,A1,b128),(r18,A1,b129),(r18,A1,b130),(r18,A1,b131),(r18,A1,b132),(r18,A1,b133),(r1 8,A1,b134),
(r18,A2,b1),(r18,A2,b2),(r18,A2,b3),(r18,A2,b4),(r18,A2,b5),(r18,A2,b6),(r18,A2,b7),(r18, A2,b8),(r18,A2,b9),(r18,A2,b10),(r18,A2,b11),(r18,A2,b12),(r18,A2,b13),(r18,A2,b14),(r1 8,A2,b15),(r18,A2,b16),(r18,A2,b17),(r18,A2,b18),(r18,A2,b19),(r18,A2,b20),(r18,A2,b21) ,(r18,A2,b22),(r18,A2,b23),(r18,A2,b24),(r18,A2,b25),(r18,A2,b26),(r18,A2,b27),(r18,A2, b28),(r18,A2,b29),(r18,A2,b30),(r18,A2,b31),(r18,A2,b32),(r18,A2,b33),(r18,A2,b34),(r18, A2,b35),(r18,A2,b36),(r18,A2,b37),(r18,A2,b38),(r18,A2,b39),(r18,A2,b40),(r18,A2,b41),( r18,A2,b42),(r18,A2,b43),(r18,A2,b44),(r18,A2,b45),(r18,A2,b46),(r18,A2,b47),(r18,A2,b 48),(r18,A2,b49),(r18,A2,b50),(r18,A2,b51),(r18,A2,b52),(r18,A2,b53),(r18,A2,b54),(r18, A2,b55),(r18,A2,b56),(r18,A2,b57),(r18,A2,b58),(r18,A2,b59),(r18,A2,b60),(r18,A2,b61),( r18,A2,b62),(r18,A2,b63),(r18,A2,b64),(r18,A2,b65),(r18,A2,b66),(r18,A2,b67),(r18,A2,b 68),(r18,A2,b69),(r18,A2,b70),(r18,A2,b71),(r18,A2,b72),(r18,A2,b73),(r18,A2,b74),(r18, A2,b75),(r18,A2,b76),(r18,A2,b77),(r18,A2,b78),(r18,A2,b79),(r18,A2,b80),(r18,A2,b81),( r18,A2,b82),(r18,A2,b83),(r18,A2,b84),(r18,A2,b85),(r18,A2,b86),(r18,A2,b87),(r18,A2,b 88),(r18,A2,b89),(r18,A2,b90),(r18,A2,b91),(r18,A2,b92),(r18,A2,b93),(r18,A2,b94),(r18, A2,b95),(r18,A2,b96),(r18,A2,b97),(r18,A2,b98),(r18,A2,b99),(r18,A2,b100),(r18,A2,b101 ),(r18,A2,b102),(r18,A2,b103),(r18,A2,b104),(r18,A2,b105),(r18,A2,b106),(r18,A2,b107), ( r18,A2,b108),(r18,A2,b109),(r18,A2,b110),(r18,A2,b111),(r18,A2,b112),(r18,A2,b113),(r1 8,A2,b114),(r18,A2,b115),(r18,A2,b116),(r18,A2,b117),(r18,A2,b118),(r18,A2,b119),(r18, A2,b120),(r18,A2,b121),(r18,A2,b122),(r18,A2,b123),(r18,A2,b124),(r18,A2,b125),(r18,A 2,b126);(r18,A2,b127),(r18,A2,b128),(r18,A2,b129),(r18,A2,b130),(r18,A2,b131),(r18,A2, b132),(r18,A2,b133),(r18,A2,b134),(r18,A3,b1),(r18,A3,b2),(r18,A3,b3),(r18,A3,b4),(r18, A3,b5),(r18,A3,b6),(r18,A3,b7),(r18,A3,b8),(r18,A3,b9),(r18,A3,b10),(r18,A3,b11),(r18,A3 ,b12),(r18,A3,b13),(r18,A3,b14),(r18,A3,b15),(r18,A3,b16),(r18,A3,b17),(r18,A3,b18),(r18 ,A3,b19),(r18,A3,b20),(r18,A3,b21),(r18,A3,b22),(r18,A3,b23),(r18,A3,b24),(r18,A3,b25),( r18,A3,b26),(r18,A3,b27),(r18,A3,b28),(r18,A3,b29),(r18,A3,b30),(r18,A3,b31),(r18,A3,b 32),(r18,A3,b33),(r18,A3,b34),(r18,A3,b35),(r18,A3,b36),(r18,A3,b37),(r18,A3,b38),(r18, A3,b39),(r18,A3,b40),(r18,A3,b41),(r18,A3,b42),(r18,A3,b43),(r18,A3,b44),(r18,A3,b45),( r18,A3,b46),(r18,A3,b47),(r18,A3,b48),(r18,A3,b49),(r18,A3,b50),(r18,A3,b51),(r18,A3,b 52),(r18,A3,b53),(r18,A3,b54),(r18,A3,b55),(r18,A3,b56),(r18,A3,b57),(r18,A3,b58),(r18, A3,b59),(r18,A3,b60),(r18,A3,b61),(r18,A3,b62),(r18,A3,b63),(r18,A3,b64),(r18,A3,b65),( r18,A3,b66),(r18,A3,b67),(r18,A3,b68),(r18,A3,b69),(r18,A3,b70),(r18,A3,b71),(r18,A3,b 72),(r18,A3,b73),(r18,A3,b74),(r18,A3,b75),(r18,A3,b76),(r18,A3,b77),(r18,A3,b78),(r18, A3,b79),(r18,A3,b80),(r18,A3,b81),(r18,A3,b82),(r18,A3,b83),(r18,A3,b84),(r18,A3,b85),( r18,A3,b86),(r18,A3,b87),(r18,A3,b88),(r18,A3,b89),(r18,A3,b90),(r18,A3,b91),(r18,A3,b 92),(r18,A3,b93),(r18,A3,b94),(r18,A3,b95),(r18,A3,b96),(r18,A3,b97),(r18,A3,b98),(r18, A3,b99),(r18,A3,b100),(r18,A3,b101),(r18,A3,b102),(r18,A3,b103),(r18,A3,b104),(r18,A3, b105),(r18,A3,b106),(r18,A3,b107),(r18,A3,b108),(r18,A3,b109),(r18,A3,b110),(r18,A3,b1 11),(r18,A3,b112),(r18,A3,b113),(r18,A3,b114),(r18,A3,b115),(r18,A3,b116),(r18,A3,b117) ,(r18,A3,b118),(r18,A3,b119),(r18,A3,b120),(r18,A3,b121),(r18,A3,b122),(r18,A3,b123),(r 18,A3,b124),(r18,A3,b125),(r18,A3,b126),(r18,A3,b127),(r18,A3,b128),(r18,A3,b129),(r18 ,A3,b130),(r18,A3,b131),(r18,A3,b132),(r18,A3,b133),(r18,A3,b134),(r18,A4,b1),(r18,A4, b2),(r18,A4,b3),(r18,A4,b4),(r18,A4,b5),(r18,A4,b6),(r18,A4,b7),(r18,A4,b8),(r18,A4,b9),( r18,A4,b10),(r18,A4,b11),(r18,A4,b12),(r18,A4,b13),(r18,A4,b14),(r18,A4,b15),(r18,A4,b1 6),(r18,A4,b17),(r18,A4,b18),(r18,A4,b19),(r18,A4,b20),(r18,A4,b21),(r18,A4,b22),(r18,A 4,b23),(r18,A4,b24),(r18,A4,b25),(r18,A4,b26),(r18,A4,b27),(r18,A4,b28),(r18,A4,b29),(r1 8,A4,b30),(r18,A4,b31),(r18,A4,b32),(r18,A4,b33),(r18,A4,b34),(r18,A4,b35),(r18,A4,b36) ,(r18,A4,b37),(r18,A4,b38),(r18,A4,b39),(r18,A4,b40),(r18,A4,b41),(r18,A4,b42),(r8,A4, b43),(r18,A4,b44),(r18,A4,b45),(r18,A4,b46),(r18,A4,b47),(r18,A4,b48),(r18,A4,b49),(r18, A4,b50),(r18,A4,b51),(r18,A4,b52),(r18,A4,b53),(r18,A4,b54),(r18,A4,b55),(r18,A4,b56),( r18,A4,b57),(r18,A4,b58),(r18,A4,b59),(r18,A4,b60),(r18,A4,b61),(r18,A4,b62),(r18,A4,b 63),(r18,A4,b64),(r18,A4,b65),(r18,A4,b66),(r18,A4,b67),(r18,A4,b68),(r18,A4,b69),(r18, A4,b70),(r18,A4,b71),(r18,A4,b72),(r18,A4,b73),(r18,A4,b74),(r18,A4,b75),(r18,A4,b76),( r18,A4,b77),(r18,A4,b78),(r18,A4,b79),(r18,A4,b80),(r18,A4,b81),(r18,A4,b82),(r18,A4,b 83),(r18,A4,b84),(r18,A4,b85),(r18,A4,b86),(r18,A4,b87),(r18,A4,b88),(r18,A4,b89),(r18, A4,b90),(r18,A4,b91),(r18,A4,b92),(r18,A4,b93),(r18,A4,b94),(r18,A4,b95),(r18,A4,b96),( r18,A4,b97),(r18,A4,b98),(r18,A4,b99),(r18,A4,b100),(r18,A4,b101),(r18,A4,b102),(r18,A 4,b103),(r18,A4,b104),(r18,A4,b105),(r18,A4,b106),(r18,A4,b107),(r18,A4,b108),(r18,A4, b109),(r18,A4,b110),(r18,A4,b111),(r18,A4,b112),(r18,A4,b113),(r18,A4,b114),(r18,A4,b1 15),(r18,A4,b116),(r18,A4,b117),(r18,A4,b118),(r18,A4,b119),(r18,A4,b120),(r18,A4,b121 ),(r18,A4,b122),(r18,A4,b123),(r18;A4,b124),(r18,A4,b125),(r18,A4,b126),(r18,A4,b127),( r18,A4,b128),(r18,A4,b129),(r18,A4,b130),(r18,A4,b131),(r18,A4,b132),(r18,A4,b133),(r1 8,A4,b134),
(r19,A1,b1),(r19,A1,b2),(r19,A1,b3),(r19,A1,b4),(r19,A1,b5),(r19,A1,b6),(r19,A1,b7),(r19, A1,b8),(r19,A1,b9),(r19,A1,b10),(r19,A1,b11),(r19,A1,b12),(r19,A1,b13),(r19,A1,b14),(r1 9,A1,b15),(r19,A1,b16),(r19,A1,b17),(r19,A1,b18),(r19,A1,b19),(r19,A1,b20),(r19,A1,b21) ,(r19,A1,b22),(r19,A1,b23),(r19,A1,b24),(r19,A1,b25),(r19,A1,b26),(r19,A1,b27),(r19,A1, b28),(r19,A1,b29),(r19,A1,b30),(r19,A1,b31),(r19,A1,b32),(r19,A1,b33),(r19,A1,b34),(r19, A1,b35),(r19,A1,b36),(r19,A1,b37),(r19,A1,b38),(r19,A1,b39),(r19,A1,b40),(r19,A1,b41),( r19,A1,b42),(r19,A1,b43),(r19,A1,b44),(r19,A1,b45),(r19,A1,b46),(r19,A1,b47),(r19,A1,b 48),(r19,A1,b49),(r19,A1,b50),(r19,A1,b51),(r19,A1,b52),(r19,A1,b53),(r19,A1,b54),(r19, A1,b55),(r19,A1,b56),(r19,A1,b57),(r19,A1,b58),(r19,A1,b59),(r19,A1,b60),(r19,Al,b61),( r19,A1,b62),(r19,A1,b63),(r19,A1,b64),(r19,A1,b65),(r19,A1,b66),(r19,A1,b67),(r19,A1,b 68),(r19,A1,b69),(r19,A1,b70),(r19,A1,b71),(r19,A1,b72),(r19,A1,b73),(r19,A1,b74),(r19, A1,b75),(r19,A1,b76),(r19,A1,b77),(r19,A1,b78),(r19,A1,b79),(r19,A1,b80),(r19,A1,b81),( r19,A1,b82),(r19,A1,b83),(r19,A1,b84),(r19,A1,b85),(r19,A1,b86),(r19,A1,b87),(r19,A1,b 88),(r19,A1,b89),(r19,A1,b90),(r19,A1,b91),(r19,A1,b92),(r19,A1,b93),(r19,A1,b94),(r19, A1,b95),(r19,A1,b96),(r19,A1,b97),(r19,A1,b98),(r19,A1,b99),(r19,A1,b100),(r19,A1,b101 ),(r19,A1,b102),(r19,A1,b103),(r19,A1,b104),(r19,A1,b105),(r19,A1,b106),(r19,A1,b107),( r19,A1,b108),(r19,A1,b109),(r19,A1,b110),(r19,A1,b111),(r19,A1,b12),(r19,A1,b113),(r1 9,A1,b114),(r19,A1,b115),(r19,A1,b116),(r19,A1,b117),(r19,A1,b118),(r19,A1,b119),(r19, A1,b120),(r19,A1,b121),(r19,A1,b122),(r19,A1,b123),(r19,A1,b124),(r19,A1,b125),(r19,A 1,b126),(r19,A1,b127),(r19,A1,b128),(r19,A1,b129),(r19,A1,b130),(r19,A1,b131),(r19,A1, b132),(r19,A1,b133),(r19,A1,b134),(r19,A2,b1),(r19,A2,b2),(r19,A2,b3),(r19,A2,b4),r19, A2,b5),(r19,A2,b6),(r19,A2,b7),(r19,A2,b8),(r19,A2,b9),(r19,A2,b10),(r19,A2,b11),(r19,A2 ,b12),(r19,A2,b13),(r19,A2,b14),(r19,A2,b15),(r19,A2,b16),(r19,A2,b17),(r19,A2,b18),(r19 ,A2,b19),(r19,A2,b20),(r19,A2,b21),(r19,A2,b22),(r19,A2,b23),(r19,A2,b24),(r19,A2,b25),( r19,A2,b26),(r19,A2,b27),(r19,A2,b28),(r19,A2,b29),(r19,A2,b30),(r19,A2,b31),(r19,A2,b 32),(r19,A2,b33),(r19,A2,b34),(r19,A2,b35),(r19,A2,b36),(r19,A2,b37),(r19,A2,b38),(r19, A2,b39),(r19,A2,b40),(r19,A2,b41),(r19,A2,b42),(r19,A2,b43),(r19,A2,b44),(r19,A2,b45),( r19,A2,b46),(r19,A2,b47),(r19,A2,b48),(r19,A2,b49),(r19,A2,b50),(r19,A2,b51),(r19,A2,b 52),(r19,A2,b53),(r19,A2,b54),(r19,A2,b55),(r19,A2,b56),(r19,A2,b57),(r19,A2,b58),(r19, A2,b59),(r19,A2,b60),(r19,A2,b61),(r19,A2,b62),(r19,A2,b63),(r19,A2,b64),(r19,A2,b65),( r19,A2,b66),(r19,A2,b67),(r19,A2,b68),(r19,A2,b69),(r19,A2,b70),(r19,A2,b71),(r19,A2,b 72),(r19,A2,b73),(r19,A2,b74),(r19,A2,b75),(r19,A2,b76),(r19,A2,b77),(r19,A2,b78),(r19, A2,b79),(r19,A2,b80),(r19,A2,b81),(r19,A2,b82),(r19,A2,b83),(r19,A2,b84),(r19,A2,b85),( r19,A2,b86),(r19,A2,b87),(r19,A2,b88),(r19,A2,b89),(r19,A2,b90),(r19,A2,b91),(r19,A2,b 92),(r19,A2,b93),(r19,A2,b94),(r19,A2,b95),(r19,A2,b96),(r19,A2,b97),(r19,A2,b98),(r19, A2,b99),(r19,A2,b100),(r19,A2,b101),(r19,A2,b102),(r19,A2,b103),(r19,A2,b104),(r19,A2, b105),(r19,A2,b106),(r19,A2,b107),(r19,A2,b108),(r19,A2,b109),(r19,A2,b110),(r19,A2,b1 11),(r19,A2,b112),(r19,A2,b113),(r19,A2,b114),(r19,A2,b115),(r19,A2,b116),(r19,A2,b117) ,(r19,A2,b118),(r19,A2,b119),(r19,A2,b120),(r19,A2,b121),(r19,A2,b122),(r19,A2,b123),(r 19,A2,b124),(r19,A2,b125),(r19,A2,b126),(r19,A2,b127),(r19,A2,b128),(r19,A2,b129),(r19 ,A2,b130),(r19,A2,b131),(r19,A2,b132),(r19,A2,b133),(r19,A2,b134),(r19,A3,b1),(r19,A3, b2),(r19,A3,b3),(r19,A3,b4),(r19,A3,b5),(r19,A3,b6),(r19,A3,b7),(r19,A3,b8),(r19,A3,b9),( r19,A3,b10),(r19,A3,b11),(r19,A3,b12),(r19,A3,b13),(r19,A3,b14),(r19,A3,b15),(r19,A3,b1 6),(r19,A3,b17),(r19,A3,b18),(r19,A3,b19),(r19,A3,b20),(r19,A3,b21),(r19,A3,b22),(r19,A 3,b23),(r19,A3,b24),(r19,A3,b25),(r19,A3,b26),(r19,A3,b27),(r19,A3,b28),(r19,A3,b29),(r1 9,A3,b30),(r19,A3,b31),(r19,A3,b32),(r19,A3,b33),(r19,A3,b34),(r19,A3,b35),(r19,A3,b36) ,(r19,A3,b37),(r19,A3,b38),(r19,A3,b39),(r19,A3,b40),(r19,A3,b41),(r19,A3,b42),(r19,A3, b43),(r19,A3,b44),(r19,A3,b45),(r19,A3,b46),(r19,A3,b47),(r19,A3,b48),(r19,A3,b49),(r19, A3,b50),(r19,A3,b51),(r19,A3,b52),(r19,A3,b53),(r19,A3,b54),(r19,A3,b55),(r19,A3,b56),( r19,A3,b57),(r19,A3,b58),(r19,A3,b59),(r19,A3,b60),(r19,A3,b61),(r19,A3,b62),(r19,A3,b 63),(r19,A3,b64),(r19,A3,b65),(r19,A3,b66),(r19,A3,b67),(r19,A3,b68),(r19,A3,b69),(r19, A3,b70),(r19,A3,b71),(r19,A3,b72),(r19,A3,b73),(r19,A3,b74),(r19,A3,b75),(r19,A3,b76),( r19,A3,b77),(r19,A3,b78),(r19,A3,b79),(r19,A3,b80),(r19,A3,b81),(r19,A3,b82),(r19,A3,b 83),(r19,A3,b84),(r19,A3,b85),(r19,A3,b86),(r19,A3,b87),(r19,A3,b88),(r19,A3,b89),(r19, A3,b90),(r19,A3,b91),(r19,A3,b92),(r19,A3,b93),(r19,A3,b94),(r19,A3,b95),(r19,A3,b96),( r19,A3,b97),(r19,A3,b98),(r19,A3,b99),(r19,A3,b100),(r19,A3,b101),(r19,A3,b102),(r19,A 3,b103),(r19,A3,b104),(r19,A3,b105),(r19,A3,b106),(r19,A3,b107),(r19,A3,b108),(r19,A3, b109),(r19,A3,b110),(r19,A3,b111),(r19,A3,b112),(r19,A3,b113),(r19,A3,b114),(r19,A3,b1 15),(r19,A3,b116),(r19,A3,b117),(r19,A3,b118),(r19,A3,b119),(r19,A3,b120),(r19,A3,b121 ),(r19,A3,b122),(r19,A3,b123),(r19,A3,b124),(r19,A3,b125),(r19,A3,b126),(r19,A3,b127),( r19,A3,b128),(r19,A3,b129),(r19,A3,b130),(r19,A3,b131),(r19,A3,b132),(r19,A3,b133),(r1 9,A3,b134),
(r19,A4,b1),(r19,A4,b2),(r19,A4,b3),(r19,A4,b4),(r19,A4,b5),(r19,A4,b6),(r19,A4,b7),(r19, A4,b8),(r19,A4,b9),(r19,A4,b10),(r19,A4,b11),(r19,A4,b12),(r19,A4,b13),(r19,A4,b14),(r1 9,A4,b15),(r19,A4,b16),(r19,A4,b17),(r19,A4,b18),(r19,A4,b19),(r19,A4,b20),(r19,A4,b21) ,(r19,A4,b22),(r19,A4,b23),(r19,A4,b24),(r19,A4,b25),(r19,A4,b26),(r19,A4,b27),(r19,A4, b28),(r19,A4,b29),(r19,A4,b30),(r19,A4,b31),(r19,A4,b32),(r19,A4,b33),(r19,A4,b34),(r19, A4,b35),(r19,A4,b36),(r19,A4,b37),(r19,A4,b38),(r19,A4,b39),(r19,A4,b40),(r19,A4,b41),( r19,A4,b42),(r19,A4,b43),(r19,A4,b44),(r19,A4,b45),(r19,A4,b46),(r19,A4,b47),(r19,A4,b 48),(r19,A4,b49),(r19,A4,b50),(r19,A4,b51),(r19,A4,b52),(r19,A4,b53),(r19,A4,b54),(r19, A4,b55),(r19,A4,b56),(r19,A4,b57),(r19,A4,b58),(r19,A4,b59),(r19,A4,b60),(r19,A4,b61),( r19,A4,b62),(r19,A4,b63),(r19,A4,b64),(r19,A4,b65),(r19,A4,b66),(r19,A4,b67),(r19,A4,b 68),(r9,A4,b69),(r19,A4,b70),(r19,A4,b71),(r19,A4,b72),(r9,A4,b73),(r19,A4,b74),(r19, A4,b75),(r19,A4,b76),(r19,A4,b77),(r19,A4,b78),(r19,A4,b79),(r19,A4,b80),(r19,A4,b81), r19,A4,b82),(r19,A4,b83),(r19,A4,b84),(r19,A4,b85),(r19,A4,b86),(r19,A4,b87),(r19,A4,b 88),(r19,A4,b89),(r9,A4,b90),(r19,A4,b91),(r19,A4,b92),(r19,A4,b93),(r19,A4,b94),(r19, A4,b95),(r19,A4,b96),(r19,A4,b97),(r19,A4,b98),(r19,A4,b99),(r19,A4,b100),(r19,A4,b101 ),(r19,A4,b102),(r19,A4,b103),(r19,A4,b104),(r19,A4,b105),(r19,A4,b106),(r19,A4,b107),( r19,A4,b108),(r19,A4,b109),(r19,A4,b110),(r19,A4,b111),(r19,A4,b112),(r19,A4,b113),(r 9,A4,b114),(r19,A4,b115),(r19,A4,b116),(r19,A4,b117),(r19,A4,b118),(r19,A4,b119),(r19, A4,b120),(r19,A4,b121),(r19,A4,b122),(r19,A4,b123),(r19,A4,b124),(r19,A4,b125),(r19,A 4,b126),(r19,A4,b127),(r19,A4,b128),(r19,A4,b129),(r19,A4,b130),(r19,A4,b131),(r19,A4, b132),(r19,A4,b133),(r19,A4,b134),(r20,A1,b1),(r20,A1,b2),(r20,A1,b3),(r20,A1,b4),(r20, A1,b5),(r20,A1,b6),(r20,A1,b7),(r20,A1,b8),(r20,A1,b9),(r20,A1,b10),(r20,A1,b11),(r20,A1 ,b12),(r20,A1,b13),(r20,A1,b14),(r20,A1,b15),(r20,A1,b16),(r20,A1,b17),(r20,A1,b18),(r20 ,A1,b19),(r20,A1,b20),(r20,A1,b21),(r20,A1,b22),(r20,A1,b23),(r20,A1,b24),(r20,A1,b25),( r20,A1,b26),(r20,A1,b27),(r20,A1,b28),(r20,A1,b29),(r20,A1,b30),(r20,A1,b31),(r20,A1,b 32),(r20,A1,b33),(r20,A1,b34),(r20,A1,b35),(r20,A1,b36),(r20,A1,b37),(r20,A1,b38),(r20, A1,b39),(r20,A1,b40),(r20,A1,b41),(r20,A1,b42),(r20,A1,b43),(r20,A1,b44),(r20,A1,b45),( r20,A1,b46),(r20,A1,b47),(r20,A1,b48),(r20,A1,b49),(r20,A1,b50),(r20,A1,b51),(r20,A1,b 52),(r20,A1,b53),(r20,A1,b54),(r20,A1,b55),(r20,A1,b56),(r20,A1,b57),(r20,A1,b58),(r20, A1,b59),(r20,A1,b60),(r20,A1,b61),(r20,A1,b62),(r20,A1,b63),(r20,A1,b64),(r20,A1,b65),( r20,A1,b66),(r20,A1,b67),(r20,A1,b68),(r20,A1,b69),(r20,A1,b70),(r20,A1,b71),(r20,A1,b 72),(r20,A1,b73),(r20,A1,b74),(r20,A1,b75),(r20,A1,b76),(r20,A1,b77),(r20,A1,b78),(r20, A1,b79),(r20,A1,b80),(r20,A1,b81),(r20,A1,b82),(r20,A1,b83),(r20,A1,b84),(r20,A1,b85),( r20,A1,b86),(r20,A1,b87),(r20,A1,b88),(r20,A1,b89),(r20,A1,b90),(r20,A1,b91),(r20,A1,b 92),(r20,A1,b93),(r20,A1,b94),(r20,A1,b95),(r20,A1,b96),(r20,A1,b97),(r20,A1,b98),(r20, A1,b99),(r20,A1,b100),(r20,A1,b101),(r20,A1,b102),(r20,A1,b103),(r20,A1,b104),(r20,A1, b105),(r20,A1,b106),(r20,A1,b107),(r20,A1,b108),(r20,A1,b109),(r20,A1,b110),(r20,A1,b1 11),(r20,A1,b112),(r20,A1,b113),(r20,A1,b114),(r20,A1,b115),(r20,A1,b116),(r20,A1,b117) ,(r20,A1,b118),(r20,A1,b119),(r20,A1,b120),(r20,A1,b121),(r20,A1,b122),(r20,A1,b123),(r 20,A1,b124),(r20,A1,b125),(r20,A1,b126),(r20,A1,b127),(r20,A1,b128),(r20,A1,b129),(r20 ,A1,b130),(r20,A1,b131),(r20,A1,b132),(r20,A1,b133),(r20,A1,b134),(r20,A2,b1),(r20,A2, b2),(r20,A2,b3),(r20,A2,b4),(r20,A2,b5),(r20,A2,b6),(r20,A2,b7),(r20,A2,b8),(r20,A2,b9),( r20,A2,b10),(r20,A2,b11),(r20,A2,b12),(r20,A2,b13),(r20,A2,b14),(r20,A2,b15),(r20,A2,b1 6),(r20,A2,b17),(r20,A2,b18),(r20,A2,b19),(r20,A2,b20),(r20,A2,b21),(r20,A2,b22),(r20,A 2,b23),(r20,A2,b24),(r20,A2,b25),(r20,A2,b26),(r20,A2,b27),(r20,A2,b28),(r20,A2,b29),(r2 0,A2,b30),(r20,A2,b31),(r20,A2,b32),(r20,A2,b33),(r20,A2,b34),(r20,A2,b35),(r20,A2,b36) ,(r20,A2,b37),(r20,A2,b38),(r20,A2,b39),(r20,A2,b40),(r20,A2,b41),(r20,A2,b42),(r20,A2, b43),(r20,A2,b44),(r20,A2,b45),(r20,A2,b46),(r20,A2,b47),(r20,A2,b48),(r20,A2,b49),(r20, A2,b50),(r20,A2,b51),(r20,A2,b52),(r20,A2,b53),(r20,A2,b54),(r20,A2,b55),(r20,A2,b56),( r20,A2,b57),(r20,A2,b58),(r20,A2,b59),(r20,A2,b60),(r20,A2,b61),(r20,A2,b62),(r20,A2,b 63),(r20,A2,b64),(r20,A2,b65),(r20,A2,b66),(r20,A2,b67),(r20,A2,b68),(r20,A2,b69),(r20, A2,b70),(r20,A2,b71),(r20,A2,b72),(r20,A2,b73),(r20,A2,b74),(r20,A2,b75),(r20,A2,b76),( r20,A2,b77),(r20,A2,b78),(r20,A2,b79),(r20,A2,b80),(r20,A2,b81),(r20,A2,b82),(r20,A2,b 83),(r20;A2,b84),(r20,A2,b85),(r20,A2,b86),(r20,A2,b87),(r20,A2,b88),(r20,A2,b89),(r20, A2,b90), (r20,A2,b91), (r20,A2,b92); (r20,A2,b93), (r20,A2,b94), (r20,A2,b95), (r20,A2,b96), ( r20,A2,b97),(r20,A2,b98),(r20,A2,b99),(r20,A2,b100),(r20,A2,b101),(r20,A2,b102),(r20,A 2,b103),(r20,A2,b104),(r20,A2,b105),(r20,A2,b106),(r20,A2,b107),(r20,A2,b108),(r20,A2, b109),(r20,A2,b110),(r20,A2,b111),(r20,A2,b112),(r20,A2,b113),(r20,A2,b114),(r20,A2,b1 15),(r20,A2,b116),(r20,A2,b117),(r20,A2,b118),(r20,A2,b119),(r20,A2,b120),(r20,A2,b121 ),(r20,A2,b122),(r20,A2,b123),(r20,A2,b124),(r20,A2,b125),(r20,A2,b126),(r20,A2,b127),( r20,A2,b128),(r20,A2,b129),(r20,A2,b130),(r20,A2,b131),(r20,A2,b132),(r20,A2,b133),(r2 0,A2,b134),
(r20,A3,b1),(r20,A3,b2),(r20,A3,b3),(r20,A3,b4),(r20,A3,b5),(r20,A3,b6),(r20,A3,b7),(r20, A3,b8),(r20,A3,b9),(r20,A3,b10),(r20,A3,b11),(r20,A3,b12),(r20,A3,b13),(r20,A3,b14),(r2 0,A3,b15),(r20,A3,b16),(r20,A3,b17),(r20,A3,b18),(r20,A3,b19),(r20,A3,b20),(r20,A3,b21) ,(r20,A3,b22),(r20,A3,b23),(r20,A3,b24),(r20,A3,b25),(r20,A3,b26),(r20,A3,b27),(r20,A3, b28),(r20,A3,b29),(r20,A3,b30),(r20,A3,b31),(r20,A3,b32),(r20,A3,b33),(r20,A3,b34),(r20, A3,b35),(r20,A3,b36),(r20,A3,b37),(r20,A3,b38),(r20,A3,b39),(r20,A3,b40),(r20,A3,b41),( r20,Δ3,b42),(r20,A3,b43),(r20,A3,b44),(r20,A3,b45),(r20,A3,b46),(r20,A3,b47),(r20,A3,b 48),(r20,A3,b49),(r20,A3,b50),(r20,A3,b51),(r20,A3,b52),(r20,A3,b53),(r20,A3,b54),(r20, A3,b55),(r20,A3,b56),(r20,A3,b57),(r20,A3,b58),(r20,A3,b59),(r20,A3,b60),(r20,A3,b61),( r20,A3,b62),(r20,A3,b63),(r20,A3,b64),(r20,A3,b65),(r20,A3,b66),(r20,A3,b67),(r20,A3,b 68),(r20,A3,b69),(r20,A3,b70),(r20,A3,b71),(r20,A3,b72),(r20,A3,b73),(r20,A3,b74),(r20, A3,b75),(r20,A3,b76),(r20,A3,b77),(r20,A3,b78),(r20,A3,b79),(r20,A3,b80),(r20,A3,b81),( r20,A3,b82),(r20,A3,b83),(r20,A3,b84),(r20,A3,b85),(r20,A3,b86),(r20,A3,b87),(r20,A3,b 88),(r20,A3,b89),(r20,A3,b90),(r20,A3,b91),(r20,A3,b92),(r20,A3,b93),(r20,A3,b94),(r20, A3,b95),(r20,A3,b96),(r20,A3,b97),(r20,A3,b98),(r20,A3,b99),(r20,A3,b100),(r20,A3,b101 ),(r20,A3,b102),(r20,A3,b103),(r20,A3,b104),(r20,A3,b105),(r20,A3,b106),(r20,A3,b107),( r20,A3,b108),(r20,A3,b109),(r20,A3,b110),(r20,A3,b111),(r20,A3,b112),(r20,A3,b113),(r2 0,A3,b114),(r20,A3,b115),(r20,A3,b116),(r20,A3,b117),(r20,A3,b118),(r20,A3,b119),(r20, A3,b120),(r20,A3,b121),(r20,A3,b122),(r20,A3,b123),(r20,A3,b124),(r20,A3,b125),(r20,A 3,b126),(r20,A3,b127),(r20,A3,b128),(r20,A3,b129),(r20,A3,b130),(r20,A3,b131),(r20,A3, b132),(r20,A3,b133),(r20,A3,b134),(r20,A4,b1),(r20,A4,b2),(r20,A4,b3),(r20,A4,b4),(r20, A4,b5),(r20,A4,b6),(r20,A4,b7),(r20,A4,b8),(r20,A4,b9),(r20,A4,b10),(r20,A4,b11),(r20,A4 ,b12),(r20,A4,b13),(r20,A4,b14),(r20,A4,b15),(r20,A4,b16),(r20,A4,b17),(r20,A4,b18),(r20 ,A4,b19),(r20,A4,b20),(r20,A4,b21),(r20,A4,b22),(r20,A4,b23),(r20,A4,b24),(r20,A4,b25),( r20,A4,b26),(r20,A4,b27),(r20,A4,b28),(r20,A4,b29),(r20,A4,b30),(r20,A4,b31),(r20,A4,b 32),(r20,A4,b33),(r20,A4,b34),(r20,A4,b35),(r20,A4,b36),(r20,A4,b37),(r20,A4,b38),(r20, A4,b39),(r20,A4,b40),(r20,A4,b41),(r20,A4,b42),(r20,A4,b43),(r20,A4,b44),(r20,A4,b45),( r20,A4,b46),(r20,A4,b47),(r20,A4,b48),(r20,A4,b49),(r20,A4,b50),(r20,A4,b51),(r20,A4,b 52),(r20,A4,b53),(r20,A4,b54),(r20,A4,b55),(r20,A4,b56),(r20,A4,b57),(r20,A4,b58),(r20, A4,b59),(r20,A4,b60),(r20,A4,b6l),(r20,A4,b62),(r20,A4,b63),(r20,A4,b64),(r20,A4,b65),( r20,A4,b66),(r20,A4,b67),(r20,A4,b68),(r20,A4,b69),(r20,A4,b70),(r20,A4,b71),(r20,A4,b 72),(r20,A4,b73),(r20,A4,b74),(r20,A4,b75),(r20,A4,b76),(r20,A4,b77),(r20,A4,b78),(r20, A4,b79),(r20,A4,b80),(r20,A4,b81),(r20,A4,b82),(r20,A4,b83),(r20,A4,b84),(r20,A4,b85),( r20,A4,b86),(r20,A4,b87),(r20,A4,b88),(r20,A4,b89),(r20,A4,b90),(r20,A4,b9l),(r20,A4,b 92),(r20,A4,b93),(r20,A4,b94),(r20,A4,b95),(r20,A4,b96),(r20,A4,b97),(r20,A4,b98),(r20, A4,b99),(r20,A4,b100),(r20,A4,b101),(r20,A4,b102),(r20,A4,b103),(r20,A4,b104),(r20,A4, b105),(r20,A4,b106),(r20,A4,b107),(r20,A4,b108),(r20,A4,b109),(r20,A4,b110),(r20,A4,b1 11),(r20,A4,b112),(r20,A4,b113),(r20,A4,b114),(r20,A4,b115),(r20,A4,b116),(r20,A4,b117) ,(r20,A4,b118),(r20,A4,b119),(r20,A4,b120),(r20,A4,b121),(r20,A4,b122),(r20,A4,b123),(r 20,A4,b124),(r20,A4,b125),(r20,A4,b126),(r20,A4,b127),(r20,A4,b128),(r20,A4,b129),(r20 ,A4,b130),(r20,A4,b131),(r20,A4,b132),(r20,A4,b133),(r20,A4,b134),(r21,A1,b1),(r21,A1, b2),(r21,A1,b3),(r21,A1,b4),(r21,A1,b5),(r21,A1,b6),(r21,A1,b7),(r21,A1,b8),(r21,A1,b9),( r21,A1,b10),(r21,A1,b11),(r21,A1,b12),(r21,A1,b13),(r21,A1,b14),(r21,A1,b15),(r21,A1,b1 6),(r21,A1,b17),(r21,A1,b18),(r21,A1,b19),(r21,A1,b20),(r21,A1,b21),(r21,A1,b22),(r21,A 1,b23),(r21,A1,b24),(r21,A1,b25),(r21,A1,b26),(r21,A1,b27),(r21,A1,b28),(r21,A1,b29),(r2 1,A1,b30),(r21,A1,b31),(r21,A1,b32),(r21,A1,b33),(r21,A1,b34),(r21,A1,b35),(r21,A1,b36) ,(r21,A1,b37),(r21,A1,b38),(r21,A1,b39),(r21,A1,b40),(r21,A1,b41),(r21,A1,b42),(r21,A1, b43),(r21,A1,b44),(r21,A1,b45),(r21,A1,b46),(r21,A1,b47),(r21,A1,b48),(r21,A1,b49),(r21, A1,b50),(r21,A1,b51),(r21,A1,b52),(r21,A1,b53),(r21,A1,b54),(r21,A1,b55),(r21,A1,b56),( r21,A1,b57),(r21,A1,b58),(r21,A1,b59),(r21,A1,b60),(r21,A1,b61),(r21,A1,b62),(r21,A1,b 63),(r21,A1,b64),(r21,A1,b65),(r21,A1,b66),(r21,A1,b67),(r21,A1,b68),(r21,A1,b69),(r21, A1,b70),(r21,A1,b71),(r21,A1,b72),(r21,A1,b73),(r21,A1,b74),(r21,A1,b75),(r21,A1,b76),( r21,A1,b77),(r21,A1,b78),(r21,A1,b79),(r21,A1,b80),(r21,A1,b81),(r21,A1,b82),(r21,A1,b 83),(r21,A1,b84),(r21,A1,b85),(r21,A1,b86),(r21,A1,b87),(r21,A1,b88),(r21_{;}A1,b89),(r21, A1,b90),(r21,A1,b91),(r21,A1,b92),(r21,A1,b93),(r21,A1,b94),(r21,A1,b95),(r21,A1,b96),( r21,A1,b97),(r21,A1,b98),(r21,A1,b99),(r21,A1,b100),(r21,A1,b101),(r21,A1,b102),(r21,A 1,b103),(r21,A1,b104),(r21,A1,b105),(r21,A1,b106),(r21,A1,b107),(r21,A1,b108),(r21,A1, b109),(r21,A1,b110),(r21,A1,b111),(r21,A1,b112),(r21,A1,b113),(r21,A1,b114),(r21,A1,b1 15),(r21,A1,b116),(r21,A1,b117),(r21,A1,b118),(r21,A1,b119),(r21,A1,b120),(r21,A1,b121 ),(r21,A1,b122),(r21,A1,b123),(r21,A1,b124),(r21,A1,b125),(r21,A1,b126),(r21,A1,b127),( r21,A1,b128),(r21,A1,b129),(r21,A1,b130),(r21,A1,b131),(r21,A1,b132),(r21,A1,b133),(r2 1,A1,b134),
(r21,A2,b1),(r21,A2,b2),(r21,A2,b3),(r21,A2,b4),(r21,A2,b5),(r21,A2,b6),(r21,A2,b7),(r21, A2,b8),(r21,A2,b9),(r21,A2,b10),(r21,A2,b11),(r21,A2,b12),(r21,A2,b13),(r21,A2,b14),(r2 1,A2,b15),(r21,A2,b16),(r21,A2,b17),(r21,A2,b18),(r21,A2,b19),(r21,A2,b20),(r21,A2,b21) ,(r21,A2,b22),(r21,A2,b23),(r21,A2,b24),(r21,A2,b25),(r21,A2,b26),(r21,A2,b27),(r21,A2, b28),(r21,A2,b29),(r21,A2,b30),(r21,A2,b31),(r21,A2,b32),(r21,A2,b33),(r21,A2,b34),(r21, A2,b35)_{;}(r21,A2,b36),(r21,A2,b37),(r21,A2,b38),(r21,A2,b39),(r21,A2,b40),(r21,A2,b41),( r21,A2,b42),(r21,A2,b43),(r21,A2,b44),(r21,A2,b45),(r21,A2,b46),(r21,A2,b47),(r21,A2,b 48),(r21,A2,b49),(r21,A2,b50),(r21,A2,b51),(r21,A2,b52),(r21,A2,b53),(r21,A2,b54),(r21, A2,b55),(r21,A2,b56),(r21,A2,b57),(r21,A2,b58),(r21,A2,b59),(r21,A2,b60),(r21,A2,b61),( r21,A2,b62),(r21,A2,b63),(r21,A2,b64),(r21,A2,b65),(r21,A2,b66),(r21,A2,b67),(r21,A2,b 68),(r21,A2,b69),(r21,A2,b70),(r21,A2,b71),(r21,A2,b72),(r21,A2,b73),(r21,A2,b74),(r21, A2,b75),(r21,A2,b76),(r21,A2,b77),(r21,A2,b78),(r21,A2,b79),(r21,A2,b80),(r21,A2,b81),( r21,A2,b82),(r21,A2,b83),(r21,A2,b84),(r21,A2,b85),(r21,A2,b86),(r21,A2,b87),(r21,A2,b 88),(r21,A2,b89),(r21,A2,b90),(r21,A2,b91),(r21,A2,b92),(r21,A2,b93),(r21_{;}A2,b94),(r21, A2,b95),(r21,A2,b96),(r21,A2,b97),(r21,A2,b98),(r21,A2,b99),(r21,A2,b100),(r21,A2,b101 ),(r21,A2,b102),(r21,A2,b103),(r21,A2,b104),(r21,A2,b105),(r21,A2,b106),(r21,A2,b107),( r21,A2,b108),(r21,A2,b109),(r21,A2,b110),(r21,A2,b111),(r21,A2,b112),(r21,A2,b113),(r2 1,A2,b114),(r21,A2,b115),(r21,A2,b116),(r21,A2,b117),(r21,A2,b118),(r21,A2,b119),(r21, A2,b120),(r21,A2,b121),(r21,A2,b122),(r21,A2,b123),(r21,A2,b124),(r21,A2,b125),(r21,A 2,b126),(r21,A2,b127),(r21,A2,b128),(r21,A2,b129),(r21,A2,b130),(r21,A2,b131),(r21,A2, b132),(r21,A2,b133),(r21,A2,b134),(r21,A3,bl),(r21,A3,b2),(r21,A3,b3),(r21,A3,b4),(r21, A3,b5),(r21,A3,b6),(r21,A3,b7),(r21,A3,b8),(r21,A3,b9),(r21,A3,b10),(r21,A3,b11),(r21,A3 ,b12),(r21,A3,b13),(r21,A3,b14),(r21,A3,b15),(r21,A3,b16),(r21,A3,b17),(r21,A3,b18),(r21 ,A3,b19),(r21,A3,b20),(r21,A3,b21),(r21,A3,b22),(r21,A3,b23),(r21,A3,b24),(r21,A3,b25),( r21,A3,b26),(r21,A3,b27),(r21,A3,b28),(r21,A3,b29),(r21,A3,b30),(r21,A3,b3l),(r21,A3,b 32),(r21,A3,b33),(r21,A3,b34),(r21,A3,b35),(r21,A3,b36),(r21,A3,b37),(r21,A3,b38),(r21, A3,b39),(r21,A3,b40),(r21,A3,b41),(r21,A3,b42),(r21,A3,b43),(r21,A3,b44),(r21,A3,b45),( r21,A3,b46),(r21,A3,b47),(r21,A3,b48),(r21,A3,b49),(r21,A3,b50),(r21,A3,b51),(r21,A3,b 52),(r21,A3,b53),(r21,A3,b54),(r21,A3,b55),(r21,A3,b56),(r21,A3,b57),(r21,A3,b58),(r21, A3,b59),(r21,A3,b60),(r21,A3,b61),(r21,A3,b62),(r21,A3,b63),(r21,A3,b64),(r21,A3,b65),( r21,A3,b66),(r21,A3,b67),(r21,A3,b68),(r21,A3,b69),(r21,A3,b70),(r21,A3,b71),(r21,A3,b 72),(r21,A3,b73),(r21,A3,b74),(r21,A3,b75),(r21,A3,b76),(r21,A3,b77),(r21,A3,b78),(r21, A3,b79),(r21,A3,b80),(r21,A3,b8l),(r21,A3,b82),(r21,A3,b83),(r21,A3,b84),(r21,A3,b85),( r21,A3,b86),(r21,A3,b87),(r21,A3,b88),(r21,A3,b89),(r21,A3,b90),(r21,A3,b91),(r21,A3,b 92),(r21,A3,b93),(r21,A3,b94),(r21,A3,b95),(r21,A3,b96),(r21,A3,b97),(r21,A3,b98),(r21, A3,b99),(r21,A3,b100),(r21,A3,b101),(r21,A3,b102),(r21,A3,b103),(r21,A3,b104),(r21,A3, b105),(r21,A3,b106),(r21,A3,b107),(r21,A3,b108),(r21,A3,b109),(r21,A3,b110),(r21,A3,b1 11),(r21,A3,b112),(r21,A3,b113),(r21,A3,b114),(r21,A3,b115),(r21,A3,b116),(r21,A3,b117) ,(r21,A3,b118),(r21,A3,b119),(r21,A3,b120),(r21,A3,b121),(r21,A3,b122),(r21,A3,b123),(r 21,A3,b124),(r21,A3,b125),(r21,A3,b126),(r21,A3,b127),(r21,A3,b128),(r21,A3,b129),(r21 ,A3,b130),(r21,A3,b131),(r21,A3,b132),(r21,A3,b133),(r21,A3,b134),(r21,A4,b1),(r21,A4, b2),(r21,A4,b3),(r21,A4,b4),(r21,A4,b5),(r21,A4,b6),(r21,A4,b7),(r21,A4,b8),(r21,A4,b9),( r21,A4,b10),(r21,A4,b11),(r21,A4,b12),(r21,A4,b13),(r21,A4,b14),(r21,A4,b15),(r21,A4,b1 6),(r21,A4,b17),(r21,A4,b18),(r21,A4,b19),(r21,A4,b20),(r21,A4,b21),(r21,A4,b22),(r21,A 4,b23),(r21,A4,b24),(r21,A4,b25),(r21,A4,b26),(r21,A4,b27),(r21,A4,b28),(r21,A4,b29),(r2 1,A4,b30),(r21,A4,b31),(r21,A4,b32),(r21,A4,b33),(r21,A4,b34),(r21,A4,b35),(r21,A4,b36) ,(r21,A4,b37),(r21,A4,b38),(r21,A4,b39),(r21,A4,b40),(r21,A4,b41),(r21,A4,b42),(r21,A4, b43),(r21,A4,b44),(r21,A4,b45),(r21,A4,b46),(r21,A4,b47),(r21,A4,b48),(r21,A4,b49),(r21, A4,b50),(r21,A4,b51),(r21,A4,b52),(r21,A4,b53),(r21,A4,b54),(r21,A4,b55),(r21,A4,b56),( r21,A4,b57),(r21,A4,b58),(r21,A4,b59),(r21,A4,b60),(r21,A4,b61),(r21,A4,b62),(r21,A4,b 63),(r21,A4,b64),(r21,A4,b65),(r21,A4,b66),(r21,A4,b67),(r21,A4,b68),(r21,A4,b69),(r21, A4,b70),(r21,A4,b71),(r21,A4,b72),(r21,A4,b73),(r21,A4,b74),(r21,A4,b75),(r21,A4,b76),( r21,A4,b77),(r21,A4,b78),(r21,A4,b79),(r21,A4,b80),(r21,A4,b81),(r21,A4,b82),(r21,A4,b 83),(r21,A4,b84),(r21,A4,b85),(r21,A4,b86),(r21,A4,b87),(r21,A4,b88),(r21,A4,b89),(r21, A4,b90),(r21,A4,b91),(r21,A4,b92),(r21,A4,b93),(r21,A4,b94),(r21,A4,b95),(r21,A4,b96),( r21,A4,b97),(r21,A4,b98),(r21,A4,b99),(r21,A4,b100),(r21,A4,b101),(r21,A4,b102),(r21,A 4,b103),(r21,A4,b104),(r21,A4,b105),(r21,A4,b106),(r21,A4,b107),(r21,A4,b108),(r21,A4, b109),(r21,A4,b110),(r21,A4,b111),(r21,A4,b112),(r21,A4,b113),(r21,A4,b114),(r21,A4,b1 15),(r21,A4,b116),(r21,A4,b117),(r21,A4,b118),(r21,A4,b119),(r21,A4,b120),(r21,A4,b121 ),(r21,A4,b122),(r21,A4,b123),(r21,A4,b124),(r21,A4,b125),(r21,A4,b126),(r21,A4,b127),( r21,A4,b128),(r21,A4,b129),(r21,A4,b130),(r21,A4,b131),(r21,A4,b132),(r21,A4,b133),(r2 1,A4,b134),
(r22,A1,b1),(r22,A1,b2),(r22,A1,b3),(r22,A1,b4),(r22,A1,b5),(r22,A1,b6),(r22,A1,b7),(r22, A1,b8),(r22,A1,b9),(r22,A1,b10),(r22,A1,b11),(r22,A1,b12),(r22,A1,b13),(r22,A1,b14),(r2 2,A1,b15),(r22,A1,b16),(r22,A1,b17),(r22,A1,b18),(r22,A1,b19),(r22,A1,b20),(r22,A1,b21) ,(r22,A1,b22),(r22,A1,b23),(r22,A1,b24),(r22,A1,b25),(r22,A1,b26),(r22,A1,b27),(r22,A1, b28),(r22,A1,b29),(r22,A1,b30),(r22,A1,b31),(r22,A1,b32),(r22,A1,b33),(r22,A1,b34),(r22, A1,b35),(r22,A1,b36),(r22,A1,b37),(r22,A1,b38),(r22,A1,b39),(r22,A1,b40),(r22,A1,b41),( r22,A1,b42),(r22,A1,b43),(r22,A1,b44),(r22,A1,b45),(r22,A1,b46),(r22,A1,b47),(r22,A1,b 48),(r22,A1,b49),(r22,A1,b50),(r22,A1,b51),(r22,A1,b52),(r22,A1,b53),(r22,A1,b54),(r22, A1,b55),(r22,A1,b56),(r22,A1,b57),(r22,A1,b58),(r22,A1,b59),(r22,A1,b60),(r22,A1,b61)_{;}( r22,A1,b62),(r22,A1,b63),(r22,A1,b64),(r22,A1,b65),(r22,A1,b66),(r22,A1,b67),(r22,A1,b 68),(r22,A1,b69),(r22,A1,b70),(r22,A1,b71),(r22,A1,b72),(r22,A1,b73),(r22,A1,b74),(r22, A1,b75),(r22,A1,b76),(r22,A1,b77),(r22,A1,b78),(r22,A1,b79),(r22,A1,b80),(r22,A1,b81),( r22,A1,b82),(r22,A1,b83),(r22,A1,b84),(r22,A1,b85),(r22,A1,b86),(r22,A1,b87),(r22,A1,b 88),(r22,A1;b89),(r22,A1,b90),(r22,A1,b91),(r22,A1,b92),(r22,A1,b93),(r22,A1,b94),(r22, A1,b95),(r22,A1,b96),(r22,A1,b97),(r22,A1,b98),(r22,A1,b99),(r22,A1,b100),(r22,A1,b101 ),(r22,A1,b102),(r22,A1,b103),(r22,A1,b104),(r22,A1,b105),(r22,A1,b106),(r22,A1,b107),( r22,A1,b108),(r22,A1,b109),(r22,A1,b110),(r22,A1,b111),(r22,A1,b112),(r22,A1,b113),(r2 2,A1,b114),(r22,A1,b115),(r22,A1,b116),(r22,A1,b117),(r22,A1,b118),(r22,A1,b119),(r22, A1,b120),(r22,A1,b121),(r22,A1,b122),(r22,A1,b123),(r22,A1,b124),(r22,A1,b125),(r22,A 1,b126),(r22,A1,b127),(r22,A1,b128),(r22,A1,b129),(r22,A1,b130),(r22,A1,b131),(r22,A1, b132),(r22,A1,b133),(r22,A1,b134),(r22,A2,b1),(r22,A2,b2),(r22,A2,b3),(r22,A2,b4),(r22, A2,b5),(r22,A2,b6),(r22,A2,b7),(r22,A2,b8),(r22,A2,b9),(r22,A2,b10),(r22,A2,b11),(r22,A2 ,b12),(r22,A2,b13),(r22,A2,b14),(r22,A2,b15),(r22,A2,b16),(r22,A2,b17),(r22,A2,b18),(r22 ,A2,b19),(r22,A2,b20),(r22,A2,b21),(r22,A2,b22),(r22,A2,b23),(r22,A2,b24),(r22,A2,b25),( r22,A2,b26),(r22,A2,b27),(r22,A2,b28),(r22,A2,b29),(r22,A2,b30),(r22,A2,b31)_{;}(r22,A2,b 32),(r22,A2,b33),(r22,A2,b34),(r22,A2,b35),(r22,A2,b36),(r22,A2,b37),(r22,A2,b38),(r22, A2,b39),(r22,A2,b40),(r22,A2,b41),(r22,A2,b42),(r22,A2,b43),(r22,A2,b44),(r22,A2,b45),( r22,A2,b46),(r22,A2,b47),(r22,A2,b48),(r22,A2,b49),(r22,A2,b50),(r22,A2,b51),(r22,A2,b 52),(r22,A2,b53),(r22,A2,b54),(r22,A2,b55),(r22,A2,b56),(r22,A2,b57),(r22,A2,b58),(r22, A2,b59),(r22,A2,b60),(r22,A2,b61),(r22,A2,b62),(r22,A2,b63),(r22,A2,b64),(r22,A2,b65),( r22,A2,b66),(r22,A2,b67),(r22,A2,b68),(r22,A2,b69),(r22,A2,b70),(r22,A2,b71),(r22,A2,b 72),(r22,A2,b73),(r22,A2,b74),(r22,A2,b75),(r22,A2,b76),(r22,A2,b77),(r22,A2,b78),(r22, A2,b79),(r22,A2,b80),(r22,A2,b81),(r22,A2,b82),(r22,A2,b83),(r22,A2,b84),(r22,A2,b85),( r22,A2,b86),(r22,A2,b87),(r22,A2,b88),(r22,A2,b89),(r22,A2,b90),(r22,A2,b91),(r22,A2,b 92),(r22,A2,b93),(r22,A2,b94),(r22,A2,b95),(r22,A2,b96),(r22,A2,b97),(r22,A2,b98),(r22, A2,b99),(r22,A2,b 100),(r22,A2,b 101),(r22,A2,b 102),(r22,A2,b103),(r22,A2,b 104),(r22,A2, b105),(r22,A2,b106),(r22,A2,b107),(r22,A2,b108),(r22,A2,b109),(r22,A2,b110),(r22,A2,b1 11),(r22,A2,b112),(r22,A2,b113),(r22,A2,b114),(r22,A2,b115),(r22,A2,b116),(r22,A2,b117) ,(r22,A2,b118),(r22,A2,b119),(r22,A2,b120),(r22,A2,b121),(r22,A2,b122),(r22,A2,b123),(r 22,A2,b124),(r22,A2,b125),(r22,A2,b126),(r22,A2,b127),(r22,A2,b128),(r22,A2,b129),(r22 ,A2,b130),(r22,A2,b131),(r22,A2,b132),(r22,A2,b133),(r22,A2,b134),(r22,A3,b1),(r22,A3, b2),(r22,A3,b3),(r22,A3,b4),(r22,A3,b5),(r22,A3,b6),(r22,A3,b7),(r22,A3,b8),(r22,A3,b9),( r22,A3,b10),(r22,A3,b11),(r22,A3,b12),(r22,A3,b13),(r22,A3,b14),(r22,A3,b15),(r22,A3,b1 6),(r22,A3,b17),(r22,A3,b18),(r22,A3,b19),(r22,A3,b20),(r22,A3,b21),(r22,A3,b22),(r22,A 3,b23),(r22,A3,b24),(r22,A3,b25),(r22,A3,b26),(r22,A3,b27),(r22,A3,b28),(r22,A3,b29),(r2 2,A3,b30),(r22,A3,b31),(r22,A3,b32),(r22,A3,b33),(r22,A3,b34),(r22,A3,b35),(r22,A3,b36) ,(r22,A3,b37),(r22,A3,b38),(r22,A3,b39),(r22,A3,b40),(r22,A3,b41),(r22,A3,b42),(r22,A3, b43), (r22,A3,b44), (r22,A3,b45), (r22,A3,b46), (r22,A3,b47), (r22,A3,b48), (r22,A3,b49), (r22, A3,b50),(r22,A3,b51),(r22,A3,b52),(r22,A3,b53),(r22,A3,b54),(r22,A3,b55),(r22,A3,b56),( r22,A3,b57),(r22,A3,b58),(r22,A3,b59),(r22,A3,b60),(r22,A3,b61),(r22,A3,b62),(r22,A3,b 63),(r22,A3,b64),(r22,A3,b65),(r22,A3,b66),(r22,A3,b07),(r22,A3,b68),(r22,A3,b69),(r22, A3,b70),(r22,A3,b71),(r22,A3,b72),(r22,A3,b73),(r22,A3,b74),(r22,A3,b75),(r22,A3,b76),( r22,A3,b77),(r22,A3,b78),(r22,A3,b79),(r22,A3,b80),(r22,A3,b81),(r22,A3,b82),(r22,A3,b 83),(r22,A3,b84),(r22,A3,b85),(r22,A3,b86),(r22,A3,b87),(r22,A3,b88),(r22,A3,b89),(r22, A3,b90),(r22,A3,b91),(r22,A3,b92),(r22,A3,b93),(r22,A3,b94),(r22,A3,b95),(r22,A3,b96),( r22,A3,b97),(r22,A3,b98),(r22,A3,b99),(r22,A3,b100),(r22,A3,b101),(r22,A3,b102),(r22,A 3,b103),(r22,A3,b104),(r22,A3,b105),(r22,A3,b106),(r22,A3,b107),(r22,A3,b108),(r22,A3, b109),(r22,A3,b110),(r22,A3,b111),(r22,A3,b112),(r22,A3,b113),(r22,A3,b114),(r22,A3,b1 15),(r22,A3,b 116),(r22,A3,b 117),(r22,A3,b 118),(r22,A3,b 119),(r22,A3,b 120),(r22,A3,b 121 ),(r22,A3,b122),(r22,A3,b123),(r22,A3,b124),(r22,A3,b125),(r22,A3,b126),(r22,A3,b127),( r22,A3,b128),(r22,A3,b129),(r22,A3,b130),(r22,A3,b131),(r22,A3,b132),(r22,A3,b133),(r2 2,A3,b134),
(r22,A4,b1),(r22,A4,b2),(r22,A4,b3),(r22,A4,b4),(r22,A4,b5),(r22,A4,b6),(r22,A4,b7),(r22, A4,b8),(r22,A4,b9),(r22,A4,b10),(r22,A4,b11),(r22,A4,b12),(r22,A4,b13),(r22,A4,b14),(r2 2,A4,b15),(r22,A4,b16),(r22,A4,b17),(r22,A4,b18),(r22,A4,b19),(r22,A4,b20),(r22,A4,b21) ,(r22,A4,b22),(r22,A4,b23),(r22,A4,b24),(r22,A4,b25),(r22,A4,b26),(r22,A4,b27),(r22,A4, b28),(r22,A4,b29),(r22,A4,b30),(r22,A4,b31),(r22,A4,b32),(r22,A4,b33),(r22,A4,b34),(r22, A4,b35),(r22,A4,b36),(r22,A4,b37),(r22,A4,b38),(r22,A4,b39),(r22,A4,b40),(r22,A4,b41),( r22,A4,b42),(r22,A4,b43),(r22,A4,b44),(r22,A4,b45),(r22,A4,b46),(r22,A4,b47),(r22,A4,b 48),(r22,A4,b49),(r22,A4,b50),(r22,A4,b51),(r22,A4,b52),(r22,A4,b53),(r22,A4,b54),(r22, A4,b55),(r22,A4,b56),(r22,A4,b57),(r22,A4,b58),(r22,A4,b59),(r22,A4,b60),(r22,A4,b61),( r22,A4,b62),(r22,A4,b63),(r22,A4,b64),(r22,A4,b65),(r22,A4,b66),(r22,A4,b67),(r22,A4,b 68),(r22,A4,b69),(r22,A4,b70),(r22,A4,b71),(r22,A4,b72),(r22,A4,b73),(r22,A4,b74),(r22, A4,b75),(r22,A4,b76),(r22,A4,b77),(r22,A4,b78),(r22,A4,b79),(r22,A4,b80),(r22,A4,b81),( r22,A4,b82),(r22,A4,b83),(r22,A4,b84),(r22,A4,b85),(r22,A4,b86),(r22,A4,b87),(r22,A4,b 88),(r22,A4,b89),(r22,A4,b90),(r22,A4,b91),(r22,A4,b92),(r22,A4,b93),(r22,A4,b94),(r22, A4,b95),(r22,A4,b96),(r22,A4,b97),(r22,A4,b98),(r22,A4,b99),(r22,A4,b100),(r22,A4,b101 ),(r22,A4,b102),(r22,A4,b103),(r22,A4,b104),(r22,A4,b105),(r22,A4,b106),(r22,A4,b107),( r22,A4,b108),(r22,A4,b109),(r22,A4,b110),(r22,A4,b111),(r22,A4,b112),(r22,A4,b113),(r2 2,A4,b114),(r22,A4,b115),(r22,A4,b116),(r22,A4,b117),(r22,A4,b118),(r22,A4,b119),(r22, A4,b120),(r22,A4,b121),(r22,A4,b122),(r22,A4,b123),(r22,A4,b124),(r22,A4,b125),(r22,A 4,b126),(r22,A4,b127),(r22,A4,b128),(r22,A4,b129),(r22,A4,b130),(r22,A4,b131),(r22,A4, b132),(r22,A4,b133),(r22,A4,b134),(r23,A1,b1),(r23,A1,b2),(r23,A1,b3),(r23,A1,b4),(r23, A1,b5),(r23,A1,b6),(r23,A1,b7),(r23,A1,b8),(r23,A1,b9),(r23,A1,b10),(r23,A1,b11),(r23,A1 ,b12),(r23,A1,b13),(r23,A1,b14),(r23,A1,b15),(r23,A1,b16),(r23,A1,b17),(r23,A1,b18),(r23 ,A1,b19),(r23,A1,b20),(r23,A1,b21),(r23,A1,b22),(r23,A1,b23),(r23,A1,b24),(r23,A1,b25),( r23,A1,b26);(r23,A1,b27)_{;}(r23,A1,b28),(r23,A1,b29),(r23,A1,b30),(r23,A1,b31),(r23,A1,b 32),(r23,A1,b33),(r23,A1,b34),(r23,A1,b35),(r23,A1,b36),(r23,A1,b37),(r23,A1,b38),(r23, A1,b39),(r23,A1,b40),(r23,A1,b41),(r23,A1,b42),(r23,A1,b43),(r23,A1,b44),(r23,A1,b45),( r23,A1,b46),(r23,A1,b47),(r23,A1,b48),(r23,A1,b49),(r23,A1,b50),(r23,A1,b51),(r23,A1,b 52),(r23,A1,b53),(r23,A1,b54),(r23,A1,b55),(r23,A1,b56),(r23,A1,b57),(r23,A1,b58),(r23, A1,b59),(r23,A1,b60),(r23,A1,b61),(r23,A1,b62),(r23,A1,b63),(r23,A1,b64),(r23,A1,b65),( r23,A1,b66),(r23,A1,b67),(r23,A1,b68),(r23,A1,b69),(r23,A1,b70),(r23,A1,b71),(r23,A1,b 72),(r23,A1,b73),(r23,A1,b74),(r23,A1,b75),(r23,A1,b76),(r23,A1,b77),(r23,A1,b78),(r23, A1,b79),(r23,A1,b80),(r23,A1,b81),(r23,A1,b82),(r23,A1,b83),(r23,A1,b84),(r23,A1,b85),( r23,A1,b86),(r23,A1,b87),(r23,A1,b88),(r23,A1,b89),(r23,A1,b90),(r23,A1,b91),(r23,A1,b 92),(r23,A1,b93),(r23,A1,b94),(r23,A1,b95),(r23,A1,b96),(r23,A1,b97),(r23,A1,b98),(r23, A1,b99),(r23,Al,b100),(r23,A1,b101),(r23,A1,b102),(r23,A1,b103),(r23,A1,b104),(r23,A1, b105),(r23,A1,b106),(r23,A1,b107),(r23,A1,b108),(r23,A1,b 109),(r23,A1,b110),(r23,A1,b1 11),(r23,A1,b112),(r23,A1,b113),(r23,A1,b114),(r23,A1,b115),(r23,A1,b116),(r23,A1,b117) ,(r23,A1,b118),(r23,A1,b119),(r23,A1,b120),(r23,A1,b121),(r23,A1,b122),(r23,A1,b123),(r 23,A1,b124),(r23,A1,b125),(r23,A1,b126),(r23,A1,b127),(r23,A1,b128),(r23,A1,b129),(r23 ,A1,b130),(r23,A1,b131),(r23,A1,b132),(r23,A1,b133),(r23,A1,b134),(r23,A2,b1),(r23,A2, b2),(r23,A2,b3),(r23,A2,b4),(r23,A2,b5),(r23,A2,b6),(r23,A2,b7),(r23,A2,b8),(r23,A2,b9),( r23,A2,b10),(r23,A2,b11),(r23,A2,b12),(r23,A2,b13),(r23,A2^{,}b14),(r23,A2,b15),(r23,A2,b1 0),(r23,A2,b17),(r23,A2,b18),(r23,A2,b19),(r23,A2,b20),(r23,A2,b21),(r23,A2,b22),(r23,A 2,b23),(r23,A2,b24),(r23,A2,b25),(r23,A2,b26),(r23,A2,b27),(r23,A2,b28),(r23,A2,b29),(r2 3,A2,b30),(r23,A2,b31),(r23,A2,b32),(r23,A2,b33),(r23,A2,b34),(r23,A2,b35),(r23,A2,b36) ,(r23,A2,b37),(r23,A2,b38),(r23,A2,b39),(r23,A2,b40),(r23,A2,b41),(r23,A2,b42),(r23,A2, b43),(r23,A2,b44),(r23,A2,b45),(r23,A2,b46);(r23,A2,b47),(r23,A2,b48),(r23,A2,b49),(r23, A2,b50),(r23,A2,b51),(r23,A2,b52),(r23,A2,b53),(r23,A2,b54),(r23,A2,b55),(r23,A2,b56),( r23,A2,b57),(r23,A2,b58),(r23,A2,b59),(r23,A2,b60),(r23,A2,b61),(r23,A2,b62),(r23,A2,b 63),(r23,A2,b64),(r23,A2,b65),(r23,A2,b66),(r23,A2,b67),(r23,A2,b68),(r23,A2,b69),(r23, A2,b70),(r23,A2,b71),(r23,A2,b72),(r23,A2,b73),(r23,A2,b74),(r23,A2,b75),(r23,A2,b76),( r23,A2,b77),(r23,A2,b78),(r23,A2,b79),(r23,A2,b80),(r23,A2,b81),(r23,A2,b82),(r23,A2,b 83),(r23,A2,b84),(r23,A2,b85),(r23,A2,b86),(r23,A2,b87),(r23,A2,b88),(r23,A2,b89),(r23, A2,b90),(r23,A2,b91),(r23,A2,b92),(r23,A2,b93),(r23,A2,b94),(r23,A2,b95),(r23,A2,b96),( r23,A2,b97),(r23,A2,b98),(r23,A2,b99),(r23,A2,b100),(r23,A2,b101),(r23,A2,b102),(r23,A 2,b103),(r23,A2,b104),(r23,A2,b105),(r23,A2,b106),(r23,A2,b107),(r23,A2,b108),(r23,A2, b109),(r23,A2,b110),(r23,A2,b111),(r23,A2,b112),(r23,A2,b113),(r23,A2,b114),(r23,A2,b1 15),(r23,A2,b116),(r23,A2,b117),(r23,A2,b118),(r23,A2,b119),(r23,A2,b120),(r23,A2,b121 ),(r23,A2,b122),(r23,A2,b123),(r23,A2,b124),(r23,A2,b125),(r23,A2,b126),(r23,A2,b127), ( r23,A2,b128),(r23,A2,b129),(r23,A2,b130),(r23,A2,b131),(r23,A2,b132),(r23,A2,b133),(r2 3,A2,b134),
(r23,A3,b1),(r23,A3,b2),(r23,A3,b3),(r23,A3,b4),(r23,A3,b5),(r23,A3,b6),(r23,A3,b7),(r23, A3,b8),(r23,A3,b9),(r23,A3,b10),(r23,A3,b11),(r23,A3,b12),(r23,A3,b13),(r23,A3,b14),(r2 3,A3,b15),(r23,A3,b16),(r23,A3,b17),(r23,A3,b18),(r23,A3,b19),(r23,A3,b20),(r23,A3,b21) ,(r23,A3,b22),(r23,A3,b23),(r23,A3,b24),(r23,A3,b25),(r23,A3,b26),(r23,A3,b27),(r23,A3, b28),(r23,A3,b29),(r23,A3,b30),(r23,A3,b31),(r23,A3,b32),(r23,A3,b33),(r23,A3,b34),(r23, A3,b35),(r23,A3,b36),(r23_{;}A3,b37),(r23,A3,b38),(r23,A3,b39),(r23,A3,b40),(r23,A3,b41), ( r23_{;}A3,b42),(r23,A3,b43),(r23,A3,b44),(r23,A3,b45),(r23,A3,b46),(r23,A3,b47),(r23,A3,b 48),(r23,A3,b49),(r23,A3,b50),(r23,A3,b51),(r23,A3,b52),(r23,A3,b53),(r23,A3,b54),(r23, A3,b55),(r23,A3,b56),(r23,A3,b57),(r23,A3,b58),(r23,A3,b59),(r23,A3,b60),(r23,A3,b61),( r23,A3_{;}b62),(r23,A3,b63),(r23,A3,b64),(r23,A3,b65),(r23,A3,b66),(r23,A3,b67),(r23,A3,b 68),(r23,A3,b69),(r23,A3,b70),(r23,A3,b71),(r23,A3,b72),(r23,A3,b73),(r23,A3,b74),(r23, A3,b75),(r23,A3,b76),(r23,A3,b77),(r23,A3,b78),(r23,A3,b79),(r23,A3,b80),(r23,A3,b81),( r23,A3,b82),(r23,A3,b83),(r23,A3,b84),(r23,A3,b85),(r23,A3,b86),(r23,A3,b87),(r23,A3,b 88),(r23,A3,b89),(r23,A3,b90),(r23,A3,b91),(r23,A3,b92),(r23,A3,b93),(r23,A3,b94),(r23, A3,b95),(r23,A3,b96),(r23,A3,b97),(r23,A3,b98),(r23,A3,b99),(r23,A3,b100),(r23,A3,b101 ),(r23,A3,b102),(r23,A3,b103),(r23,A3,b104),(r23,A3,b105),(r23,A3,b106),(r23,A3,b107),( r23,A3,b108),(r23,A3,b109),(r23,A3,b110),(r23,A3,b111),(r23,A3,b112),(r23,A3,b113),(r2 3,A3,b114),(r23,A3,b115),(r23,A3,b116),(r23,A3,b117),(r23,A3,b118),(r23,A3,b119),(r23, A3,b120),(r23,A3,b121),(r23,A3,b122),(r23,A3,b123),(r23,A3,b124),(r23,A3,b125),(r23,A 3,b126),(r23,A3,b127),(r23,A3,b128),(r23,A3,b129),(r23,A3,b130),(r23,A3,b131),(r23,A3, b132),(r23,A3,b133),(r23,A3,b134),(r23,A4,b1),(r23,A4,b2),(r23,A4,b3),(r23,A4,b4),(r23, A4,b5),(r23,A4,b6),(r23,A4,b7),(r23,A4,b8),(r23,A4,b9),(r23,A4,b10),(r23,A4,b11),(r23,A4 ,b12),(r23,A4,b13),(r23,A4,b14),(r23,A4,b15),(r23,A4,b16),(r23,A4,b17),(r23,A4,b18),(r23 ,A4,b19),(r23,A4,b20),(r23,A4,b21),(r23,A4,b22),(r23,A4,b23),(r23,A4,b24),(r23,A4,b25),( r23,A4,b26),(r23,A4,b27),(r23,A4,b28),(r23,A4,b29),(r23,A4,b30),(r23,A4,b31),(r23,A4,b 32),(r23,A4,b33),(r23,A4,b34),(r23,A4,b35),(r23,A4,b36),(r23,A4,b37),(r23,A4,b38),(r23, A4,b39),(r23,A4,b40),(r23,A4,b41),(r23,A4,b42),(r23,A4,b43),(r23,A4,b44),(r23,A4,b45),( r23,A4,b46),(r23,A4,b47),(r23,A4,b48),(r23,A4,b49),(r23,A4,b50),(r23,A4,b51),(r23,A4,b 52),(r23,A4,b53),(r23,A4,b54),(r23,A4,b55),(r23,A4,b56),(r23,A4,b57),(r23,A4,b58),(r23, A4,b59),(r23,A4,b60),(r23,A4,b61),(r23,A4,b62),(r23,A4,b63),(r23,A4,b64),(r23,A4,b65),( r23,A4,b66),(r23,A4,b67),(r23,A4,b68),(r23,A4,b69),(r23,A4,b70),(r23,A4,b71),(r23,A4,b 72),(r23,A4,b73),(r23,A4,b74),(r23,A4,b75),(r23,A4,b76),(r23,A4,b77),(r23,A4,b78),(r23, A4,b79),(r23,A4,b80),(r23,A4,b81),(r23,A4,b82),(r23,A4,b83),(r23,A4,b84),(r23,A4,b85),( r23,A4,b86),(r23,A4,b87),(r23,A4,b88),(r23,A4,b89),(r23,A4,b90),(r23,A4,b91),(r23,A4,b 92),(r23,A4,b93),(r23,A4,b94),(r23,A4,b95),(r23,A4,b96),(r23,A4,b97),(r23,A4,b98),(r23, A4,b99),(r23,A4,b 100),(r23,A4,b 101),(r23,A4,b 102),(r23,A4,b 103),(r23,A4,b 104),(r23,A4, b105),(r23,A4,b106),(r23,A4,b107),(r23,A4,b108),(r23,A4,b109),(r23,A4,bl10),(r23,A4,b1 11),(r23,A4,b112),(r23,A4,b113),(r23,A4,b114),(r23,A4,b115),(r23,A4,b116),(r23,A4,b117) ,(r23,A4,b118),(r23,A4,b119),(r23,A4,b120),(r23,A4,b121),(r23,A4,b122),(r23,A4,b123),(r 23,A4,b124),(r23,A4,b125),(r23,A4,b126),(r23,A4,b127),(r23,A4,b128),(r23,A4,b129),(r23 ,A4,b130),(r23,A4,b131),(r23,A4,b132),(r23,A4,b133),(r23,A4,b134),(r24,A1,b1),(r24,A1, b2),(r24,A1,b3),(r24,A1,b4),(r24,A1,b5),(r24,A1,b6),(r24,A1,b7),(r24,A1,b8),(r24,A1,b9),( r24,A1,b10),(r24,A1,b11),(r24,A1,b12),(r24,A1,b13),(r24,A1,b14),(r24,A1,b15),(r24,A1,b1 6),(r24,A1,b17),(r24,A1,b18),(r24,A1,b19),(r24,A1,b20),(r24,A1,b21),(r24,A1,b22),(r24,A 1,b23),(r24,A1,b24),(r24,A1,b25),(r24,A1,b26),(r24,A1,b27),(r24,A1,b28),(r24,A1,b29),(r2 4,A1,b30),(r24,A1,b31),(r24,A1,b32),(r24,A1,b33),(r24,A1,b34),(r24,A1,b35),(r24,A1,b36) ,(r24,A1,b37),(r24,A1,b38),(r24,A1,b39),(r24,A1,b40),(r24,A1,b41),(r24,A1,b42),(r24,A1, b43),(r24,A1,b44),(r24,A1,b45),(r24,A1,b46),(r24,A1,b47),(r24,A1,b48),(r24,A1,b49),(r24, A1,b50),(r24,A1,b51),(r24,A1,b52),(r24,A1,b53),(r24,A1,b54),(r24,A1,b55),(r24,A1,b56),( r24,A1,b57),(r24,A1,b58),(r24,A1,b59),(r24,A1,b60),(r24,A1,b61),(r24,A1,b62),(r24,A1,b 63),(r24,A1,b64),(r24,A1,b65),(r24,A1,b66),(r24,A1,b67),(r24,A1,b68),(r24,A1,b69),(r24, A1,b70),(r24,A1,b71),(r24,A1,b72),(r24,A1,b73),(r24,A1,b74),(r24,A1,b75),(r24,A1,b76),( r24,A1,b77),(t24,A1,b78),(r24,A1,b79),(r24,A1,b80),(r24,A1,b81),(r24,A1,b82),(r24,A1,b 83),(r24,A1,b84),(r24,A1,b85),(r24,A1,b86),(r24,A1,b87),(r24,A1,b88),(r24,A1,b89),(r24, A1,b90),(r24,A1,b91),(r24,A1,b92),(r24,A1,b93),(r24,A1,b94),(r24,A1,b95),(r24,A1,b96),( r24,A1,b97),(r24,A1,b98),(r24,A1,b99),(r24,A1,b100),(r24,A1,b101),(r24,A1,b102),(r24,A 1,b103),(r24,A1,b104),(r24,A1,b105),(r24,A1,b106),(r24,A1,b107),(r24,A1,b108),(r24,A1, b109),(r24,A1,b110),(r24,A1,b111),(r24,A1,b112),(r24,A1,b113),(r24,A1,b114),(r24,A1,b1 15),(r24,A1,b116),(r24,A1,b117),(r24,A1,b118),(r24,A1,b119),(r24,A1,b120),(r24,A1,b121 ),(r24,A1,b122),(r24,A1,b123),(r24,A1,b124),(r24,A1,b125),(r24,A1,b126),(r24,A1,b127),( r24,A1,b128),(r24,A1,b129),(r24,A1,b130),(r24,A1,b131),(r24,A1,b132),(r24,A1,b133),(r2 4,A1,b134),
(r24,A2,b1),(r24,A2,b2),(r24,A2,b3),(r24,A2,b4),(r24,A2,b5),(r24,A2,b6),(r24,A2,b7),(r24, A2,b8),(r24,A2,b9),(r24,A2,b10),(r24,A2,b11),(r24,A2,b12),(r24,A2,b13),(r24,A2,b14),(r2 4,A2,b15),(r24,A2,b16),(r24,A2,b17),(r24,A2,b18),(r24,A2,b19),(r24,A2,b20),(r24,A2,b21) ,(r24,A2,b22),(r24,A2,b23),(r24,A2,b24),(r24,A2,b25),(r24,A2,b26),(r24,A2,b27),(r24,A2, b28);(r24,A2,b29);(r24,A2,b30),(r24,A2,b31),(r24,A2,b32),(r24,A2,b33),(r24,A2,b34),(r24, A2,b35),(r24,A2,b36),(r24,A2,b37),(r24,A2,b38),(r24,A2,b39),(r24,A2,b40),(r24,A2,b41),( r24,A2,b42),(r24,A2,b43),(r24,A2,b44),(r24,A2,b45),(r24,A2,b46),(r24,A2,b47),(r24,A2,b 48),(r24,A2,b49),(r24,A2,b50),(r24,A2,b51),(r24,A2,b52),(r24,A2,b53),(r24,A2,b54),(r24, A2,b55),(r24,A2,b56),(r24,A2,b57),(r24,A2,b58),(r24,A2,b59),(r24,A2,b60),(r24,A2,b61),( r24,A2,b62),(r24,A2,b63),(r24,A2,b64),(r24,A2,b65),(r24,A2,b66),(r24,A2,b67),(r24,A2,b 68),(r24,A2,b69),(r24,A2,b70),(r24,A2,b71),(r24,A2,b72),(r24,A2,b73),(r24,A2,b74),(r24, A2,b75),(r24,A2,b76),(r24,A2,b77),(r24,A2,b78),(r24,A2,b79),(r24,A2,b80),(r24,A2,b81),( r24,A2,b82),(r24,A2,b83),(r24,A2,b84),(r24,A2,b85),(r24,A2,b86),(r24,A2,b87),(r24,A2,b 88),(r24,A2,b89),(r24,A2,b90),(r24,A2,b91),(r24,A2,b92),(r24,A2,b93),(r24,A2,b94),(r24, A2,b95),(r24,A2,b96),(r24,A2,b97),(r24,A2,b98),(r24,A2,b99),(r24,A2,b100),(r24,A2,b101 ),(r24,A2,b102),(r24,A2,b103),(r24,A2,b104),(r24,A2,b105),(r24,A2,b106),(r24,A2,b107), ( r24,A2,b108),(r24,A2,b109),(r24,A2,b110),(r24,A2,b111),(r24,A2,b112),(r24,A2,b113),(r2 4,A2,b114),(r24,A2,b115),(r24,A2,b116),(r24,A2,b117),(r24,A2,b118),(r24,A2,b119),(r24, A2,b120),(r24,A2,b121),(r24,A2,b122),(r24,A2,b123),(r24,A2,b124),(r24,A2,b125),(r24,A 2,b126),(r24,A2,b127),(r24,A2,b128),(r24,A2,b129),(r24,A2,b130),(r24,A2,b131),(r24,A2, b132),(r24,A2,b133),(r24,A2,b134),(r24,A3,b1),(r24,A3,b2),(r24,A3,b3),(r24,A3,b4),(r24, A3,b5),(r24,A3,b6),(r24,A3,b7),(r24,A3,b8),(r24,A3,b9),(r24,A3,b10),(r24,A3,b11),(r24,A3 ,b12),(r24,A3,b13),(r24,A3,b14),(r24,A3,b15),(r24,A3,b16),(r24,A3,b17),(r24,A3,b18),(r24 ,A3,b19),(r24,A3,b20),(r24,A3,b21),(r24,A3,b22),(r24,A3,b23),(r24,A3,b24),(r24,A3,b25),( r24,A3,b26),(r24,A3,b27),(r24,A3,b28),(r24,A3,b29),(r24,A3,b30),(r24,A3,b31),(r24,A3,b 32),(r24,A3,b33),(r24,A3,b34),(r24,A3,b35),(r24,A3,b36),(r24,A3,b37),(r24,A3,b38),(r24, A3,b39),(r24,A3,b40),(r24,A3,b41),(r24,A3,b42),(r24,A3,b43),(r24,A3,b44),(r24,A3,b45),( r24,A3,b46),(r24,A3,b47),(r24,A3,b48),(r24,A3,b49),(r24,A3,b50),(r24,A3,b51),(r24,A3,b 52),(r24,A3,b53),(r24,A3,b54),(r24,A3,b55),(r24,A3,b56),(r24,A3,b57),(r24,A3,b58),(r24, A3,b59),(r24,A3,b60),(r24,A3,b61),(r24,A3,b62),(r24,A3,b63),(r24,A3,b64),(r24,A3,b65),( r24,A3,b66),(r24,A3,b67),(r24,A3,b68),(r24,A3,b69),(r24,A3,b70),(r24,A3,b71),(r24,A3,b 72),(r24,A3,b73),(r24,A3,b74),(r24,A3,b75),(r24,A3,b76),(r24,A3,b77),(r24,A3,b78),(r24, A3,b79),(r24,A3,b80),(r24,A3,b81),(r24_{;}A3,b82),(r24,A3,b83),(r24,A3,b84),(r24,A3,b85),( r24,A3,b86),(r24,A3,b87),(r24,A3,b88),(r24,A3,b89),(r24,A3,b90),(r24,A3,b91),(r24,A3,b 92),(r24,A3,b93),(r24,A3,b94),(r24,A3,b95),(r24,A3,b96),(r24,A3,b97),(r24,A3,b98),(r24, A3,b99),(r24,A3,b 100),(r24,A3,b 101),(r24,A3,b 102),(r24,A3,b 103),(r24,A3,b 104),(r24,A3, b105),(r24,A3,b106),(r24,A3,b107),(r24,A3,b108),(r24,A3,b109),(r24,A3,b110),(r24,A3,b1 11),(r24,A3,b112),(r24,A3,b113),(r24,A3,b114),(r24,A3,b115),(r24,A3,b116),(r24,A3,b117) ,(r24,A3,b118),(r24,A3,b119),(r24,A3,b120),(r24,A3,b121),(r24,A3,b122),(r24,A3,b123),(r 24,A3,b124),(r24,A3,b125),(r24,A3,b126),(r24,A3,b127),(r24,A3,b128),(r24,A3,b129),(r24 ,A3,b130),(r24,A3,b131),(r24,A3,b132),(r24,A3,b133),(r24,A3,b134),(r24,A4,b1),(r24,A4, b2),(r24,A4,b3),(r24,A4,b4),(r24,A4,b5),(r24,A4,b6),(r24,A4,b7),(r24,A4,b8),(r24,A4,b9),( r24,A4,b10),(r24,A4,b11),(r24,A4,b12),(r24,A4,b13),(r24,A4,b14),(r24,A4,b15),(r24,A4,b1 6),(r24,A4,b17),(r24,A4,b18),(r24,A4,b19),(r24,A4,b20),(r24,A4,b21),(r24,A4,b22),(r24,A 4,b23),(r24,A4,b24),(r24,A4,b25),(r24,A4,b26),(r24,A4,b27),(r24,A4,b28),(r24,A4,b29),(r2 4,A4,b30),(r24,A4,b31),(r24,A4,b32),(r24,A4,b33),(r24,A4,b34),(r24,A4,b35),(r24,A4,b36) ,(r24,A4,b37),(r24,A4,b38),(r24,A4,b39),(r24,A4,b40),(r24,A4,b41),(r24,A4,b42),(r24,A4, b43),(r24,A4,b44),(r24,A4,b45),(r24,A4,b46),(r24,A4,b47),(r24,A4,b48),(r24,A4,b49),(r24, A4,b50)_{;}(r24,A4,b51),(r24,A4,b52),(r24,A4,b53),(r24,A4,b54),(r24,A4,b55),(r24,A4,b56),( r24,A4,b57),(r24,A4,b58),(r24,A4,b59),(r24,A4,b60),(r24,A4,b6l),(r24,A4,b62),(r24,A4,b 63),(r24,A4,b64),(r24,A4,b65),(r24,A4,b66),(r24,A4,b67),(r24,A4,b68),(r24,A4,b69),(r24, A4,b70),(r24,A4,b71),(r24,A4,b72),(r24,A4,b73),(r24,A4,b74),(r24,A4,b75),(r24,A4,b76),( r24,A4,b77),(r24,A4,b78),(r24,A4,b79),(r24,A4,b80),(r24,A4,b81),(r24,A4,b82),(r24,A4,b 83),(r24,A4,b84),(r24,A4,b85),(r24,A4,b86),(r24,A4,b87),(r24,A4,b88),(r24,A4,b89),(r24, A4,b90),(r24,A4,b9l),(r24,A4,b92),(r24,A4,b93),(r24,A4,b94),(r24,A4,b95),(r24,A4,b96),( r24,A4,b97),(r24,A4,b98),(r24,A4,b99),(r24,A4,b100),(r24,A4,b101),(r24,A4,b102),(r24,A 4,b103),(r24,A4,b104),(r24,A4,b105),(r24,A4,b106),(r24,A4,b107),(r24,A4,b108),(r24,A4, b109),(r24,A4,b110),(r24,A4,b111),(r24,A4,b112),(r24,A4,b113),(r24,A4,b114),(r24,A4,b1 15),(r24,A4,b116),(r24,A4,b117),(r24,A4,b118),(r24,A4,b119),(r24,A4,b120),(r24,A4,b121 ),(r24,A4,b122),(r24,A4,b123),(r24,A4,b124),(r24,A4,b125),(r24,A4,b126),(r24,A4,b127),( r24,A4,b128),(r24,A4,b129),(r24,A4,b130),(r24,A4,b131),(r24,A4,b132),(r24,A4,b133),(r2 4,A4,b134),
(r25,A1,b1),(r25,A1,b2),(r25,A1,b3),(r25,A1,b4),(r25,A1,b5),(r25,A1,b6),(r25,A1,b7),(r25, A1,b8),(r25,A1,b9),(r25,A1,b10),(r25,A1,b11),(r25,A1,b12),(r25,A1,b13),(r25,A1,b14),(r2 5,A1,b15),(r25,A1,b16),(r25,A1,b17),(r25,A1,b18),(r25,A1,b19),(r25,A1,b20),(r25,A1,b21) ,(r25,A1,b22),(r25,A1,b23),(r25,A1,b24),(r25,A1,b25),(r25,A1,b26),(r25,A1,b27),(r25,A1, b28),(r25,A1,b29),(r25,A1,b30),(r25,A1,b31),(r25,A1,b32),(r25,A1,b33),(r25,A1,b34),(r25, A1,b35),(r25,A1,b36),(r25,A1,b37),(r25,A1,b38),(r25,A1,b39),(r25,A1,b40),(r25,A1,b41),( r25,A1,b42),(r25,A1,b43),(r25,A1,b44),(r25,A1,b45),(r25,A1,b46),(r25,A1,b47),(r25,A1,b 48),(r25,A1,b49),(r25,A1,b50),(r25,A1,b51),(r25,A1,b52),(r25,A1,b53),(r25,A1,b54),(r25, A1,b55),(r25,A1,b56),(r25,A1,b57),(r25,A1_{;}b58),(r25,A1,b59),(r25,A1,b60),(r25,A1,b61),( r25,A1,b62),(r25,A1,b63),(r25,A1,b64),(r25,A1,b65),(r25,A1,b66),(r25,A1,b67),(r25,A1,b 68),(r25,A1,b69),(r25,A1,b70),(r25,A1,b71),(r25,A1,b72),(r25,A1,b73),(r25,A1,b74),(r25, A1,b75),(r25,A1,b76),(r25,A1,b77),(r25,A1,b78),(r25,A1,b79),(r25,A1,b80),(r25,A1,b81),( r25,A1,b82),(r25,A1,b83),(r25,A1,b84),(r25,A1,b85),(r25,A1,b86),(r25,A1,b87),(r25,A1,b 88),(r25,A1,b89),(r25,A1,b90),(r25,A1,b91),(r25,A1,b92),(r25,A1,b93),(r25,A1,b94),(r25, A1,b95),(r25,A1,b96),(r25,A1,b97),(r25,A1,b98),(r25,A1,b99),(r25,A1,b100),(r25,A1,b101 ),(r25,A1,b102),(r25,A1,b103),(r25,A1,b104),(r25,A1,b105),(r25,A1,b106),(r25,A1,b107), ( r25,A1,b108),(r25,A1,b109),(r25,A1,b110),(r25,A1,b111),(r25,A1,b112),(r25,A1,b113),(r2 5,A1,b114),(r25,A1,b115),(r25,A1,b116),(r25,A1,b117),(r25,A1,b118),(r25,A1,b119),(r25, A1,b120),(r25,A1,b121),(r25,A1,b122),(r25,A1,b123),(r25,A1,b124),(r25,A1,b125),(r25,A 1,b126),(r25,A1,b127),(r25,A1,b128),(r25,A1,b129),(r25,A1,b130),(r25,A1,b131),(r25,A1, b132),(r25,A1,b133),(r25,A1,b134),(r25,A2,b1),(r25,A2,b2),(r25,A2,b3),(r25,A2,b4),(r25, A2,b5),(r25,A2,b6),(r25,A2,b7),(r25,A2,b8),(r25,A2,b9),(r25,A2,b10),(r25,A2,b11),(r25,A2 ,b12),(r25,A2,b13),(r25,A2,b14),(r25,A2,b15),(r25,A2,b16),(r25,A2,b17),(r25,A2,b18),(r25 ,A2,b19),(r25,A2,b20),(r25,A2,b21),(r25,A2,b22),(r25,A2,b23),(r25,A2,b24),(r25,A2,b25),( r25,A2,b26),(r25,A2,b27),(r25,A2,b28),(r25,A2,b29),(r25,A2,b30),(r25,A2,b31),(r25,A2,b 32),(r25,A2,b33),(r25,A2,b34),(r25,A2,b35),(r25,A2,b36),(r25,A2,b37),(r25,A2,b38),(r25, A2,b39),(r25,A2,b40),(r25,A2,b41),(r25,A2,b42),(r25,A2,b43),(r25,A2,b44),(r25,A2,b45),( r25,A2,b46),(r25,A2,b47),(r25,A2,b48),(r25,A2,b49),(r25,A2,b50),(r25,A2,b51),(r25;A2,b 52),(r25,A2,b53),(r25,A2,b54),(r25,A2,b55),(r25,A2,b56),(r25,A2,b57),(r25,A2,b58),(r25, A2,b59),(r25,A2,b60),(r25,A2,b61),(r25,A2,b62),(r25,A2,b63),(r25,A2,b64),(r25,A2,b65),( r25,A2,b66),(r25,A2,b67),(r25,A2,b68),(r25,A2,b69),(r25,A2,b70),(r25,A2,b71),(r25,A2,b 72),(r25,A2,b73),(r25,A2,b74),(r25,A2,b75),(r25,A2,b76),(r25,A2,b77),(r25,A2,b78),(r25, A2,b79),(r25,A2,b80),(r25,A2,b81),(r25,A2,b82),(r25,A2,b83),(r25,A2,b84),(.r25,A2,b85),( r25,A2,b86),(r25,A2,b87),(r25,A2,b88),(r25,A2,b89),(r25,A2,b90),(r25,A2,b91),(r25,A2,b 92),(r25,A2,b93),(r25,A2,b94),(r25,A2,b95),(r25,A2,b96),(r25,A2,b97),(r25,A2,b98),(r25, A2,b99),(r25,A2,b100),(r25,A2,b101),(r25,A2,b102),(r25,A2,b103),(r25,A2,b104),(r25,A2, b105),(r25,A2,b106),(r25,A2,b107),(r25,A2,b108),(r25,A2,b109),(r25,A2,b110),(r25,A2,b1 11),(r25,A2,b112),(r25,A2,b113),(r25,A2,b114),(r25,A2,b115),(r25,A2,b116),(r25,A2,b117) ,(r25,A2,b118),(r25,A2,b119),(r25,A2,b120),(r25,A2,b121),(r25,A2,b122),(r25,A2,b123),(r 25,A2,b124),(r25,A2,b125),(r25,A2,b126),(r25,A2,b127),(r25,A2,b128),(r25,A2,b129),(r25 ,A2,b130),(r25,A2,b131),(r25,A2,b132),(r25,A2,b133),(r25,A2,b134),(r25,A3,b1),(r25,A3, b2),(r25,A3,b3),(r25,A3,b4),(r25,A3,b5),(r25,A3,b6),(r25,A3,b7),(r25,A3,b8),(r25,A3,b9),( r25,A3,b 10),(r25,A3,b 11),(r25,A3,b 12),(r25,A3,b 13),(r25,A3,b 14),(r25,A3,b 15),(r25,A3,b 1 6),(r25,A3,b17),(r25,A3,b18),(r25,A3,b19),(r25,A3,b20),(r25,A3,b21),(r25,A3,b22),(r25,A 3,b23),(r25,A3,b24),(r25,A3,b25),(r25,A3,b26),(r25,A3,b27),(r25,A3,b28),(r25,A3,b29),(r2 5,A3,b30),(r25,A3,b31),(r25,A3,b32),(r25,A3,b33),(r25,A3,b34),(r25,A3,b35),(r25,A3,b36) ,(r25,A3,b37),(r25,A3,b38),(r25,A3,b39),(r25,A3,b40),(r25,A3,b41),(r25,A3,b42),(r25,A3, b43),(r25,A3,b44),(r25,A3,b45),(r25,A3,b46),(r25,A3,b47),(r25,A3,b48),(r25,A3,b49),(r25, A3,b50),(r25,A3,b51),(r25,A3,b52),(r25,A3,b53),(r25,A3,b54),(r25,A3,b55),(r25,A3,b56),( r25,A3,b57),(r25,A3,b58),(r25,A3,b59),(r25,A3,b60),(r25,A3,b61),(r25,A3,b62),(r25,A3,b 63),(r25,A3_{;}b64),(r25,A3,b65),(r25,A3,b66),(r25,A3,b67),(r25,A3,b68),(r25,A3,b69),(r25, A3,b70),(r25,A3,b71),(r25,A3,b72),(r25,A3,b73),(r25,A3,b74),(r25,A3,b75),(r25,A3,b76),( r25,A3,b77),(r25,A3,b78),(r25,A3,b79),(r25,A3,b80),(r25,A3,b81),(r25,A3,b82),(r25,A3,b 83),(r25,A3,b84),(r25,A3,b85),(r25,A3,b86),(r25,A3,b87),(r25,A3,b88),(r25,A3,b89),(r25, A3,b90),(r25,A3,b91),(r25,A3,b92),(r25,A3,b93),(r25,A3,b94),(r25,A3,b95),(r25,A3,b96),( r25,A3,b97),(r25,A3,b98),(r25,A3,b99),(r25,A3,b100),(r25,A3,b101),(r25,A3,b102),(r25,A 3,b103),(r25,A3,b104),(r25,A3,b105),(r25,A3,b106),(r25,A3,b107),(r25,A3,b108),(r25,A3, b109),(r25,A3,b110),(r25,A3,b111),(r25,A3,b112),(r25,A3,b113),(r25,A3,b114),(r25,A3,b1 15),(r25,A3,b116),(r25,A3,b117),(r25,A3,b118),(r25,A3,b119),(r25,A3,b120),(r25,A3,b121 ),(r25,A3,b122),(r25,A3,b123),(r25,A3,b124),(r25,A3,b125),(r25,A3,b126),(r25,A3,b127),( r25,A3,b128),(r25,A3,b129),(r25,A3,b130),(r25,A3,b131),(r25,A3,b132),(r25,A3,b133),(r2 5,A3,b134),
(r25,A4,b1),(r25,A4,b2),(r25,A4,b3),(r25,A4,b4),(r25,A4,b5),(r25,A4,b6),(r25,A4,b7),(r25, A4,b8),(r25,A4,b9),(r25,A4,b10),(r25,A4,b11),(r25,A4,b12),(r25,A4,b13),(r25,A4,b14),(r2 5,A4,b15),(r25,A4,b16),(r25,A4,b17),(r25,A4,b18),(r25,A4,b19),(r25,A4,b20),(r25,A4,b21) ,(r25,A4,b22),(r25,A4,b23),(r25,A4,b24),(r25,A4,b25),(r25,A4,b26),(r25,A4,b27),(r25,A4, b28),(r25,A4,b29),(r25,A4,b30),(r25,A4,b31),(r25,A4,b32),(r25,A4,b33),(r25,A4,b34),(r25, A4,b35),(r25,A4,b36),(r25,A4,b37),(r25,A4,b38),(r25,A4,b39),(r25,A4,b40),(r25,A4,b41),( r25,A4,b42),(r25,A4,b43),(r25,A4,b44),(r25,A4,b45),(r25,A4,b46),(r25,A4,b47),(r25,A4,b 48),(r25,A4,b49),(r25,A4,b50),(r25,A4,b51),(r25,A4,b52),(r25,A4,b53),(r25,A4,b54),(r25, A4,b55),(r25,A4,b56),(r25,A4,b57),(r25,A4,b58),(r25,A4,b59),(r25,A4,b60),(r25,A4,b61),( r25,A4,b62),(r25,A4,b63),(r25,A4,b64),(r25,A4,b65),(r25,A4,b66),(r25,A4,b67),(r25,A4,b 68),(r25,A4,b69),(r25,A4,b70),(r25,A4,b7l),(r25,A4,b72),(r25,A4,b73),(r25,A4,b74),(r25, A4,b75),(r25,A4,b76),(r25,A4_{;}b77),(r25,A4,b78),(r25,A4,b79),(r25,A4,b80),(r25,A4,b81),( r25,A4,b82),(r25,A4,b83),(r25,A4,b84),(r25,A4,b85),(r25,A4,b86),(r25,A4,b87),(r25,A4,b 88),(r25,A4,b89),(r25,A4,b90),(r25,A4,b91),(r25,A4,b92),(r25,A4,b93),(r25,A4,b94),(r25, A4,b95),(r25,A4,b96),(r25,A4,b97),(r25,A4,b98),(r25,A4,b99),(r25,A4,b100),(r25,A4,b101 ),(r25,A4,b102),(r25,A4,b103),(r25,A4,b104),(r25,A4,b105),(r25,A4,b106),(r25,A4,b107), ( r25,A4,b108),(r25,A4,b109),(r25,A4,b110),(r25,A4,b111),(r25,A4,b112),(r25,A4,b113),(r2 5,A4,b114),(r25,A4,b115),(r25,A4,b116),(r25,A4,b117),(r25,A4,b118),(r25,A4,b119),(r25, A4,b 120),(r25,A4,b121),(r25,A4,b122),(r25,A4,b123),(r25,A4,b124),(r25,A4,b125), (r25,A 4,b126),(r25,A4,b127),(r25,A4,b128),(r25,A4,b129),(r25,A4,b130),(r25,A4,b131),(r25,A4, b132),(r25,A4,b133),(r25,A4,b134),(r26,A1,b1),(r26,A1,b2),(r26,A1,b3),(r26,A1,b4),(r26, A1,b5),(r26,Al,b6),(r26,A1,b7),(r26,A1,b8),(r26,A1,b9),(r26,A1,b10),(r26,A1,b11),(r26,A1 ,b12),(r26,A1,b13),(r26,A1,b14),(r26,A1,b15),(r26,A1,b16),(r26,A1,b17),(r26,A1,b18),(r26 ,A1,b19),(r26,A1,b20),(r26,A1,b21),(r26,A1,b22),(r26,A1,b23),(r26,A1,b24),(r26,A1,b25),( r26,A1,b26),(r26,A1,b27),(r26,A1,b28),(r26,A1,b29),(r26,A1,b30),(r26,A1,b31),(r26,Al,b 32),(r26,A1,b33),(r26,A1,b34),(r26,A1,b35),(r26,A1,b36),(r26,A1,b37),(r26,A1,b38),(r26, A1,b39),(r26,A1,b40),(r26,A1,b41),(r26,A1,b42),(r26,A1,b43),(r26,A1,b44),(r26,A1,b45),( r26,A1,b46),(r26,A1,b47),(r26,A1,b48),(r26,A1,b49),(r26,A1,b50),(r26,A1,b51),(r26,A1,b 52),(r26,A1,b53),(r26,A1,b54),(r26,A1,b55),(r26,A1,b56),(r26,A1,b57),(r26,A1,b58),(r26, A1,b59),(r26,A1,b60),(r26,A1,b61),(r26,A1,b62),(r26,A1,b63),(r26,A1,b64),(r26,A1,b65),( r26,A1,b66),(r26,A1,b67),(r26,A1,b68),(r26,A1,b69),(r26,A1,b70),(r26,A1,b71),(r26,A1,b 72),(r26,A1,b73),(r26,A1,b74),(r26,A1,b75),(r26,A1,b76),(r26,A1,b77),(r26,A1,b78),(r26, A1,b79),(r26,A1,b80),(r26,A1,b81),(r26,A1,b82),(r26,A1,b83),(r26,A1,b84),(r26,A1,b85), ( r26,A1,b86),(r26,A1,b87),(r26,A1,b88),(r26,A1,b89),(r26,A1,b90),(r26,A1,b91),(r26,A1,b 92),(r26,A1,b93),(r26,A1,b94),(r26,A1,b95),(r26,A1,b96),(r26,A1,b97),(r26,A1,b98),(r26, A1,b99),(r26,A1,b100),(r26,A1,b101),(r26,A,b102),(r26,A1,b103),(r26,A1,b104),(r26,A1, b105),(r26,A1,b106),(r26,A1,b107),(r26,A1,b108),(r26,A1,b109),(r26,A1,b110),(r26,A1,b1 11),(r26,A1,b112),(r26,A1,b113),(r26,A1,b114),(r26,A1,b115),(r26,A1,b116),(r26,A1,b117) ,(r26,A1,b118),(r26,A1,b119),(r26,A1,b120),(r26,A1,b121),(r26,A1,b122),(r26,A1,b123),(r 26,A1,b124),(r26,A1,b125),(r26,A1,b126),(r26,A1,b127),(r26,A1,b128),(r26,A1,b129),(r26 ,A1,b130),(r26,A1,b131),(r26,A1,b132),(r26,A1,b133),(r26,A1,b134),(r26,A2,b),(r26,A2, b2),(r26,A2,b3),(r26,A2,b4),(r26,A2,b5),(r26,A2,b6),(r26,A2,b7),(r26,A2,b8),(r26,A2,b9),( r26,A2,b10),(r26,A2,b11),(r26,A2,b12),(r26,A2,b13),(r26,A2,b14),(r26,A2,b15),(r26,A2,b1 6),(r26,A2,b17),(r26,A2,b18),(r26,A2,b19),(r26,A2,b20),(r26,A2,b21),(r26,A2,b22),(r26,A 2,b23),(r26,A2,b24),(r26,A2,b25),(r26,A2,b26),(r26,A2,b27),(r26,A2,b28),(r26,A2,b29),(r2 6,A2,b30),(r26,A2,b31),(r26,A2,b32),(r26,A2,b33),(r26,A2,b34),(r26,A2,b35),(r26,A2,b36) ,(r26,A2,b37),(r26,A2,b38),(r26,A2,b39),(r26,A2,b40),(r26,A2,b41),(r26,A2,b42),(r26,A2, b43),(r26,A2,b44),(r26,A2,b45).(r26,A2,b46),(r26,A2,b47),(r26,A2,b48),(r26,A2,b49),(r26, A2,b50),(r26,A2,b51),(r26,A2,b52),(r26,A2,b53),(r26,A2,b54),(r26,A2,b55),(r26,A2,b56),( r26,A2,b57),(r26,A2,b58),(r26,A2,b59),(r26,A2,b60),(r26,A2,b61),(r26,A2,b62),(r26,A2,b 63),(r26,A2,b64),(r26,A2,b65),(r26,A2,b66),(r26,A2,b67),(r26,A2,b68),(r26,A2,b69),(r26, A2,b70),(r26,A2,b71),(r26,A2,b72),(r26,A2,b73),(r26,A2,b74),(r26,A2,b75),(r26,A2,b76),( r26,A2,b77),(r26,A2,b78),(r26,A2,b79),(r26,A2,b80),(r26,A2,b81),(r26,A2,b82),(r26,A2,b 83),(r26,A2,b84),(r26,A2,b85),(r26,A2,b86),(r26,A2,b87),(r26,A2,b88),(r26,A2,b89),(r26, A2,b90),(r26,A2,b91),(r26,A2,b92),(r26,A2,b93),(r26,A2,b94),(r26,A2,b95),(r26,A2,b96),( r26,A2,b97),(r26,A2,b98),(r26,A2,b99),(r26,A2,b100),(r26,A2,b101),(r26,A2,b102),(r26,A 2,b103),(r26,A2,b104),(r26,A2,b105),(r26,A2,b106),(r26,A2,b107),(r26,A2,b108),(r26,A2, b109),(r26,A2,b110),(r26,A2,b111),(r26,A2,b112),(r26,A2,b113),(r26,A2,b114),(r26,A2,b1 15),(r26,A2,b116),(r26,A2,b117),(r26,A2,b118),(r26,A2,b119),(r26,A2,b120),(r26,A2,b121 ),(r26,A2,b122),(r26,A2,b123),(r26,A2,b124),(r26,A2,b125),(r26,A2,b126),(r26,A2,b127),( r26,A2,b128),(r26,A2,b129),(r26,A2,b130),(r26,A2,b131),(r26,A2,b132),(r26,A2,b133),(r2 6,A2,b134),
(r26,A3,b1),(r26,A3,b2),(r26,A3,b3),(r26,A3,b4),(r26,A3,b5),(r26,A3,b6),(r26,A3,b7),(r26, A3,b8),(r26,A3,b9),(r26,A3,b10),(r26,A3,b11),(r26,A3,b12),(r26,A3,b13),(r26,A3,b14),(r2 6,A3,b15),(r26,A3,b16),(r26,A3,b17),(r26,A3,b18),(r26,A3,b19),(r26,A3,b20),(r26,A3,b21) ,(r26,A3,b22),(r26,A3,b23),(r26,A3,b24),(r26,A3,b25),(r26,A3,b26),(r26,A3,b27),(r26,A3, b28),(r26,A3,b29),(r26,A3,b30),(r26,A3,b31),(r26,A3,b32),(r26,A3,b33),(r26,A3,b34),(r26, A3,b35),(r26,A3,b36),(r26,A3,b37),(r26,A3,b38),(r26,A3,b39),(r26,A3,b40),(r26,A3,b41),( r26,A3,b42),(r26,A3,b43),(r26,A3,b44),(r26,A3,b45),(r26,A3,b46),(r26,A3,b47),(r26,A3,b 48),(r26,A3,b49),(r26,A3,b50),(r26,A3,b51),(r26,A3,b52),(r26,A3,b53),(r26,A3,b54),(r26, A3,b55),(r26,A3,b56),(r26,A3,b57),(r26,A3,b58),(r26,A3,b59),(r26,A3,b60),(r26,A3,b61),( r26,A3,b62),(r26,A3,b63),(r26,A3,b64),(r26,A3,b65),(r26,A3,b66),(r26,A3,b67),(r26,A3,b 68),(r26,A3,b69),(r26,A3,b70),(r26,A3,b71),(r26,A3,b72),(r26,A3,b73),(r26,A3,b74),(r26, A3,b75),(r26,A3,b76),(r26,A3,b77),(r26,A3,b78),(r26,A3,b79),(r26,A3,b80)_{;}(r26,A3,b81),( r26,A3,b82),(r26;A3,b83),(r26,A3,b84),(r26,A3,b85),(r26,A3,b86),(r26,A3,b87),(r26,A3,b 88),(r26,A3,b89),(r26,A3,b90),(r26,A3,b91),(r26,A3,b92),(r26,A3,b93),(r26,A3,b94),(r26, A3,b95),(r26,A3,b96),(r26,A3,b97),(r26,A3,b98),(r26,A3,b99),(r26,A3,b100),(r26,A3,b101 ),(r26,A3,b102),(r26,A3,b103),(r26,A3,b104),(r26,A3,b105),(r26,A3,b106),(r26,A3,b107),( r26,A3,b108),(r26,A3,b109),(r26,A3,b110),(r26,A3,b111),(r26,A3,b112),(r26,A3,b113),(r2 6,A3,b114),(r26,A3,b115),(r26,A3,b116),(r26,A3,b117),(r26,A3,b118),(r26,A3,b119),(r26, A3,b120),(r26,A3,b121),(r26,A3,b122),(r26,A3,b123),(r26,A3,b124),(r26,A3,b125),(r26,A 3,b126),(r26,A3,b127),(r26,A3,b128),(r26,A3,b129),(r26,A3,b130),(r26,A3,b131),(r26,A3, b132),(r26,A3,b133),(r26,A3,b134),(r26,A4,b1),(r26,A4,b2),(r26,A4,b3),(r26,A4,b4),(r26, A4,b5),(r26,A4,b6),(r26,A4,b7),(r26,A4,b8),(r26,A4,b9),(r26,A4,b10),(r26,A4,b11),(r26,A4 ,b12),(r26,A4,b13),(r26,A4,b14),(r26,A4,b15),(r26,A4,b16),(r26,A4,b17),(r26,A4,b18),(r26 ,A4,b 19), (r26,A4,b20), (r26,A4,b21), (r26,A4,b22), (r26,A4,b23), (r26,A4,b24), (r26,A4,b25), ( r26,A4,b26),(r26,A4,b27),(r26,A4,b28),(r26,A4,b29),(r26,A4,b30),(r26,A4,b31),(r26,A4,b 32),(r26,A4,b33),(r26,A4,b34),(r26,A4,b35),(r26,A4,b36),(r26,A4,b37),(r26,A4,b38),(r26, A4,b39),(r26,A4,b40),(r26,A4,b41),(r26,A4,b42),(r26,A4,b43),(r26,A4,b44),(r26,A4,b45),( r26,A4,b46),(r26,A4,b47),(r26,A4,b48),(r26,A4,b49),(r26,A4,b50),(r26,A4,b51),(r26,A4,b 52),(r26,A4,b53),(r26,A4,b54),(r26,A4,b55),(r26,A4,b56),(r26,A4,b57),(r26,A4,b58),(r26, A4,b59),(r26,A4,b60),(r26,A4,b61),(r26,A4,b62),(r26,A4,b63),(r26,A4,b64),(r26,A4,b65),( r26,A4,b66),(r26,A4,b67),(r26,A4,b68),(r26,A4,b69),(r26,A4,b70),(r26,A4,b71),(r26,A4,b 72),(r26,A4,b73),(r26,A4,b74),(r26,A4,b75),(r26,A4,b76),(r26,A4,b77),(r26,A4,b78),(r26, A4,b79),(r26,A4,b80),(r26,A4,b81),(r26,A4,b82),(r26,A4,b83),(r26,A4,b84),(r26,A4,b85),( r26,A4,b86),(r26,A4,b87),(r26,A4,b88),(r26,A4,b89),(r26,A4,b90),(r26,A4,b91),(r26,A4,b 92),(r26,A4,b93),(r26,A4,b94),(r26,A4,b95),(r26,A4,b96),(r26,A4,b97),(r26,A4,b98),(r26, A4,b99),(r26,A4,b100),(r26,A4,b101),(r26,A4,b102),(r26,A4,b103);(r26,A4,b104),(r26,A4, b105),(r26,A4,b106),(r26,A4,b107),(r26,A4,b108),(r26,A4,b109),(r26,A4,b110),(r26,A4,b1 11),(r2,6,A4,b112),(r26,A4,b113),(r26,A4,b114),(r26,A4,b115),(r26,A4,b116),(r26,A4,b117) , (r26;A4,b118),(r26,A4,b119),(r26,A4,b120),(r26,A4,b121),(r26,A4,b122),(r26,A4,b123),(r 26,A4,b124),(r26,A4,b125),(r26,A4,b126),(r26,A4,b127),(r26,A4,b128),(r26,A4,b129),(r26 ,A4,b130),(r26,A4,b131),(r26,A4,b132),(r26,A4,b133),(r26,A4,b134),(r27,A1,b1),(r27,A1, b2),(r27,A1,b3),(r27,A1,b4),(r27,A1,b5),(r27,A1,b6),(r27,A1,b7),(r27,A1,b8),(r27,A1,b9)_{;}( r27,A1,b10),(r27,A1,b11),(r27,A1,b12),(r27,A1,b13),(r27,A1,b14),(r27,A1,b14),(r27,A1,b1 6),(r27,A1,b17),(r27,A1,b18),(r27,A1,b19),(r27,A1,b20),(r27,A1,b21),(r27,A1,b22),(r27,A 1,b23),(r27,A1,b24),(r27,A1,b25),(r27,A1,b26),(r27,A1,b27),(r27,A1,b28),(r27,A1,b29),(r2 7,A1,b30),(r27,A1,b31),(r27,A1,b32),(r27,A1,b33),(r27,A1,b34),(r27,A1,b35),(r27,A1,b36) ,(r27,A1,b37),(r27,A1,b38),(r27,A1,b39),(r27,A1,b40),(r27,A1,b41),(r27,A1,b42),(r27,A1, b43),(r27,A1,b44),(r27,A1,b45),(r27,A1,b46),(r27,A1,b47),(r27,A1,b48),(r27,A1,b49),(r27, A1,b50),(r27,A1,b51),(r27,A1,b52),(r27,A1,b53),(r27,A1,b54),(r27,A1,b55),(r27,A1,b56),( r27,A1,b57),(r27,A1,b58),(r27,A1,b59),(r27,A1,b60),(r27,A1,b61),(r27,A1,b62),(r27,A1,b 63),(r27,A1,b64),(r27,A1,b65),(r27,A1,b66),(r27,A1,b67),(r27,A1,b68),(r27,A1,b69),(r27, A1,b70),(r27,A1,b71),(r27,A1,b72),(r27,A1,b73),(r27,A1,b74),(r27,A1,b75),(r27,A1,b76),( r27,A1,b77),(r27,A1,b78),(r27,A1,b79),(r27,A1,b80),(r27,A1,b81),(r27,A1,b82),(r27,A1,b 83),(r27,A1,b84),(r27,A1,b85),(r27,A1,b86),(r27,A1,b87),(r27,A1,b88),(r27,A1,b89),(r27, A1,b90),(r27,A1,b91),(r27,A1,b92),(r27,A1,b93),(r27,A1,b94),(r27,A1,b95),(r27,A1,b96),( r27,A1,b97),(r27,A1,b98),(r27,A1,b99),(r27,A1,b100),(r27,A1,b101),(r27,A1,b102),(r27,A 1,b103),(r27,A1,b104),(r27,A1,b105),(r27,A1,b106),(r27,A1,b107),(r27,A1,b108),(r27,A1, b109),(r27,A1,b110),(r27,A1,b111),(r27,A1,b112),(r27,A1,b113),(r27,A1,b114),(r27,A1,b1 15),(r27,A1,b116),(r27,A1,b117),(r27,A1,b118),(r27,A1,b119),(r27,A1,b120),(r27,A1,b121 ),(r27,A1,b122),(r27,A1,b123),(r27,A1,b124),(r27,A1,b125),(r27,A1,b126),(r27,A1,b127),( r27,A1,b128),(r27,A1,b129),(r27,A1,b130),(r27,A1,b131),(r27,A1,b132),(r27,A1,b133),(r2 7,A1,b134),
(r27,A2,b1),(r27,A2,b2),(r27,A2,b3),(r27,A2,b4),(r27,A2,b5),(r27,A2,b6),(r27,A2,b7),(r27, A2,b8),(r27,A2,b9),(r27,A2,b10),(r27,A2,b11),(r27,A2,b12),(r27,A2,b13),(r27,A2,b14),(r2 7,A2,b15),(r27,A2,b16),(r27,A2,b17),(r27,A2,b18),(r27,A2,b19),(r27,A2,b20),(r27,A2,b21) ,(r27,A2,b22),(r27,A2,b23),(r27,A2,b24),(r27,A2,b25),(r27,A2,b26),(r27,A2,b27),(r27,A2, b28),(r27,A2,b29),(r27,A2,b30),(r27,A2,b31),(r27,A2,b32),(r27,A2,b33),(r27,A2,b34),(r27, A2,b35),(r27,A2,b36),(r27,A2,b37),(r27,A2,b38),(r27,A2,b39),(r27,A2,b40),(r27,A2,b41),( r27,A2,b42),(r27,A2,b43),(r27,A2,b44),(r27,A2,b45),(r27,A2,b46),(r27,A2,b47),(r27,A2,b 48),(r27,A2,b49),(r27,A2,b50),(r27,A2,b51),(r27,A2,b52),(r27,A2,b53),(r27,A2,b54),(r27, A2,b55),(r27,A2,b56),(r27,A2,b57),(r27,A2,b58),(r27,A2,b59),(r27,A2,b60),(r27,A2,b61),( r27,A2,b62),(r27,A2,b63),(r27,A2,b64),(r27,A2,b65),(r27,A2,b66),(r27,A2,b67),(r27,A2,b 68),(r27,A2,b69),(r27,A2,b70),(r27,A2,b71),(r27,A2,b72),(r27,A2,b73),(r27,A2,b74),(r27, A2,b75),(r27,A2,b76),(r27,A2,b77),(r27,A2,b78),(r27,A2,b79),(r27,A2,b80),(r27,A2,b81),( r27,A2,b82),(r27,A2,b83),(r27,A2,b84),(r27,A2,b85),(r27,A2,b86),(r27,A2,b87),(r27,A2,b 88),(r27,A2,b89),(r27,A2,b90),(r27,A2,b91),(r27,A2,b92),(r27,A2,b93),(r27,A2,b94),(r27, A2,b95),(r27,A2,b96),(r27,A2,b97),(r27,A2,b98),(r27,A2,b99),(r27,A2,b100),(r27,A2,b101 ),(r27,A2,b102),(r27,A2,b103),(r27,A2,b104),(r27,A2,b105),(r27,A2,b106),(r27,A2,b107),( r27,A2,b108),(r27,A2,b109),(r27,A2,b110),(r27,A2,b111),(r27,A2,b112),(r27,A2,b113),(r2 7,A2,b114),(r27,A2,b115),(r27,A2,b116),(r27,A2,b117),(r27,A2,b118),(r27,A2,b119),(r27, A2,b120),(r27,A2,b121),(r27,A2,b122),(r27,A2,b123),(r27,A2,b124),(r27,A2,b125),(r27,A 2,b126),(r27,A2,b127),(r27,A2,b128),(r27,A2,b129),(r27,A2,b130),(r27,A2,b131),(r27,A2, b132),(r27,A2,b133),(r27,A2,b134),(r27,A3,b1),(r27,A3,b2),(r27,A3,b3),(r27,A3,b4),(r27, A3,b5),(r27,A3,b6),(r27,A3,b7),(r27,A3,b8),(r27,A3,b9),(r27,A3,b10),(r27,A3,b11),(r27,A3 ,b 12),(r27,A3,b 13),(r27,A3,b 14),(r27,A3,b 15),(r27,A3,b 16),(r27,A3,b 17),(r27,A3,b 18),(r27 ,A3,b19),(r27,A3,b20),(r27,A3,b21),(r27,A3,b22),(r27,A3,b23),(r27,A3,b24),(r27,A3,b25),( r27,A3,b26),(r27,A3,b27),(r27,A3,b28),(r27,A3,b29),(r27,A3,b30),(r27,A3,b31),(r27,A3,b 32),(r27,A3,b33),(r27,A3,b34),(r27,A3,b35),(r27,A3,b36),(r27,A3,b37),(r27,A3,b38),(r27, A3,b39),(r27,A3,b40),(r27,A3,b41),(r27,A3,b42),(r27,A3,b43),(r27,A3,b44),(r27,A3,b45),( r27,A3,b46),(r27,A3,b47),(r27,A3,b48),(r27,A3,b49),(r27,A3,b50),(r27,A3,b51),(r27,A3,b 52),(r27,A3,b53),(r27,A3,b54),(r27,A3,b55),(r27,A3,b56),(r27,A3,b57),(r27,A3,b58),(r27, A3,b59),(r27,A3,b60),(r27,A3,b61),(r27,A3,b62),(r27,A3,b63),(r27,A3,b64),(r27,A3,b65),( r27,A3,b66),(r27,A3,b67),(r27,A3,b68),(r27,A3,b69),(r27,A3,b70),(r27,A3,b71),(r27,A3,b 72),(r27,A3,b73),(r27,A3,b74),(r27,A3,b75),(r27,A3,b76),(r27,A3,b77),(r27,A3,b78),(r27, A3,b79),(r27,A3,b80),(r27,A3,b81),(r27,A3,b82),(r27,A3,b83),(r27,A3,b84),(r27,A3,b85),( r27,A3,b86),(r27,A3,b87),(r27,A3,b88),(r27,A3,b89),(r27,A3,b90),(r27,A3,b91),(r27,A3,b 92),(r27,A3,b93),(r27,A3,b94),(r27,A3,b95),(r27,A3,b96),(r27,A3,b97),(r27,A3,b98),(r27, A3,b99),(r27,A3,b100),(r27,A3,b101),(r27,A3,b102),(r27,A3,b103),(r27,A3,b104),(r27,A3, b105),(r27,A3,b106),(r27,A3,b107),(r27,A3,b108),(r27,A3,b109),(r27,A3,b110),(r27,A3,b1 11),(r27,A3,b112),(r27,A3,b113),(r27,A3,b114),(r27,A3,b115),(r27,A3,b116),(r27,A3,b117) ,(r27,A3,b118),(r27,A3,b119),(r27,A3,b120),(r27,A3,b121),(r27,A3,b122),(r27,A3,b123),(r 27,A3,b124),(r27,A3,b125),(r27,A3,b126),(r27,A3,b127),(r27,A3,b128),(r27,A3,b129),(r27 ,A3,b130),(r27,A3,b131),(r27,A3,b132),(r27,A3,b133),(r27,A3,b134),(r27,A4,b1),(r27,A4, b2),(r27,A4,b3),(r27,A4,b4),(r27,A4,b5),(r27,A4,b6),(r27,A4,b7),(r27,A4,b8),(r27,A4,b9),( r27,A4,b10),(r27,A4,b11),(r27,A4,b12),(r27,A4,b13),(r27,A4,b14),(r27,A4,b15),(r27,A4,b1 6),(r27,A4,b17),(r27,A4,b18),(r27,A4,b19),(r27,A4,b20),(r27,A4,b21),(r27,A4,b22),(r27,A 4,b23),(r27,A4,b24),(r27,A4,b25),(r27,A4,b26),(r27,A4,b27),(r27,A4,b28),(r27,A4,b29),(r2 7,A4,b30),(r27,A4,b31),(r27,A4,b32),(r27,A4,b33),(r27,A4,b34),(r27,A4,b35),(r27,A4,b36) ,(r27,A4,b37),(r27,A4,b38),(r27,A4,b39),(r27,A4,b40),(r27,A4,b41),(r27,A4,b42),(r27,A4, b43),(r27,A4,b44),(r27,A4,b45),(r27,A4,b46),(r27,A4,b47),(r27,A4,b48),(r27,A4,b49),(r27, A4,b50),(r27,A4,b51),(r27,A4,b52),(r27,A4,b53),(r27,A4,b54),(r27,A4,b55),(r27,A4,b56),( r27,A4,b57),(r27,A4,b58),(r27,A4,b59),(r27,A4,b60),(r27,A4,b61),(r27,A4,b62),(r27,A4,b 63),(r27,A4,b64),(r27,A4,b65),(r27,A4,b66),(r27,A4,b67),(r27,A4,b68),(r27,A4,b69),(r27, A4,b70),(r27,A4,b71),(r27,A4,b72),(r27,A4,b73),(r27,A4,b74),(r27,A4,b75),(r27,A4,b76),( r27,A4,b77),(r27,A4,b78),(r27,A4,b79),(r27,A4,b80),(r27,A4,b81),(r27,A4,b82),(r27,A4,b 83),(r27,A4,b84),(r27,A4,b85),(r27,A4,b86),(r27,A4,b87),(r27,A4,b88),(r27,A4,b89),(r27, A4,b90),(r27,A4,b91),(r27,A4,b92),(r27,A4,b93),(r27,A4,b94),(r27,A4,b95),(r27,A4,b96),( r27,A4,b97),(r27,A4,b98),(r27,A4,b99),(r27,A4,b100),(r27,A4,b101),(r27,A4,b102),(r27,A 4,b103),(r27,A4,b104),(r27,A4,b105),(r27,A4,b106),(r27,A4,b107),(r27,A4,b108),(r27,A4, b109),(r27,A4,b110),(r27,A4,b111),(r27,A4,b112),(r27,A4,b113),(r27,A4,b114),(r27,A4,b1 15),(r27,A4,b116),(r27,A4,b117),(r27,A4,b118),(r27,A4,b119),(r27,A4,b120),(r27,A4,b121 ),(r27,A4,b122),(r27,A4,b123),(r27,A4,b124),(r27,A4,b125),(r27,A4,b126),(r27,A4,b127),( r27,A4,b128),(r27,A4,b129),(r27,A4,b130),(r27,A4,b131),(r27,A4,b132),(r27,A4,b133),(r2 7,A4,b134),
(r28,A1,b1),(r28,A1,b2),(r28,A1,b3),(r28,A1,b4),(r28,A1,b5),(r28,A1,b6),(r28,A1,b7),(r28, A1,b8),(r28,A1,b9),(r28,A1,b10),(r28,A1,b11),(r28,A1,b12),(r28,A1,b13),(r28,A1,b14),(r2 8,A1,b15),(r28,A1,b16),(r28,A1,b17),(r28,A1,b18),(r28,A1,b19),(r28,A1,b20),(r28,A1,b21) ,(r28,A1,b22),(r28,A1,b23),(r28,A1,b24),(r28,A1,b25),(r28,A1,b26),(r28,A1,b27),(r28,A1, b28),(r28,A1,b29),(r28,A1,b30),(r28,A1,b31),(r28,A1,b32),(r28,A1,b33),(r28,A1,b34),(r28, A1,b35),(r28,A1,b36),(r28,A1,b37),(r28,A1,b38),(r28,A1,b39),(r28,A1,b40),(r28,A1,b41),( r28,A1,b42),(r28,A1,b43),(r28,A1,b44),(r28,A1,b45),(r28,A1,b46),(r28,A1,b47),(r28,A1,b 48),(r28,A1,b49),(r28,A1,b50),(r28,A1,b51),(r28,A1,b52),(r28,A1,b53),(r28,A1,b54),(r28, A1,b55),(r28,A1,b56),(r28,A1,b57),(r28,A1,b58),(r28,A1,b59),(r28,A1,b60),(r28,A1,b61),( r28,A1,b62),(r28,A1,b63),(r28,A1,b64),(r28,A1,b65),(r28,A1,b66),(r28,A1,b67),(r28,A1,b 68),(r28,A1,b69),(r28,A1,b70),(r28,A1,b71),(r28,A1,b72),(r28,A1,b73),(r28,A1,b74),(r28, A1,b75),(r28,A1,b76),(r28,A1,b77),(r28,A1,b78),(r28,A1,b79),(r28,A1,b80),(r28,A1,b81),( r28,A1,b82),(r28,A1,b83),(r28,A1,b84),(r28,A1,b85),(r28,A1,b86),(r28,A1,b87),(r28,A1,b 88),(r28,A1,b89),(r28,A1,b90),(r28,A1,b91),(r28,A1,b92),(r28,A1,b93),(r28,A1,b94),(r28, A1,b95),(r28,A1,b96),(r28,A1,b97),(r28,A1,b98),(r28,A1,b99),(r28,A1,b100),(r28,A1,b101 ),(r28,A1,b102),(r28,A1,b103),(r28,A1,b104),(r28,A1,b105),(r28,A1,b106),(r28,A1,b107),( r28,A1,b108),(r28,A1,b109),(r28,A1,b110),(r28,A1,b111),(r28,A1,b112),(r28,A1,b113),(r2 8,A1,b114),(r28,A1,b115),(r28,A1,b116),(r28,A1,b117),(r28,A1,b118),(r28,A1,b119),(r28, A1,b120),(r28,A1,b121),(r28,A1,b122),(r28,A1,b123),(r28,A1,b124),(r28,A1,b125),(r28,A 1,b126),(r28,A1,b127),(r28,A1,b128),(r28,A1,b129),(r28,A1,b130),(r28,A1,b131),(r28,A1, b132),(r28,A1,b133),(r28,A1,b134),(r28,A2,b1),(r28,A2,b2),(r28,A2,b3),(r28,A2,b4),(r28, A2,b5),(r28,A2,b6),(r28,A2,b7),(r28,A2,b8),(r28,A2,b9),(r28,A2,b10),(r28,A2,b11),(r28,A2 ,b12),(r28,A2,b13),(r28,A2,b14),(r28,A2,b15),(r28,A2,b16),(r28,A2,b17),(r28,A2,b18),(r28 ,A2,b19),(r28,A2,b20),(r28,A2,b21),(r28,A2,b22),(r28,A2,b23),(r28,A2,b24),(r28,A2,b25),( r28,A2,b26),(r28,A2,b27),(r28,A2,b28),(r28,A2,b29),(r28,A2,b30),(r28,A2,b31),(r28,A2,b 32),(r28,A2,b33),(r28,A2,b34),(r28,A2,b35),(r28,A2,b36),(r28,A2,b37),(r28,A2,b38),(r28, A2,b39),(r28,A2,b40),(r28,A2,b41),(r28,A2,b42),(r28,A2,b43),(r28,A2,b44),(r28,A2,b45),( r28,A2,b46),(r28,A2,b47),(r28,A2,b48),(r28,A2,b49),(r28,A2,b50),(r28,A2,b51),(r28,A2,b 52),(r28,A2,b53),(r28,A2,b54),(r28,A2,b55),(r28,A2,b56),(r28,A2,b57),(r28,A2,b58),(r28, A2,b59),(r28,A2,b60),(r28,A2,b61),(r28,A2,b62),(r28,A2,b63),(r28,A2,b64),(r28,A2,b65),( r28,A2,b66),(r28,A2,b67),(r28,A2,b68),(r28,A2,b69),(r28,A2,b70),(r28,A2,b71),(r28,A2,b 72),(r28,A2,b73),(r28,A2,b74),(r28,A2,b75),(r28,A2,b76),(r28,A2,b77),(r28,A2,b78),(r28, A2,b79),(r28,A2,b80),(r28,A2,b81),(r28,A2,b82),(r28,A2,b83),(r28,A2,b84),(r28,A2,b85),( r28,A2,b86),(r28,A2,b87),(r28,A2,b88),(r28,A2,b89),(r28,A2,b90),(r28,A2,b91),(r28,A2,b 92),(r28,A2,b93),(r28,A2,b94),(r28,A2,b95),(r28,A2,b96),(r28,A2,b97),(r28,A2,b98),(r28, A2,b99),(r28,A2,b100),(r28,A2,b101),(r28,A2,b102),(r28,A2,b103),(r28,A2,b104),(r28,A2, b105),(r28,A2,b106),(r28,A2,b107),(r28,A2,b108),(r28,A2,b109),(r28,A2,b110),(r28,A2,b1 11),(r28,A2,b112),(r28,A2,b113),(r28,A2,b114),(r28,A2,b115),(r28,A2,b116),(r28,A2,b117) ,(r28,A2,b118),(r28,A2,b119),(r28,A2,b120),(r28,A2,b121),(r28,A2,b122),(r28,A2,b123),(r 28,A2,b124),(r28,A2,b125),(r28,A2,b126),(r28,A2,b127),(r28,A2,b128),(r28,A2,b129),(r28 ,A2,b130),(r28,A2,b131),(r28,A2,b132),(r28,A2,b133),(r28,A2,b134),(r28,A3,b1),(r28,A3, b2),(r28,A3,b3),(r28,A3,b4),(r28,A3,b5),(r28,A3,b6),(r28,A3,b7),(r28,A3,b8),(r28,A3,b9),( r28,A3,b10),(r28,A3,b11),(r28,A3,b12),(r28,A3,b13),(r28,A3,b14),(r28,A3,b15),(r28,A3,b1 6),(r28,A3,b17),(r28,A3,b18),(r28,A3,b19),(r28,A3,b20),(r28,A3,b21),(r28,A3,b22),(r28,A 3,b23),(r28,A3,b24),(r28,A3,b25),(r28,A3,b26),(r28,A3,b27),(r28,A3,b28),(r28,A3,b29),(r2 8,A3,b30),(r28,A3,b31),(r28,A3,b32),(r28,A3,b33),(r28,A3,b34),(r28,A3,b35),(r28,A3,b36) ,(r28,A3,b37),(r28,A3,b38),(r28,A3,b39),(r28,A3,b40),(r28,A3,b41),(r28,A3,b42),(r28,A3, b43),(r28,A3,b44),(r28,A3,b45),(r28,A3,b46),(r28,A3,b47),(r28,A3,b48),(r28,A3,b49),(r28, A3,b50),(r28,A3,b51),(r28,A3,b52),(r28,A3,b53),(r28,A3,b54),(r28,A3,b55),(r28,A3,b56),( r28,A3,b57),(r28,A3,b58),(r28,A3,b59),(r28,A3,b60),(r28,A3,b61),(r28,A3,b62),(r28,A3,b 63),(r28,A3,b64),(r28,A3,b65),(r28,A3,b66),(r28,A3,b67),(r28,A3,b68),(r28,A3,b69),(r28, A3,b70),(r28,A3,b71),(r28,A3,b72),(r28,A3,b73),(r28,A3,b74),(r28,A3,b75),(r28,A3,b76),( r28,A3,b77),(r28,A3,b78),(r28,A3,b79),(r28,A3,b80),(r28,A3,b81),(r28,A3,b82),(r28,A3,b 83),(r28,A3,b84),(r28,A3,b85),(r28,A3,b86),(r28,A3,b87),(r28,A3,b88),(r28,A3,b89),(r28, A3,b90),(r28,A3,b91),(r28,A3,b92),(r28,A3,b93),(r28,A3,b94),(r28,A3,b95),(r28,A3,b96),( r28,A3,b97),(r28,A3,b98),(r28,A3,b99),(r28,A3,b100),(r28,A3,b101),(r28,A3,b102),(r28,A 3,b103),(r28,A3,b104),(r28,A3,b105),(r28,A3,b106),(r28,A3,b107),(r28,A3,b108),(r28,A3, b109),(r28,A3,b110),(r28,A3,b111),(r28,A3,b112),(r28,A3,b113),(r28,A3,b114),(r28,A3,b1 15),(r28,A3,b116),(r28,A3,b117),(r28,A3,b118),(r28,A3,b119),(r28,A3,b120),(r28,A3,b121 ),(r28,A3,b122),(r28,A3,b123),(r28,A3,b124),(r28,A3,b125),(r28,A3,b126),(r28,A3,b127),( r28,A3,b128),(r28,A3,b129),(r28,A3,b130),(r28,A3,b131),(r28,A3,b132),(r28,A3,b133),(r2 8,A3,b134),
(r28,A4,b1),(r28,A4,b2),(r28,A4,b3),(r28,A4,b4),(r28,A4,b5),(r28,A4,b6),(r28,A4,b7),(r28, A4,b8),(r28,A4,b9),(r28,A4,b10),(r28,A4,b11),(r28,A4,b12),(r28,A4,b13),(r28,A4,b14),(r2 8,A4,b15),(r28,A4,b16),(r28,A4,b17),(r28,A4,b18),(r28,A4,b19),(r28,A4,b20),(r28,A4,b21) ,(r28,A4,b22),(r28,A4,b23),(r28,A4,b24),(r28,A4,b25),(r28,A4,b26),(r28,A4,b27),(r28,A4, b28),(r28,A4,b29),(r28,A4,b30),(r28,A4,b31),(r28,A4,b32),(r28,A4,b33),(r28,A4,b34),(r28, A4,b35),(r28,A4,b36),(r28,A4,b37),(r28,A4,b38),(r28,A4,b39),(r28,A4,b40),(r28,A4,b41),( r28,A4,b42),(r28,A4,b43),(r28,A4,b44),(r28,A4,b45),(r28,A4,b46),(r28,A4,b47),(r28,A4,b 48),(r28,A4,b49),(r28,A4,b50),(r28,A4,b51),(r28,A4,b52),(r28,A4,b53),(r28,A4,b54),(r28, A4,b55),(r28,A4,b56),(r28,A4,b57),(r28,A4,b58),(r28,A4,b59),(r28,A4,b60),(r28,A4,b61),( r28,A4,b62),(r28,A4,b63),(r28,A4,b64),(r28,A4,b65),(r28,A4,b66),(r28,A4,b67),(r28,A4,b 68),(r28,A4,b69),(r28,A4,b70),(r28,A4,b71),(r28,A4,b72),(r28,A4,b73),(r28,A4,b74),(r28, A4,b75),(r28,A4,b76),(r28,A4,b77),(r28,A4,b78),(r28,A4,b79),(r28,A4,b80),(r28,A4,b81),( r28,A4,b82),(r28,A4,b83),(r28,A4,b84),(r28,A4,b85),(r28,A4,b86),(r28,A4,b87),(r28,A4,b 88),(r28,A4,b89),(r28,A4,b90),(r28,A4,b91),(r28,A4,b92),(r28,A4,b93),(r28,A4,b94),(r28, A4,b95),(r28,A4,b96),(r28,A4,b97),(r28,A4,b98),(r28,A4,b99),(r28,A4,b100),(r28,A4,b101 ),(r28,A4,b102),(r28,A4,b103),(r28,A4,b104),(r28,A4,b105),(r28,A4,b106),(r28,A4,b107),( r28,A4,b108),(r28,A4,b109),(r28,A4,b110),(r28,A4,b111),(r28,A4,b112),(r28,A4,b113),(r2 8,A4,b114),(r28,A4,b115),(r28,A4,b116),(r28,A4,b117),(r28,A4,b118),(r28,A4,b119),(r28, A4,b120),(r28,A4,b121),(r28,A4,b122),(r28,A4,b123),(r28,A4,b124),(r28,A4,b125),(r28,A 4,b126),(r28,A4,b127),(r28,A4,b128),(r28,A4,b129),(r28,A4,b130),(r28,A4,b131),(r28,A4, b132),(r28,A4,b133),(r28,A4,b134),(r29,A1,b1),(r29,A1,b2),(r29,A1,b3),(r29,A1,b4),(r29, A1,b5),(r29,A1,b6),(r29,A1,b7),(r29,A1,b8),(r29,A1,b9),(r29,A1,b10),(r29,A1,b11),(r29,A1 ,b12),(r29,A1,b13),(r29,A1,b14),(r29,A1,b15),(r29,A1,b16),(r29,A1,b17),(r29,A1,b18),(r29 ,A1,b19),(r29,A1,b20),(r29,A1,b21),(r29,A1,b22),(r29,A1,b23),(r29,A1,b24),(r29,A1,b25),( r29,A1,b26),(r29,A1,b27),(r29,A1,b28),(r29,A1,b29),(r29,A1,b30),(r29,A1,b31),(r29,A1,b 32),(r29,A1,b33),(r29,A1,b34),(r29,A1,b35),(r29,A1,b36),(r29,A1,b37),(r29,A1,b38),(r29, A1,b39),(r29,A1,b40),(r29,A1,b41),(r29,A1,b42),(r29,A1,b43),(r29,A1,b44),(r29,A1,b45),( r29,A1,b46),(r29,A1,b47),(r29,A1,b48),(r29,A1,b49),(r29,A1,b50),(r29,A1,b51),(r29,A1,b 52),(r29,A1,b53),(r29,A1,b54),(r29,A1,b55),(r29,A1,b56),(r29,A1,b57),(r29,A1,b58),(r29, A1,b59),(r29,A1,b60),(r29,A1,b61),(r29,A1,b62),(r29,A1,b63),(r29,A1,b64),(r29,A1,b65),( r29,A1,b66),(r29,A1,b67),(r29,A1,b68),(r29,A1,b69),(r29,A1,b70),(r29,A1,b71),(r29,A1,b 72),(r29,A1,b73),(r29,A1,b74),(r29,A1,b75),(r29,A1,b76),(r29,A1,b77),(r29,A1,b78),(r29, A1,b79),(r29,A1,b80),(r29,A1,b81),(r29,A1,b82),(r29,A1,b83),(r29,A1,b84),(r29,A1,b85),( r29,A1,b86),(r29,A1,b87),(r29,A1,b88),(r29,A1,b89),(r29,A1,b90),(r29,A1,b91),(r29,A1,b 92),(r29,A1,b93),(r29,A1,b94),(r29,A1,b95),(r29,A1,b96),(r29,A1,b97),(r29,A1,b98),(r29, A1,b99),(r29,A1,b100),(r29,A1,b101),(r29,A1,b102),(r29,A1,b103),(r29,A1,b104),(r29,A1, b105),(r29,A1,b106),(r29,A1,b107),(r29,A1,b108),(r29,A1,b109),(r29,A1,b110),(r29,A1,b1 11),(r29,A1,b112),(r29,A1,b113),(r29,A1,b114),(r29,A1,b115),(r29,A1,b116),(r29,A1,b117) ,(r29,A1,b118),(r29,A1,b119),(r29,A1,b120),(r29,A1,b121),(r29,A1,b122),(r29,A1,b123),(r 29,A1,b124),(r29,A1,b125),(r29,A1,b126),(r29,A1,b127),(r29,A1,b128),(r29,A1,b129),(r29 ,A1,b130),(r29,A1,b131),(r29,A1,b132),(r29,A1,b133),(r29,A1,b134),(r29,A2,b1),(r29,A2, b2),(r29,A2,b3),(r29,A2,b4),(r29,A2,b5),(r29,A2,b6),(r29,A2,b7),(r29,A2,b8),(r29,A2,b9),( r29,A2,b10),(r29,A2,b11),(r29,A2,b12),(r29,A2,b13),(r29,A2,b14),(r29,A2,b15),(r29,A2,b1 6),(r29,A2,b17),(r29,A2,b18),(r29,A2,b19),(r29,A2,b20),(r29,A2,b21),(r29,A2,b22),(r29,A 2,b23),(r29,A2,b24),(r29,A2,b25),(r29,A2,b26),(r29,A2,b27),(r29,A2,b28),(r29,A2,b29),(r2 9,A2,b30),(r29,A2,b31),(r29,A2,b32),(r29,A2,b33),(r29,A2,b34),(r29,A2,b35),(r29,A2,b36) ,(r29,A2,b37),(r29,A2,b38),(r29,A2,b39),(r29,A2,b40),(r29,A2,b41),(r29,A2,b42),(r29,A2, b43),(r29,A2,b44),(r29,A2,b45),(r29,A2,b46),(r29,A2,b47),(r29,A2,b48),(r29,A2,b49),(r29, A2,b50),(r29,A2,b51),(r29,A2,b52),(r29,A2,b53),(r29,A2,b54),(r29,A2,b55),(r29,A2,b56),( r29,A2,b57),(r29,A2,b58),(r29,A2,b59),(r29,A2,b60),(r29,A2,b61),(r29,A2,b62),(r29,A2,b 63),(r29,A2,b64),(r29,A2,b65),(r29,A2,b66),(r29,A2,b67),(r29,A2,b68),(r29,A2,b69),(r29, A2,b70),(r29,A2,b71),(r29,A2,b72),(r29,A2,b73),(r29,A2,b74),(r29,A2,b75),(r29,A2,b76),( r29,A2,b77),(r29,A2,b78),(r29,A2,b79),(r29,A2,b80),(r29,A2,b81),(r29,A2,b82),(r29,A2,b 83),(r29,A2,b84),(r29,A2,b85),(r29,A2,b86),(r29,A2,b87),(r29,A2,b88),(r29,A2,b89),(r29, A2,b90),(r29,A2,b91),(r29,A2,b92),(r29,A2,b93),(r29,A2,b94),(r29,A2,b95),(r29,A2,b96),( r29,A2,b97),(r29,A2,b98),(r29,A2,b99),(r29,A2,b100),(r29,A2,b101),(r29,A2,b102),(r29,A 2,b103),(r29,A2,b104),(r29,A2,b105),(r29,A2,b106),(r29,A2,b107),(r29,A2,b108),(r29,A2, b109),(r29,A2,b110),(r29,A2,b111),(r29,A2,b112),(r29,A2,b113),(r29,A2,b114),(r29,A2,b1 15),(r29,A2,b116),(r29,A2,b117),(r29,A2,b118),(r29,A2,b119),(r29,A2,b120),(r29,A2,b121 ),(r29,A2,b122),(r29,A2,b123),(r29,A2,b124),(r29,A2,b125),(r29,A2,b126),(r29,A2,b127),( r29,A2,b128),(r29,A2,b129),(r29,A2,b130),(r29,A2,b131),(r29,A2,b132),(r29,A2,b133),(r2 9,A2,b134),
(r29,A3,b1),(r29,A3,b2),(r29,A3,b3),(r29,A3,b4),(r29,A3,b5),(r29,A3,b6),(r29,A3,b7),(r29, A3,b8),(r29,A3,b9),(r29,A3,b10),(r29,A3,b11),(r29,A3,b12),(r29,A3,b13),(r29,A3,b14),(r2 9,A3,b15),(r29,A3,b16),(r29,A3,b17),(r29,A3,b18),(r29,A3,b19),(r29,A3,b20),(r29,A3,b21) ,(r29,A3,b22),(r29,A3,b23),(r29,A3,b24),(r29,A3,b25),(r29,A3,b26),(r29,A3,b27),(r29,A3, b28),(r29,A3,b29),(r29,A3,b30),(r29,A3,b31),(r29,A3,b32),(r29,A3,b33),(r29,A3,b34),(r29, A3,b35),(r29,A3,b36),(r29,A3,b37),(r29,A3,b38),(r29,A3,b39),(r29,A3,b40),(r29,A3,b41),( r29,A3,b42),(r29,A3,b43),(r29,A3,b44),(r29,A3,b45),(r29,A3,b46),(r29,A3,b47),(r29,A3,b 48),(r29,A3,b49),(r29,A3,b50),(r29,A3,b51),(r29,A3,b52),(r29,A3,b53),(r29,A3,b54),(r29, A3,b55),(r29,A3,b56),(r29,A3,b57),(r29,A3,b58),(r29,A3,b59),(r29,A3,b60),(r29,A3,b61),( r29,A3,b62),(r29,A3,b63),(r29,A3,b64),(r29,A3,b65),(r29,A3,b66),(r29,A3,b67),(r29,A3,b 68),(r29,A3,b69),(r29,A3,b70),(r29,A3,b71),(r29,A3,b72),(r29,A3,b73),(r29,A3,b74),(r29, A3,b75),(r29,A3,b76),(r29,A3,b77),(r29,A3,b78),(r29,A3,b79);(r29,A3,b80),(r29,A3,b81),( r29,A3,b82),(r29,A3,b83),(r29,A3,b84),(r29,A3,b85),(r29,A3,b86),(r29,A3,b87),(r29,A3,b 88),(r29,A3,b89),(r29,A3,b90),(r29,A3,b91),(r29,A3,b92),(r29,A3,b93),(r29,A3,b94),(r29, A3,b95),(r29,A3,b96),(r29,A3,b97),(r29,A3,b98),(r29,A3,b99),(r29,A3,b100),(r29,A3,b101 ),(r29,A3,b102),(r29,A3,b103),(r29,A3,b104),(r29,A3,b105),(r29,A3,b106),(r29,A3,b107),( r29,A3,b108),(r29,A3,b109),(r29,A3,b110),(r29,A3,b111),(r29,A3,b112),(r29,A3,b113),(r2 9,A3,b114),(r29,A3,b115),(r29,A3,b116),(r29,A3,b117),(r29,A3,b118),(r29,A3,b119),(r29, A3,b120),(r29,A3,b121),(r29,A3,b122),(r29,A3,b123),(r29,A3,b124),(r29,A3,b125),(r29,A 3,b126),(r29,A3,b127),(r29,A3,b128),(r29,A3,b129),(r29,A3,b130),(r29,A3,b131),(r29,A3, b132),(r29,A3,b133),(r29,A3,b134),(r29,A4,b1),(r29,A4,b2),(r29,A4,b3),(r29,A4,b4),(r29, A4,b5),(r29,A4,b6),(r29,A4,b7),(r29,A4,b8),(r29,A4,b9),(r29,A4,b10),(r29,A4,b11),(r29,A4 ,b12),(r29,A4,b13),(r29,A4,b14),(r29,A4,b15),(r29,A4,b16),(r29,A4,b17),(r29,A4,b18),(r29 ,A4,b19),(r29,A4,b20),(r29,A4,b21),(r29,A4,b22),(r29,A4,b23),(r29,A4,b24),(r29,A4,b25),( r29,A4,b26),(r29,A4,b27),(r29,A4,b28),(r29,A4,b29),(r29,A4,b30),(r29,A4,b31),(r29,A4,b 32),(r29,A4,b33),(r29,A4,b34),(r29,A4,b35),(r29,A4,b36),(r29,A4,b37),(r29,A4,b38),(r29, A4,b39),(r29,A4,b40),(r29,A4,b41),(r29,A4,b42),(r29,A4,b43),(r29,A4,b44),(r29,A4,b45),( r29,A4,b46),(r29,A4,b47),(r29,A4,b48),(r29,A4,b49),(r29,A4,b50),(r29,A4,b51),(r29,A4,b 52),(r29,A4,b53),(r29,A4,b54),(r29,A4,b55),(r29,A4,b56),(r29,A4,b57),(r29,A4,b58),(r29, A4,b59),(r29,A4,b60),(r29,A4,b61),(r29,A4,b62),(r29,A4,b63),(r29,A4,b64),(r29,A4,b65),( r29,A4,b66),(r29,A4,b67),(r29,A4,b68),(r29,A4,b69),(r29,A4,b70),(r29,A4,b71),(r29,A4,b 72),(r29,A4,b73),(r29,A4,b74),(r29,A4,b75),(r29,A4,b76),(r29,A4,b77),(r29,A4,b78),(r29, A4,b79),(r29,A4,b80),(r29,A4,b81),(r29,A4,b82),(r29,A4,b83),(r29,A4,b84),(r29,A4,b85),( r29,A4,b86),(r29,A4,b87),(r29,A4,b88),(r29,A4,b89),(r29,A4,b90),(r29,A4,b91),(r29,A4,b 92),(r29,A4,b93),(r29,A4,b94),(r29,A4,b95),(r29,A4,b96),(r29,A4,b97),(r29,A4,b98),(r29, A4,b99),(r29,A4,b100),(r29,A4,b101),(r29,A4,b102),(r29,A4,b103),(r29,A4,b104),(r29,A4, b105),(r29,A4,b106),(r29,A4,b107),(r29,A4,b108),(r29,A4,b109),(r29,A4,b110),(r29,A4,b1 11),(r29,A4,b112),(r29,A4,b113),(r29,A4,b114),(r29,A4,b115),(r29,A4,b116),(r29,A4,b117) ,(r29,A4,b118),(r29,A4,b119),(r29,A4,b120),(r29,A4,b121),(r29,A4,b122),(r29,A4,b123),(r 29,A4,b124),(r29,A4,b125),(r29,A4,b126),(r29,A4,b127),(r29,A4,b128),(r29,A4,b129),(r29 ,A4,b130),(r29,A4,b131),(r29,A4,b132),(r29,A4,b133),(r29,A4,b134),(r30,A1,b1),(r30,A1, b2),(r30,A1,b3),(r30,A1,b4),(r30,A1,b5),(r30,A1,b6),(r30,A1,b7),(r30,A1,b8),(r30,A1,b9),( r30,A1,b10),(r30,A1,b11),(r30,A1,b12),(r30,A1,b13),(r30,A1,b14),(r30,A1,b15),(r30,A1,b1 6),(r30,A1,b17),(r30,A1,b18),(r30,A1,b19),(r30,A1,b20),(r30,A1,b21),(r30,A1,b22),(r30,A 1,b23),(r30,A1,b24),(r30,A1,b25),(r30,A1,b26),(r30,A1,b27),(r30,A1,b28),(r30,A1,b29),(r3 0,A1,b30),(r30,A1,b31),(r30,A1,b32),(r30,A1,b33),(r30,A1,b34),(r30,A1,b35),(r30,A1,b36) ,(r30,A1,b37),(r30,A1,b38),(r30,A1,b39),(r30,A1,b40),(r30,A1,b41),(r30,A1,b42),(r30,A1, b43),(r30,A1,b44),(r30,A1,b45),(r30,A1,b46),(r30,A1,b47),(r30,A1,b48),(r30,A1,b49),(r30, A1,b50),(r30,A1,b51),(r30,A1,b52),(r30,A1,b53),(r30,A1,b54),(r30,A1,b55),(r30,A1,b56),( r30,A1,b57),(r30,A1,b58),(r30,A1,b59),(r30,A1,b60),(r30,A1,b61),(r30,A1,b62),(r30,A1,b 63),(r30,A1,b64),(r30,A1,b65),(r30,A1,b66),(r30,A1,b67),(r30,A1,b68),(r30,A1,b69),(r30, A1,b70),(r30,A1,b71),(r30,A1,b72),(r30,A1,b73),(r30,A1,b74),(r30,A1,b75),(r30,A1,b76),( r30,A1,b77),(r30,A1,b78),(r30,A1,b79),(r30,A1,b80),(r30,A1,b81),(r30,A1,b82),(r30,A1,b 83),(r30,A1,b84),(r30,A1,b85),(r30,A1,b86),(r30,A1,b87),(r30,A1,b88),(r30,A1,b89),(r30, A1,b90),(r30,A1,b91),(r30,A1,b92),(r30,A1,b93),(r30,A1,b94),(r30,A1,b95),(r30,A1,b96),( r30,A1,b97),(r30,A1,b98),(r30,A1,b99),(r30,A1,b100),(r30,A1,b101),(r30,A1,b102),(r30,A 1,b103),(r30,A1,b104),(r30,A1,b105),(r30,A1,b106),(r30,A1,b107),(r30,A1,b108),(r30,A1, b109),(r30,A1,b110),(r30,A1,b111),(r30,A1,b112),(r30,A1,b113),(r30,A1,b114),(r30,A1,b1 15),(r30,A1,b116),(r30,A1,b117),(r30,A1,b118),(r30,A1,b119),(r30,A1,b120),(r30,A1,b121 ),(r30,A1,b122),(r30,A1,b123),(r30,A1,b124),(r30,A1,b125),(r30,A1,b126),(r30,A1,b127),( r30,A1,b128),(r30,A1,b129),(r30,A1,b130),(r30,A1,b131),(r30,A1,b132),(r30,A1,b133),(r3 0,A1,b134),
(r30,A2,b1),(r30,A2,b2),(r30,A2,b3),(r30,A2,b4),(r30,A2,b5),(r30,A2,b6),(r30,A2,b7),(r30, A2,b8),(r30,A2,b9),(r30,A2,b10),(r30,A2,b11),(r30,A2,b12),(r30,A2,b13),(r30,A2,b14),(r3 0,A2,b15),(r30,A2,b16),(r30,A2,b17),(r30,A2,b18),(r30,A2,b19),(r30,A2,b20),(r30,A2,b21) ,(r30,A2,b22),(r30,A2,b23),(r30,A2,b24),(r30,A2,b25),(r30,A2,b26),(r30,A2,b27),(r30,A2, b28),(r30,A2,b29),(r30,A2,b30),(r30,A2,b31),(r30,A2,b32),(r30,A2,b33),(r30,A2,b34),(r30, A2,b35),(r30,A2,b36),(r30,A2,b37),(r30,A2,b38),(r30,A2,b39),(r30,A2,b40),(r30,A2,b41),( r30,A2,b42),(r30,A2,b43),(r30,A2,b44),(r30,A2,b45),(r30,A2,b46),(r30,A2,b47),(r30,A2,b 48),(r30,A2,b49),(r30,A2,b50),(r30,A2,b51),(r30,A2,b52),(r30,A2,b53),(r30,A2,b54),(r30, A2,b55),(r30,A2,b56),(r30,A2,b57),(r30,A2,b58),(r30,A2,b59),(r30,A2,b60),(r30,A2,b61),( r30,A2,b62),(r30,A2,b63),(r30,A2,b64),(r30,A2,b65),(r30,A2,b66),(r30,A2,b67),(r30,A2,b 68),(r30,A2,b69),(r30,A2,b70),(r30,A2,b71),(r30,A2,b72),(r30,A2,b73),(r30,A2,b74),(r30, A2,b75),(r30,A2,b76),(r30,A2,b77),(r30,A2,b78),(r30,A2,b79),(r30,A2,b80),(r30,A2,b81),( r30,A2,b82),(r30,A2,b83),(r30,A2,b84),(r30,A2,b85),(r30,A2,b86),(r30,A2,b87),(r30,A2,b 88),(r30,A2,b89),(r30,A2,b90),(r30,A2,b91),(r30,A2,b92),(r30,A2,b93),(r30,A2,b94),(r30, A2,b95),(r30,A2,b96),(r30,A2,b97),(r30,A2,b98),(r30,A2,b99),(r30,A2,b100),(r30,A2,b101 ),(r30,A2,b102),(r30,A2,b103),(r30,A2,b104),(r30,A2,b105),(r30,A2,b106),(r30,A2,b107),( r30,A2,b108),(r30,A2,b109),(r30,A2,b110),(r30,A2,b111),(r30,A2,b112),(r30,A2,b113),(r3 0,A2,b114),(r30,A2,b115),(r30,A2,b116),(r30,A2,b117),(r30,A2,b118),(r30,A2,b119),(r30, A2,b120),(r30,A2,b121),(r30,A2,b122),(r30,A2,b123),(r30,A2,b124),(r30,A2,b125),(r30,A 2,b126),(r30,A2,b127),(r30,A2,b128),(r30,A2,b129),(r30,A2,b130),(r30,A2,b131),(r30,A2, b132),(r30,A2,b133),(r30,A2,b134),(r30,A3,b1),(r30,A3,b2),(r30,A3,b3),(r30,A3,b4),(r30, A3,b5),(r30,A3,b6),(r30,A3,b7),(r30,A3,b8),(r30,A3,b9),(r30,A3,b10),(r30,A3,b11),(r30,A3 ,b12),(r30,A3,b13),(r30,A3,b14),(r30,A3,b15),(r30,A3,b16),(r30,A3,b17),(r30,A3,b18),(r30 ,A3,b19),(r30,A3,b20),(r30,A3,b21),(r30,A3,b22),(r30,A3,b23),(r30,A3,b24),(r30,A3,b25),( r30,A3,b26),(r30,A3,b27),(r30,A3,b28),(r30,A3,b29),(r30,A3,b30),(r30,A3,b31),(r30,A3,b 32),(r30,A3,b33),(r30,A3,b34),(r30,A3,b35),(r30,A3,b36),(r30,A3,b37),(r30,A3,b38),(r30, A3,b39),(r30,A3,b40),(r30,A3,b41),(r30,A3,b42),(r30,A3,b43),(r30,A3,b44),(r30,A3,b45),( r30,A3,b46),(r30,A3,b47),(r30,A3,b48),(r30,A3,b49),(r30,A3,b50),(r30,A3,b51),(r30,A3,b 52),(r30,A3,b53),(r30,A3,b54),(r30,A3,b55),(r30,A3,b56),(r30,A3,b57),(r30,A3,b58),(r30, A3,b59),(r30,A3,b60),(r30,A3,b61),(r30,A3,b62),(r30,A3,b63),(r30,A3,b64),(r30,A3,b65),( r30,A3,b66),(r30,A3,b67),(r30,A3,b68),(r30,A3,b69),(r30,A3,b70),(r30,A3,b71),(r30,A3,b 72),(r30,A3,b73),(r30,A3,b74),(r30,A3,b75),(r30,A3,b76),(r30,A3,b77),(r30,A3,b78),(r30, A3,b79),(r30,A3,b80),(r30,A3,b81),(r30,A3,b82),(r30,A3,b83),(r30,A3,b84),(r30,A3,b85),( r30,A3,b86),(r30,A3,b87),(r30,A3,b88),(r30,A3,b89),(r30,A3,b90),(r30,A3,b91),(r30,A3,b 92),(r30,A3,b93),(r30,A3,b94),(r30,A3,b95),(r30,A3,b96),(r30,A3,b97),(r30,A3,b98),(r30, A3,b99),(r30,A3,b100),(r30,A3,b101),(r30,A3,b102),(r30,A3,b103),(r30,A3,b104),(r30,A3, b105),(r30,A3,b106),(r30,A3,b107),(r30,A3,b108),(r30,A3,b109),(r30,A3,b110),(r30,A3,b1 11),(r30,A3,b112),(r30,A3,b113),(r30,A3,b114),(r30,A3,b115),(r30,A3,b116),(r30,A3,b117) ,(r30,A3,b118),(r30,A3,b119),(r30,A3,b120),(r30,A3,b121),(r30,A3,b122),(r30,A3,b123),(r 30,A3,b124),(r30,A3,b125),(r30,A3,b126),(r30,A3,b127),(r30,A3,b128),(r30,A3,b129),(r30 ,A3,b130),(r30,A3,b131),(r30,A3,b132),(r30,A3,b 133),(r30,A3,b134),(r30,A4,b1),(r30,A4, b2),(r30,A4,b3),(r30,A4,b4),(r30,A4,b5),(r30,A4,b6),(r30,A4,b7),(r30,A4,b8),(r30,A4,b9),( r30,A4,b10),(r30,A4,b11),(r30,A4,b12),(r30,A4,b13),(r30,A4,b14),(r30,A4,b15),(r30,A4,b1 6),(r30,A4,b 17),(r30,A4,b 18),(r30,A4,b19),(r30,A4,b20),(r30,A4,b21),(r30,A4,b22),(r30,A 4,b23),(r30,A4,b24),(r30,A4,b25),(r30,A4,b26),(r30,A4,b27),(r30,A4,b28),(r30,A4,b29),(r3 0,A4,b30),(r30,A4,b31),(r30,A4,b32),(r30,A4,b33),(r30,A4,b34),(r30,A4,b35),(r30,A4,b36) ,(r30,A4,b37),(r30,A4,b38),(r30,A4,b39),(r30,A4,b40),(r30,A4,b41),(r30,A4,b42),(r30,A4, b43),(r30,A4,b44),(r30,A4,b45),(r30,A4,b46),(r30,A4,b47),(r30,A4,b48),(r30,A4,b49),(r30, A4,b50),(r30,A4,b51),(r30,A4,b52),(r30,A4,b53),(r30,A4,b54),(r30,A4,b55),(r30,A4,b56),( r30,A4,b57),(r30,A4,b58),(r30,A4,b59),(r30,A4,b60),(r30,A4,b61),(r30,A4,b62),(r30,A4,b 63),(r30,A4,b64),(r30,A4,b65),(r30,A4,b66),(r30,A4,b67),(r30,A4,b68),(r30,A4,b69),(r30, A4,b70),(r30,A4,b71),(r30,A4,b72),(r30,A4,b73),(r30,A4,b74),(r30,A4,b75),(r30,A4,b76),( r30,A4,b77),(r30,A4,b78),(r30,A4,b79),(r30,A4,b80),(r30,A4,b81),(r30,A4,b82),(r30,A4,b 83),(r30,A4,b84),(r30,A4,b85),(r30,A4,b86),(r30,A4,b87),(r30,A4,b88),(r30,A4,b89),(r30, A4,b90),(r30,A4,b91),(r30,A4,b92),(r30,A4,b93),(r30,A4,b94),(r30,A4,b95),(r30,A4,b96),( r30,A4,b97),(r30,A4,b98),(r30,A4,b99),(r30,A4,b100),(r30,A4,b 101),(r30,A4,b102),(r30,A 4,b103),(r30,A4,b104),(r30,A4,b105),(r30,A4,b106),(r30,A4,b107),(r30,A4,b108),(r30,A4, b 109),(r30,A4,b110),(r30,A4,b111),(r30,A4,b 112),(r30,A4,b113),(r30,A4,b114),(r30,A4,b1 15), (r30,A4,b 116),(r30,A4,b117),(r30,A4,b118),(r30,A4,b119),(r30,A4,b120),(r30,A4,b121 ),(r30,A4,b122),(r30,A4,b123),(r30,A4,b124),(r30,A4,b125),(r30,A4,b126),(r30,A4,b127),( r30,A4,b128),(r30,A4,b129),(r30,A4,b130),(r30,A4,b131),(r30,A4,b132),(r30,A4,b133) or (r30,A4,b134).

The present compounds are useful in disease induced by the production, secretion or deposition of amyloid β protein, and are effective in treatment and/or prevention, and symptom improvement of such as dementia of the Alzheimer's type (Alzheimer's disease, senile dementia of Alzheimer type), Down's syndrome, memory impairment, prion disease (Creutzfeldt-Jakob disease), mild cognitive impairment (MCI), Dutch type of hereditary cerebral hemorrhage with amyloidosis, cerebral amyloid angiopathy, other type of degenerative dementia, mixed dementia with Alzheimer's and vascular type, dementia with Parkinson's Disease, dementia with progressive supranuclear palsy, dementia with Cortico-basal degeneration, Alzheimer's disease with diffuse Lewy body disease, age-related macular degeneration, Parkinson's Disease, amyloid angiopathy and so on.

In the present invention, "treating Alzheimer's disease" includes prevention of aggravation of MCI and prevention of familial Alzheimer's disease.

In the present invention, "a pharmaceutical composition which is a medicament for treating Alzheimer's disease" includes a pharmaceutical composition which is a medicament for prevention of aggravation of MCI and prevention of familial Alzheimer's disease.

Since the present compound has high inhibitory activity on BACE1, and/or has high selectivity on other enzymes, it can be a medicament with reduced side effect. Further, since the compound has high effect of reducing amyloid β production in a cell system, particularly, has high effect of reducing amyloid β production in brain, it can be an excellent medicament. In addition, by converting the compound into an optically active compound having suitable stereochemistry, the compound can be a medicament having a wider safety margin on the side effect. In addition, the present compound also has advantages that metabolism stability is high, solubility is high, oral absorbability is high, good bioavailability is exhibited, clearance is good, brain transference is high, a half life is high, non-protein binding rate is high, hERG channel inhibition is low, CYP inhibition is low, CYP MBI (mechanism-based inhibition) is low, and/or an Ames test is negative.

The present compounds can be administrated in combination with other pharmaceutical agents such as other therapeutic drugs for Alzheimer's disease, e.g., acetylcholinesterase and the like. The present compounds can be treated with concomitantly with the anti-dementia agents such as Donepezil Hydrochloride, Tacrine, Galantamine, Rivastigmine, Zanapezil, Memantine, and Vinpocetine.

When the present compound is administered to a human, it can be administered orally as powders, granules, tablets, capsules, pills, solutions, or the like, or parenterally as injectables, suppositories, transdermal absorbable agents, inhalations, or the like. In addition, the present compound can be formulated into pharmaceutical preparations by adding pharmaceutical additives such as excipients, binders, wetting agents, disintegrating agents, lubricants and the like, which are suitable for formulations and an effective amount of the present compound.

A dose is different depending on state of disease, an administration route, and an age and a weight of a patient, and is usually 0.1 µg to 1 g/day, preferably 0.01 to 200 mg/day when orally administered to an adult, and is usually 1 µg to 10 g/day, preferably 0.1 to 2 g/day when parenterally administered.

### [Examples]

Following examples and test examples illustrate the present invention in more detail, but the present invention is not limited by these examples.

¹H-NMR was measured in deuterium chloroform (CDCl₃) using tetramethylsilane as an internal standard. δ values were shown as ppm. Binding constants (J) were shown as Hz. In the data, s, d, t, m, br, and brs means singlet, doublet, triplet, multiplet, broad-line and broad-singlet, respectively. In the data, "and" means "and", "each" means "respectively".

In example, the meaning of each abbreviation is as follows:
Me methyl
Et ethyl
Bz benzoyl
Boc t-butoxycarbonyl
THF tetrahydrofuran
TOSMIC tosylmethyl isocyanide
HATU O-(7-azabenzotriazole-1-yl)-N,N,N',N'-tetramethyluronium hexafluorophosphate

LC/MS data of the present compound were measured under the following conditions, and a retention time and [M+H]⁺ are shown.
Compounds I-1 to I-52, I-55 to I-57, I-63, I-65 and I-66
Column: Shim-pack XR-ODS (2.2 µm, i.d. 50 x 3.0 mm) (Shimadzu)
Flow rate: 1.6 mL/min
Column oven: 50 °C
UV detection wavelength: 254 nm
Mobile phase: [A]is 0.1% formic acid-containing aqueous solution, [B] is 0.1% formic acid-containing acetonitrile solution
Gradient: Linear gradient of 10% to 100% solvent [B] for 3 minutes was performed, and 100% solvent [B] was maintained for 1 minutes.
Compounds I-53, I-54 to I-58 to I-62, I-64 and intermediate of example 12
Column: XBridge C18 (5 µm, i.d. 4.6 x 50 mm) (Waters)
Flow rate: 3 mL/min
UV detection wavelength: 254 nm
Mobile phase: [A]is 0.1% formic acid-containing aqueous solution, [B] is 0.1% formic acid-containing acetonitrile solution
Gradient: Linear gradient of 10% to 100% solvent [B] for 3 minutes was performed, and 100% solvent [B] was maintained for 1 minutes.

Reference Example 1

### Synthesis of intermediate compound (6)

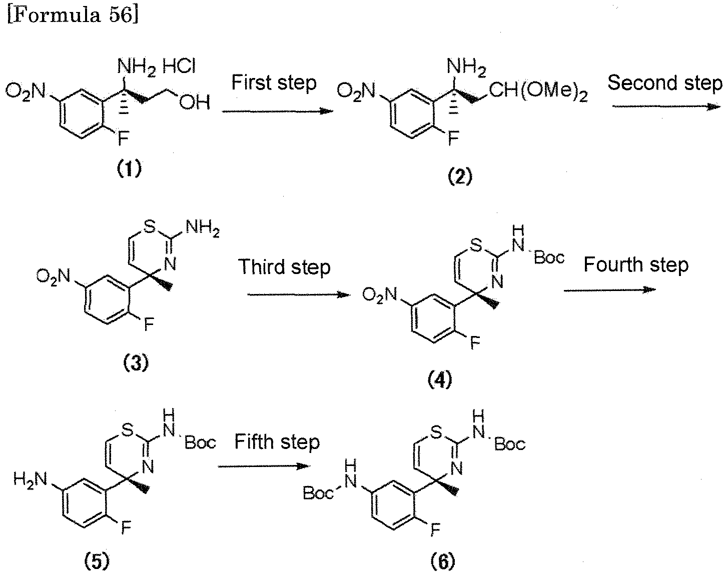

### First step

Aminoalcohol was prepared by the method such as those described in Patent Literature 2 (WO 2008/133274) and was converted to hydrochloride salt, the compound (1), according to the conventional manner. To a solution of the compound (1) (6.1 g) in the tetrahydrofuran (60 ml) were added water (30 ml), sodium hydrogen carbonate (3.9 g) and N-((9-fluorenyl)methoxycarbonyloxy) succinic acid imide (7.8 g). The solution was stirred for 2 hours and 30 minutes at room temperature. After extraction with ethyl acetate, the organic layer was dried over anhydrous magnesium sulfate. The solvent was evaporated under reduced pressure. The residue was dissolved in dimethyl sulfoxide (30 ml). 2-iodoxy benzoic acid (6.5 g) was added thereto. It was stirred at room temperature overnight. Water was added to the reaction mixture. After extraction by ethyl acetate, the organic layer was dried over anhydrous magnesium sulfate. The solvent was evaporated under reduced pressure. The residue was dissolved in methanol (100 ml). Amberlite 15 (2.0 g) was added thereto. It was heated under reflux for 2 hours and 30 minutes. After filtering, the solvent was evaporated under reduced pressure. The residue was dissolved in dimethylformamide. To the solution was added piperidine (2.8 ml). It was stirred at room temperature for 1 hour. Water was added thereto. The reaction mixture was extracted with ethyl acetate, washed with water and dried over anhydrous magnesium sulfate. The solvent was evaporated under reduced pressure. The residue was purified by column chromatography to give the compound (2) (3.4 g).
¹H-NMR(CDCl₃):1.56(3H,d,J=0.9Hz),2.10(1H,ddd,J=14.4,6.9,0.9Hz),2.39(1H,ddd,J=14. 4,4.2,2.1Hz),3.18(3H,s),3.19(3H,s),4.13(1H,dd,J=6.9,4.2Hz),7.16(1H,dd,J=10.8,8.9Hz),8. 16(1H,ddd,J=8.9,4.0,3.0Hz),8.62(1H,dd,J=6.9,3.0Hz).

### Second step

To a solution in the acetone (12 ml) of the compound (2) (3.4 g) obtained in the first step was added at 0 °C, benzoyl isothiocyanate (1.7 ml). It was stirred for 30 minutes. The solvent was evaporated under reduced pressure. To the residue was added concentrated sulfuric acid (13 ml). It was stirred at 40 °C overnight. After adding ice (40 g) and tetrahydrofuran (10 ml) and making it alkaline with an aqueous ammonia 28%, it was extracted with ethyl acetate and dried over anhydrous magnesium sulfate. The solvent was removed under reduced pressure. The residue was purified by column chromatography to give the compound (3) (2.6 g).
¹H-NMR (CDCl₃)
δ:1.72(3H,d,J=0.9Hz),6.26(1H,dd,J=9.5,4.6Hz),6.35(1H,d,J=9.5Hz),7.16(1H,dd,J=10.5,8. 9Hz),8.13(1H,ddd,J=8.9,4.1,3.0Hz),8.41(1H,dd,J=6.9,3.0Hz).

### Third step

The compound (3) (600 mg) obtained in the second step was dissolved in tetrahydrofuran(5 ml). Di-tert-butyl carbonate (686 mg) was added at room temperature, and the mixture was stirred at 60°C for 6 hours. Water was added to the reaction solution, and the mixture was extracted with ethyl acetate. The organic layer was washed with brine, and dried over anhydrous sodium sulfate. The solvent was evaporated under reduced pressure to afford the compound (4) (820 mg).

### Fourth step

The compound (4) (820 mg) obtained in the third step was dissolved in a mixed solvent of tetrahydrofuran (10 ml), methanol (10 ml) and water (5ml). Ammonium chloride (537 mg) and iron (436 mg) were added, and the mixture was stirred at 60°C for 5 hours. After the mixture was alkalized with 28% aqueous ammonia and the resulting insoluble material was removed by filtration, the filtrate was extracted with ethyl acetate. After dried over anhydrous magnesium sulfate, the solvent was evaporated under reduced pressure to afford the compound (5) (600 mg).

### Fifth step

The compound (5) (1.0 g) obtained in the fourth step was dissolved in dichloromethane (10 ml). Di-tert-butyl carbonate (712 mg) was added at room temperature, and the mixture was stirred at 40°C for 4 hours. An aqueous saturated sodium bicarbonate solution was added to the reaction mixture and the mixture was extracted with ethyl acetate. The organic layer was washed with brine, dried over anhydrous sodium sulfate, and the solvent was evaporated under reduced pressure. The residue was purified by column chromatography to afford the compound (6) (1.0 g). ¹H-NMR (CDCl₃) δ:
1.51(9H,s),1.52(9H,s),1.75(3H,s),6.16(1H,dd,J=9.89,3.30Hz),6.22(1H,d,J=9.63Hz),6.44(1 H,s),6.99(1H,dd,J=11.15,8.62Hz),7.17(1H,dd,J=7.10,2.53Hz),7.39(1H,s).

Example 1

### Synthesis of compound (I-38)

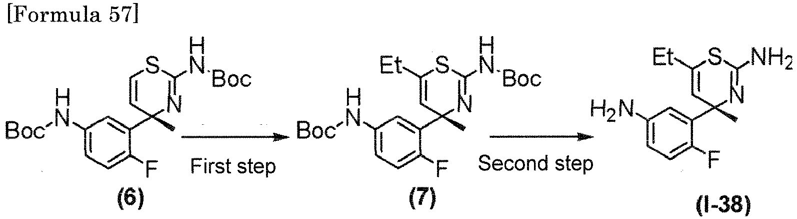

### First step

A solution of tetramethylethylene diamine (172 µl) in THF (3 ml) was cooled to -78°C, and a solution of 2.76 mol/L n-butyllithium in n-hexane (414 µl) was added dropwise thereto at -78°C. A solution of compound (6) (100 mg) in tetrahydrofuran (1 ml) was added dropwise, the mixture was stirred at -78°C for 1 hour, and a solution of iodoethane (28 µl) in tetrahydrofuran (1 ml) was added dropwisely. After the mixture was stirred at -78°C for 1 hour, a saturated ammonium chloride aqueous solution was added, then the mixture was extracted with ethyl acetate. The organic layer was dried over anhydrous magnesium sulfate, and the solvent was evaporated under reduced pressure. The residue was purified by column chromatography to afford the compound (7) (57 mg).
¹H-NMR (CDCl₃) δ:
1.13(3H,t,J=7.35Hz),1.51(9H,s),1.52(9H,s),1.74(3H,s),2.26(2H,q,J=7.44Hz),5.89(1H,s),6. 42(1H,s),6.98(1H,dd,J=11.41,8.87Hz),7.11(1H,dd,J=7.10,2.53Hz),7.38-7.41(1H,m).

### Second step

The compound (7) (50 mg) obtained in the first step was dissolved in dichloromethane (1 ml). Trifluoroacetic acid (1 ml) was added to the solution, and the mixture was stirred at room temperature for 1 hour. The reaction solution was concentrated under reduced pressure. An aqueous saturated sodium bicarbonate solution and water were added to the resulting residue and the solid was precipitated. The precipitated solid was collected by filtration using Kiriyama funnel to afford the compound (I-38) (19 mg). ¹H-NMR (CDCl₃) δ:
1.10(3H,t,J=7.35Hz),1.65(3H,s),2.25(2H,q,J=7.44Hz),3.50(2H,s),4.56(2H,brs),5.96(1H,d, J=5.07Hz),6.46-6.49(1H,m),6.75(1H,dd,J=6.84,2.79Hz),6.80(1H,dd,J=11.66,8.62Hz).

Example 2

### Synthesis of compound (I-39)

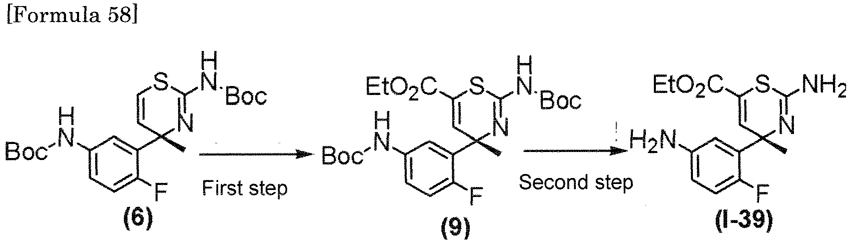

### First step

A solution of tetramethylethylene diamine (137 µl) in THF (3 ml) was cooled to -78°C, and a solution of 2.76 mol/L n-butyllithium in n-hexane (328 µl) was dropwisely added thereto at -78°C. A solution of compound (6) (120 mg) in tetrahydrofuran (1 ml) was added dropwise and the mixture was stirred at -78°C for 1 hour. A solution of diethyl carbonate (50 µl) in tetrahydrofuran (1 ml) was dropwisely added to the mixture. The mixture was stirred at -78°C for 1 hour, an aqueous saturated ammonium chloride solution was added, and the mixture was extracted with ethyl acetate. The organic layer was dried over anhydrous magnesium sulfate, and the solvent was evaporated under reduced pressure. The residue was purified by column chromatography to afford the compound (9) (101 mg).

### Second step

The compound (9) (120 mg) obtained in the first step was dissolved in dichloromethane (1 ml). To the solution was added trifluoroacetic acid (1 ml), and the mixture was stirred at room temperature for 1 hour. The reaction solution was concentrated under reduced pressure. An aqueous saturated sodium bicarbonate solution and water were added to the resulting residue and the mixture was extracted with ethyl acetate. The organic layer was dried over anhydrous magnesium sulfate, and the solvent was evaporated under reduced pressure. The residue was purified by column chromatography to afford the compound (I-39) (55 mg).
¹H-NMR (CDCl₃) δ:
1.31(3H,q,J=8.45Hz),1.72(3H,s),3.53(2H,s),4.28(2H,ddd,J=18.12,6.97,4.18Hz),4.63(2H,b rs),6.49-6.53(1H,m),6.74(1H,dd,J=6.59,2.53Hz),6.83(1H,dd,J=11.15,8.62Hz),7.32(1H,d,J =4.56Hz).

Example 3

### Synthesis of compound (I-40)

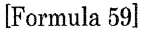

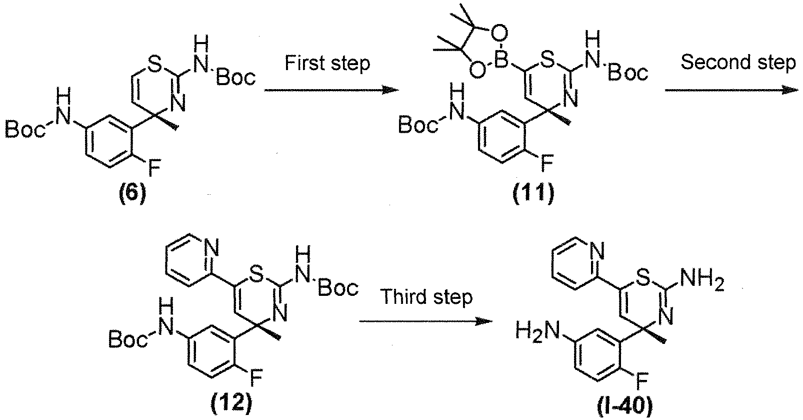

### First step

A solution of tetramethylethylene diamine (517 µl) in THF (3 ml) was cooled to -78°C, and a solution of n-butyllithium in n-hexane (1.24 ml) was added dropwise thereto at -78°C. A solution of compound (6) (300 mg) in tetrahydrofuran (1 ml) was dropwisely added to the mixture. After the mixture was stirred at -78°C for 1 hour, a solution of 2-isopropoxy-4,4,5,5-tetramethyl-1,3,2-dioxaborane (208 µl) in tetrahydrofuran (1 ml) was added dropwisely. The mixture was stirred at -78°C for 1 hour, an aqueous saturated ammonium chloride solution was added and the mixture was extracted with ethyl acetate. The organic layer was dried over anhydrous magnesium sulfate, and the solvent was evaporated under reduced pressure. The residue was purified by column chromatography to afford the compound (11) (480 mg) as a crude product.
¹H-NMR (CDCl₃)
δ:1.26(11H,s),1.50(9H,s),1.51(9H,s),1.74(3H,s),6.43(1H,s),6.76(1H,d,J=3.55Hz),6.98(1H, dd,J=11.15,8.62Hz),7.11(1H,d,J=4.06Hz),7.45(1H,s).

### Second step

The compound (11) (90 mg) obtained in the first step and 2-bromopyridin (25 mg) were dissolved in a mixed solvent of dimethylformamide (2.7 ml) and water (0.3ml).
To the mixture were added potassium carbonate (66.2 mg) and 1,1'-bis (di-tert-butylphosphino) ferrocene palladium dichloride (10 mg), and the mixture was stirred at room temperature for 5 hours. An aqueous saturated sodium bicarbonate solution was added to the reaction solution and the mixture was extracted with ethyl acetate. The organic layer was dried over anhydrous magnesium sulfate, and the solvent was evaporated under reduced pressure. The residue was purified by column chromatography to afford the compound (12) (27 mg).
¹H-NMR (CDCl₃)
δ:1.50(9H,s),1.53(9H,s),1.85(3H,s),6.44(1H,s),6.90(1H,s),7.00(1H,dd,J=11.15,9.12Hz),7.1 7(1H,s),7.22(1H,t,J=6.08Hz),7.48(1H,s),7.56(1H,d,J=8.11Hz),7.68(1H,t,J=7.86Hz),8.58( 1H,d,J=4.56Hz).

### Third step

The compound (12) (50 mg) obtained in the second step was dissolved in dichloromethane(1 ml). Trifluoroacetic acid (1 ml) was added to the solution and the mixture was stirred at room temperature for 1 hour. The reaction solution was concentrated under reduced pressure. An aqueous saturated sodium bicarbonate solution and water were added to the resulting residue, and the mixture was extracted with ethyl acetate. The organic layer was dried over anhydrous magnesium sulfate, and the solvent was evaporated under reduced pressure to afford the compound (I-40) (30 mg).
MS[M+1] 315; LC/MS retention time 0.86 min.

Example 4

### Synthesis of compound (I-41)

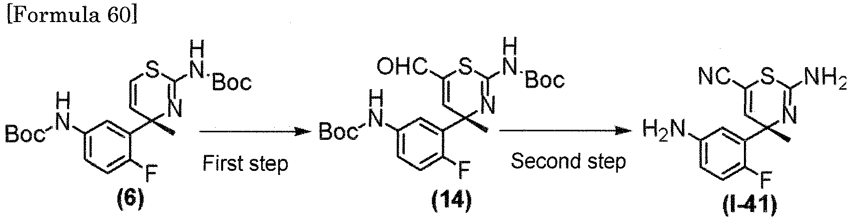

### First step

A solution of tetramethylethylene diamine (690 µl) in THF (3 ml) was cooled to -78°C, and a solution of 2.76 mol/L n-butyllithium in n-hexane (1.66 ml) was dropwisely added thereto at -78°C. A solution of compound (6) (400 mg) in tetrahydrofuran (1 ml) was added dropwise and the mixture was stirred at -78°C for 1 hour. A solution of ethylformate (102 mg) in tetrahydrofuran (1 ml) was dropwisely added to the reaction mixture. After the mixture was stirred at -78°C for 1 hour, an aqueous saturated ammonium chloride solution was added, and the mixture was extracted with ethyl acetate. The organic layer was dried over anhydrous magnesium sulfate, and the solvent was evaporated under reduced pressure. The residue was purified by column chromatography to afford the compound (14) (348 mg).
¹H-NMR (CDCl₃)
δ:1.51(9H,s),1.52(9H,s),1.76(3H,s),6.46(1H,s),7.01-7.03(2H,m),7.30(1H,dd,J=6.84,2.28H z),7.41(1H,s),9.50(1H,s).

### Second step

The compound (14) (55 mg) obtained in the first step was dissolved in a mixed solvent of acetonitrile (3 ml) and water (0.3 ml). To the solution was added aminooxy sulfonic acid (17.37 mg), and the mixture was stirred at 65°C for 1 hour. The reaction solution was concentrated under reduced pressure. An aqueous saturated sodium bicarbonate solution and water were added to the resulting residue, and the precipitated solid was collected by filtration using Kiriyama funnel. Thionyl chloride (1 ml) was added to the obtained solid (40 mg) at 0 °C. After the mixture was allowed to warm up to room temperature, the mixture was stirred for 3 hours. The reaction solution was poured into ice water, and the solution was alkalized with an aqueous sodium hydroxide solution. The mixture was extracted with ethyl acetate, and the organic layer was dried over anhydrous magnesium sulfate. The solvent was evaporated under reduced pressure to afford the compound (I-41) (20 mg).
MS[M+1] 263; LC/MS retention time 0.66 min.

### Example 5

### Synthesis of compound (I-42)

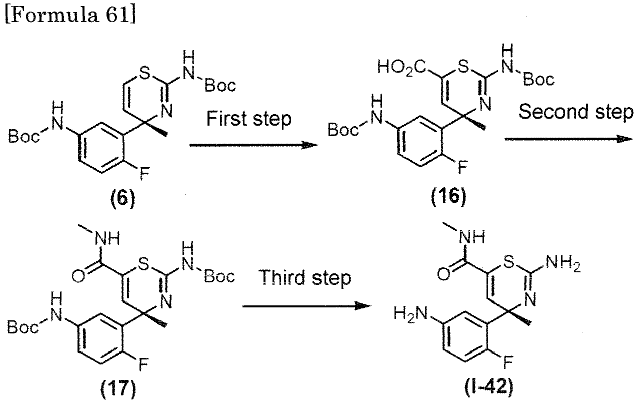

### First step

A solution of tetramethylethylene diamine (345 µl) in THF (3 ml) was cooled to -78°C, and a solution of 2.76 mol/L n-butyllithium in n-hexane (828 µl) was dropwisely added thereto at -78°C. A solution of compound (6) (200 mg) in tetrahydrofuran (1 ml) was dropwisely added to the solution. The mixture was stirred at -78°C for 1 hour, and dry ice (about 500 mg) was added. The mixture was stirred at -78°C for 1 hour and an aqueous saturated ammonium chloride solution was added. The mixture was extracted with ethyl acetate and the organic layer was dried over anhydrous magnesium sulfate. The solvent was evaporated under reduced pressure to afford the white solid. The residue was purified by recrystallization (ethyl acetate/ hexane) to afford the compound (16) (184 mg).
¹H-NMR(DMSO-d₆)δ:1.42(9H,s),1.45(9H,s),1.61(3H,s),6.64(1H,s),7.05(1H,dd,J=11.66,9. 12Hz),7.36(1H,s),7.43(1H,d,J=7,10Hz),9.33(1H,s).

### Second step

The compound (16) (76 mg) obtained in the first step was dissolved in tetrahydrofuran (3 ml), and cooled to 0°C. To the solution were added oxalyl chloride (18 µl) and dimethylformamide (1 µl) at 0°C. The mixture was warmed to room temperature, and stirred for 30 minutes.

The above reaction solution was dropwisely added to a solution of triethylamine (33 µl) and 2.0 mol/L methylamine (2 ml) in THF at 0 °C, and the mixture was stirred at 0°C for 1 hour. An aqueous saturated sodium bicarbonate solution was added to the reaction solution and the mixture was extracted with ethyl acetate. The organic layer was dried over anhydrous magnesium sulfate. The solvent was evaporated under reduced pressure to afford the compound (17) (78 mg) as a crude product.
¹H-NMR (CDCl₃) δ:
1.50(9H,s),1.52(9H,s),1.74(3H,s),2.89(3H,d,J=5.07Hz),6.07(1H,d,J=4.56Hz),6.47(1H,s),6 .96-6.99(2H,m),7.18(1H,s),7.40(1H,s).

### Third step

The compound (17) (78 mg) obtained in the second step was dissolved in dichloromethane (1 ml). Trifluoroacetic acid (1 ml) was added to the solution, and the mixture was stirred at room temperature for 1 hour. The reaction solution was concentrated under reduced pressure. An aqueous saturated sodium bicarbonate solution and water were added to the residue, and the mixture was extracted with ethyl acetate. The organic layer was dried over anhydrous magnesium sulfate, and the solvent was evaporated under reduced pressure to afford the compound (I-42) (46 mg). ¹H-NMR(DMSO-d₆)δ:1.60(3H,s),2.65(3H,d,J=4.56Hz),4.96(2H,brs),6.44(1H,s),6.60(1H,d d,J=6.84,2.79Hz),6.80(1H,t,J=10.14Hz),6.90(1H,s),8.35(1H,brs).

### Example 6

### Synthesis of compound (I-43)

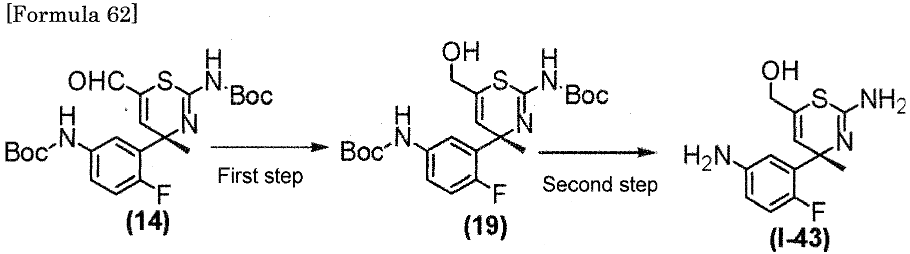

### First step

The compound (14) (130 mg) was dissolved in tetrahydrofuran (3 ml), and cooled to 0°C. To the solution was added sodium borohydride (12 mg) at 0°C, and the mixture was stirred at room temperature for 1 hour. The reaction solution was cooled to 0°C, and 0.1 mol/L hydrochloric acid was added thereto. The mixture was stirred for 30 minutes and extracted with ethyl acetate. The organic layer was washed with a saturated sodium bicarbonate aqueous solution and brine, and dried over anhydrous magnesium sulfate. The solvent was evaporated under reduced pressure to afford the compound (19) (130 mg).
¹H-NMR(CDCl₃)δ:1.50(9H,s),1.51(9H,s),1.76(3H,s),4.27(2H,dd,J=23.07,13.43Hz),6.15(1 H,s),6.46(1H,s),6.98(1H,dd,J=11.15,8.62Hz),7.26-7.29(1H,m),7.32-7.34(1H,m).

### Second step

The compound (19) (130 mg) obtained in the first step was dissolved in dichloromethane (1 ml). Trifluoroacetic acid (1 ml) was added to the solution, and the mixture was stirred at room temperature for 1 hour. The reaction solution was concentrated under reduced pressure. An aqueous saturated sodium bicarbonate solution and water were added to the residue, and the mixture was extracted with ethyl acetate. The organic layer was dried over anhydrous magnesium sulfate, and the solvent was evaporated under reduced pressure to afford the compound (I-43) (70 mg).
¹H-NMR(CDCl₃)δ:1.67(3H,s),3.49(1H,s),4.24(2H,s),6.24(1H,d,J=5.07Hz),6.48-6.50(1H,m ),6.74(1H,dd,J=6.59,3.04Hz),6.81(1H,dd,J=11.15,8.62Hz).

### Example 7

### Synthesis of compound (I-44)

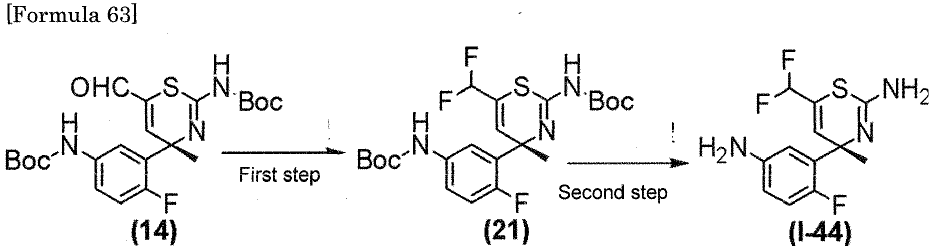

### First step

The compound (14) (89 mg) was dissolved in dichloromethane (5 ml). To the solution was added (diethylamino)sulfur trifluoride (62 µl) at 0°C, and the mixture was stirred at room temperature for 8 hours. The reaction solution was cooled to 0°C, and an aqueous saturated sodium bicarbonate solution was added thereto. The mixture was extracted with dichloromethane. The organic layer was washed with brine, and dried over anhydrous magnesium sulfate. The solvent was evaporated under reduced pressure, and the residue was purified by column chromatography to afford the compound (21) (56 mg).
¹H-NMR (CDCl₃) δ:
1.51(9H,s),1.52(9H,s),1.71(3H,s),6.14(1H,t,J=55.26Hz),6.43(2H,s),7.00(1H,dd,J=11.15,9. 12Hz),7.26(1H,s),7.40(1H,s).

### Second step

The compound (21) (50 mg) obtained in the first step was dissolved in dichloromethane (1 ml). Trifluoroacetic acid (1 ml) was added to the solution, and the mixture was stirred at room temperature for 1 hour. The reaction solution was concentrated under reduced pressure. An aqueous saturated sodium bicarbonate solution and water were added to the residue, and the mixture was extracted with ethyl acetate. The organic layer was dried over anhydrous magnesium sulfate, and the solvent was evaporated under reduced pressure to afford the compound (I-44) (29 mg).
¹H-NMR(CDCl₃)δ:1.69(3H,s),3.55(2H,brs),6.17(1H,t,J=55.51Hz),6.51-6.52(1H,m),6.58-6. 59(1H,m),6.74-6.77(1H,m),6.80-6.85(1H,m).

Example 8

### Synthesis of compound (I-45)

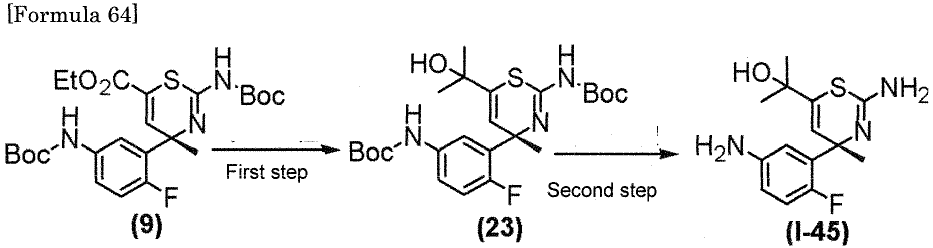

### First step

The compound (9) (260 mg) was dissolved in THF (5 ml), and the mixture was cooled to -78°C. A solution of 1.12 mol/L methyl lithium in n-hexane (1.815 ml) was dropwisely added to the solution, and the mixture was stirred at -78°C for 1 hour. An aqueous saturated ammonium chloride solution was added to the reaction solution, and the mixture was extracted with ethyl acetate. The organic layer was washed with brine, and dried over anhydrous magnesium sulfate. The solvent was evaporated under reduced pressure, and the residue was purified by silica gel column chromatography to afford the compound (23) (153 mg).
¹H-NMR(CDCl₃)δ:1.46(3H,s),1.50(9H,s),1.52(9H,s),1.52(3H,s),1.80(3H,s),2.12(1H,s),6.15 (1H,d,J=3.04Hz),6.45(1H,s),6.96(1H,dd,J=11.15,9.12Hz),7.22(1H,d,J=8.11Hz),7.38(1H,d ,J=5.07Hz).

### Second step

The compound (23) (80 mg) obtained in the first step was dissolved in dichloromethane (1 ml). Trifluoroacetic acid (1 ml) was added to the solution, and the mixture was stirred at room temperature for 1 hour. The reaction solution was concentrated under reduced pressure. An aqueous saturated sodium bicarbonate solution and water were added to the residue, and the mixture was extracted with ethyl acetate. The organic layer was dried over anhydrous magnesium sulfate, and the solvent was evaporated under reduced pressure to afford the compound (I-45) (44 mg). ¹H-NMR(CDCl₃)δ:1.44(6H,d,J=4.56Hz),1.67(3H,s),3.52(2H,brs),6.22(1H,d,J=5.58Hz),6.4 6-6.50(1H,m),6.74(1H,dd,J=6.59,2.53Hz),6.80(1H,dd,J=11.15, 8.62Hz).

Example 9

### Synthesis of compound (I-46)

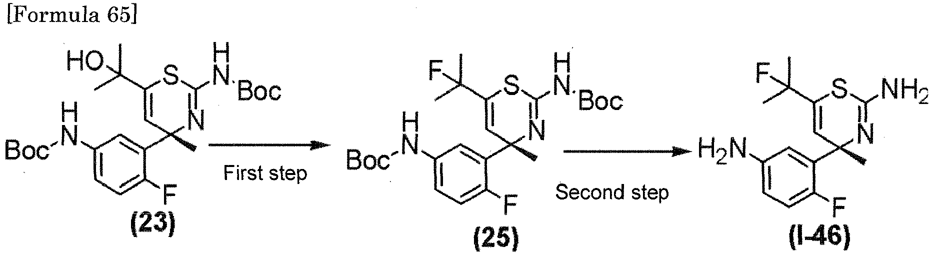

### First step

The compound (23) (68 mg) was dissolved in dichloromethane (3 ml). To the solution was added (diethylamino)sulfur trifluoride (22 µl) at 0°C, and the mixture was stirred at 0°C 1 hour. An aqueous saturated sodium bicarbonate solution was added to the reaction solution and the mixture was extracted with dichloromethane. The organic layer was washed with brine, and dried over anhydrous magnesium sulfate. The solvent was evaporated under reduced pressure, and the residue was purified by column chromatography to afford the compound (25) (45 mg).
¹H-NMH(CDCl₃) δ:
1.51(12H,s),1.52(14H,s),1.76(3H,s),6.17(1H,s),6.43(1H,s),6.98(1H,dd,J=11.41,8.87Hz),7. 20(1H,s),7.36-7.38(1H,m).

### Second step

The compound (25) (43 mg) obtained in the first step was dissolved in dichloromethane (1 ml). Trifluoroacetic acid (1 ml) was added to the solution, and the mixture was stirred at room temperature for 1 hour. The reaction solution was concentrated under reduced pressure. An aqueous saturated sodium bicarbonate solution and water were added to the residue, and the mixture was extracted with ethyl acetate. The organic layer was dried over anhydrous magnesium sulfate, and the solvent was evaporated under reduced pressure to afford the compound (I-46) (26 mg). MS[M+1] 298; LC/MS retention time 0.89 min.

Example 10

### Synthesis of compound (I-6)

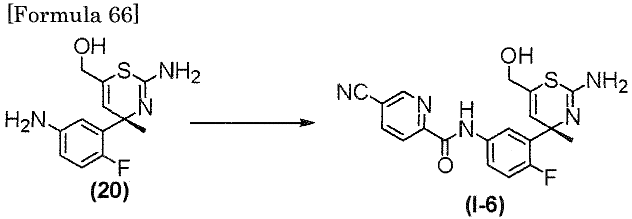

The compound (20) and 5-cyano picolinic acid (20 mg) were dissolved in methanol (0.45 ml). To the solution was added a 2 mol/L hydrochloric acid (65 µl), and the mixture was cooled to 0°C. To the reaction mixture was added N-ethyl-N'-(3-dimethylaminopropyl) carbodiimide hydrochloride (26 mg), and the mixture was stirred at 0°C for 1 hour. A 2 mol/L sodium hydroxide aqueous solution (65 µl) and an aqueous saturated sodium bicarbonate solution were added to the reaction solution, and the mixture was extracted with ethyl acetate. The organic layer was washed with brine, and dried over anhydrous magnesium sulfate. The solvent was evaporated under reduced pressure to afford the crude product (47 mg). The compound (I-6) (33 mg) was prepared by recrystallization.

Example 11

### Synthesis of compound (I-1)

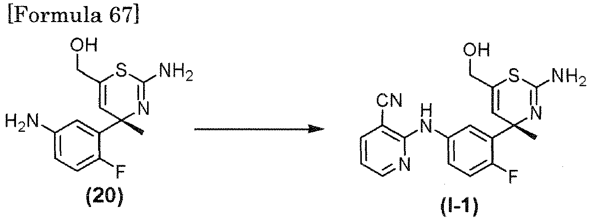

The compound (20) (30 mg) and 2-chloronicotinonitrile (47 mg) were dissolved in n-butanol (3 ml). Aconcentrated sulfuric acid (19 µl) was added thereto, and the mixture was heated at reflux for 8 hours. An aqueous sodium hydroxide solution was added to the reaction solution, and the mixture was extracted with ethyl acetate. The organic layer was washed with brine, and dried over anhydrous magnesium sulfate. The solvent was evaporated under reduced pressure, and the residue was purified by column chromatography to afford the crude product (15 mg). Compound (I-1) (10 mg) was prepared by recrystallization with diethyl ether/hexane.

Example 12

### Synthesis of compound (I-58)

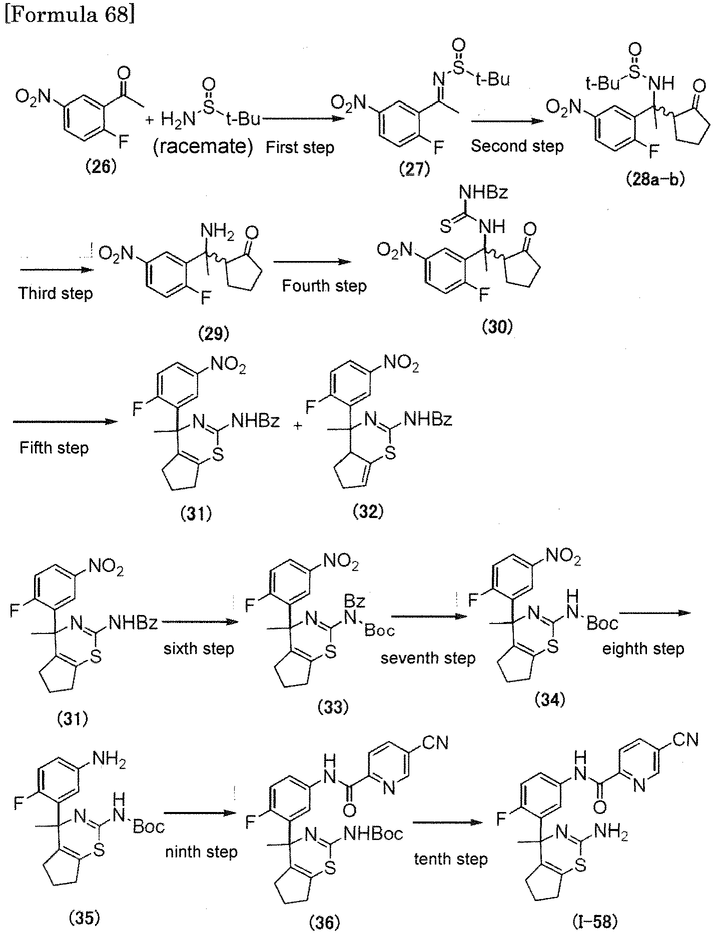

### First step

Titanium tetraethoxide (19.89 g) was dissolved in tetrahydrofuran (44 ml). To the solution were added 2-methyl-2-propanesulfinamide (4.65 g) and the compound (26) (6.39 g). The mixture was stirred at reflux in an oil bath at 85°C under a nitrogen stream.

After 3 hours, the insoluble material was removed by filtration with Celite (10 g). The filtrate was washed with water and brine. The organic layer was dried over sodium sulfate, and the solvent was evaporated under reduced pressure. Diisopropyl ether and n-hexane were added to the residue (10.67 g), and the mixture was filtered under ice-cooling to afford the compound (27) (8.24 g).
LCMS: 287(M+H)⁺ retention time 1.74 min
¹H-NMR(CDCl₃):1.34(9H,s),2.82(3H,d,J=3.3Hz),7.30(1H,t,J=9.6Hz),8.31-8.34(1H,m),8.5 5-8.58(1H,m).

### Second step

To tetrahydrofuran (58 ml) was added a solution of lithiumhexamethyl disilazide in 1.6M tetrahydrofuran (10 ml) at -78°C. A solution of cyclopentanone (1.346 g) in tetrahydrofuran (35 ml) was dropwisely added to the solution and the mixture was stirred for 0.5 hour. A solution of the compound (27) (2.863 g) in tetrahydrofuran (58 ml) was dropwisely added to the reaction solution, and the mixture was stirred for 5 hours. After a saturated solution of ammonium chloride in methanol (50 ml) was added to the reaction solution, water and ethyl acetate was added to the mixture and extracted with ethyl acetate. The organic layer was dried over sodium sulfate, and the solvent was evaporated under reduced pressure.

The residue was purified by column chromatography to afford the compound (28a) (564 mg) and the compound (28b) (150 mg).
(28a):
¹H-NMR(CDCl₃):1.22(9H,s),1.59-1.81(3H,m),1.87(3H,s),1.92-2.05(1H,m),2.14-2.27(1H,m ),2.39-2.48(1H,m),3.05-3.12(1H,m),5.74(1H,bs),7.19(1H,dd,J=9.0,11.4Hz),8.21-8.26(1H, m),8.39(1H,dd,J=2.7,6.6Hz).
LCMS:371(M+H)⁺ retention time 1.80 min
(28b):¹H-NMR(CDCl₃):1.25(9H,s),1.75-1.91(2H,m),1.96(3H,d,J=0.9Hz),2.04-2.43(4H,m), 3.09-3.16(1H,m),5.38(1H,bs),7.17(1H,dd,J=9.0,11.4Hz),8.15-8.20(1H,m),8.30(1H,dd,J=2. 7,6.9Hz).
LCMS: 371(M+H)⁺ retention time 1.73 min

### Third step

The compound (28a) (564 mg) and the compound (28b) (150 mg) were dissolved in methanol (5.6 ml), and the mixture was stirred at room temperature. To the solution was added a 5-10% solution of hydrochloride in methanol (9.4 ml), and the mixture was stirred.

After 1.75 hours, the reaction solution was poured into ice and ethyl acetate (80 ml), then an aqueous saturated sodium bicarbonate solution (30 ml) was added thereto and the mixture was extracted with ethyl acetate.. The organic layer was washed once with brine (30ml), and dried over sodium sulfate. The solvent was evaporated under reduced pressure and the residue (560 mg) was purified by silica gel column chromatography to afford the compound (29) (462 mg, 90%).
LCMS: 267(M+H)⁺ retention time 0.57min
¹H-NMR(CDCl₃):1.50 and 1.79(3H,each
s),1.55-2.18(5H,m),2.28-2.41(1H,m),2.90-2.97(1H,m),7.11-7.19(1H,m),8.12-8.18(1H,m),8. 49-8.55(1H,m).

### Fourth step

The compound (29) (462 mg) was dissolved in tetrahydrofuran (11.6 ml). To the solution was added benzoyl isothiocyanates (0.233 ml) and the mixture was stirred under ice-cooling and a nitrogen stream. After 1 hour, the mixture was stirred at room temperature. After 1 hour, the reaction solution was evaporated under reduced pressure.

The residue was purified by silica gel column chromatography to afford the compound (30) (780 mg, 104.7%).
LCMS: 430(M+H)⁺ retention time 2.12 and 2.19 min
¹H-NMR(CDCl₃):1.98 and 2.11(3H,each s),1.59-2.30(5H,m),2.30-2.55(1H,m),3.06 and 3.86(1H,each
m),7.08-7.15(1H,m),7.48-7.59(2H,m),7.59-7.66(1H,m),7.83-7.89(2H,m),8.15-8.20(1H,m), 8.27 and 8.42(1H,each dd,J=2.7,6.9Hz),8.77(1H,s),11.56 and 12.13(1H,each s).

### Fifth step

The compound (30) (714 mg) was cooled with ice-bath, and to the mixture was added an ice cooled concentrated sulfuric acid (14.3 ml). After stirred for 1 hour, the reaction solution was gradually added to ice under stirring ice-cooling, and a solution of sodium hydroxide (19.5 g) in water (20 ml) was added during 15 min (pH 3-4). After ethyl acetate (30 ml) was added, 50% aqueous potassium carbonate solution (5 ml) was added (pH 8-9). The mixture was poured into separating funnel, ethyl acetate (50 ml) was added, and separated. The aqueous layer was extracted with ethyl acetate (40 ml) and the combined organic layers were washed with brine (20 ml). The organic layer was dried over sodium sulfate, and the solvent was evaporated under reduced pressure.

The residue was purified by silica gel column chromatography to afford the compound (31) (211 mg, 32.3%), the compound (32) (170 mg, 26%), and the mixture of the compounds (31) and (32) (1:1) (151 mg, 23.1%).
Compound (31)
LCMS: 412(M+H)+ retention time 2.50 min
¹H-NMR(CDCl₃) :1.94(3H,s),2.00-2.12(2H,m),2.20-2.34(1H,m),2.50-2.58(1H,m),2.65-2.7 0(2H,m),7.20-7.24(1H,m),7.38-7.47(2H,m),7.40-7.52(1H,m),8.07-8.10(2H,m),8.16-8.25(2 H,m).
Compound (32)
LCMS: 412(M+H)+ retention time 2.46 min
¹H-NMR(CDCl₃) :1.66(3H,s),1.87-1.96(1H,m),2.46-2.55(2H,m),3.42(1H,m),5.95(1H,q,J= 2.4Hz),7.25(1H,m),7.29(1H,dd,J=9.3,11.1Hz),7.39-7.43(2H,m),7.47-7.52(1H,m),8.05(2H, bd,J=7.2Hz),8.24-8.29(1H,m),8.37(1H,dd,J=2.7,6.6Hz).

### Sixth step

To the compound (31) (171 mg) and N,N-dimethyl aminopyridine (61 mg) was added tetrahydrofuran (8.6 ml) and the mixture was suspended. To the suspension was added di-tert-butyl dicarbonate (0.134 ml) at a time at room temperature with stirring under a nitrogen stream.

After stirring for 1.5 hours, the solvent was evaporated under reduced pressure, and the residue was purified by silica gel column chromatography to afford the compound (33) (213 mg, 100%).
LCMS: 512(M+H)+ retention time 2.81 min
¹H-NMR(CDCl₃):1.37(9H,s),1.64(3H,d,J=1.2Hz),1.90-2.04(3H,m),2.35-2.45(1H,m),2.54-2.58(2H,m),7.08(1H,dd,J=9.0,10.2Hz),7.25-7.42(2H,m),7.47-7.52(1H,m),7.69-7.72(2H,m) ,8.10-8.15(1H,m),8.21(1H,dd,J=3.0,6.6Hz).

### Seventh step

The compound (33) (213 mg) was dissolved in a mixture of tetrahydrofuran and methanol (I:1) (8.6 ml). After addition of water (2.6 ml), potassium carbonate (173 mg) was added at a time. After stirring for 2.5 hours, the reaction solution was poured into iced water and the mixture was extracted with ethyl acetate (40 ml). The organic layer was washed with brine, dried over sodium sulfate, and the solvent was evaporated under reduced pressure. The residue was purified by silica gel column chromatography to afford the compound (34) (157 mg, 92.6%).
LCMS: 408(M+H)⁺ retention time 2.47 min
¹H-NMR(CDCl₃) :1.48(9H,s),1.81(3H,bs),1.88-2.24(3H,m),2.40-2.52(1H,m),2.56-2.61(2H, m),7.22(1H,t,J=9.3Hz),8.18-8.21(2H,m).

### Eighth step

The compound (34) (157 mg) was dissolved in tetrahydrofuran (2.4 ml) and methanol (1.6 ml). To the solution were added iron powder (119 mg), ammonium chloride (92 mg) and water (0.63 ml), and the mixture was stirred at 60°C in an oil bath. After 4 hours, the reaction mixture was filtered with Celite (10 g), and washed with ethyl acetate (40 ml). The filtrate was poured into ice and an aqueous saturated sodium bicarbonate solution (3 ml) and extracted. The organic layer was washed with brine, dried over anhydrous magnesium sulfate, and the solvent was evaporated under reduced pressure.

The residue was purified by silica gel column chromatography to afford the compound (35) (95 mg, 65.36%).
LCMS: 378(M+H)⁺ retention time 1.61min
¹H-NMR(CDCl₃):1.48(9H,s),1.80(3H,s),1.90-2.02(2H,m),2.25-2.36(1H,m),2.53-2.58(2H, m),6.49(1H,dd.J=3.0,6.6Hz),6.52-6.58(1H,m),6.83(1H,dd,J=8.4,11.4Hz).

### Ninth step

To the compound (35) (30 mg) and 3-cyanopyridine-6-carboxylic acid monohydrate (17.2 mg) was added dimethylformamide (0.9 ml) and dissolved. To the solution were added triethylamine (0.0166 ml) and HATU (39.2 mg) under stirring at room temperature, and the mixture was stirred. After 1.25 hours, the reaction solution was added to an aqueous saturated sodium bicarbonate solution (2 ml) under ice-cooling, and the mixture was extracted once with ethyl acetate. The organic layer was washed twice with water, dried over sodium sulfate, and the solvent was evaporated under reduced pressure. The residue was purified by silica gel column chromatography to afford the compound (36) (43 mg, 106.7%).
LCMS: 508(M+H)⁺ retention time 3.21min
¹H-NMR(CDCl₃) :1.49(9H,s),1.86(3H,s),1.98-2.03(2H,m),2.25-2.38(1H,m),2.46-2.51(1H, m),2.57-2.61(2H,m),7.09(1H,dd,J=8.7,10.8Hz),7.67-7.72(2H,m),8.21(1H,dd,J=2.1,8.4Hz) ,8.41(1H,dd,J=0.6,8.1Hz),8.90(1H,dd,J=0.6,2.1Hz),9.82(1H,bs).

### Tenth step

The compound (36) (64 mg) was dissolved in formic acid (0.39 ml), and the mixture was stirred at 40°C in an oil bath. After 1 hour, the reaction solution was evaporated under reduced pressure. The residue was dissolved in water and ethyl acetate under ice-cooling. The mixture was poured into 50% aqueous potassium carbonate solution (5 ml) and ice, and extracted. Then, insoluble material was extracted twice with a solution of chloroform in methanol (9:1) and the combined organic layers were washed with brine. The organic layer was dried over sodium sulfate, and the solvent was evaporated under reduced pressure.

The residue was dissolved in ethyl acetate and methanol. The solution was concentrated under reduced pressure and diethyl ether was added thereto. The precipitated solid was collected by filtration to afford the compound (I-58) (36 mg, 74.2%).
LCMS: 408(M+H)⁺ retention time 1.27 min
¹H-NMR(d6-DMSO) :1.59(3H,s),1.84(2H,m),2.12(1H,m),2.34(1H,m),6.09(2H,bs),7.07(1H ,dd,J=9.0,11.4Hz),7.79-7.82(1H,m),7.86-7.89(1H,m),8.27(1H,d,J=8.1Hz),8.57(1H,dd,J=1. 8,8.1Hz),9.19(1H,s),10.84(1H,bs).

Example 13

### Synthesis of compound (I-59)

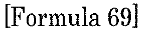

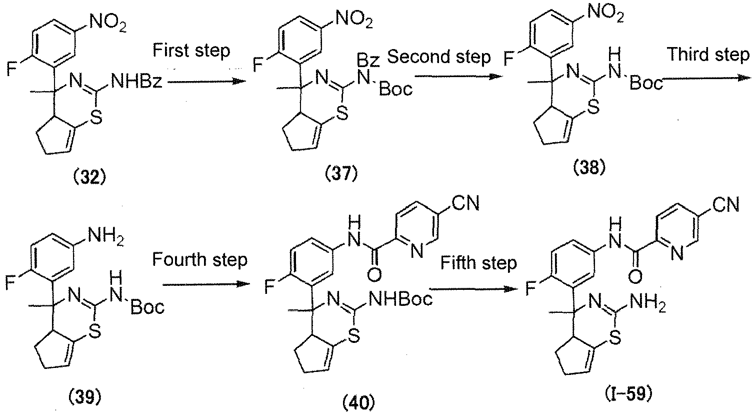

### First step

The compound (37) (183 mg) was prepared from the compound (32) (147 mg) in a similar manner to sixth step in Example 12.
LCMS: 512(M+H)⁺ retention time 2.79 min
¹H-NMR(CDCl₃):1.39(9H,s),1.43(3H,d,J=1.8Hz),1.68-1.83(2H,m),2.34-2.56(2H,m),2.91-2.97(1H,m),5.86(1H,m),7.15(1H,dd,J=8.7,10.5Hz),7.39-7.44(2H,m),7.49-7.54(1H,m),7.70 -7.73(2H,m),8.12-8.17(1H,m),8.25(1H,dd,J=2.7,6.6Hz).

### Second step

The compound (38) (133 mg, 91.4%) was prepared from the compound (37) (183 mg) in a similar manner to seventh step in Example 12.
LCMS: 408(M+H)⁺ retention time 2.40 min
¹H-NMR(CDCl₃):1.48(9H,s),1.52(3H,s),1.65-1.73(1H,m),1.79-1.89(1H,m),2.39-2.49(2H, m),3.01(1H,bs),5.85-5.87(1H,m),7.18-7.25(1H,m),8.15-8.24(1H,m),8.35(1H,dd,J=2.7,6.6 Hz).

### Third step

The compound (39) (85 mg, 69.1%) was prepared from the compound (38) (133 mg) in a similar manner to eighth step in Example 12.
LCMS: 378(M+H)⁺ retention time 1.41 min
¹H-NMR(CDCl₃) :1.47(9H,s),1.53(3H,s),1.47-1.69(1H,m),1.80-1.93(1H,m),2.36-2.48(2H, m),3.30-3.42(1H,m),3.50-3.70(2H,m),5.80(1H,dd,J=2.1,4.5Hz),6.58-6.62(1H,m),6.67(1H, dd,J=2.7,6.6Hz),6.89(1H,dd,J=8.4,12.0Hz).

### Fourth step

The compound (40) (24 mg, 89.2%) was prepared from the compound (39) (20 mg) in a similar manner to ninth step in Example 12.
LCMS: 508(M+H)⁺ retention time 2.69 min
¹H-NMR(CDCl₃) :1.47(9H,s),1.59(3H,s),1.55-1.70(1H,m),1.86-1.95(1H,m),2.40-2.50(2H, m),3.37(1H,bs),5.83(1H,m),7.14(1H,dd,J=8.4,11.4Hz),7.69-7.74(1H,m),7.85-7.87(1H,m), 8.22(1H,dd,J=2.1,8.1Hz),8.42(1H,d,J=7.8Hz),8.90(1H,m),9.88(1H,bs).

### Fifth step

The compound (I-59) (21 mg, 63.8%) was prepared from the compound (40) (41 mg) in a similar manner to tenth step in Example 12.
LCMS: 408(M+H)⁺ retention time 1.22 min
¹H-NMR(d6-DMSO) :1.30(3H,s),1.45-1.60(1H,m),1.63-1.76(1H,m),2.31(2H,m),2.82(1H, m),5.71(1H,m),6.00(2H,bs),7.10(1H,dd,J=2.7,11.7Hz),7.85-7.90(1H,m),7.98(1H,dd,J=1.4, 7.2Hz),8.27(1H,d,J=8.1Hz),8.58(1H,dd,J=2.1,8.1Hz),9.19(1H,m),10.84(1H,bs).

Example 14

### Synthesis of compound (I-65)

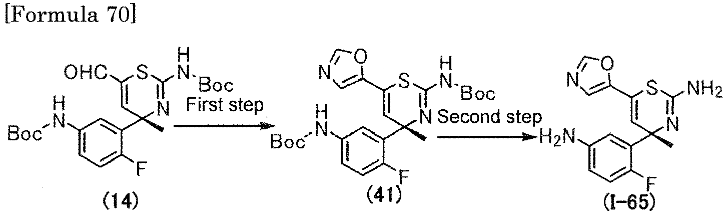

### First step

The compound (14) (80 mg) was dissolved in methanol (2 ml). To the solution were added a solution of 1M sodium methoxide (NaOMe) in methanol (0.722 ml) and TOSMIC (40.3 mg) in this order, and the mixture was stirred at room temperature for 1 hour. An aqueous saturated sodium bicarbonate solution (NaHCO₃) was added to the reaction solution, and the precipitated solid was collected using Kiriyama funnel to afford the compound (41) (84 mg).
¹H-NMR (CDCl₃)
8:7.84(1H,s),7.43(1H,t,J=10.14Hz),7.18(1H,s),7.00(1H,dd,J=11.41,8.87Hz),6.60(1H,s),6. 42(1H,s),1.54(3H,s), 1.54(9H,s), 1.51(9H,s).

### Second step

The compound (41) (84 mg) obtained in the first step was dissolved in dichloromethane (1 ml). Trifluoroacetic acid (1 ml) was added to the solution, and the mixture was stirred at room temperature for 1 hour. The reaction solution was concentrated under reduced pressure. An aqueous saturated sodium bicarbonate solution and water were added to the resulting residue, and the mixture was extracted with ethyl acetate. The organic layer was dried anhydrous magnesium sulfate, and the solvent was evaporated under reduced pressure to afford the compound (I-65) (50 mg).

Example 15

### Synthesis of compound (I-66)

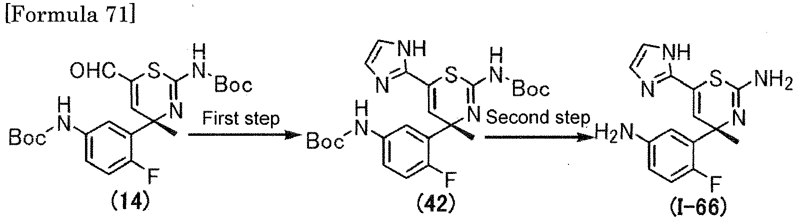

### First step

The compound (14) (70 mg) was dissolved in a mixed solvent of methanol (4 ml) and water (1 ml). To the solution were added oxalaldehyde (0.034 ml) and NH₄CO₃H (47.5 mg), and the mixture was stirred at room temperature for 5 days. To the mixture were added oxalaldehyde (0.034 ml) and NH₄CO₃H (47.6 mg), and the mixture was stirred at 50°C for 2 days. The reaction solution was concentrated under reduced pressure. A 10% aqueous potassium carbonate solution was added to the resulting residue, and the solid was precipitated. The precipitated solid was filtered with Kiriyama funnel to afford yellow solid. The solid was purified by aminosilica gel column chromatography to afford the compound (42) (26 mg).
¹H-NMR (CDCl₃)
δ:9.76(1H,s),7.41(1H,s),7.18(1H,d,J=4.56Hz),7.13(1H,s),7.03(1H,s),7.00-6.94(1H,m),6.65 (1H,s),6.52(1H,s),1.76(3H,s),1.52(9H,s),1.50(9H,s).

### Second step

The compound (42) (25 mg) obtained in the first step was dissolved in methanol (1ml). To the solution was added a 10% solution of hydrochloric acid (HCl) in methanol (1 ml), and the mixture was stirred at 50°C for 4 hours. The reaction solution was concentrated under reduced pressure to afford the compound (I-66) (20 mg).

The following compounds are prepared according to the above examples. In the tables, RT represents a retention time (minutes).

**[Table 1-1]**

| **Compound No.** | **Structure** | **NMR (Solvent: shift value ascending order)** | **MS [M+1]** | **LC/MS RT** |
|---|---|---|---|---|
| 1-1 | 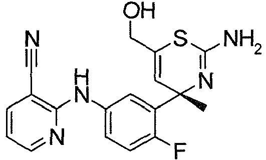 | 1H-NMR (CDCl3) δ 1 71 (3H, s), 4.25 (2H, d, J = 7.60 Hz), 6.27 (1H, d, J = 4.56 Hz), 6.77 (1H, dd, J = 7.60, 5.07 Hz), 6 95 (1H, s), 7.03 (1H, dd, J = 11.15, 8.62 Hz), 7.45 (1H, dd, J = 6 84, 2.79 Hz), 7.59-7 63 (1H, m), 7 77 (1H, dd, J = 7.86, 1.77 Hz) | 370 | 0.97 |
| I-2 | 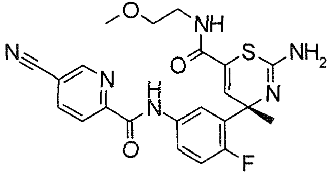 | 1H-NMR (DMSO-d6) δ 1.60 (3H, s), 3 23 (3H, s), 3 29 (2H, t, J = 5.32 Hz), 3 37 (2H, t, J = 5.58 Hz), 6.42 (2H, s), 6.95 (1H, d, J = 4 06 Hz), 7 17 (1H, dd, J = 11.41, 8.87 Hz), 7 77-7.81 (1H, m), 7 96 (1H, dd, J = 7 10, 2 53 Hz), 8 28 (1H, d, J = 8 11 Hz) 8 39 (1H, t, J = 2 03 Hz), 9 19-9 20 (1H, m), 1081 (1H,s) | 469 | 0.91 |
| I-3 | 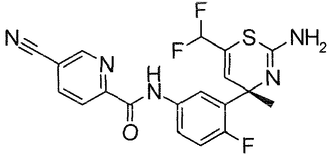 | 1H-NMR (CDCl3) δ 1 73 (3H, s), 4 78 (2H, br s), 6 19 (1H, t, J = 55 51 Hz), 6 61-6 63 (1H, m), 7 09 (1H, dd, J = 11 15, 8 62 Hz), 7 73 (1H, dd, J = 6 84, 2 79 Hz), 7 84 (1H, ddd, J = 8 87, 4 44, 2.22 Hz), 8 20 (1H, dd, J = 8 11, 2 03 Hz), 8 42 (1H, d, J = 8.11 Hz), 8 88-8 89 (1H, m), 9 81 (1H, s) | 418 | 1.17 |

**[Table 1-2]**

| **Compound No.** | **Structure** | **NMR (Solvent: shift value ascending order)** | **MS [M+1]** | **LC/MS RT** |
|---|---|---|---|---|
| **I-4** | 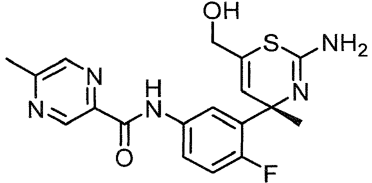 | 1H-NMR (DMSO-d6) δ 1.56 (3H, s), 2.63 (3H, s), 4.02 (2H, d, J = 5.58 Hz), 5.20 (1H, t, J = 5.83 Hz), 609 (1H, d, J = 4.06 Hz), 6 29 (2H, s), 7 13 (1H, dd, J = 11 66, 8.62 Hz), 7.71-7 75 (1H, m), 7.90 (1H, dd, J = 7.35, 2.79 Hz), 8 69 (1H, s), 9.14 (1H, s), 10 59(1H, s). | **388** | **0.88** |
| **I-5** | 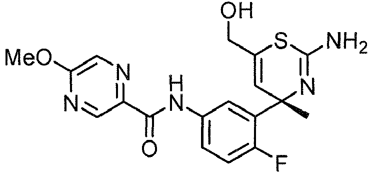 | 1H-NMR (DMSO-d6) δ 1.55 (3H, s), 4.02 (5H, s), 520 (1H, t, J = 5.83 Hz), 6.09 (1H, d, J = 406 Hz), 6.29 (2H, s), 7.12 (1H, dd, J = 11.66, 8.62 Hz), 7.71 (1H, dt, J = 8.45, 3 42 Hz), 7 87 (1H, dd, J = 7 35, 2.79 Hz), 8 41 (1H, d. J = 1 01 Hz), 8 88 (1H, d, J = 1 01 Hz), 10.40 (1H, s) | **404** | **1.00** |
| **I-6** | 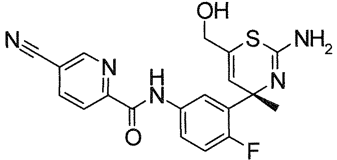 | 1H-NMR (DMSO-d6) 6. 1.56 (3H, s), 4.02 (2H, d, J = 6 08 Hz), 5.21 (1H, t, J = 6 08 Hz), 6 09 (1H, d, J = 4.06 Hz), 6.31 (2H, s), 7 14 (1H,dd,J=11.41,887 Hz), 7 73-7.77 (1H, m), 7 90 (1H, dd, J = 7 35, 2 79 Hz), 8 27 (1H, d, J = 8 11 Hz), 8 58 (1H, dd,J = 8 11, 2 03 Hz), 9 19 (1H, d, J = 2 03 Hz), 10 74 (1H, s) | **398** | **0.94** |

**[Table 1-3]**

| **Compound No.** | **Structure** | **NMR (Solvent: shift value ascending order)** | **MS [M+1]** | **LC/MS RT** |
|---|---|---|---|---|
| **I-7** | 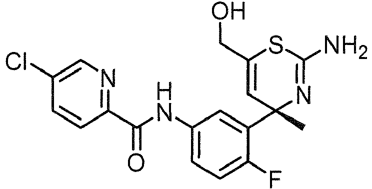 | 1H-NMR (DMSO-d6) δ 1 56 (3H, s), 4.02 (2H, d, J = 5.07 Hz), 5 20 (1H, t, J = 6 08 Hz), 6.09 (1H, d, J = 4.56 Hz), 6.30 (2H, s), 7 13 (1H, dd, J = 11.41, 887 Hz), 7 74 (1H, td, J = 5 70, 3.21 Hz), 7.87 (1H, dd, J = 735, 2.79 Hz), 8.13-8.20 (2H, m), 8.78 (1H, d, J = 2.03 Hz), 10.56 (1H,s). | **407** | **1.15** |
| **I-8** | 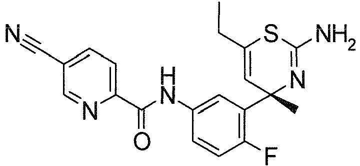 | 1H-NMR(CDCl3) δ 1.11 (3H, t, J = 7 35 Hz), 1.69 (3H, s), 2.28 (2H, q, J = 7.44 Hz), 4 65 (2H, br s), 5.99 (1H, d, J = 5.07 Hz), 7.06 (1H, dd, J = 11 15, 8.62 Hz), 7 61 (1H, dd, J = 6.84, 2.79 Hz), 7 85-7 87 (1H, m), 8.19 (1H, dd, J = 811,2.03Hz),8.42(1H,d, J = 8 11 Hz), 8 88-8 88 (1H, m), 9 79 (1H, s). | 396 | 1.22 |
| **I-9** | 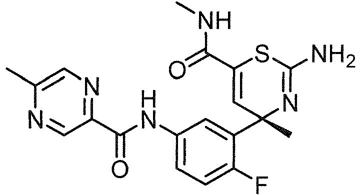 | 1H-NMR (DMSO-d6) δ 1.60 (3H, s), 2 63 (3H, s), 2 65 (3H, d, J = 4 06 Hz), 6 41 (2H, s), 6 89 (1H, d, J = 4.06 Hz), 7 15 (1H, dd, J = 1014, 5.07 Hz), 7.74-7 78 (1H, m), 7 94 (1H, d, J = 7 10 Hz), 8 28 (1H, d, J = 4 56 Hz), 8 69 (1H, s), 9 14 (1H, s), 10 65 (1H, s) | **415** | **0.93** |

**[Table 1-4]**

| **Compound No.** | **Structure** | **NMR (Solvent: shift value ascending order)** | **MS [M+1]** | **LC/MS RT** |
|---|---|---|---|---|
| **I-10** | 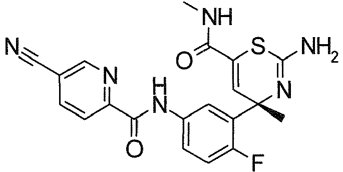 | 1H-NMR (DMSO-d6) δ 1.60 (3H, s), 2.65 (3H, d, J = 4.56 Hz), 6.42 (2H, s), 6 89 (1H, d, J = 4.06 Hz), 7 17 (1H, dd, J = 11 41, 8 87 Hz), 7.77-7 81 (1H, m), 7.95 (1H, dd, J = 7.35, 2.79 Hz), 8 26-8.30 (2H, m), 8.58 (1H, dd, J = 8 11, 2.03 Hz), 9 20 (1H, s). | **425** | **1.01** |
| **I-11** | 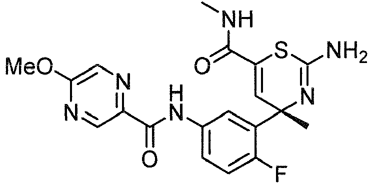 | 1H-NMR (DMSO-d6) δ 1 59 (3H, s), 2.65 (3H, d, J = 4.56 Hz), 4.02 (3H, s), 6.40 (2H, s), 6.89 (1H, d, J = 3.55 Hz), 7 14 (1H, dd, J = 11.15, 9.12 Hz), 7.73-7.77 (1H, m), 7.92 (1H, dd, J = 3.55, 1.77 Hz), 8 28 (1H, d, J = 4.56 Hz), 8.41 (1H, s), 8 88 (1H, s), 10.46 (1H,s) | **431** | **1.04** |
| **I-12** | 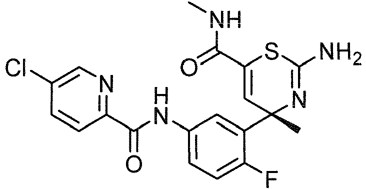 | 1H-NMR (DMSO-d6) δ 1 59 (3H, s), 2.65 (3H, d, J = 4.56 Hz), 6.41 (2H, s), 6 89 (1H, d, J = 4.06 Hz), 715 (1H, dd, J = 1141, 887 Hz), 775-779 (1H, m), 7 92 (1H, dd, J = 7 10, 2.53 Hz), 813-8.21 (2H, m), 828 (1H, d, J = 4.56 Hz), 8 78 (1H, d, J = 2 53 Hz), 10 63 (1H, s) | 434 | **1.14** |

**[Table 1-5]**

| **Compound No.** | **Structure** | **NMR (Solvent: shift value ascending order)** | **MS [M+1]** | **LC/MS RT** |
|---|---|---|---|---|
| **I-13** | 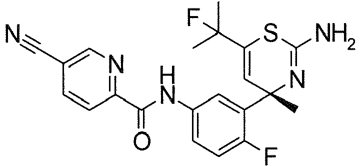 | 1H-NMR (DMSO-d6) δ 1 52 (6H, dd, J = 21 80, 4 56 Hz), 1 58 (3H, s), 6 20 (1H, dd, J = 5.07, 2.03 Hz), 6 45 (2H, s), 7.15 (1H, dd, J = 11.41, 8.87 Hz), 7.71-7.75 (1H, m), 7 95 (1H, dd, J = 7 10, 3 04 Hz), 8.27 (1H, d, J = 8.11 Hz), 8.58 (1H, dd, J = 8.62, 2 03 Hz), 9 19 (1H, d, J = 1 01 Hz), 10 77 (1H, s). | **428** | **1.3** |
| **I-14** | 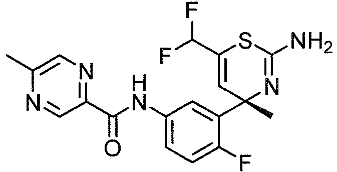 | 1H-NMR (DMSO-d6) δ : 1.60 (3H, s), 2.63 (3H, s), 6.64-6.82 (4H, m), 7.17 (1H, dd, J = 11 15, 8.62 Hz), 7.74-7.77 (1H, m), 7.96 (1H, dd, J = 3 55, 1 77 Hz), 8.69 (1H, s), 9.14 (1H, s), 10.67 (1H, s). | **408** | **1.13** |
| **1-15** | 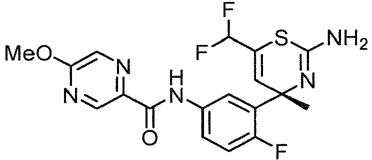 | 1H-NMR (DMSO-d6) δ : 1.60 (3H, s), 4 02 (3H, s), 664-682 (4H, m), 7.16 (1H, dd, J = 11 15, 862 Hz), 772-7.76 (1H, m), 7 94 (1H, dd, J = 7.35, 2.79 Hz), 8 41 (1H, s), 8 88 (1H, s), 10 48 (1H, s). | **424** | **1.25** |
| **I-16** | 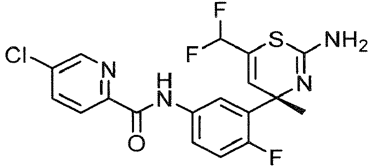 | 1H-NMR (DMSO-d6) δ 1 60 (3H, s), 6 59-6 86 (4H, m), 7.16 (1H, dd, J = 11 41 8 87 Hz), 7 75-7 79 (1H, m), 794 (1H, dd, J = 7.35, 2 79 Hz), 8 15-8 19 (2H, m), 8 78 (1H, d, J = 1 52 Hz), 10 65 (1H, s) | **427** | **1.41** |

**[Table 1-6]**

| **Compound No.** | **Structure** | **NMR (Solvent: shift value ascending order)** | **MS [M+1]** | **LC/MS RT** |
|---|---|---|---|---|
| **I-17** | 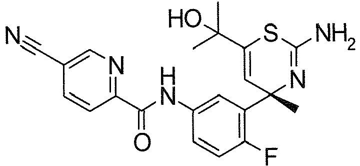 | 1H-NMR (CDCl3) δ 1 47 (6H, d, J = 14 19 Hz), 1.74 (3H, s), 1.94 (1H, br s), 4.72 (2H, br s), 6.22 (1H, d, J = 4 56 Hz), 7.06 (1H, dd, J = 12 17, 8.62 Hz), 7.72-7.76 (2H, m), 8.19 (1H, dd, J = 8.36, 1.77 Hz), 8 42 (1H, d, J = 8.11 Hz), 8 88 (1H, d, J = 2.03 Hz), 9.81 (1H,s). | **426** | **1.11** |
| 1-18 | 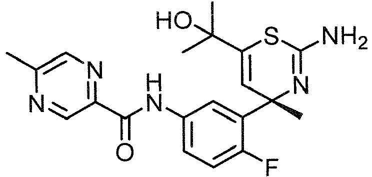 | 1H-NMR (DMSO-d6) δ 1 24 (1H, s), 1 28 (6H, d, J = 10 65 Hz), 1.54 (3H, s), 2.63 (3H, s), 508 (1H, s), 6 07 (1H, d, J = 4.56 Hz), 6.25 (2H, s), 7.12 (1H, dd, J = 11 41, 8.87 Hz), 7.68-7 72 (1H, m), 7.90 (1H, dd, J = 7 60, 2.53 Hz), 8.69 (1H, s), 9.14 (1H, s), 10.56 (1H, s). | **416** | **1.03** |
| **I-19** | 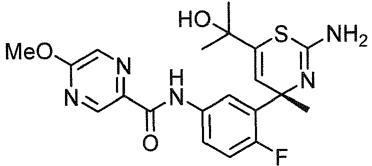 | 1H-NMR (DMSO-d6) δ 1 24 (1H, s), 1 27 (6H, d, J = 11 15 Hz), 1 54 (3H, s), 4 02 (3H, s), 5.08 (1H, s), 607 (1H, d, J = 4.56 Hz), 6 25 (2H, s), 7.11 (1H, dd, J = 11 66, 8 62 Hz), 7 67-7.71 (1H, m), 7 87 (1H, dd, J = 7.35, 2 79 Hz), 8.41 (1H, s), 8 87 (1H, s), 10 37 (1H, s) | **432** | **1.15** |

**[Table 1-7]**

| **Compound No.** | **Structure** | **NMR (Solvent: shift value ascending order)** | **MS [M+1]** | **LC/MS RT** |
|---|---|---|---|---|
| **I-20** | 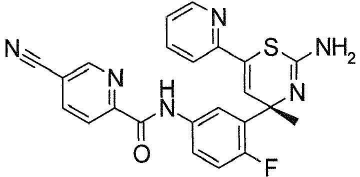 | 1H-NMR (DMSO-d6) 6 1 69 (3H, s), 6.43 (2H, s), 7.08 (1H, d, J = 4.56 Hz), 7.15(1H,dd,J=1141, 8.87 Hz), 7 34 (1H, dd, J = 7.10, 4 56 Hz), 7.75-7.84 (3H, m), 7.98 (1H, dd, J = 7 10, 2.53 Hz), 8.28 (1H, d, J = 8 11 Hz), 8 54 (1H, d, J = 4 06 Hz), 8 58 (1H, dd, J = 8.11, 2 03 Hz), 9 20 (1H, d, J = 1.01 Hz), 10.81 (1H, s) | **445** | **1.29** |
| **I-21** | 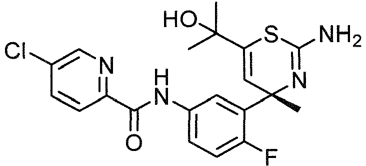 | 1H-NMR (DMSO-d6) δ 1.24 (1H, s), 1.28 (6H, d, J = 10 65 Hz), 1.54 (3H, s), 5 08 (1H, s), 6 07 (1H, d, J = 4.56 Hz), 6 26 (2H, s), 7.12 (1H, dd, J = 11.41, 887 Hz), 7.69-7.73 (1H, m), 7 88 (1H, dd, J = 7.35, 2.79 Hz), 8.13-820 (2H, m), 8.78 (1H, d, J = 203 Hz), 10 54 (1H,s). | **435** | **1.28** |
| **I-22** | 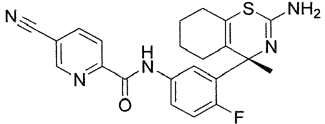 | | | |
| **I-23** | 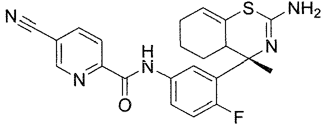 | | | |
| **I-24** | 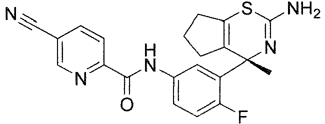 | | | |

**[Table 1-8]**

| **Compound No.** | **Structure** | **NMR (Solvent: shift value ascending order)** | **MS [M+1]** | **LC/MS RT** |
|---|---|---|---|---|
| **I-25** | 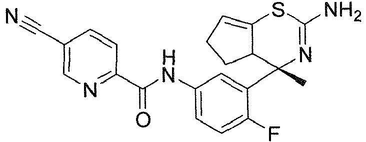 | | | |
| 1-26 | 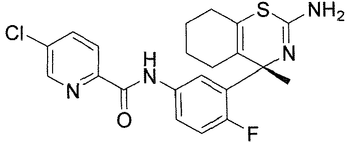 | | | |
| **I-27** | 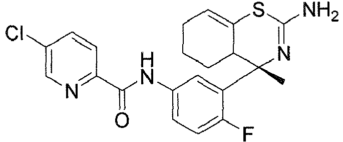 | | | |
| **I-28** | 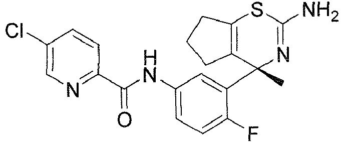 | | | |
| **I-29** | 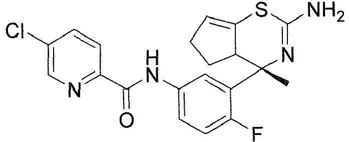 | | | |
| **I-30** | 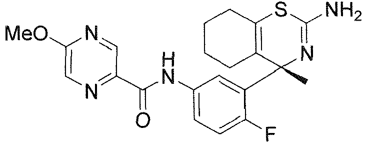 | | | |
| **I-31** | 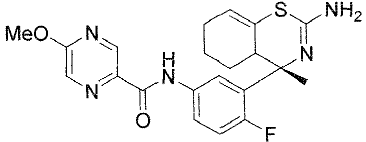 | | | |

**[Table 1-9]**

| **Compound No.** | **Structure** | **NMR (Solvent: shift value ascending order)** | **MS [M+1]** | **LC/MS RT** |
|---|---|---|---|---|
| **I-32** | 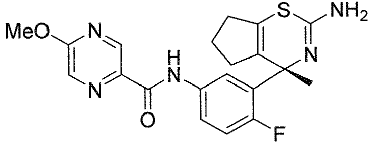 | | | |
| **I-33** | 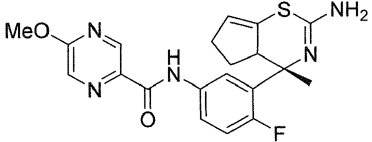 | | | |
| **I-34** | 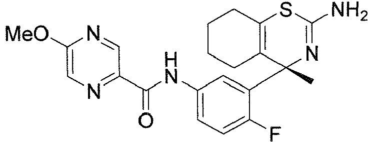 | | | |
| **I-35** | 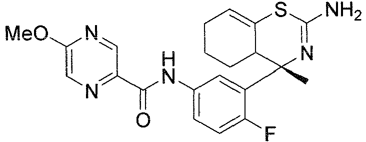 | | | |
| **I-36** | 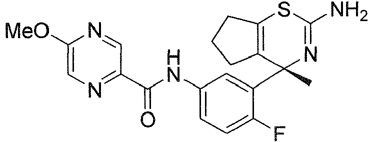 | | | |
| **I-37** | 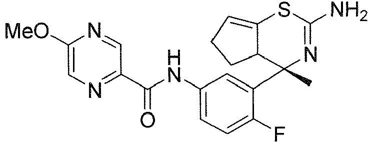 | | | |

**[Table 1-10]**

| **Compound No.** | **Structure** | **NMR (Solvent: shift value ascending order)** | **MS [M+1]** | **LC/MS RT** |
|---|---|---|---|---|
| 1-38 | 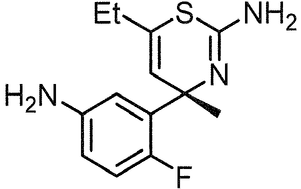 | 1H-NMR (CDCl3) δ 1 10(3H,t,J=7 35 Hz),1.65(3H,s),2 25(2H,q,J =7 44 Hz),3 50(2H,s),4.56(2H,br s),5.96(1H,d,J=5.07 Hz),6.46-6.49(1H,m),6.75(1H,dd,J=6 84, 2.79 Hz),6 80(1H,dd,J=11 66, 8.62 Hz). | | |
| **I-39** | 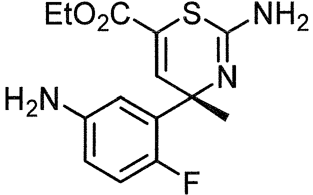 | 1H-NMR (CDCl3) δ 1.31(3H,q,J=8.45 Hz),1.72(3H,s),3.53(2H,s),4 28(2H,ddd,J=18 12, 6.97, 4.18 Hz),4.63(2H,br s),6.49-6.53(1H,m),6 74(1H,dd,J=6 59, 2 53 Hz),6 83(1H,dd,J=11.15, 8 62 Hz),7 32(1H,d,J=4.56 Hz) | | |
| **I-40** | 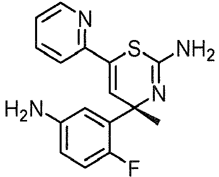 | | **315** | **0.86** |
| **1-41** | 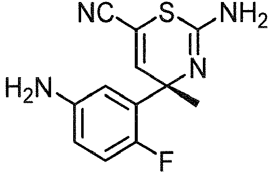 | | **263** | **0.66** |

**[Table 1-11]**

| **Compound No.** | **Structure** | **NMR (Solvent: shift value ascending order)** | **MS [M+1]** | **LC/MS RT** |
|---|---|---|---|---|
| **1-42** | 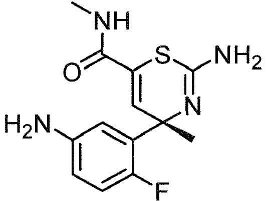 | 1H-NMR (DMSO-d6) δ:1.60(3H,s),2.65(3H,d,J=4. 56 Hz),4.96(2H,br s),6.44(1H,s),6.60(1H,dd,J= 6.84,2.79 Hz),6 80(1H,t,J=10.14 Hz),6 90(1H,s),8.35(1 H,br s). | | |
| **I-43** | 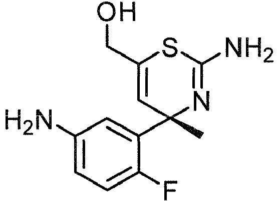 | 1H-NMR (CDCl3) δ:1.67(3H,s),3.49(1H,s),4.2 4(2H,s),6.24(1H,d,J=5.07 Hz),6.48-6.50(1H,m),6 74(1H,dd,J=6 59, 3.04 Hz),6.81(1H,dd,J=11.15, 8.62 Hz). | | |
| **I-44** | 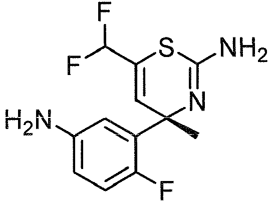 | 1H-NMR (CDCl3) δ 1.69(3H,s),3.55(2H,br s),6.17(1H,t,J=55 51 Hz),6 51-6.52(1H,m),6 58-6 59(1H,m),6 74-6.77(1H,m),6 80-6 85(1H,m). | | |
| **I-45** | 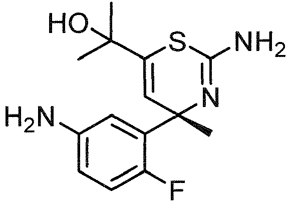 | 1H-NMR (CDCl3) δ 1.44(6H,d,J=4 56 Hz),1 67(3H,s),3.52(2H,br s),6.22(1H,d,J=5.58 Hz),6.46-6.50(1H,m),6 74(1H,dd,J=6 59, 2.53 Hz),6.80(1H,dd,J=11 15, 8 62 Hz). | | |
| **I-46** | 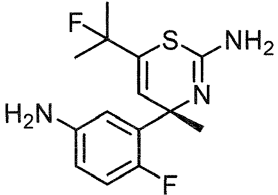 | | **298** | **0.89** |

**[Table 1-12]**

| **Compound No.** | **Structure** | **NMR (Solvent: shift value ascending order)** | **MS [M+1]** | **LC/MS RT** |
|---|---|---|---|---|
| **I-47** | 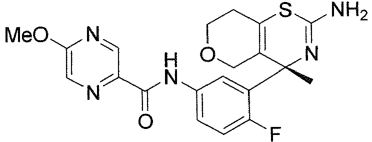 | | | |
| **I-48** | 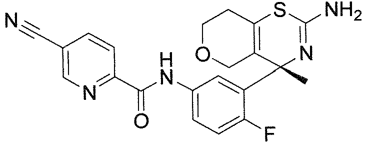 | | | |
| **I-49** | 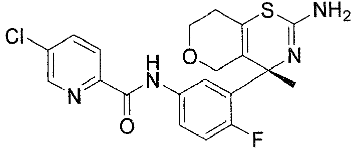 | | | |
| **I-50** | 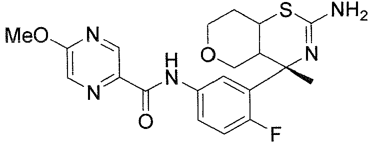 | | | |
| **I-51** | 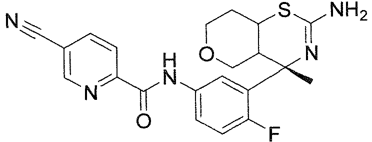 | | | |
| **I-52** | 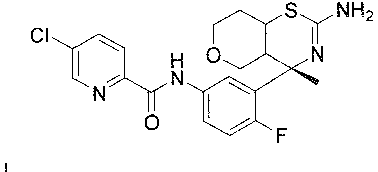 | | | |

**[Table 1-13]**

| **Compound No.** | **Structure** | **NMR (Solvent: shift value ascending order)** | **MS [M+1]** | **LC/MS RT** |
|---|---|---|---|---|
| **I-53** | 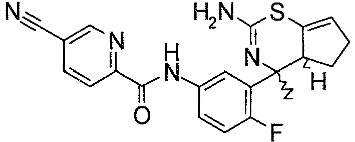 | 1H-NMR (CDCI3+CD3OD)) δ: 1.85 (3H, d, J = 4 5 Hz), 1.99-2 06(2H, m), 2.74(1H, m), 5.60(1H, m), 7.03(1H, dd, J = 8 7, 12 0 Hz), 7.38(1H, dd, J = 2.7, 6 9 Hz), 7.72-7.77(1H, m), 8 21(1H, dd, J = 2.1, 8 1 Hz), 8.41(1H, d, J = 8.1 Hz), 8.91 (1H, d, J = 1.2 Hz). | **408** | **1.19** |
| I-54 | 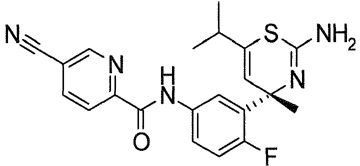 | 1H-NMR (CDCl3) δ: 1.13 (6H, d, J = 6 9 Hz), 1.73 (3H, s), 2.45-2.58 (1H, m), 4.77 (1H, s), 6.02 (1H, dd, J = 5 2, 0.9 Hz), 7.05 (1H, dd, J = 11 1, 8.7 Hz), 7.61 (1H, dd, J = 6.9, 2.7 Hz), 7.82-7 88 (1H, m), 8.18 (1H, dd, J = 8.1, 2.0 Hz), 8.41 (1H, dd, J = 8.1, 0.9 Hz), 8.87 (1H, dd, J = 2.0, 0.9 Hz), 9.80 (1H, s) | **125** | **1.32** |
| **I-55** | 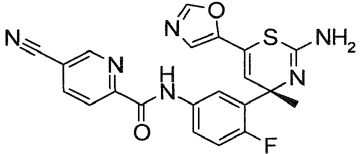 | 1H-NMR (DMSO-d6) δ 10 80 (1H, s), 9 19 (1H, dd, J = 2.03, 1 01 Hz), 8.57 (1H, dd, J = 8 62, 2 03 Hz), 8 42 (1H, s), 8.27 (1H, d, J = 8 11 Hz), 7.96 (1H, dd, J = 7.35, 2 79 Hz), 7.79-7.76 (1H, m), 7 26 (1H, s), 7 17 (1H, dd, J = 11 41, 8 87 Hz), 6 67 (1H, d, J = 4.56 Hz), 6 62 (2H, s), 1 67 (3H, s) | **435** | **1.11** |
| **I-56** | 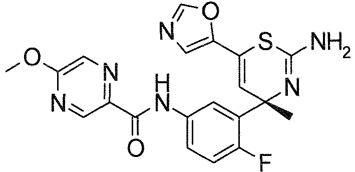 | 1H-NMR (DMSO-d6) δ 10 47 (1H, s), 8 88 (1H, d, J = 1 01 Hz), 8 42 (1H, s), 8 41 (1H, d, J = 1 01 Hz), 7.94 (1H, dd, J = 7.60, 2 53 Hz), 7 75-7 72 (1H, m), 7 26 (1H, s), 7 14 (1H, dd, J = 11 41, 8 87 Hz), 6 67 (1H, d J = 5.07 Hz), 6 61 (2H, s), 4 02 (3H, s), 1 66 (3H, s) | **441** | **1.15** |

**[Table 1-14]**

| **Compound No.** | **Structure** | **NMR (Solvent: shift value ascending order)** | **MS [M+1]** | **LC/MS RT** |
|---|---|---|---|---|
| **I-57** | 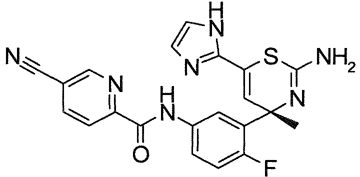 | 1H-NMR (DMSO-d6) δ: 12.47 (1H, s), 10.79 (1H, s), 9.20-9.19 (1H, m), 8.57 (1H, dd, J = 8 11, 2 03 Hz), 8.27 (1H, d, J = 8 11 Hz), 7.96 (1H, dd, J = 7.10, 2 53 Hz), 7.79 (1H, td, J = 6.21, 3.21 Hz), 7 19-7.14 (2H, m), 6 91 (1H, s), 6.78 (1H, d, J = 3 55 Hz), 6 37 (2H, s), 1 65 (3H, s). | **434** | **1.02** |
| **I-58** | 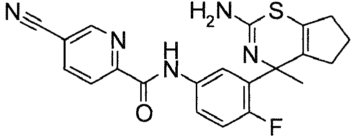 | 1H-NMR (DMSO-d6) d. 1 59 (3H, s), 1.84(2H, m), 2.12(1H, m), 2.34(1H, m), 6.09(2H, bs), 7.07(1H, dd, J = 9.0, 11.4 Hz), 7.79-7 82(1H, m), 7 86-7.89(1H, m), 8.27(1H, d, J = 8.1 Hz), 8 57(1H, dd, J = 1 8, 8 1 Hz), 9.19(1H, s), 10.84(1H, bs) | **408** | **1.27** |
| **I-59** | 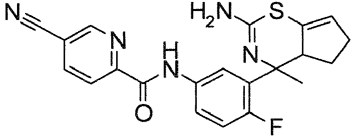 | 1H-NMR (DMSO-d6) d 1 30 (3H, s), 1.45-1.60(1H, m), 1 63-1 76(1H, m), 2 31 (2H, m), 2.82(1H, m), 5 71 (1H, m), 6 00(2H, bs), 7 10(1H, dd, J = 2 7, 11 7 Hz), 7 85-7 90(1H, m), 7 98(1H, dd, J = 2 4, 7 2 Hz), 8.27(1H, d, J = 8 1 Hz), 8 55(1H, dd, J = 2 1, 8 1 Hz), 9 19(1H, m), 10 84(1H, bs) | **408** | **1.22** |

**[Table 1-15]**

| **Compound No.** | **Structure** | **NMR (Solvent: shift value ascending order)** | **MS [M+1]** | **LC/MS RT** |
|---|---|---|---|---|
| **I-60** | 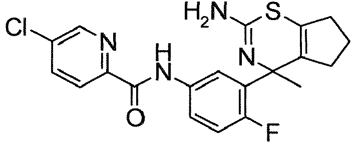 | 1H-NMR (DMSO-d6) δ 1 59 (3H, s), 1.83(2H, m), 2.13(1H, m), 2 34(1H, m), 6 09(2H, bs), 7.06(1H, dd, J = 8.7, 11.4 Hz), 7.77-7 82(1H, m), 7 86(1H, dd, J = 2.7, 7 2 Hz), 8 12-8 21(2H, m), 8 77(1H, m), 10.68(1H, bs). | **417** | **1.45** |
| **I-61** | 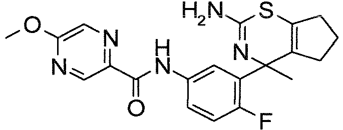 | 1H-NMR (DMSO-d6) δ 1 58 (3H, s), 1.83(2H, m), 2.12(1H, m), 2.34(1H, m), 4 02(3H, s), 6.08(2H, bs), 7.05(1H, dd, J = 2.7, 11 4 Hz), 7.74-7.79(1H, m), 7.85(1H, dd, J = 2 4, 7.5 Hz), 8 40(1H, d, J = 0 9 Hz), 10.51 (1H, bs). | **414** | **1.3** |
| **I-62** | 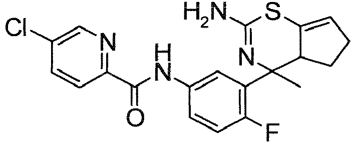 | 1H-NMR (DMSO-d6) δ: 1 29 (3H, s), 1.55(1H. m), 1 68(1H, m), 2 31(2H, m), 2.82(1H, m), 5.71(1H, m), 5.95(2H, bs), 7 08(1H, dd, J = 9.0, 12 3 Hz), 7 83-7 88(1H, m), 7 96(1H, dd, J = 2.4, 7 5 Hz), 8 16-8 21(2H, m), 8.79(1H, d, J = 2 1 Hz), 10.68(1H, bs). | **417** | **1.45** |
| **I-63** | 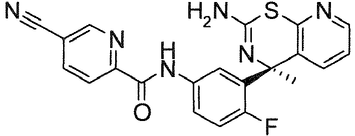 | 1H-NMR (CDCl3) δ 2 01 (s, 3H), 4 97 (br, 2H), 6 99 (dd, J = 10.9, 8 7 Hz, 1H), 7 18 (dd, J = 7 7, 4 5 Hz, 1H), 7 56 (ddd, J = 7 7, 2 0, 1.7 Hz, 1H), 7 71-7 82 (m, 2H), 8 20 (dd, J = 8.6, 1 7 Hz, 1H), 8 37-8 44 (m, 2H), 8 87 (d, J = 2 0 Hz, 1H), 9 82 (brs, 1H) | **419** | **1.07** |

**[Table 1-16]**

| **Compound No.** | **Structure** | **NMR (Solvent: shift value ascending order)** | **MS [M+1]** | **LC/MS RT** |
|---|---|---|---|---|
| **I-64** | 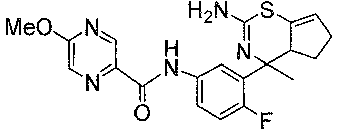 | 1H-NMR(d6-DMSO) δ 1.29(3H, s), 1.54(1H, m), 1.68(1H, m), 2 31 (2H, m), 2.83(1H, m), 4 02(3H, s), 5 71(1H, m), 5 99(2H, bs), 7.08(1H, dd, J= 8.7, 12 0Hz), 7 80-7.85(1H, m), 7 96(1H, dd, J= 2.4, 7 5Hz), 8.40(1H, m), 8 88(1H, m), 10.51(1H, bs). | **414** | **1.30** |
| **I-65** | 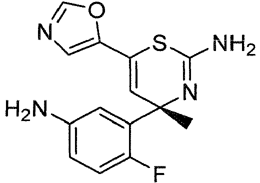 | | **305** | **0.68** |
| **I-66** | 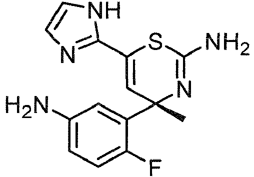 | | **304** | **0.5** |

The effect of the present compound is confirmed by the following test Examples.

### (Test Example 1: Assay of BACE1 inhibiting activity)

48.5 µL of substrate peptide solution (Biotin-XSEVNLDAEFRHDSGC-Eu: X=ε-amino-n-capronic acid, Eu=Europium cryptate) was added to each well of 96-hole half-area plate (a black plate: Corning Incorporated), and after addition of 0.5 µl of the test compound (dissolved in N,N'-dimethylformaldehyde) and 1 µl of Recombinant human BACE1(R&D Systems), the reaction mixture was incubated at 30°C for 3 hours. The substrate peptide was synthesized by reacting Cryptate TBPCOOH mono SMP (CIS bio international) with Biotin-XSEVNLDAEFRHDSGC (Peptide Institute, Inc.). The final concentrations of the substrate peptide and Recombinant human BACE1 were adjusted to 18 nM and 7.4 nM respectively, and the reaction was performed in sodium acetate buffer (50 mM sodium acetate, pH 5.0, 0.008% Triton X-100).
After the incubation for reaction, 50 µl of 8.0 µg/ml Streptavidin-XL665(CIS bio international) dissolved in phosphate buffer (150 mM K₂ HPO₄ -KH₂ PO₄, pH 7.0, 0.008 % Triton X-100, 0.8 M KF) was added to each well and left stand at 30°C for an hour. After then, fluorescence intensity was measured (excitation wavelength: 320 nm, measuring wavelength: 620 nm and 665 nm) using Wallac 1420 multilabel counter (Perkin Elmer life sciences). Enzymatic activity was determined from counting ratio of each wavelength (10,000 x Count 665/Count 620) and 50% inhibitory concentration against the enzymatic activity was calculated. IC₅₀ values of the compound I-8 is 0.012 µmol/L. IC₅₀ values of the compound I-1 to I-7, I-9 to I-21, I-53 to I-58, I-60, I-61, and I-63 are less than 1 µmol/L, respectively.

### (Test Example 2 : Measurement of β-amyloid (Aβ) production inhibitory effect in cell)

Neuroblastoma SH-SY5Y cells (SH/APPwt) with human wild-type β-APP excessively expressed therein were prepared at 8×10⁵ cells/mL, and 150 µl portions thereof were inoculated into respective wells of a 96-well culture plate (Falcon). The cells were cultured for 2 hours at 37°C in a 5% gaseous carbon dioxide incubator. Then, a solution which had been preliminarily prepared by adding and suspending the test compound (DMSO (dimethyl sulfoxide) solution) so as to be 2 µl/50 µl medium was added to the cell sap. Namely, the final DMSO concentration was 1%, and the amount of the cell culture was 200 µl. After the incubation was performed for 24 hours from the addition of the test compound, 100 µl of the culture supernatant was collected from each fraction. The amount of the Aβ in each fraction was measured.

The Aβ amount was measured as follows. 10 µl of a homogeneous time resolved fluorescence (HTRF) measurement reagent (Amyloid-β 1-40 peptide; IBA Molecular Holding, S.A.) and 10 µl of the culture supernatant were put into a 384-well half area microplate (black microplate, Costar) and mixed with each other, and then left standing overnight at 4°C while the light was shielded. Then, the fluorescence intensity (excitation wavelength: 337 nm, measurement wavelength: 620 nm and 665 nm) was measured with a Wallac 1420 multilabel counter (Perkin Elmer life sciences). The Aβ amount was determined from the count rate at each measurement wavelength (10000 × Count 665/Count 620), and the amount needed to inhibit Aβ production by 50 % (IC₅₀) was calculated from at least six different dosages. IC₅₀ values of the compound I-8 is 0.002 µmol/L. IC₅₀ values of the compound I-1to 7, 9 to 21, and 53 -63 are less than 5 µmol/L, respectively.

### (Test Example 3 : Lowering effect on brain β amyloid in rats)

A test compound is suspended in 0.5% methylcellulose, the final concentration is adjusted to 2 mg/mL, and this is orally administered to male Crj:SD rat (7 to 9 weeks old) at 10 mg/kg. In a vehicle control group, only 0.5% methylcellulose is administered, and an administration test is performed at 3 to 8 animals per group. A brain is isolated 3 hours after administration, a cerebral hemisphere is isolated, a weight thereof is measured, the hemisphere is rapidly frozen in liquid nitrogen, and stored at -80°C until extraction date. The frozen cerebral hemisphere is transferred to a homogenizer manufactured by Teflon (registered trade name) under ice cooling, a 5-fold volume of a weight of an extraction buffer (containing 1% CHAPS ({3-[(3-chloroamidopropyl)dimethylammoniol-1-propanesulfonate}), 20 mmol/L Tris-HCl (pH 8.0), 150 mmol/L NaCl, Complete (Roche) protease inhibitor) is added, up and down movement is repeated, and this is homogenized to solubilize for 2 minutes. The suspension is transferred to a centrifugation tube, allowed to stand on an ice for 3 hours or more and, thereafter centrifuged at 100,000 x g, 4°C for 20 minutes. After centrifugation, the supernatant is transferred to an ELISA plate (product No. 294-62501, Wako Junyaku Kogyo) for measuring β amyloid 1-40. ELISA measurement is performed according to the attached instruction. The lowering effect is calculated as a ratio compared to the brain β amyloid 1-40 level of vehicle control group of each test.

### (Test Example 4 : CYP3A4 fluorescent MBI test)

The CYP3A4 fluorescent MBI test is a test of investigating enhancement of CYP3A4 inhibition of a compound by a metabolism reaction, and the test was performed using, as CYP3A4 enzyme expressed in *Escherichia coli* and employing, as an index, a reaction in which 7-benzyloxytrifluoromethylchmarin (7-BFC) is debenzylated by the CYP3A4 enzyme to produce a metabolite, 7-hydroxytrifluoromethylchmarin (HFC) emitting fluorescent light.

The reaction conditions were as follows: substrate, 5.6 µmol/L 7-BFC; pre-reaction time, 0 or 30 minutes; reaction time, 15 minutes; reaction temperature, 25°C (room temperature); CYP3A4 content (expressed in *Escherichia coli*)*,* at pre-reaction 62.5 pmol/mL, at reaction 6.25 pmol/mL (at 10-fold dilution); test drug concentration, 0.625, 1.25, 2.5, 5, 10, 20 µmol/L (six points).

An enzyme in a K-Pi buffer (pH 7.4) and a test drug solution as a pre-reaction solution were added to a 96-well plate at the composition of the pre-reaction, a part of it was transferred to another 96-well plate so that it was 1/10 diluted by a substrate in a K-Pi buffer, NADPH as a co-factor was added to initiate a reaction as an index (without preincubation) and, after a predetermined time of a reaction, acetonitrile/0.5 mol/L Tris (trishydroxyaminomethane) = 4/1 was added to stop the reaction. In addition, NADPH was added to a remaining preincubation solution to initiate a preincubation (with preincubation) and, after a predetermined time of a preincubation, a part was transferred to another plate so that it was 1/10 diluted with a substrate and a K-Pi buffer to initiate a reaction as an index. After a predetermined time of a reaction, acetonitrile/0.5 mol/L Tris (trishydroxyaminomethane) = 4/1 was added to stop the reaction. For the plate on which each index reaction had been performed, a fluorescent value of 7-HFC which is a metabolite was measured with a fluorescent plate reader. (Ex = 420 nm, Em = 535 nm).

Addition of only DMSO which is a solvent dissolving a drug to a reaction system was adopted as a control (100%), remaining activity (%) was calculated at each concentration of a test drug added as the solution, and IC₅₀ was calculated by reverse-presumption by a logistic model using a concentration and an inhibition rate. When a difference between IC₅₀ values is 5 µM or more, this was defined as (+) and, when the difference is 3 µM or less, this was defined as (-).

### (Result)

### Compound I-20: (-)

### (Test Example 5 : CYP inhibition test)

Using commercially available pooled human hepatic microsome, and employing, as markers, 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenedine hydroxylation (CYP3A4) as typical substrate metabolism reactions of human main five CYP enzyme forms (CYP1A2, 2C9, 2C19, 2D6, 3A4), an inhibitory degree of each metabolite production amount by a test compound was assessed.

The reaction conditions were as follows: substrate, 0.5 µmol/L ethoxyresorufin (CYP1A2), 100 µmol/L tolbutamide (CYP2C9), 50 µmol/L S-mephenitoin (CYP2C19), 5 µmol/L dextromethorphan (CYP2D6), 1 µmol/L terfenedine (CYP3A4); reaction time, 15 minutes; reaction temperature, 37°C; enzyme, pooled human hepatic microsome 0.2 mg protein/mL; test drug concentration, 1, 5, 10, 20 µmol/L (four points).

Each five kinds of substrates, human hepatic microsome, or a test drug in 50 mM Hepes buffer as a reaction solution was added to a 96-well plate at the composition as described above, NADPH, as a cofactor was added to initiate metabolism reactions as markers and, after the incubation at 37°C for 15 minutes, a methanol/acetonitrile = 1/1 (v/v) solution was added to stop the reaction. After the centrifugation at 3000 rpm for 15 minutes, resorufin (CYP1A2 metabolite) in the supernatant was quantified by a fluorescent multilabel counter and tributamide hydroxide (CYP2CP metabolite), mephenytoin 4' hydroxide (CYP2C19 metabolite), dextromethorphan (CYP2D6 metabolite), and terfenadine alcohol (CYP3A4 metabolite) were quantified by LC/MS/MS.

Addition of only DMSO being a solvent dissolving a drug to a reaction system was adopted as a control (100%), remaining activity (%) was calculated at each concentration of a test drug added as the solution and IC₅₀ was calculated by reverse presumption by a logistic model using a concentration and an inhibition rate.

### (Result)

### Compound I-2: five kinds > 21 µM

### (Test Example 6 : FAT Test)

Each 20 µL of freeze-stored *Salmonella typhimurium* (TA98 and TA100 strain) is inoculated in 10 mL of liquid nutrient medium (2.5% Oxoid nutrient broth No.2), and the cultures are incubated at 37°C under shaking for 10 hours. 9 mL of TA98 culture is centrifuged (2000 × g, 10 minutes) to remove medium, and the bacteria is suspended in 9 mL of Micro F buffer (K₂HPO₄: 3.5 g/L, KH₂PO₄: 1 g/L, (NH₄)₂SO₄: 1 g/L, trisodium citrate dihydrate: 0.25 g/L, MgSO₄ • 7H₂O: 0.1 g/L),and the suspension is added to 110 mL of Exposure medium (Micro F buffer containing Biotin: 8 µg/mL, histidine: 0.2 µg/mL, glucose: 8 mg/mL). 3.16 mL of TA100 culture is added to 120 mL of Exposure medium to prepare the test bacterial solution. 588 µL of the test bacterial solution (or mixed solution of 498 µl of the test bacterial solution and 90 µL of the S9 mix in the case with metabolic activation system) are mixed with each 12 µL of the following solution: DMSO solution of the test substance (eight dose levels from maximum dose 50 mg/mL at 2-fold ratio); DMSO as negative control; 50 µg/mL of 4-nitroquinoline-1-oxide DMSO solution as positive control for TA98 without metabolic activation system; 0.25 µg/mL of 2-(2-furyl)-3-(5-nitro-2-furyl)acrylamide DMSO solution as positive control for TA100 without metabolic activation system; 40 µg/mL of 2-aminoanthracene DMSO solution as positive control for TA98 with metabolic activation system; or 20 µg/mL of 2-aminoanthracene DMSO solution as positive control for TA100 with metabolic activation system. 12 µL of the solution and 588 µL of the test bacterial solution (a mixed solution of 498 µl of the test bacterial solution and 90 µL of S9 mix with metabolic activation condition) were mixed and incubated at 37°C under shaking for 90 minutes. 460 µL of the bacterial solution exposed to the test substance is mixed with 2300 µL of Indicator medium (Micro F buffer containing biotin: 8 µg/mL, histidine: 0.2 µg/mL, glucose: 8 mg/mL, Bromo Cresol Purple: 37.5 µg/mL), each 50 µL is dispensed into 48 wells per dose in the microwell plates, and is subjected to stationary cultivation at 37°C for 3 days. A well containing the bacteria, which has obtained the ability of proliferation by mutation in the gene coding amino acid (histidine) synthetase, turns the color from purple to yellow due to pH change. The number of the yellow wells among the 48 total wells per dose is counted, and evaluate the mutagenicity by comparing with the negative control group. (-) means that mutagenicity is negative and (+) means positive.

### (Test Example 7 : Solubility Test)

A 2-fold dilution series (12 points) of a 10 mM solution of a test compound in DMSO was added to a medium (JP-I, JP-II) (2%), and solubility was assessed by 3 stages (High; > 40 µM, Medium; 3-40 µM, Low; < 3 µM) from a turbidity after 4 hours (crystallization information).

The preparation method of solution was as follows: A 10 mM solution of the compound was prepared with DMSO, and 6 µL of the compound solution was added to 594 µL of an artificial intestinal juice (water and 118 mL of 0.2 mol/L NaOH reagent were added to 250 mL of 0.2 mol/L potassium dihydrogen phosphate reagent to reach 1000 mL) with a pH of 6.8. The mixture was left standing for 16 hours at 25°C, and the mixture was vacuum-filtered. The filtrate was two-fold diluted with methanol/water = 1/1.

### (Result)

### Compound I-6: High (JP-I)

### (Test Example 8 : Metabolism Stability Test)

Using a commercially available pooled human hepatic microsomes, a test compound was reacted for a constant time, a remaining rate was calculated by comparing a reacted sample and an unreacted sample, thereby, a degree of metabolism in liver was assessed.

A reaction was performed (oxidative reaction) at 37°C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human liver microsomes. After the reaction, 50 µL of the reaction solution was added to 100 µL of a methanol/acetonitrile = 1/1 (v/v)"mixed and centrifuged at 3000 rpm for 15 minutes. The test compound in the supernatant was quantified by LC/MS/MS, and a remaining amount of the test compound after the reaction was calculated, letting a compound amount at 0 minute reaction time to be 100%.

### (Result)

### Compound I-19: 96.4%

### (Test Example 9 : hERG Test)

For the purpose of assessing risk of an electrocardiogram QT interval prolongation, effects on delayed rectifier K+ current (I_{Kr}), which plays an important role in the ventricular repolarization process, was studied using HEK293 cells expressing human ether-a-go-go related gene (hERG) channel.

After a cell was retained at a membrane potential of -80 mV by whole cell patch clamp method using an automated patch clamp system (PatchXpress 7000A, Axon Instruments Inc.), I_{Kr} induced by depolarization pulse stimulation at +50 mV for 2 seconds and, further, repolarization pulse stimulation at -50 mV for 2 seconds was recorded. After the generated current was stabilized, extracellular solution (NaCl: 137 mmol/L, KCl: 4 mmol/L, CaCl₂. 2H₂O: 1.8 mmol/L, MgCl₂ • 6H₂O: 1 mmol/L, glucose: 10 mmol/L, HEPES (4-(2-hydroxyethyl)-1-piperazineethanesulfonic acid): 10 mmol/L, pH=7.4) in which the test compound had been dissolved at an objective concentration was applied to the cell under the room temperature condition for 10 minutes. From the recording I_{Kr}, an absolute value of the tail peak current was measured based on the current value at the resting membrane potential using an analysis software (DataXpress ver.1, Molecular Devices Corporation). Further, the % inhibition relative to the tail peak current before application of the test substance was calculated, and compared with the vehicle-applied group (0.1% dimethyl sulfoxide solution) to assess influence of the test substance on I_{Kr}.

### (Result)

### Compound I-14: 3.8%

### (Test Example 10 : Powder solubility test)

Appropriate amounts of the test substances are put into appropriate containers. To the respective containers are added 200 µL of JP-1 fluid (sodium chloride 2.0 g, hydrochloric acid 7.0 mL and water to reach 1000 mL), 200 µL of JP-2 fluid (phosphate buffer (pH 6.8) 500 mL and water 500 mL), and 200 µL of 20 mmol/L TCA (sodium taurocholate)/JP-2 fluid (TCA 1.08 g and water to reach 100 mL). In the case that the test compound is dissolved after the addition of the test fluid, the bulk powder is added as appropriate. The containers are sealed, and shaken for 1 hour at 37°C. The mixtures are filtered, and 100 µL of methanol is added to each of the filtrate (100 µL) so that the filtrates are two-fold diluted. The dilution ratio may be changed if necessary. The dilutions are observed for bubbles and precipitates, and then the containers are sealed and shaken. Quantification is performed by HPLC with an absolute calibration method.

### (Test Example 11 : BA test)

### Materials and methods for studies on oral absorption

(1) Animal: SD rats
(2) Breeding conditions: rats are allowed to freely take solid feed and sterilized tap water
(3) Dose and grouping: orally or intravenously administered at a predetermined dose; grouping is as follows (Dose depends on the compound)
   Oral administration: 1 to 30 mg/kg (n=2 to 3)
   Intravenous administration: 0.5 to 10 mg/kg (n=2 to 3)
(4) Preparation of dosing solution: for oral administration, in a solution or a suspension state; for intravenous administration, in a solubilized state
(5) Administration method: in oral administration, forcedly administer into ventriculus with oral probe; in intravenous administration, administer from caudal vein with a needle-equipped syringe
(6) Evaluation items: blood is collected over time, and the plasma concentration of drug is measured by LC/MS/MS
(7) Statistical analysis: regarding the transition of the plasma concentration, the area under the plasma concentration-time curve (AUC) is calculated by non-linear least squares program WinNonlin (Registered trade name), and the bioavailability (BA) is calculated from the AUCs of the oral administration group and intravenous administration group.

### (Test Example 12: Brain distribution studies)

Compound of the present invention was intravenously administered to a rat at 0.5 mg/mL/kg dosage. 30 minutes later, all blood was drawn from vena cava inferior under isoflurane anesthesia for death from exsanguination. The brain was enucleated and 20-25% of homogenate thereof was prepared with distilled water. The obtained blood was used as plasma after centrifuging. To the brain sample was added the control plasma at 1:1. To the plasma samples was added the control brains at 1:1. Each sample was measured using LC/MS/MS. The obtained area ratio (a brain/plasma) was used for the brain Kp value.

### (Result)

### Compound I-54: 2.5.

### Preparation Example 1

A granule containing the following ingredients is produced.

| | | |
|---|---|---|
| Ingredient | Compound of formula (I) | 10 mg |
| | Lactose | 700 mg |
| | Corn starch | 274 mg |
| | HPC-L | 16 mg |
| | | 1000 mg |

The compound of formula (I), and lactose are passed through a 60 mesh sieve. Corn starch is passed through a 120 mesh sieve. These are mixed with a V-type mixer. To the mixed powder is added a HPC-L (low viscosity hydroxypropylcellulose) aqueous solution, this is kneaded, granulated (extrusion granulation, pore diameter 0.5 to 1 mm), and dried. The resulting dry granule is passed through a vibration sieve (12/60 mesh) to obtain a granule.

### Preparation Example 2

A granule for filling a capsule containing the following ingredients is produced.

| | | |
|---|---|---|
| Ingredient | Compound of formula (I) | 15 mg |
| | Lactose | 90 mg |
| | Corn starch | 42 mg |
| | HPC-L | 3 mg |
| | | 150 mg |

The compound of formula (I), and lactose are passed through a 60 mesh sieve. Corn starch is passed through a 120 mesh sieve. These are mixed, a HPC-L solution is added to the mixed powder, this is kneaded, granulated, and dried. The resulting dry granule is adjusted in a size, and 150 mg of it is filled into a No.4 hard gelatin capsule.

### Preparation Example 3

A tablet containing the following ingredients is produced.

| | | |
|---|---|---|
| Ingredient | Compound of formula (I) | 10 mg |
| | Lactose | 90 mg |
| | Microcrystalline cellulose | 30 mg |
| | CMC-Na | 15 mg |
| | Magnesium stearate | 5 mg |
| | | 150 mg |

The compound of formula (I), lactose, microcrystalline cellulose, and CMC-Na (carboxymethylcellulose sodium salt) are passed through a 60 mesh sieve, and mixed. Magnesium stearate is mixed into the mixed powder to obtain a mixed powder for tabletting. The present mixed powder is directly compressed to obtain a 150 mg of a tablet.

### Preparation Example 4

The following ingredients are warmed, mixed, and sterilized to obtain an injectable.

| | | |
|---|---|---|
| Ingredient | Compound of formula (I) | 3 mg |
| | Nonionic surfactant | 15 mg |
| | Purified water for injection | 1 ml |

### [Industrial applicability]

The present compound can be a medicament useful as an agent for treating a disease induced by production, secretion and/or deposition of amyloid β protein.

## Claims

1. A compound of formula (I): 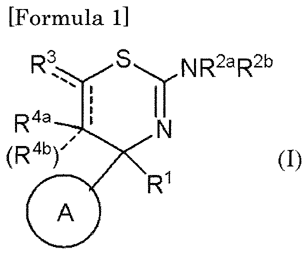 wherein
R¹ is substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group, R^{2a} and R^{2b} are each independently hydrogen, substituted or unsubstituted alkyl, substituted or unsubstituted acyl, substituted or unsubstituted alkoxycarbonyl or substituted or unsubstituted carbamoyl, 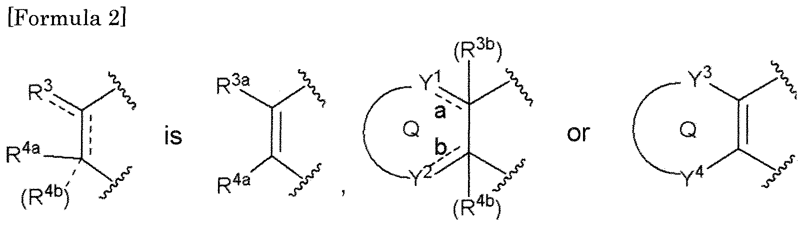 wherein
R^{3a} is halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl, R^{4a} is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
ring Q is a substituted or unsubstituted carbocycle or a substituted or unsubstituted heterocycle,
R^{3b} and R^{4b} are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, carboxy, substituted or unsubstituted alkoxycarbonyl, substituted or unsubstituted alkenyloxycarbonyl, substituted or unsubstituted alkynyloxycarbonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heteroyclylsulfinyl or substituted or unsubstituted heterocyclylsulfonyl,
dashed line a and dashed line b each independently mean the presence or absence of a bond,
when dashed line a means the presence of a bond, then R^{3b} is absent,
when dashed line b means the presence of a bond, then R^{4b} is absent,
Y¹ and Y² are each independently -C(R⁵)(R⁶)-, -C(R⁵)=, -N(R⁷)-, -N=, -S-, -SO-, -SO₂ -or -O-,
Y³ and Y⁴ are each independently -C(R⁵)(R⁶)-, -N(R⁷)-, -S-, -SO-, -SO₂ -or -O-,
R⁵ and R⁶ are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl,
R⁷ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, substituted or unsubstituted carbamoyl, substituted or unsubstituted thiocarbamoyl, substituted or unsubstituted sulfamoyl, substituted or unsubstituted amino, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl, and
ring A is a substituted or unsubstituted carbocycle or a substituted or unsubstituted heterocycle,
excluding the following compounds: 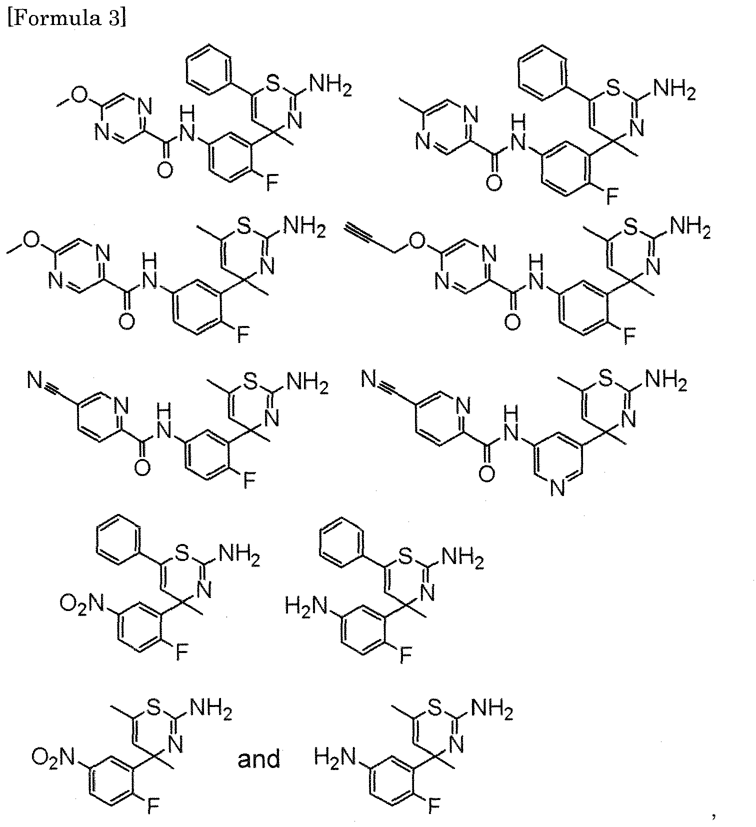 its pharmaceutically acceptable salt or a solvate thereof.

2. The compound according to claim 1 wherein ring A is 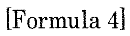 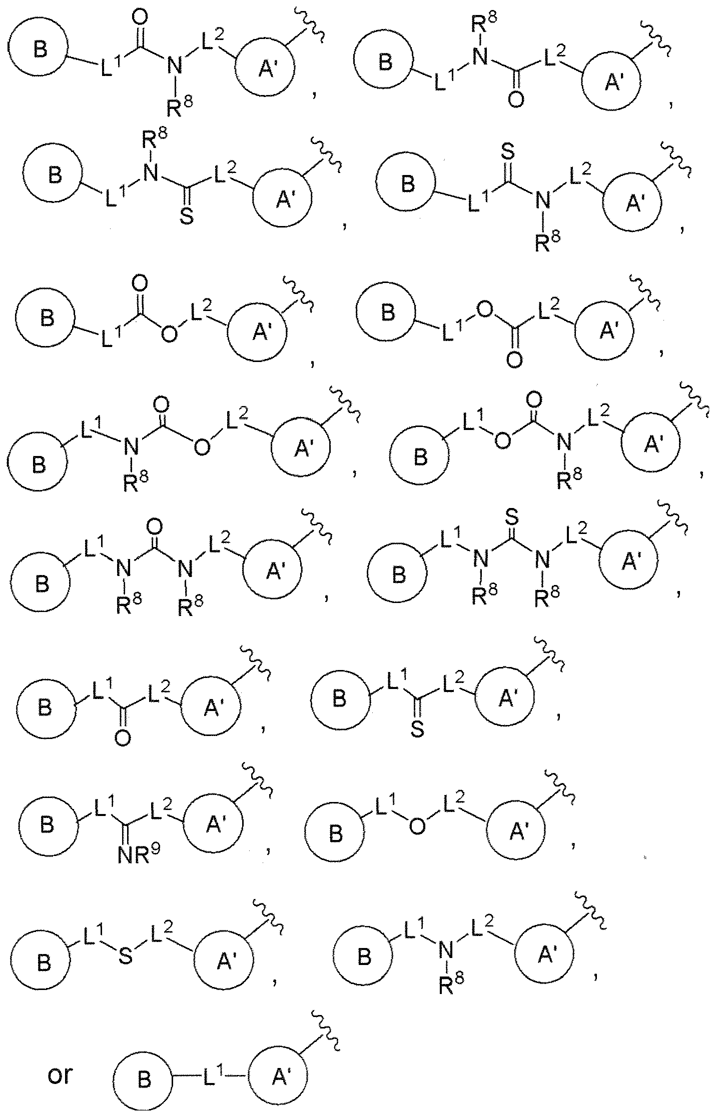 wherein ring A' and ring B are each independently a substituted or unsubstituted carbocycle or a substituted or unsubstituted heterocycle,
L¹ and L² are each independently a bond, substituted or unsubstituted alkylene, substituted or unsubstituted alkenylene or substituted or unsubstituted alkynylene,
R⁸ is hydrogen, substituted or unsubstituted alkyl, substituted or unsubstituted alkenyl, substituted or unsubstituted alkynyl or substituted or unsubstituted acyl,
R⁹ is hydrogen, hydroxy, substituted or unsubstituted alkyl, substituted or unsubstituted alkenyl, substituted or unsubstituted alkynyl or substituted or unsubstituted acyl,
its pharmaceutically acceptable salt or a solvate thereof.

3. The compound according to claim 2 wherein ring A is 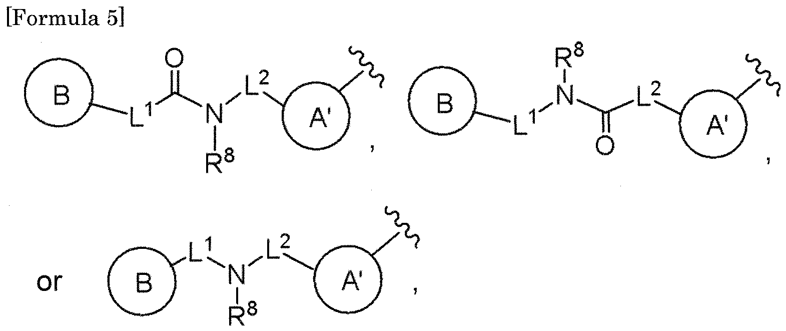 its pharmaceutically acceptable salt or a solvate thereof.

4. The compound according to any one of claims 1 to 3 wherein R^{3a} is substituted or unsubstituted alkyl, cyano, substituted or unsubstituted alkoxycarbonyl, substituted or unsubstituted carbamoyl or a substituted or unsubstituted heterocyclic group, its pharmaceutically acceptable salt or a solvate thereof.

5. The compound according to any one of claims 1 to 4 wherein R^{4a} is hydrogen, its pharmaceutically acceptable salt or a solvate thereof.

6. The compound according to any one of claims 1 to 5 wherein 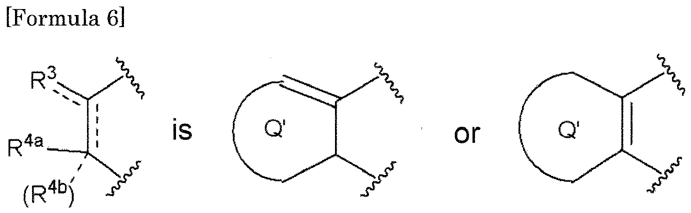 wherein ring Q' is a substituted or unsubstituted carbocycle,
its pharmaceutically acceptable salt or a solvate thereof.

7. The compound according to any one of claims 2 to 6 wherein ring A' is substituted or unsubstituted benzene, ring B is substituted or unsubstituted pyridine, substituted or unsubstituted pyrimidine, or substituted or unsubstituted pyrazine, its pharmaceutically acceptable salt or a solvate thereof.

8. The compound according to any one of claims 1 to 7 wherein R¹ is C1 to C3 unsubstituted alkyl, its pharmaceutically acceptable salt or a solvate thereof.

9. The compound according to any one of claims 1 to 8 wherein both of R^{2a} and R^{2b} are hydrogen, its pharmaceutically acceptable salt or a solvate thereof.

10. A pharmaceutical composition comprising the compound according to any one of claims 1 to 9, its pharmaceutically acceptable salt or a solvate thereof as an active ingredient.

11. A pharmaceutical composition having BACE1 inhibitory activity comprising the compound according to any one of claims 1 to 9, its pharmaceutically acceptable salt or a solvate thereof as an active ingredient.

12. The pharmaceutical composition according to claim 10 or 11, which is a medicament for treating or preventing the diseases induced by production, secretion or deposition of amyloid-β proteins.

13. The pharmaceutical composition according to claim 10 or 11, which is a medicament for treating or preventing Alzheimer's disease.

14. A method for inhibiting BACE1 activity comprising administering the compound according to any one of claims 1 to 9, its pharmaceutically acceptable salt or a solvate thereof.

15. The compound according to any one of claims 1 to 9, its pharmaceutically acceptable salt or a solvate thereof for use in a method for inhibiting BACE1 activity.

16. A method for treating or preventing diseases induced by production, secretion or deposition of amyloid-β proteins comprising administering the compound according to any one of claims 1 to 9, its pharmaceutically acceptable salt or a solvate thereof.

17. The compound according to any one of claims 1 to 9, its pharmaceutically acceptable salt or a solvate thereof for use in a method for treating or preventing diseases induced by production, secretion or deposition of amyloid-β proteins.

18. A method for treating or preventing Alzheimer's disease comprising administering the compound according to any one of claims 1 to 9, its pharmaceutically acceptable salt or a solvate thereof.

19. The compound according to any one of claims 1 to 9, its pharmaceutically acceptable salt or a solvate thereof for use in a method for treating or preventing Alzheimer's disease.
